# EUROPEAN PATENT APPLICATION

(11) **EP 4 678 644 A1**
(43) Date of publication of application: **14.01.2026**
(21) Application number: 24788187.3
(22) Date of filing: 12.04.2024
(51) Int. Cl.: C07D 519/00, C07D 495/04, A61K 31/519, A61P 3/00, A61P 25/00, A61P 29/00

(54) **SUBSTITUTED DIHYDROTHIENOPYRIMIDINE COMPOUND, PREPARATION METHOD THEREFOR, AND USE THEREOF**

(30) Priority: 13.04.2023 CN 202310393487; 20.03.2024 CN 202410319993
(71) Applicant: Shanghai Yidi Biotechnology Co., Ltd., Shanghai 201208 (CN)
(72) Inventor: YE, Bin, Shanghai 201208 (CN); YU, Jun, Shanghai 201208 (CN)
(74) Representative: Mewburn Ellis LLP
(86) International application number: PCT/CN2024/087397
(87) International publication number: WO 2024/213089

(57) **Abstract**

The present invention relates to a substituted dihydrothienopyrimidine compound, a preparation method therefor, and use thereof, and belongs to the field of biological medicines. Specifically, the present invention relates to a compound represented by general formula (I), a preparation method therefor, a pharmaceutical composition comprising the compound, and use thereof in the preparation of a drug for treating an inflammatory disease, an autoimmune disease, a metabolic disease, a nervous system disease, and related diseases, wherein the definition of each substituent in general formula (I) is the same as that in the specification.

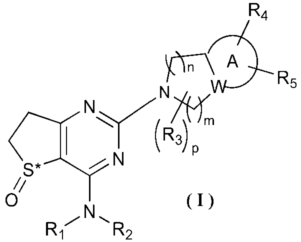

## Description

The present application claims priority to Chinese Patent Application No. 2023103934873, filed on April 13, 2023, and claims priority to Chinese Patent Application No. 2024103199932, filed on March 20, 2024. The above-mentioned Chinese patent applications are incorporated in the present application by reference in their entireties.

### TECHNICAL FIELD

The present invention belongs to the field of biomedicine and particularly relates to a substituted dihydrothienopyrimidine compound, a preparation method therefor, and use thereof.

### BACKGROUND

PDE4 is the most extensive member of the PDE family. The PDE family has 11 members, ranging from PDE1 to PDE11. These members differ in their expression locations and catalyzed substrate ranges. PDE4 is expressed at a high level in cells involved in various physiological processes of brain, kidney, smooth muscle, heart and endothelial cells, and is also expressed in all hematopoietic system cells. PDE4 is responsible for selectively catalyzing the hydrolysis of cAMP in various organs and cells and plays a very important role in cAMP-mediated signal pathways, such as cAMP/PKA/CREB, EPAC/Rap1 and ERK/BCL-6 signal pathways.

The PDE4 gene family includes four subtypes PDE4A-D, and each PDE4 subtype gene can express 3-11 proteins. At least 25 different PDE4 isoforms can be produced. PDE4 subtypes and isoforms exhibit distinct cell-type-specific and tissue-type-specific intracellular distributions, which contributes to specific functions thereof in terms of cell functions. Different PDE4 subtypes and isoforms can regulate spatially distinct cAMP signal cascades. PDE4 enzyme is expressed in inflammatory cells such as T cells, B cells, eosinophils, neutrophils, airway epithelial cells, and endothelial cells. It can specifically hydrolyze a 3',5' phosphodiester bond of cAMP to produce 5' adenosine monophosphate (5'-AMP), thus regulating the production of pro-inflammatory and anti-inflammatory cytokines. A PDE4 inhibitor can effectively increase the level of cAMP by inhibiting the hydrolysis of cAMP and activate protein kinase A (PKA), thereby inhibiting signal pathways such as NFκB and NFAT to reduce the release of downstream cytokines and chemokines, thus inhibiting inflammation. These factors control the expression of inflammatory mediators such as IL-2, IL-4, IL-6, IL-31, and TNF-α, thereby regulating inflammatory responses in T cells, Th2 cells and other cells, e.g., neutrophil degranulation, chemotaxis, and adhesion to endothelial cells. PDE4 inhibition can suppress inflammatory responses in macrophages, dendritic cells (DCs), Th1 cells, and Th17 cells, in addition to T cells and Th2 cells, and interfere with the phenotype and function of B cells. Therefore, the PDE4 target is widely used in the development of drugs for various inflammatory diseases, such as respiratory diseases (chronic obstructive pulmonary disease and asthma), various skin diseases (psoriasis, atopic dermatitis, etc.), and immune system diseases (systemic lupus erythematosus, rheumatoid arthritis, etc.); in addition, it is also used for developing drugs for diseases such as cognitive and emotional disorders, fragile X syndrome, autoimmune diseases, and tumors.

According to literature reports, PDE4B is a primary pro-inflammatory factor, and PDE4D is more related to side effects. PDE4D is expressed in various tissues of human body. The brain is the main expression region of PDE4D, and there are also many related studies. Vomiting is the main side effect of PDE4 inhibitors, and PDE4D exists in the area postrema, nucleus tractus solitarius, and locus coeruleus, all of which are related to an emetic action caused by PDE4 inhibition. It can be seen that there is a need for selective inhibitors to ensure safety while maintaining therapeutic effects.

Many PDE4 inhibitors have been published and disclosed. WO2006/1111549, WO2007/118793, US20070259846, WO2009/050236, WO2009/050242, WO2009/052268, WO2009/050248, and WO2013/026797 (all from Boehringer Ingelheim International), and WO2019/057806, WO2019/11577, WO2019/115775, and WO2019/115776 ((all from Leo Pharma, A/S) all disclose substituted dihydrothienopyrimidines, which are used for treating respiratory or inflammatory diseases. These compounds are said to nhibit PDE4 enzyme. WO2014/066659 (Tetra Discovery Partners) discloses bicyclic heteroaryl compounds said to be PDE4 inhibitors.

At present, it is still necessary to continuously develop new PDE4 inhibitors having a more favorable therapeutic window and less side effects.

An object of the present invention is to provide a novel class of substituted dihydrothienopyrimidine compounds. The present invention relates to a PDE4 inhibitor, which can be used as a therapeutic agent for PDE4-mediated diseases, including chronic obstructive pulmonary disease (COPD), asthma, psoriasis, rheumatoid arthritis, psoriatic arthritis, atopic dermatitis, idiopathic pulmonary fibrosis, nervous system diseases, cognitive and emotional disorders, fragile X syndrome, other inflammatory allergic diseases and autoimmune diseases, lung injury, tumors, etc.

The compounds of the present invention can have good oral bioavailability, solubility, absorption, and metabolic stability. They also have good safety, which makes them more tolerant than other PDE4 inhibitors. Equivalent to other PDE4 inhibitors, the compounds of the present invention have certain selectivity for PDE4D, and can have improved windows in terms of nausea and vomiting side effects, thus allowing them to be administered in higher multiples to achieve greater medical effects.

### SUMMARY

An object of the present invention is to provide a compound represented by general formula (I), a stereoisomer thereof, or a pharmaceutically acceptable salt thereof. A structure of the compound represented by general formula (I) is as follows: 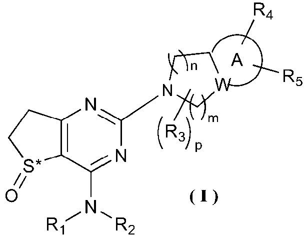 where:
S* represents a chiral sulfur atom and has R configuration or S configuration;
W is selected from N, CH, or C;
ring A is a monocyclic group or a bicyclic group, where the monocyclic group is selected from 5- to 8-membered heteroaryl or C₆₋₁₀ aryl, and the bicyclic group is selected from 5- to 6-membered heteroaryl-fused 5- to 6-membered heteroaryl, 5- to 6-membered heteroaryl-fused phenyl, 5- to 6-membered cycloalkyl-fused phenyl, 5- to 6-membered cycloalkyl-fused 5- to 6-membered heteroaryl, 5- to 6-membered heterocyclyl-fused phenyl, 5- to 6-membered heterocyclyl-fused 5- to 6-membered heteroaryl, benzo 5- to 6-membered heterocyclyl, or benzo-fused 5- to 6-membered heteroaryl;
R₁ is selected from hydrogen or C₁₋₆ alkyl;
R₂ is selected from C₁₋₆ alkyl, C₃₋₈ cycloalkyl, 3- to 8-membered heterocyclyl, C₆₋₁₀ aryl, or 5- to 10-membered heteroaryl, where the C₁₋₆ alkyl, C₃₋₈ cycloalkyl, 3- to 8-membered heterocyclyl, C₆₋₁₀ aryl, and 5- to 10-membered heteroaryl are optionally further substituted with one or more groups selected from deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, 3- to 6-membered heterocyclyl, 5- to 6-membered heteroaryl, 5- to 6-membered heteroaryl-substituted C₁₋₆ alkyl, R^{2.1}-substituted C₁₋₆ alkyl, C₁₋₆ alkyl-substituted 5- to 6-membered heteroaryl, C₁₋₆ alkyl-substituted 3- to 6-membered heterocyclyl, -C(O)-R^{2.1}, -S(O)₂-R^{2.2}, -C(O)O-R^{2.1}, - C(O)NR^{2.3}R^{2.4}, -SR^{2.5}, -SOR^{2.2}, -OR^{2.5}, or -NR^{2.3}R^{2.4};
or R₁ and R₂ are connected to form 3- to 6-membered heterocyclyl or 5- to 6-membered heteroaryl, where the 3- to 6-membered heterocyclyl and the 5- to 6-membered heteroaryl are optionally further substituted with one or more groups selected from hydgron, deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, or 3- to 6-membered heterocyclyl;
each R₃ is independently selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, or 3- to 6-membered heterocyclyl;
or any R₃, together with carbon atoms on a ring, forms C₃₋₈ cycloalkyl or 3- to 8-membered heterocyclyl;
R₄ is selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, 7- to 9-membered heterocyclyl, 5- to 8-membered heteroaryl, -C(O)-R^{4.1}, -S(O)₂-R^{4.2} -C(O)O-R^{4.1}, -C(O)NR^{4.3}R^{4.4}, -SOR^{4.2}, -OR^{4.5}, -SR^{4.5}, or -NR^{4.3}R^{4.4}, where the C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, 7- to 9-membered heterocyclyl, and 5- to 8-membered heteroaryl are capable of being optionally further substituted with one or more groups selected from deuterium, halogen, cyano, hydroxyl, oxo, C₁₋₆ alkyl, C₁₋₆ haloalkyl, C₁₋₆ hydroxyalkyl, C₁₋₆ alkoxy, C₁₋₆ alkoxy C₁₋₆ alkyl, -NR^{4.3}R^{4.4}, C₃₋₆ cycloalkyl, or 3- to 6-membered heterocyclyl;
R₅ is selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, -C(O)-R^{5.1}, -S(O)₂-R^{5.2}, -C(O)O-R⁵⁻¹ - C(O)NR^{5.3}R^{5.4}, -SOR^{5.2}, -OR^{5.5}, -SR^{5.5}, or -NR^{5.3}R^{5.4}.
or R₄ is connected to R₅ to form 5- to 6-membered heteroaryl or C₆₋₁₀ aryl, where the 5- to 6-membered heteroaryl and C₆₋₁₀ aryl are capable of being optionally further substituted with one or more groups selected from deuterium, halogen, cyano, hydroxyl, oxo, C₁₋₆ alkyl, C₁₋₆ hydroxyalkyl, C₁₋₆ alkoxy, C₃₋₆ cycloalkyl, or 3- to 6-membered heterocyclyl;
R^{2.1}, R^{2.2}, R^{2.3}, R^{2.4} R^{2.5}, R^{4.1}, R^{4.2}, R^{4.3}, R^{4.4}, R^{4.5}, R^{5.1}, R^{5.2}, R^{5.3} R^{5.4}, and R^{5.5} are each independently selected from hydrogen, deuterium, halogen, amino, hydroxyl, cyano, carboxyl, C₁₋₆ alkyl, C₁₋₆ hydroxyalkyl, C₁₋₆ haloalkyl, C₁₋₆ alkoxy, -C(O)NH₂, C₃₋₆ cycloalkyl, or 3- to 6-membered heterocyclyl;
m and n are each independently selected from 1, 2, or 3; and
p is selected from 1 or 2.

In a preferred embodiment of the present invention, the compound described above is further as represented by formula (II-A), formula (II-B), formula (II-C), or formula (II-D):

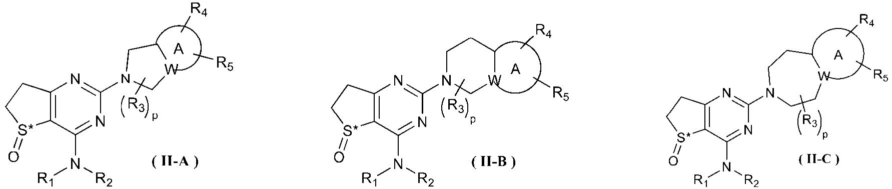

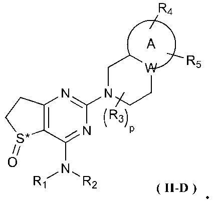

In a preferred embodiment of the present invention, the ring A described above is a monocyclic group selected from 5-membered heteroaryl, 6-membered heteroaryl, or phenyl; preferably, the ring A is selected from thiazolyl, oxazolyl, oxadiazolyl, thiadiazolyl, phenyl, pyrazolyl, thienyl, furyl, imidazolyl, triazolyl, tetrazolyl, pyridinyl, pyrimidinyl, pyrazinyl, or pyridazinyl.

In a preferred embodiment of the present invention, the ring A described above is a bicyclic group. The bicyclic group is selected from 5-membered heteroaryl-fused 6-membered heteroaryl, 5-membered heteroaryl-fused phenyl, 6-membered cycloalkyl-fused phenyl, 6-membered heterocyclyl-fused phenyl, benzo-fused 5-membered heterocyclyl, benzo-fused 5-membered heteroaryl, 6-membered heteroaryl-fused 5-membered heteroaryl, 6-membered heterocyclyl-fused 6-membered heteroaryl, or 6-membered heteroaryl-fused phenyl;
preferably, ring A is selected from pyrazolopyrimidinyl, imidazophenyl, imidazopyridinyl, pyrazolopyridinyl, cyclohexyl-fused phenyl, oxacyclohexyl-fused phenyl, benzodioxazolyl, 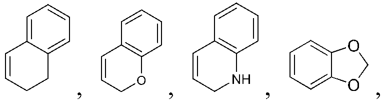 benzoxazolyl, 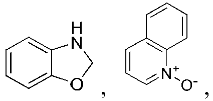 oxacyclohexyl-fused pyridinyl, pyridone-fused phenyl, pyridopyrazolyl, benzopyrazolyl, thienophenyl, or pyridophenyl.

In some embodiments of the present invention, the 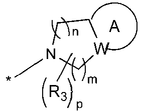 described above is selected from

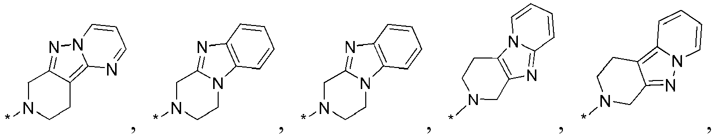

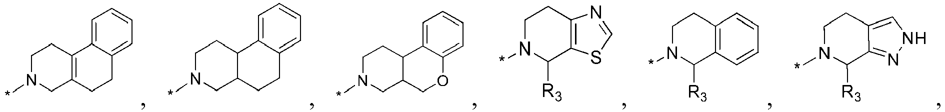

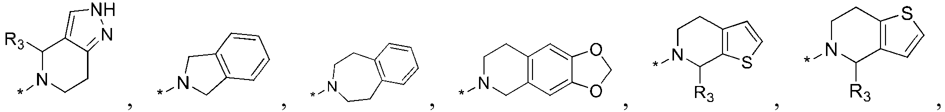

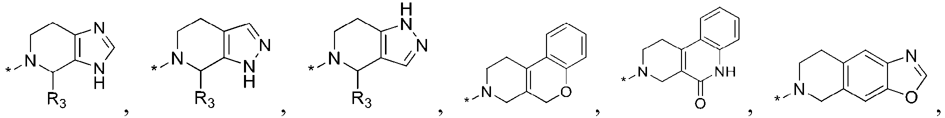

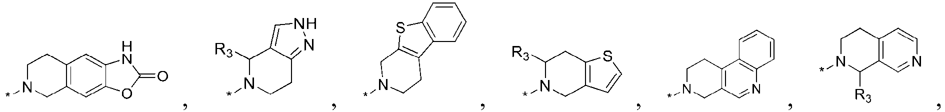

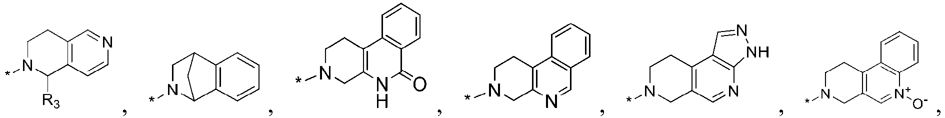

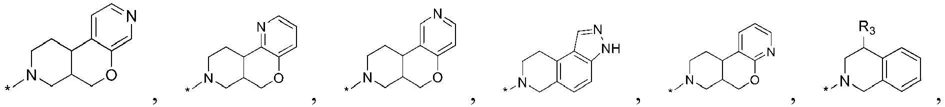

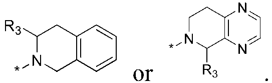

In a preferred embodiment of the present invention, the compound of the present invention is further as represented by formula (III-A) or formula (III-B):

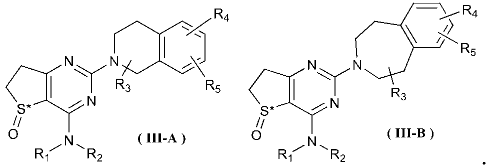

In a preferred embodiment of the present invention, the formula (III-A) of the present invention is further as represented by formula (IV): 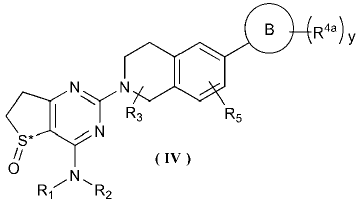 where ring B is C₃₋₆ cycloalkyl, 3- to 6-membered heterocyclyl, 7- to 9-membered heterocyclyl, phenyl, or 5- to 6-membered heteroaryl; each R^{4a} is independently selected from deuterium, halogen, amino, hydroxyl, cyano, carboxyl, C₁₋₃ alkyl, C₁₋₃ hydroxyalkyl, C₁₋₃ haloalkyl, C₁₋₃ alkoxy, C₁₋₃ alkoxyalkyl, -N(CH₃)₂, -C(O)NH₂, C₃₋₆ cycloalkyl, or 3- to 6-membered heterocyclyl; and y is 0, 1, or 2.

In a preferred embodiment of the present invention, the ring B described above is C₃₋₆ cycloalkyl, 3- to 6-membered heterocyclyl, 7- to 9-membered heterocyclyl, phenyl, or 5-to 6-membered heteroaryl; each R^{4a} is independently selected from deuterium, halogen, amino, hydroxyl, cyano, carboxyl, oxo, C₁₋₃ alkyl, C₁₋₃ hydroxyalkyl, C₁₋₃ haloalkyl, C₁₋₃ alkoxy, C₁₋₃ alkoxyalkyl, -N(CH₃)₂, -C(O)NH₂, C₃₋₆ cycloalkyl, or 3- to 6-membered heterocyclyl; and y is 0, 1, or 2.

In a preferred embodiment of the present invention, the ring B described above is selected from cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, pyrazolyl, oxazolyl, thiazolyl, 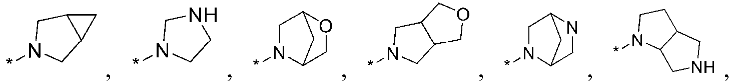 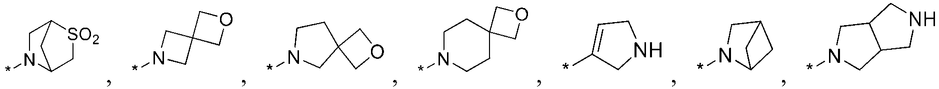 or

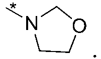

In a preferred embodiment of the present invention, the R₁ described above is selected from hydrogen, and the R₂ is selected from C₁₋₃ alkyl, C₃₋₆ cycloalkyl, 3- to 6-membered heterocyclyl, phenyl, or 5- to 6-membered heteroaryl, where the C₁₋₃ alkyl, C₃₋₆ cycloalkyl, 3- to 6-membered heterocyclyl, phenyl, and 5- to 6-membered heteroaryl are optionally further substituted with one or more groups selected from deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, 3- to 6-membered heterocyclyl, 5- to 6-membered heteroaryl, 5- to 6-membered heteroaryl-substituted C₁₋₆ alkyl, R^{2.1}-substituted C₁₋₆ alkyl, C₁₋₆ alkyl-substituted 5- to 6-membered heteroaryl, C₁₋₆ alkyl-substituted 3- to 6-membered heterocyclyl, -C(O)-R^{2.1}, -S(O)₂-R^{2.2}, -C(O)O-R^{2.1}, - C(O)NR^{2.3}R^{2.4}, -SR^{2.5}, -SOR^{2.2}, -OR^{2.5}, or -NR^{2.3}R^{2.4}.

In a preferred embodiment of the present invention, each R^{4a} described above is independently selected from deuterium, fluoro, chloro, amino, hydroxyl, cyano, carboxyl, oxo, methyl, ethyl, -CH₂OCH₃, -N(CH₃)₂, methoxy, ethoxy, trifluoromethyl, hydroxymethyl, or hydroxyethyl.

In a preferred embodiment of the present invention, the R^{2.1}, R^{2.2}, R^{2.3}, R^{2.4}, and R^{2.5} described above are each independently selected from hydrogen, deuterium, fluoro, chloro, bromo, amino, hydroxyl, cyano, carboxyl, methyl, ethyl, propyl, isopropyl, hydroxymethyl, hydroxyethyl, -CF₃, -CHF₂, -CH₂F, methoxy, ethoxy, propoxy, -C(O)NH₂, cyclopropyl, cyclobutyl, cyclopentyl, oxiranyl, oxetanyl, aziridinyl, azetidinyl, tetrahydropyrrolyl, or tetrahydrofuryl.

In a preferred embodiment of the present invention, the R₂ described above is selected from C₁₋₃ alkyl, C₃₋₆ cycloalkyl, 3- to 6-membered heterocyclyl, phenyl, or 5- to 6-membered heteroaryl, where the C₁₋₃ alkyl, C₃₋₆ cycloalkyl, 3- to 6-membered heterocyclyl, phenyl, and 5- to 6-membered heteroaryl are optionally further substituted with one or more groups selected from deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, 3- to 6-membered heterocyclyl, 5- to 6-membered heteroaryl, 5- to 6-membered heteroaryl-substituted C₁₋₆ alkyl, R^{2.1}-substituted C₁₋₆ alkyl, C₁₋₆ alkyl-substituted 5- to 6-membered heteroaryl, C₁₋₆ alkyl-substituted 3- to 6-membered heterocyclyl, -C(O)-R^{2.1}, -S(O)₂-R^{2.2}, -C(O)O-R ^{2.1}, -C(O)NR^{2.3}R^{2.4}, -SR^{2.5}, -SOR^{2.2}, -OR^{2.5}, or -NR^{2.3}R^{2.4}.

In a preferred embodiment of the present invention, the R₁ described above is selected from hydrogen or C₁₋₆ alkyl;
or R₂ is as represented by formula (III): 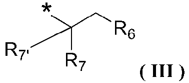 where:
R₆ is selected from hydroxyl, cyano, amino, or halogen;
R₇ and R_{7'} are each independently selected from hydrogen and C₁₋₆ alkyl, where the C₁₋₆ alkyl is optionally further substituted with one or more groups selected from deuterium, halogen, amino, hydroxyl, cyano, oxo, C₁₋₃ alkyl, C₁₋₃ haloalkyl, C₁₋₃ hydroxyalkyl, C₁₋₃ alkoxy, -C(O)-R^{2.1}, -S(O)₂-R ^{2.2}, -C(O)O-R^{2.1}, -C(O)NR^{2.3}R^{2.4}, -SR^{2.5}, -SOR^{2.2}, -OR^{2.5}, or -NR^{2.3}R^{2.4};
preferably, R₇ and R_{7'} are each independently selected from hydrogen, methyl, ethyl, propyl, isopropyl, or butyl;
or R₂ is selected from methyl, ethyl, cyclopropyl, cyclobutyl, cyclopentyl, cyclohexyl, oxacyclohexyl, cycloheptyl, phenyl, pyridinyl, pyrazolyl, triazolyl, tetrazolyl, oxetanyl, azetidinyl, thietanyl, tetrahydropyranyl, tetrahydrofuryl, tetrahydrothiopyran, pyrrolidinyl, tetrahydropyrrolidinyl, tetrahydrothienyl, piperidinyl, piperazinyl, morpholinyl, thiomorpholinyl, azepanyl, oxaspiroheptyl, benzopyridinyl, pyridopyridinyl, benzimidazolyl, benzopyrimidinyl, or naphthyl, where the methyl, ethyl, cyclopropyl, cyclobutyl, cyclopentyl, cyclohexyl, oxacyclohexyl, cycloheptyl, phenyl, pyridinyl, pyrazolyl, triazolyl, tetrazolyl, oxetanyl, azetidinyl, thietanyl, tetrahydropyranyl, tetrahydrofuryl, tetrahydrothiopyran, pyrrolidinyl, tetrahydropyrrolidinyl, tetrahydrothienyl, piperidinyl, piperazinyl, morpholinyl, thiomorpholinyl, azepanyl, oxaspiroheptyl, benzopyridinyl, pyridopyridinyl, benzimidazolyl, benzopyrimidinyl, and naphthyl are capable of being optionally further substituted with one or more groups selected from deuterium, fluoro, chloro, bromo, amino, hydroxyl, mercapto, cyano, oxo, methyl, ethyl, propyl, isopropyl, butyl, isobutyl, hydroxymethyl, hydroxyethyl, cyclopropyl, -CF₃, -CHF₂, -CH₂F, methoxy, ethoxy, propoxy, oxiranyl, oxetanyl, aziridinyl, azetidinyl, -(CH₂)₂CN, -C(O)-R^{2.1}, -S(O)₂-R^{2.2}, -C(O)O-R^{2.1}, -C(O)NR^{2.3}R^{2.4}, -SR^{2.5}, -SOR^{2.2}, -OR^{2.5}, or -NR^{2.3}R^{2.4};
or each R₃ is independently selected from hydrogen, deuterium, halogen, amino, nitro, cyano, hydroxyl, oxo, C₁₋₃ alkyl, C₁₋₃ haloalkyl, C₁₋₃ deuteroalkyl, C₁₋₃ hydroxyalkyl, C₁₋₃ alkoxy, C₃₋₆ cycloalkyl, or 3- to 6-membered heterocyclyl;
or R₄ is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, -C(O)-R^{4.1}, -S(O)₂-R^{4.2}, -C(O)O-R^{4.1}, -C(O)NR^{4.3}R^{4.4}, -SOR^{4.2}, -OR^{4.5}, -SR^{4.5}, -NR^{4.3}R^{4.4}, C₃₋₆ cycloalkyl, 3- to 6-membered heterocyclyl, 7- to 9-membered heterocyclyl, phenyl, or 5- to 6-membered heteroaryl, where the C₃₋₆ cycloalkyl, 3- to 6-membered heterocyclyl, 7- to 9-membered heterocyclyl, phenyl, and 5- to 6-membered heteroaryl are capable of being optionally further substituted with one or more substituents selected from deuterium, halogen, amino, nitro, cyano, hydroxyl, oxo, C₁₋₃ alkyl, C₁₋₃ hydroxyalkyl, C₁₋₃ alkoxy, -NR^{4.3}R^{4.4}, C₁₋₃ alkoxy C₁₋₃ alkyl, or C₁₋₃ haloalkyl;
or R₅ is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, -C(O)-R^{5.1}, -S(O)₂-R^{5.2}, -C(O)O-R^{5.1}, -C(O)NR^{5.3}R^{5.4}, -SOR^{5.2}, -OR^{5.5}, -SR^{5.5}, -NR^{5.3}R^{5.4}, C₃₋₆ cycloalkyl, 3- to 6-membered heterocyclyl, phenyl, or 5- to 6-membered heteroaryl, where the C₃₋₆ cycloalkyl, 3-to 6-membered heterocyclyl, phenyl, and 5- to 6-membered heteroaryl are capable of being optionally further substituted with one or more substituents selected from deuterium, halogen, amino, nitro, cyano, hydroxyl, oxo, C₁₋₃ alkyl, C₁₋₃ hydroxyalkyl, C₁₋₃ alkoxy, or C₁₋₃ haloalkyl;
the R^{2.1}, R^{2.2}, R^{2.3}, R^{2.4}, R^{2.5}, R^{4.1}, R^{4.2}, R^{4.3}, R^{4.4}, R^{4.5}, R^{5.1}, R^{5.2}, R^{5.3} , R^{5.4}, and R^{5.5} are each independently selected from hydrogen, deuterium, halogen, amino, hydroxyl, cyano, carboxyl, C₁₋₃ alkyl, C₁₋₃ hydroxyalkyl, C₁₋₃ haloalkyl, C₁₋₃ alkoxy, -C(O)NH₂, C₃₋₆ cycloalkyl, or 3- to 6-membered heterocyclyl;
preferably, the R^{2.1}, R^{2.2}, R^{2.3}, R^{2.4}, R^{2.5}, R^{4.1}, R^{4.2}, R^{4.3}, R^{4.4}, R^{4.5}, R^{5.1}, R^{5.2}, R^{5.3} R^{5.4}, and R^{5.5} are each independently selected from hydrogen, deuterium, fluoro, chloro, bromo, amino, hydroxyl, cyano, carboxyl, methyl, ethyl, propyl, isopropyl, hydroxymethyl, hydroxyethyl, -CF₃, -CHF₂, -CH₂F, methoxy, ethoxy, propoxy, -C(O)NH₂, cyclopropyl, cyclobutyl, cyclopentyl, oxiranyl, oxetanyl, aziridinyl, azetidinyl, tetrahydropyrrolyl, or tetrahydrofuryl.

In a preferred embodiment of the present invention, the R₄ described above is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, or pyrazolyl, where the C₁₋₃ alkyl, C₁₋₃ hydroxyalkyl, C₁₋₃ alkoxy, C₁₋₃ haloalkyl, cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, and pyrazolyl are optionally substituted with one or more substituents selected from deuterium, halogen, amino, nitro, cyano, hydroxyl, fluoro, chloro, oxo, methyl, ethyl, methoxy, ethoxy, trifluoromethyl, hydroxymethyl, hydroxyethyl, trifluoroethyl, -CH₂OCH₃, or -N(CH₃)₂.

In a preferred embodiment of the present invention, the R₄ described above is selected from hydrogen, deuterium, fluoro, chloro, bromo, iodo, nitro, cyano, hydroxyl, amino, oxo, methyl, ethyl, propyl, isopropyl, methoxy, ethoxy, propoxy, isopropoxy, cyclopropyl, cyclobutyl, oxiranyl, oxetanyl, aziridinyl, azetidinyl, -CF₃, -CHF₂, -CH₂F, -NMe₂, -SO₂Me, - SO₂Et, -CONH₂, -CONHMe, -CO₂Me, 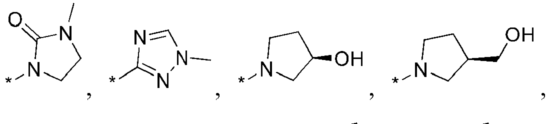 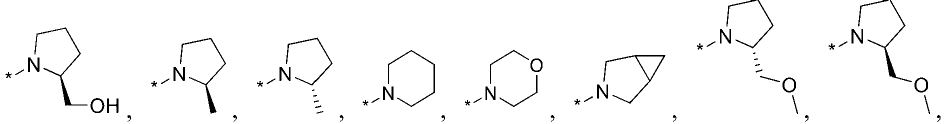 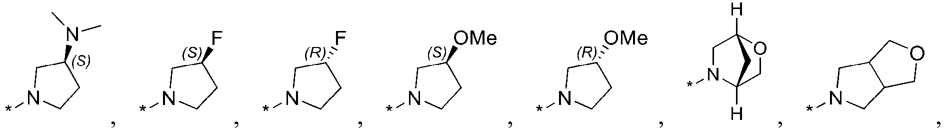 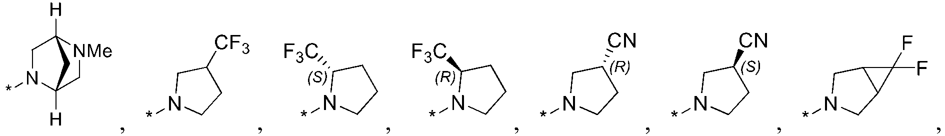 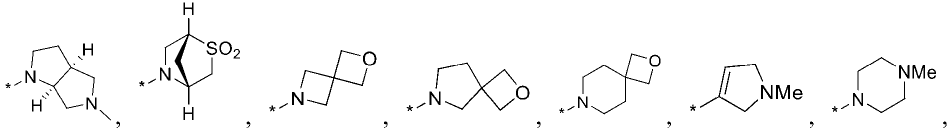 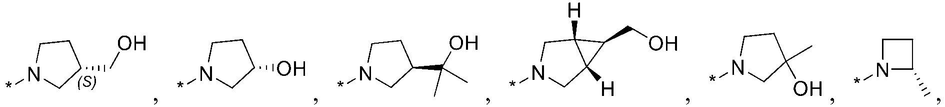 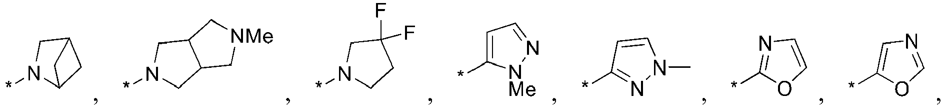 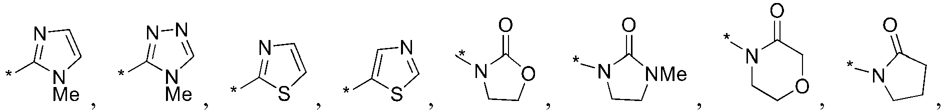 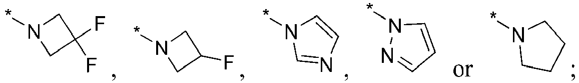 or R₅ is selected from hydrogen, deuterium, fluoro, chloro, bromo, iodo, nitro, cyano, hydroxyl, amino, oxo, methyl, ethyl, propyl, isopropyl, methoxy, ethoxy, propoxy, isopropoxy, cyclopropyl, cyclobutyl, oxiranyl, oxetanyl, aziridinyl, azetidinyl, -NH-(CH₂)₂-OH, -CF₃, -CHF₂, -CH₂F, -NMe₂, -SO₂Me, -SO₂Et, -CONH₂, -CONHMe, or -CO₂Me.

In a preferred embodiment of the present invention, 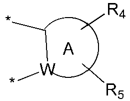 in each of formula (II-A), formula (II-B), formula (II-C), and formula (II-D) described above is independently selected from 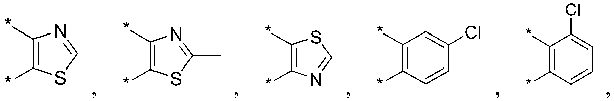 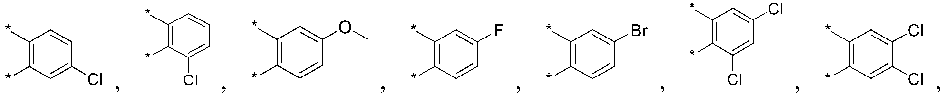 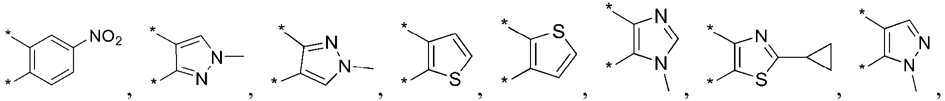 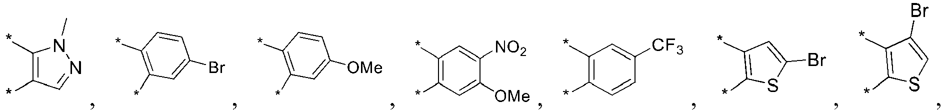 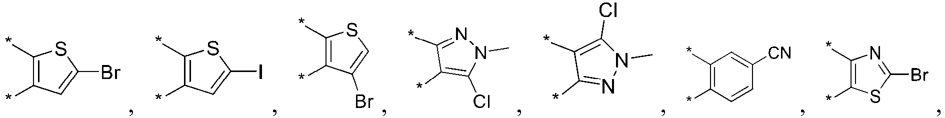 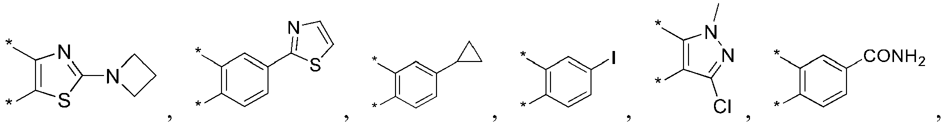 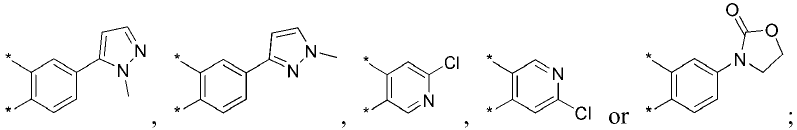 or 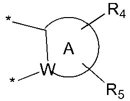 in each of formula (II-A), formula (II-B), formula (II-C), and formula (II-D) is independently selected from

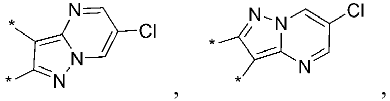

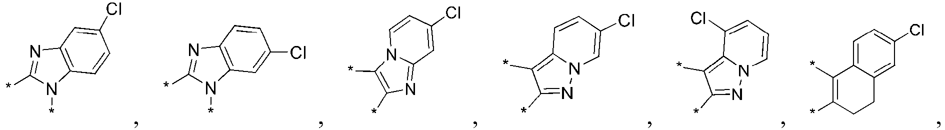

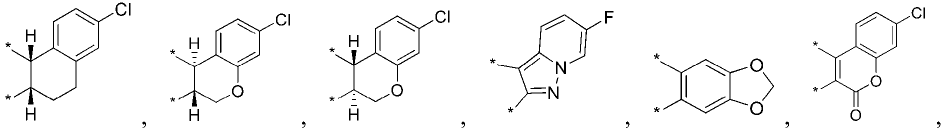

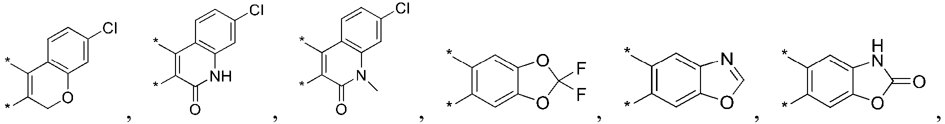

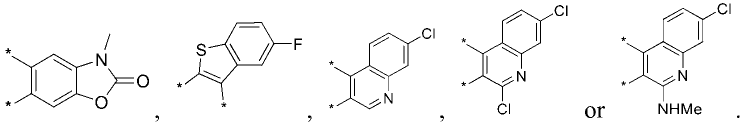

In a preferred embodiment of the present invention, the R₁ described above is selected from hydrogen and C₁₋₃ alkyl;
or the R₂ is selected from the following groups: 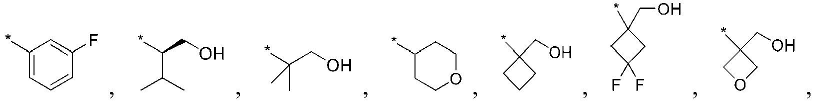 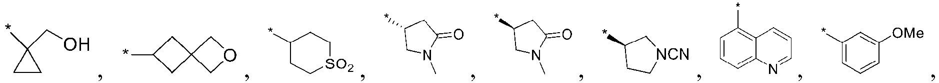 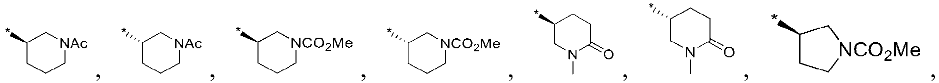 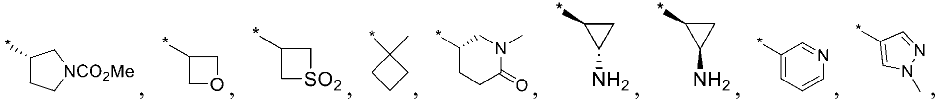 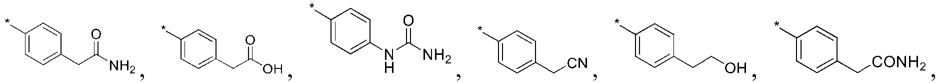 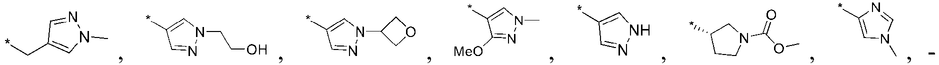 (CH₂)₂OH, 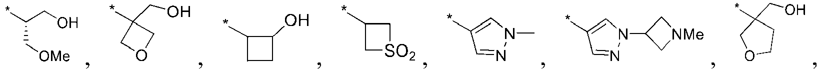 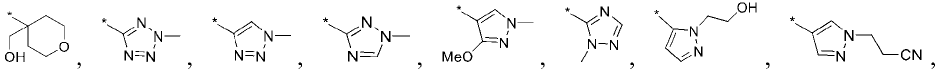 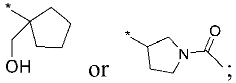
or each R₃ is independently selected from hydrogen, deuterium, fluoro, chloro, bromo, cyano, oxo, methyl, ethyl, propyl, isopropyl, butyl, isobutyl, methoxy, ethoxy, propoxy, isopropoxy, -CF₃, -CHF₂, -CH₂F, cyclopropyl, cyclobutyl, cyclopentyl, oxiranyl, oxetanyl, aziridinyl, azetidinyl, tetrahydropyrrolyl, tetrahydrofuryl, -CH₂OH, -CF₃, -CHF₂, -CH₂F, or - CD₃.

In a preferred embodiment of the present invention, the compound of the present invention is selected from compounds having the following structures:

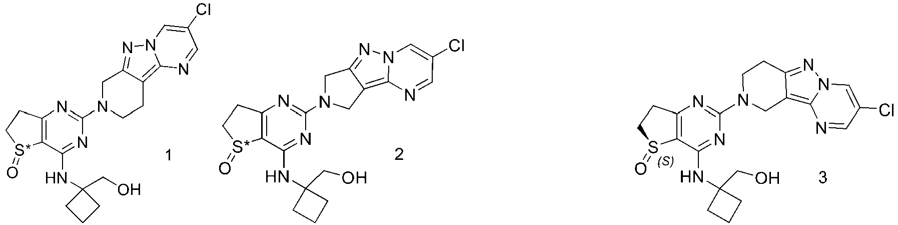

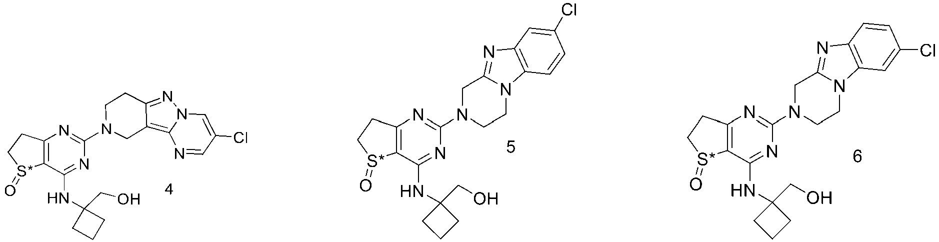

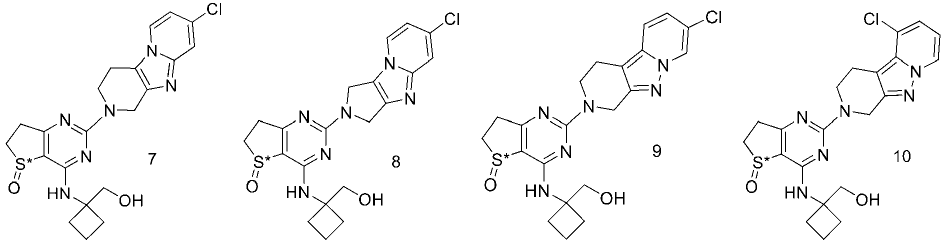

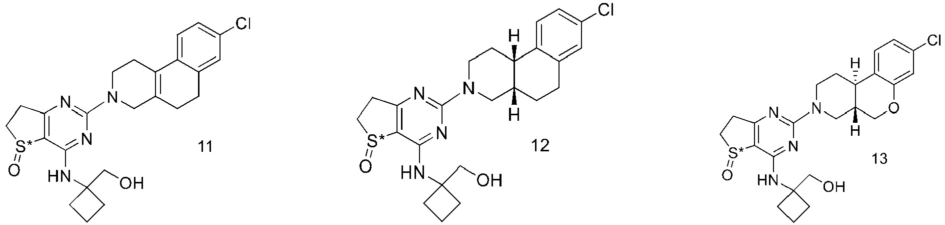

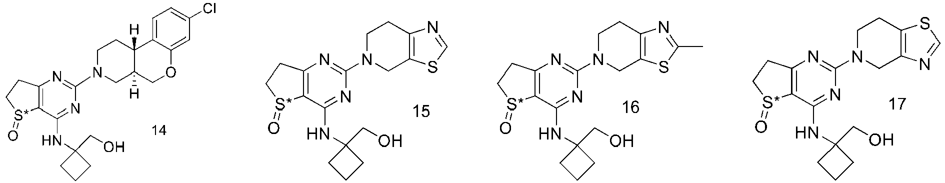

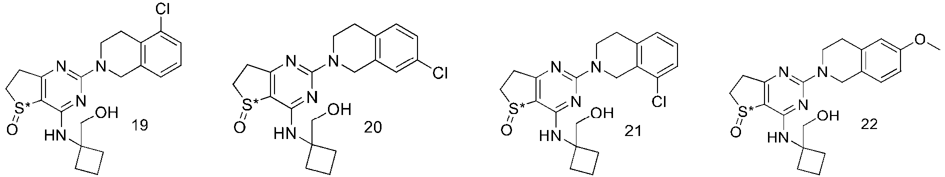

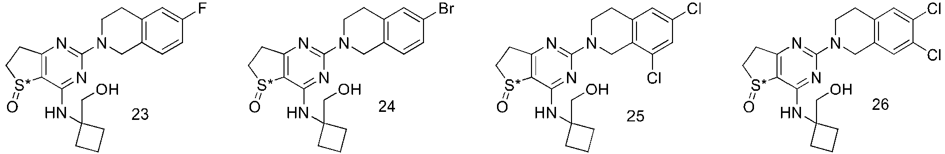

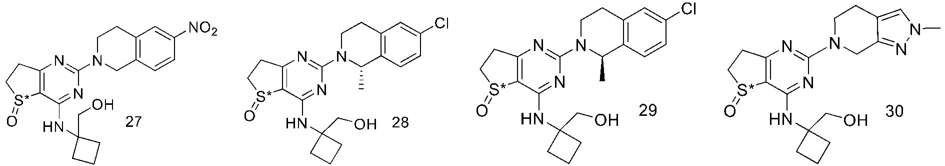

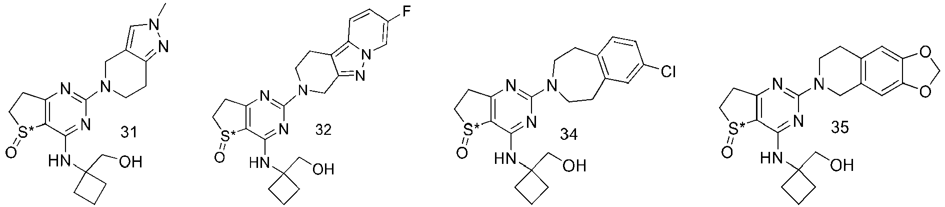

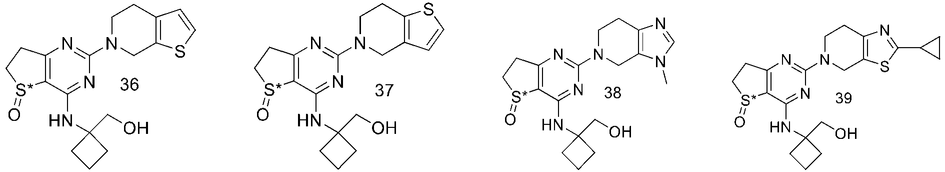

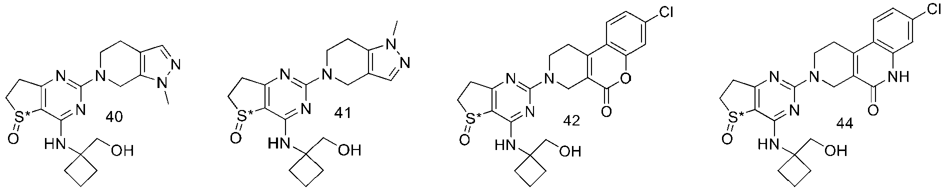

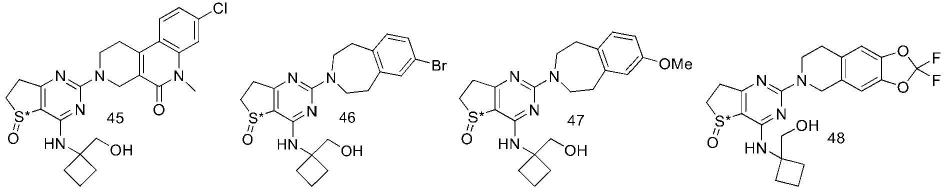

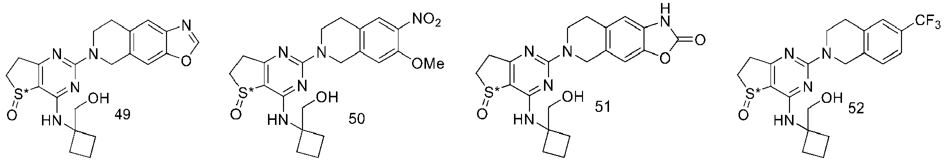

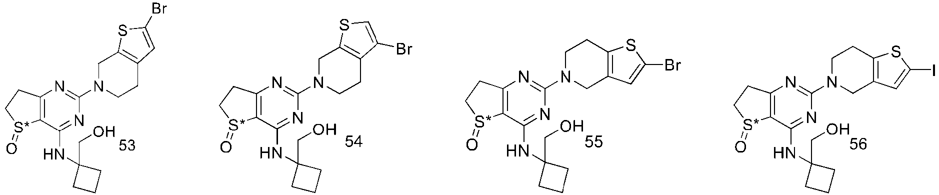

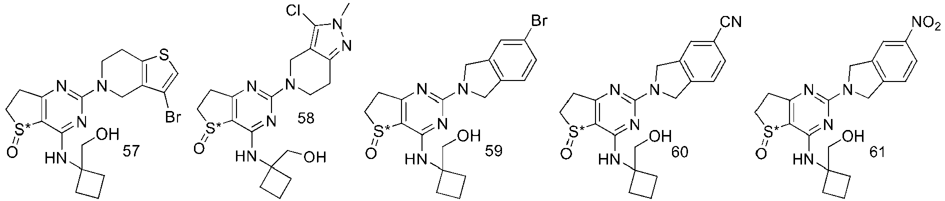

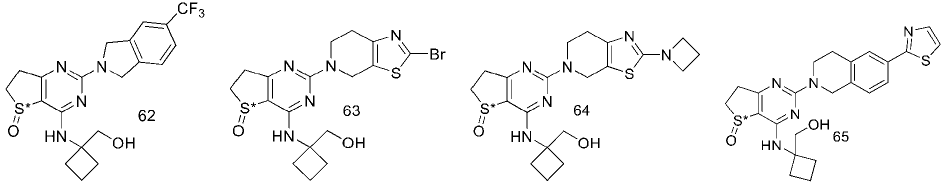

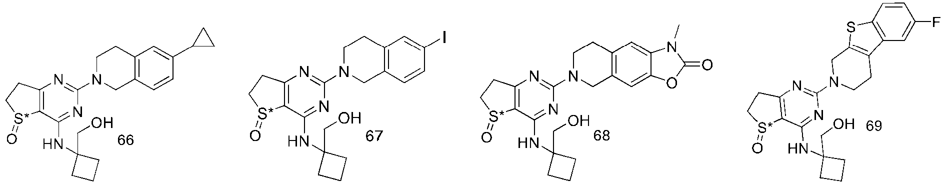

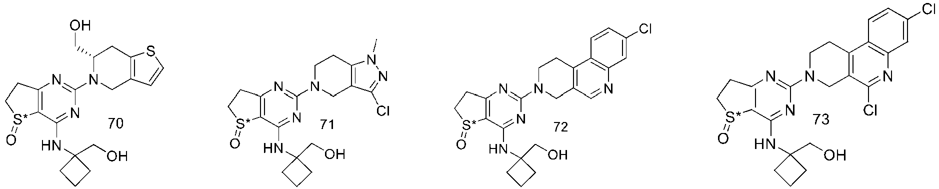

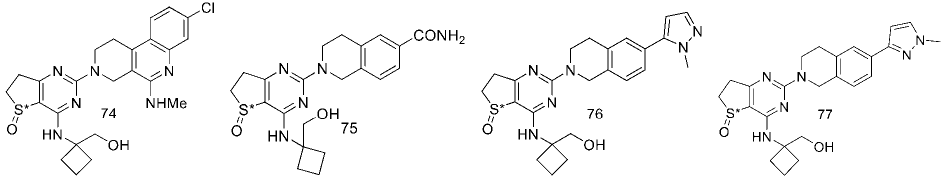

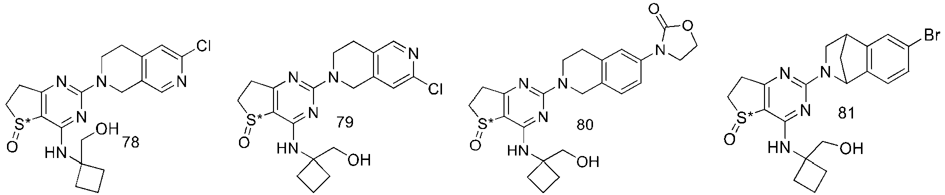

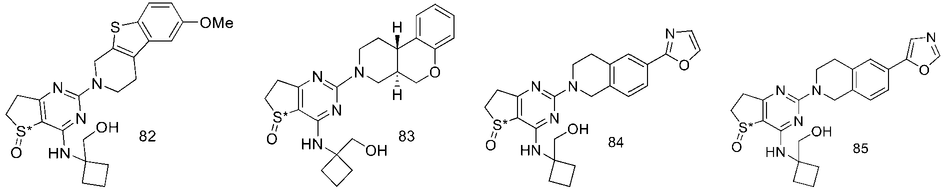

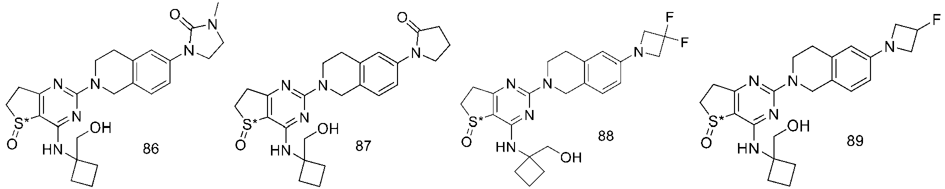

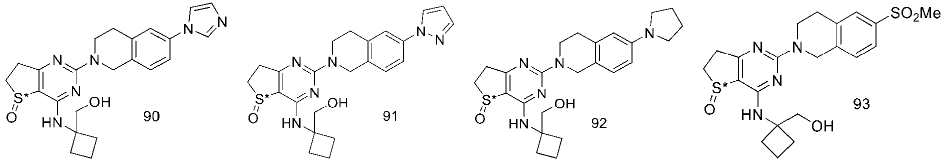

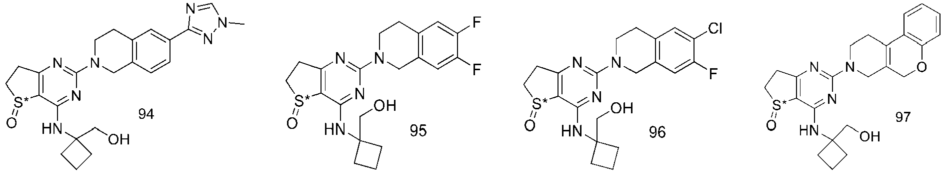

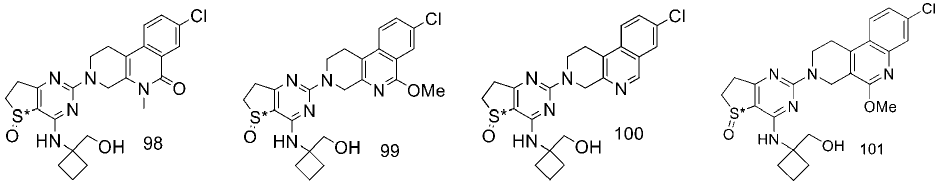

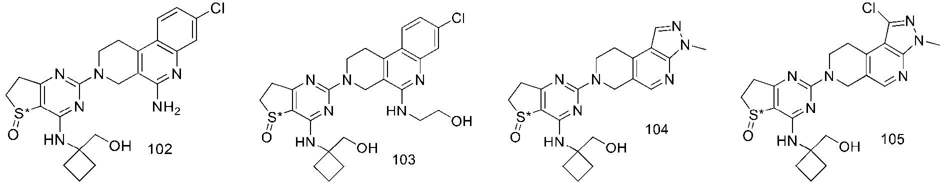

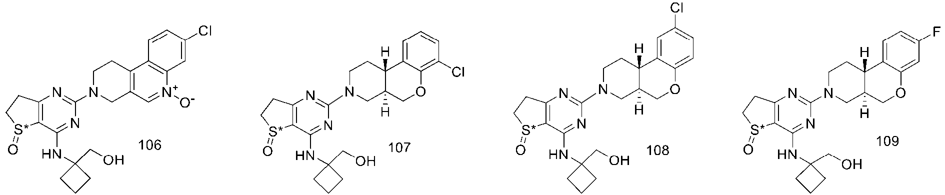

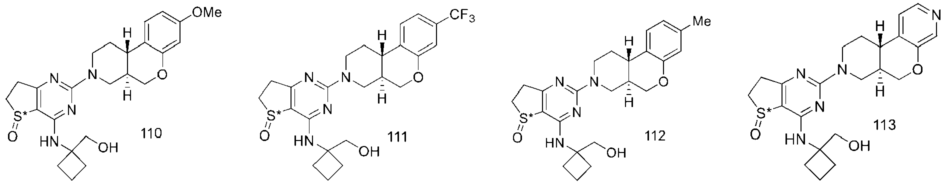

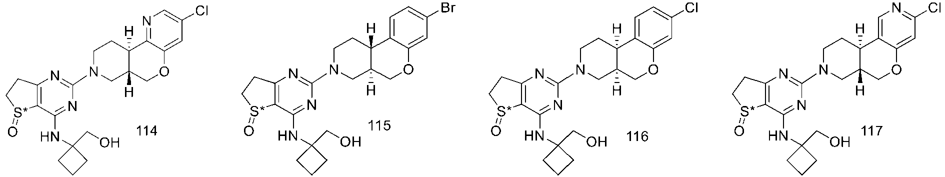

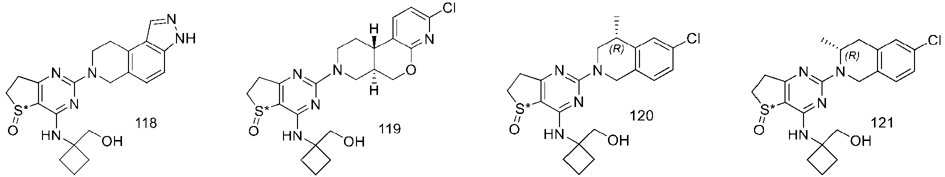

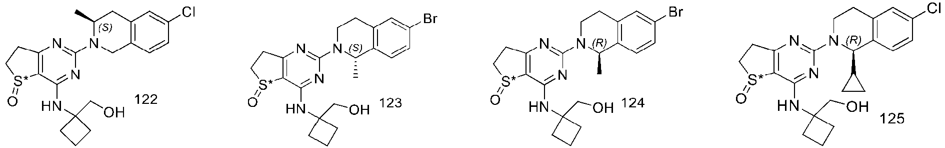

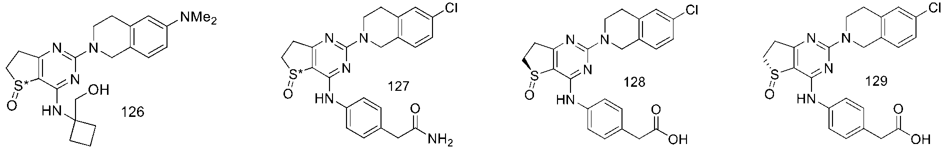

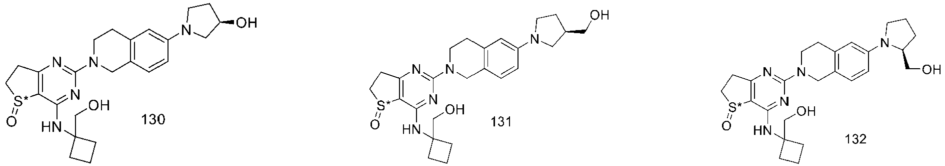

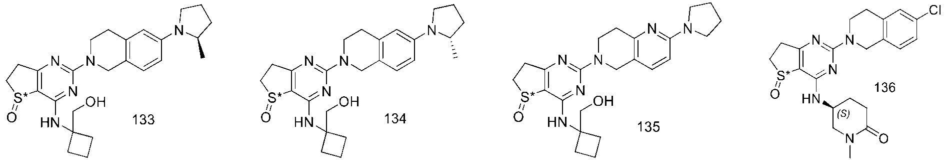

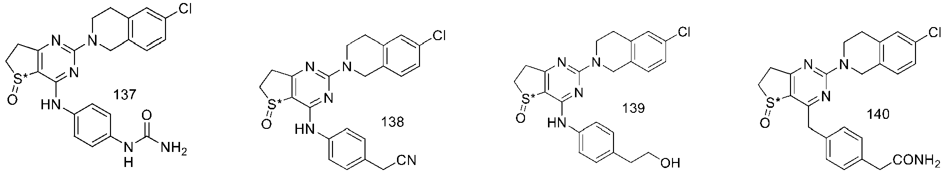

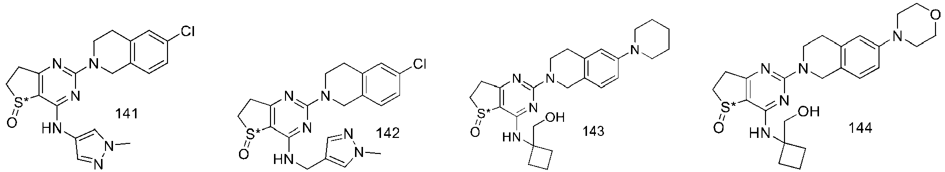

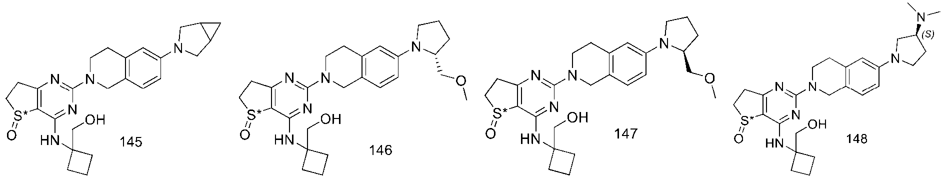

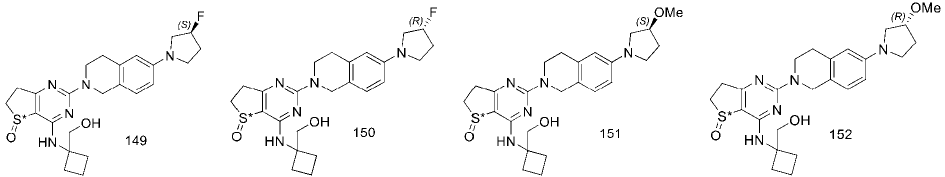

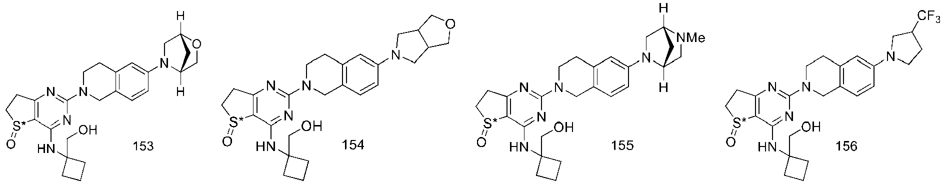

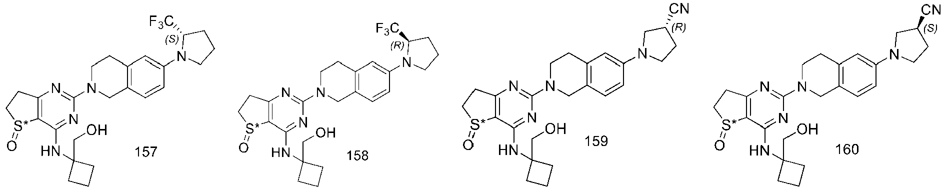

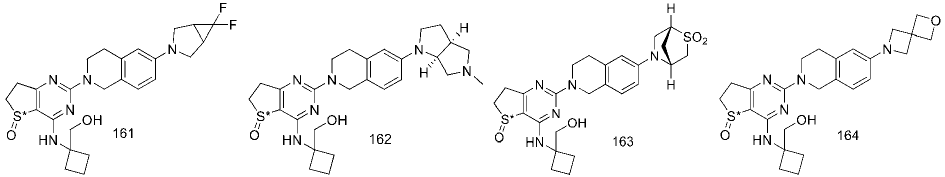

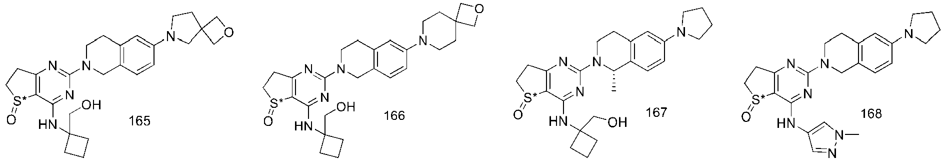

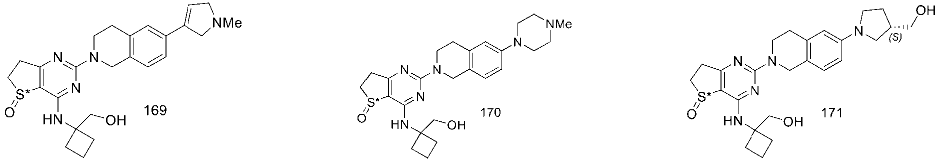

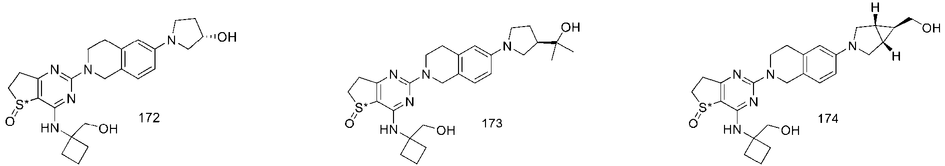

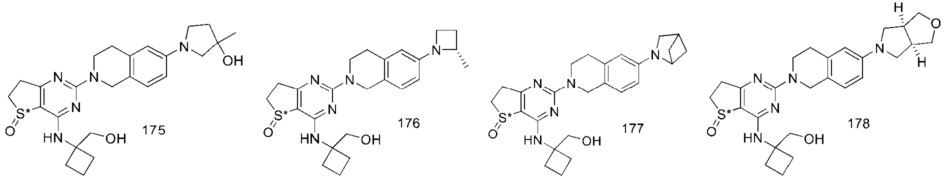

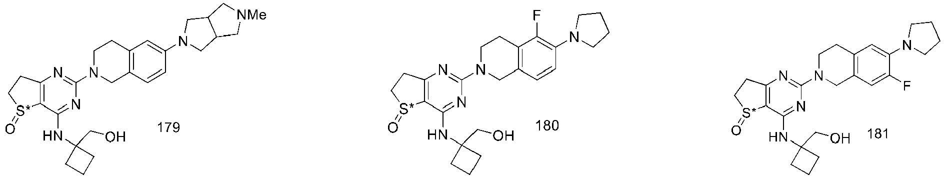

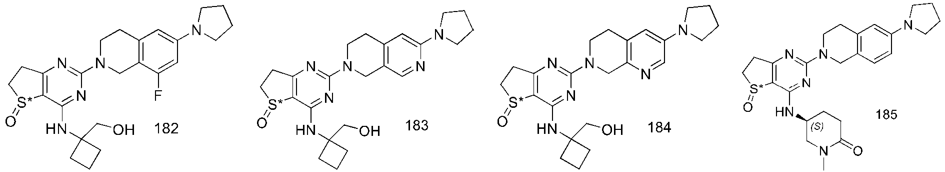

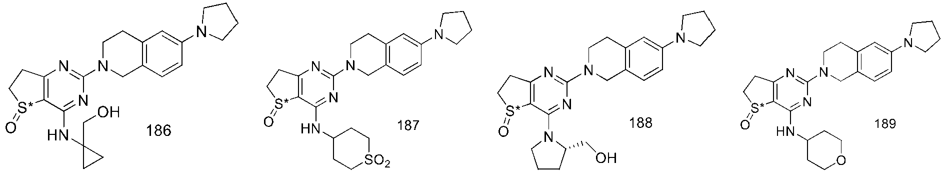

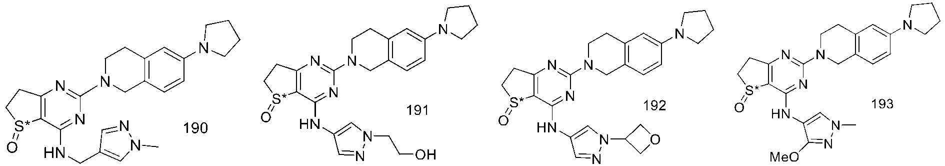

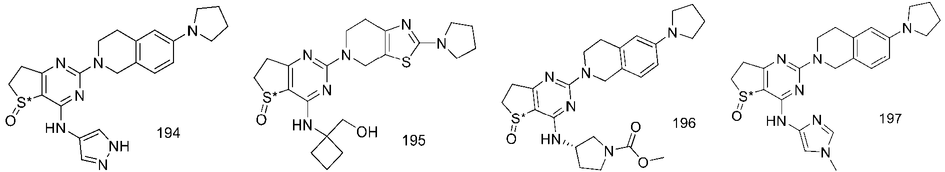

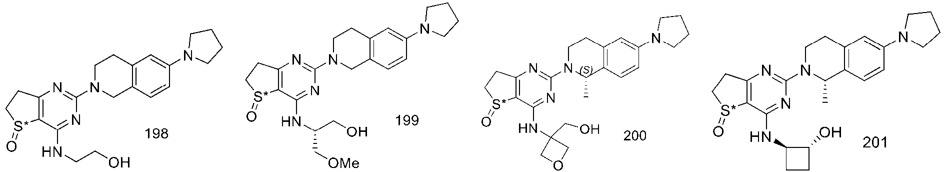

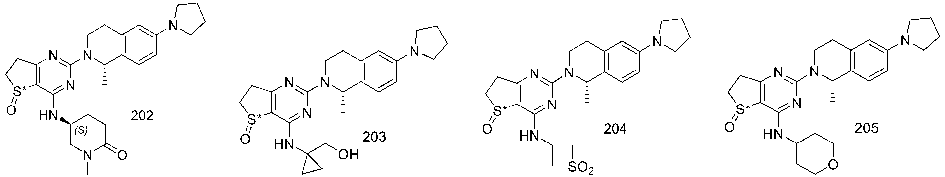

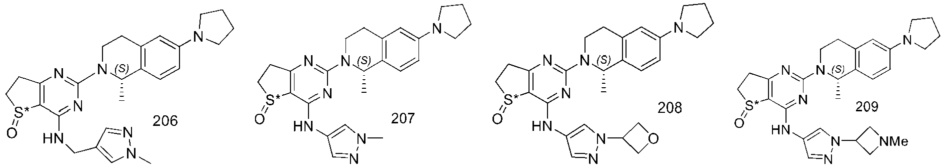

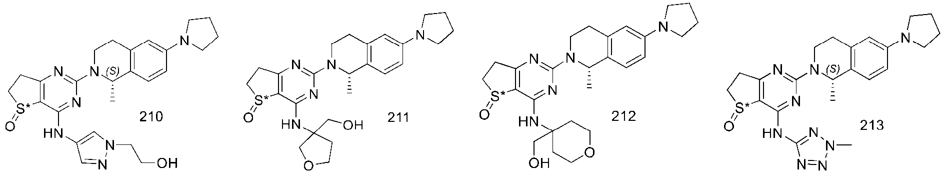

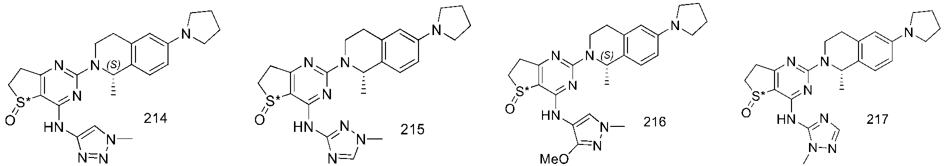

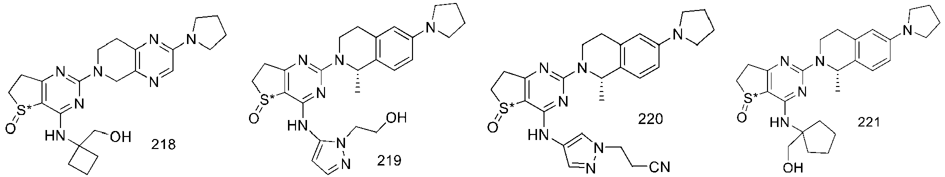

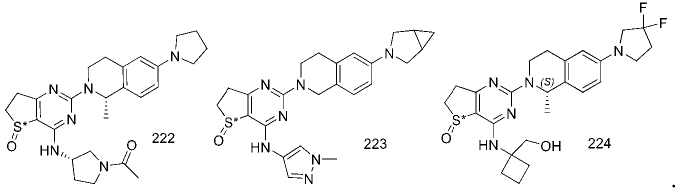

The present invention further provides a preferred solution, which relates to a pharmaceutical composition including a therapeutically effective amount of any one of the above-mentioned compounds or compounds of the general formula, stereoisomers thereof, or pharmaceutically acceptable salts thereof described above, and one or more pharmaceutically acceptable carriers or excipients.

The present invention further relates to use of any one of the compound, the stereoisomer thereof, or the pharmaceutically acceptable salt thereof as described above, or the pharmaceutical composition as described above in the preparation of a PDE4 inhibitor drug.

The present invention further relates to use of any one of the compound, the stereoisomer thereof, or the pharmaceutically acceptable salt thereof as described above, or the pharmaceutical composition as described above in the preparation of a medicament for treating or preventing inflammatory diseases, autoimmune diseases, metabolic diseases, nervous system diseases, and related diseases.

### DETAILED DESCRIPTION

Unless stated to the contrary, the terms used in the specification and claims have the following meanings.

The term "alkyl" refers to a saturated aliphatic hydrocarbon group, which is a linear or branched group containing 1 to 20 carbon atoms, preferably an alkyl group containing 1 to 8 carbon atoms, more preferably an alkyl group containing 1 to 6 carbon atoms, and most preferably an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, etc., and various branched isomers thereof, etc. Preferred in the present invention are methyl, ethyl, isopropyl, tert-butyl, haloalkyl, deuteroalkyl, alkoxy-substituted alkyl, and hydroxyl-substituted alkyl.

The term "alkenyl" refers to an alkyl group as defined above consisting of at least two carbon atoms and at least one carbon-carbon double bond, e.g., vinyl, 1-propenyl, 2-propenyl, and 1-, 2-, or 3- butenyl. The alkenyl may be substituted or unsubstituted. When the alkenyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, and heterocycloalkylthio.

The term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms, more preferably 3 to 8 carbon atoms, and further preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and the monocyclic cycloalkyl includes spiro, fused, and bridged cycloalkyl groups, non-limiting examples of which include 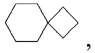 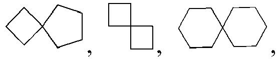 etc.

The cycloalkyl ring can be fused to an aryl, heteroaryl, or heterocycloalkyl ring, where a ring connected to a parent structure is cycloalkyl, preferably 5- to 6-membered cycloalkyl-fused phenyl and 5- to 6-membered cycloalkyl-fused 5- to 6-membered heteroaryl. Non-limiting examples include indanyl, tetrahydronaphthyl, benzocycloheptanyl, etc.

The cycloalkyl may be optionally substituted or unsubstituted. When the cycloalkyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "heterocyclyl" refers to a saturated or partially unsaturated monocyclic or polycyclic hydrocarbon substituent, which contains 3 to 20 ring atoms, among which one or more ring atoms are heteroatoms selected from nitrogen, oxygen, C(O), S(O)(=NH), or S(O)ₘ (where m is an integer of 0 to 2), excluding -O-O-, -O-S-, or -S-S- moiety, while the remaining ring atoms are carbon. The group preferably contains 3 to 12 ring atoms, among which 1 to 4 ring atoms are heteroatoms; more preferably contains 3 to 12 ring atoms; most preferably contains 3 to 6 ring atoms, among which the number of heteroatoms is 1 or 2. Non-limiting examples of monocyclic heterocyclyl include oxetanyl, thietanyl, azetidinyl, tetrahydropyranyl, azepanyl, pyrrolidinyl, imidazolidinyl, tetrahydrofuryl, tetrahydrothienyl, dihydroimidazolyl, dihydrofuryl, dihydropyrazolyl, dihydropyrrolyl, piperidinyl, pyrrolidinyl, piperazinyl, morpholinyl, thiomorpholinyl, homopiperazinyl, pyranyl, pyridinonyl, etc., preferably oxetane, thietane, azetidine, tetrahydrofuryl, tetrahydropyranyl, 1-imino-1-oxothiopyran, azepanyl, pyrrolidinyl, piperidinyl, and piperazinyl. Polycyclic heterocyclyl includes spiro, fused, and bridged heterocyclyl, non-limiting examples of which include 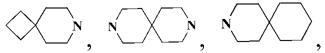 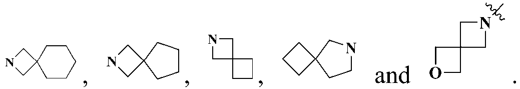 The heterocyclyl may be substituted or unsubstituted. When the heterocyclyl is substituted, the substituent is preferably one or more groups independently selected from hydrogen, alkyl, hydroxyalkyl, amino, imino, cyano, oxo, cycloalkyl, heterocycloalkyl, aryl, and heteroaryl.

The heterocyclyl may be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The heterocyclyl may be fused to a heteroaryl, heterocyclyl, or cycloalkyl ring, where a ring connected to a parent structure is heteroaryl, preferably 5- to 6-membered heterocyclyl-fused phenyl or 5- to 6-membered heterocyclyl-fused 5- to 6-membered **heteroaryl;** e.g.,

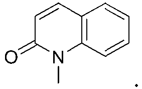

The term "aryl" refers to a 6- to 14-membered all-carbon monocyclic or fused polycyclic ring (i.e., a ring sharing a pair of adjacent carbon atoms) group having a conjugated *π* electron system, which is preferably 6- to 10-membered, e.g., phenyl and naphthyl. Phenyl is more preferred.

The aryl ring may be fused to a heteroaryl, heterocyclyl, or cycloalkyl ring, where a ring connected to a parent structure is an aryl ring, preferably benzo-fused 5- to 6-membered heterocyclyl or benzo-fused 5- to 6-membered heteroaryl; e.g.,

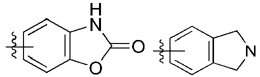

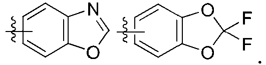

The aryl may be substituted or unsubstituted. When the aryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "heteroaryl" refers to a heteroaromatic system containing 1 to 4 heteroatoms and 5 to 14 ring atoms, where the heteroatoms are selected from oxygen, sulfur, and nitrogen. The heteroaryl is preferably 5- to 8-membered monocyclic heteroaryl or 7- to 14-membered bicyclic heteroaryl, more preferably 5-membered monocyclic heteroaryl, 6-membered monocyclic heteroaryl, 8-membered bicyclic heteroaryl, 9-membered bicyclic heteroaryl, or 10-membered bicyclic heteroaryl, e.g., imidazolyl, furyl, thienyl, thiazolyl, pyrazolyl, pyrrolyl, triazolyl, tetrazolyl, pyridinyl, pyrimidinyl, pyrazinyl, pyridazinyl, piperazinyl, pyridoimidazolyl, pyrimidoimidazolyl, etc., preferably pyridoimidazolyl, pyrimidoimidazolyl, benzothiazolyl, thiazolothienyl, imidazothiazolyl, thienothiazolyl, thiazolothiazolyl, thiazolotriazolyl, pyrazolothiazolyl, thiazolopyrazolyl, imidazothienyl, indolyl, quinazolinyl, benzisoxazolyl, benzoxazolyl, triazolopyridinyl, benzofuryl, isobenzofuryl, thiadiazolyl, tetrazolyl, triazolothiadiazolyl, or oxadiazolyl.

The heteroaryl ring may be fused to a heteroaryl, heterocyclyl, or cycloalkyl ring to form a fused ring, in which a ring connected to a parent structure is a heteroaryl ring, preferably 5- to 6-membered heteroaryl-fused 5- to 6-membered heteroaryl or 5- to 6-membered heteroaryl-fused phenyl; e.g.,

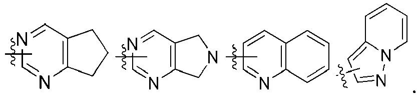

The heteroaryl may be optionally substituted or unsubstituted. When the heteroarylis substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "alkoxy" refers to -O-(alkyl) and -O-(unsubstituted cycloalkyl), where the alkyl is as defined above. Non-limiting examples of alkoxy include methoxy, ethoxy, propoxy, butoxy, cyclopropoxy, cyclobutoxy, cyclopentoxy, and cyclohexoxy. The alkoxy may be optionally substituted or unsubstituted. When the alkoxyis substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

"Haloalkyl" refers to alkyl substituted with one or more halogens, where the alkyl is as defined above. Non-limiting examples include trifluoromethyl and difluoromethyl.

"Haloalkoxy" refers to alkoxy substituted with one or more halogens, where the alkoxy is as defined above.

"Hydroxyalkyl" refers to hydroxy-substituted alkyl, where the alkyl is as defined above. Non-limiting examples include -C(CH₃)₂(OH).

Different expressions, such as "X is selected from A, B, or C", "X is selected from A, B, and C", "X is A, B, or C", and "X is A, B, and C", all indicate the same meaning, which means that X can be any one or more of A, B, and C.

In the present invention, "------" means a bond or absence.

A chiral carbon in the compound of the present invention can be in either R configuration or S configuration.

The "*" or 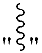 on a substituent of the present invention indicates a position where the substituent is connected to a substitution site.

Each hydrogen described in the present invention can be replaced by its isotope deuterium, and any hydrogen in example compounds involved in the present invention can also be replaced by a deuterium atom.

"Optional" or "optionally" means that the subsequently described event or environment may, but not necessarily, occur, and this expression includes occasions when the event or environment occurs or does not occur. For example, "heterocyclyl optionally substituted with alkyl" means that the alkyl may, but not necessarily, exist, and this expression includes the case where the heterocyclyl is substituted with the alkyl and the case where the heterocyclyl is not substituted with alkyl.

"Substituted" means that one or more hydrogen atoms in a group, preferably at most 5, more preferably 1-3 hydrogen atoms, are each independently replaced by a corresponding number of substituents. It goes without saying that substituents are only in their possible chemical positions, and those skilled in the art can determine (by experiment or theory) possible or impossible substitutions without excessive efforts. For example, amino or hydroxyl having a free hydrogen may be unstable when bonded with a carbon atom having an unsaturated (such as olefinic) bond.

"Pharmaceutical composition" means a mixture containing one or more compounds described herein or physiologically/pharmaceutically acceptable salts or prodrugs thereof and other chemical components, as well as other components such as physiologically/pharmaceutically acceptable carriers and excipients. The purpose of the pharmaceutical composition is to promote the administration to organisms, facilitate the absorption of active ingredients and further exert biological activity.

"Pharmaceutically acceptable salt" refers to a salt of the compound of the present invention, which is safe and effective when used in mammals and has due biological activity.

### PARTICULAR EMBODIMENTS

The present invention will be described in detail by the following examples, but it does not mean that the present invention is adversely limited in any way. The compound of the present invention can be prepared by a variety of synthesis methods well known to those skilled in the art, including specific embodiments listed below, embodiments formed by combining them with other chemical synthesis methods, and equivalent alternatives well known to those skilled in the art. However, preferred embodiments are not limited to the examples of the present invention. All reagents used in the present invention are available from commercial channels or prepared with reference to the prior art. The names of all compounds except reagents are generated by ChemDrew 20.0.

General method: The compounds of the present invention can be prepared according to the following non-limiting general methods and examples.

Scheme 1: Synthesis of a compound of general formula (1), where R₁, R₂, R₃, R₄, and R₅ are as defined above:

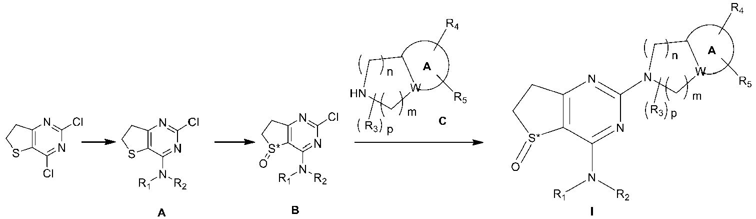

As shown in scheme 1, a compound of general formula A can be prepared by reacting commercially available 2,4-dichloro-6,7-dihydrothieno[3,2-d]pyrimidine with an amine of formula R₁R₂NH. Typical reaction conditions include heating a solution of 2,4-dichloro-6,7-dihydrothieno[3,2-d]pyrimidine and an appropriate amine in an appropriate protic or polar aprotic solvent at a temperature from room temperature to the boiling point of the solvent in the presence of an added organic or inorganic base. The amine of R₁R₂NH is commercially available or can be easily synthesized by a method known to those skilled in the art. Suitable conditions include reacting 2,4-dichloro-6,7-dihydrothieno[3,2-d]pyrimidine with the amine of formula R₁R₂NH in DMF in the presence of DIPEA at 120°C, as listed in Preparation Example 1.

In addition, the compound of general formula A can also be prepared by reacting 2,4-dichloro-6,7-dihydrothieno[3,2-d]pyrimidine with an appropriate amine in an appropriate solvent or solvent mixture such as 1,4-dioxane in the presence of a palladium catalyst and an appropriate inorganic or organic base such as potassium tert-butoxide, potassium carbonate or cesium carbonate.

As shown in Scheme 1, a sulfoxide compound (a compound of general formula B) can be prepared by reacting the compound of general formula A with an appropriate oxidant in an appropriate solvent, optionally in the presence of a chiral catalyst. For example, a solution of the compound of general formula A in DCM/water can be oxidized by treatment with ^{t}BuOOH at 0°C-room temperature in the presence of Ti(OiPr)4 and (S)-(-)-1,1'-bi(2-naphthol).

The sulfoxide compound, i.e., the compound of general formula B, can be prepared in the form of a single enantiomer, or in the form of an enantiomeric mixture, and then separated by a method known to those skilled in the art. Alternatively, it may also be preferable to subject the compound to a subsequent reaction in the form of an enantiomeric mixture and to separate enantiomers at a later stage.

As shown in Scheme 1, the compound of general formula (I) can be prepared by reacting the compound of general formula B with the amine of the compound of general formula C, which is commercially available or easily synthesized by a method known to those skilled in the art.

Typical reaction conditions include heating a solution of the compound of general formula B and the amine of the compound of general formula C in an appropriate protic or polar aprotic solvent at a temperature from room temperature to the boiling point of the solvent in the presence of an added organic or inorganic base. For example, a solution of the compound of general formula B in DMSO is reacted with the amine of the compound of general formula C in the presence of DIPEA.

Alternatively, the compound (the compound of general formula (I)) can also be prepared by reacting the compound of general formula B with the amine of the compound of general formula C in an appropriate solvent or solvent mixture such as 1,4-dioxane in the presence of a palladium catalyst and an appropriate inorganic or organic base such as potassium tert-butoxide, potassium carbonate or cesium carbonate.

### Preparation Examples and Examples

The following examples are illustrative of the present invention and do not limit the present invention in any way. Unless otherwise specified, all fractions are by weight and temperatures are in degrees Celsius. Pressures are at or near atmospheric pressure. All data are measured by Agilent (Agilent 6120 and/or 1100). Except for synthesized intermediates, all reagents used in the present invention are available from commercial channels. The names of all compounds except reagents are generated by ChemDrew 20.0.

The following abbreviations are used:
(Boc)₂O: di-tert-butyl pyrocarbonate; BH₃: borane; DIEPA: N,N-diisopropylethylamine; EDCI: 1-(3-dimethylaminopropyl)-3-ethylcarbodiimide hydrochloride; EA: ethyl acetate; Et3N: triethylamine; HATU: 2-(7-azabenzotriazole)-N,N,N',N'-tetramethyluronium hexafluorophosphate; HBTU: O-benzotriazole-tetramethyluronium hexafluorophosphate; HOAc: acetic acid; HOBt: 1-hydroxybenzotriazole; ee: enantiomeric excess; NCS: N-chlorosuccinimide; Rochelle's salt: potassium sodium tartrate tetrahydrate; PE: petroleum ether; Pd(dppf)₂Cl₂: [1,1'-bis(diphenylphosphino)ferrocene]palladium dichloride; Pd₂(dba)₃: tris(dibenzylideneacetone)dipalladium; Pd(PPh₃)₄: tetrakis(triphenylphosphine)palladium; PMB: p-methoxybenzyl cyanide; PPh₃:triphenylphosphine; Pin₂B₂: pinacol diboronate; THF: tetrahydrofuran; TFA: trifluoroacetic acid; TsOH: 4-toluenesulfonic acid; Xphos: 2-dicyclohexylphosphino-2',4',6'-triisopropylbiphenyl; and Burgess reagent: methyl N-(triethylammoniosulfonyl)carbamate.

Preparation Example 1: (R)-2-chloro-4-((1-(hydroxymethyl)cyclobutyl)amino)-6,7-dihydrothieno[3,2-d]pyrimidine 5-oxide (I01)

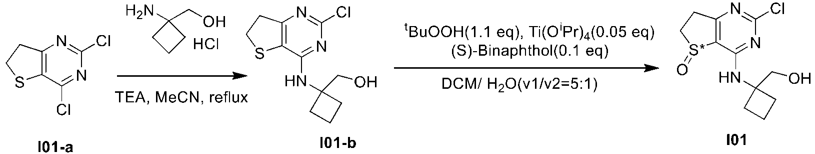

### 1) (1-((2-Chloro-6,7-dihydrothieno[3,2-d]pyrimidin-4-yl)amino)cyclobutyl)methanol (I01-b)

At room temperature, to a solution of 2,4-dichloro-6,7-dihydrothieno[3,2-d]pyrimidine (I01-a, 22.6 g,110 mmol,1 eq) in acetonitrile (200 mL), (1-aminocyclobutyl)methanol hydrochloride (15 g, 110.00 mmol, 1 eq) and triethylamine (80 ml, 550.00 mmol, 5 eq) were successively added. The resulting material was heated to 65-70°C, and stirring was continued for 12 hours. Then, water (1.2 L) was slowly added over 20 minutes and the resulting material was cooled to 25°C over 2 hours. After continued stirring for 12 hours, the resulting material was filtered. The filter cake was washed with a 2 : 1 water and acetonitrile solution(400 mL) and water (200 mL) successively. The resulting solid was dried in vacuum at 50°C for 12 hours to obtain I01-b (15 g, 50.5 %). LCMS: 272.15 [M+H]⁺.

### 2) (R)-2-chloro-4-((1-(hydroxymethyl)cyclobutyl)amino)-6,7-dihydrothieno[3,2-d]pyrimidine 5-oxide (I01)

At room temperature, to a solution of I01-b (10.36 g, 38 mmol) in dichloromethane (50 mL), (S)-(-)-1.1'-bi-2-naphthol (4.4 g, 15.20 mmol, 0.2 eq), Ti(OⁱPr)₄ (540 mg, 1.90 mmol, 0.05 eq), and water (8 mL) was added successively. After stirring for 1 hour, tert-butyl hydrogenperoxide (70% in water, 3.79 g, 42 mmol, 1.1 eq) was added in one portion. The reaction solution became homogeneous, and the reaction temperature reached about 40°C. The resulting material was naturally cooled to room temperature. Stirring was continued for 1.5 hours, followed by filtration. The filter cake was washed with isopropyl acetate (243 mL x 2) and air-dried to obtain I01 (6.2 g, 56.5%). LCMS: 288.25 [M+H]⁺. ¹H NMR (400 MHz, CD₃OD) δ 3.89 (d, J = 2.0 Hz, 2H), 3.71 - 3.62 (m, 1H), 3.51 - 3.41 (m, 1H), 3.25 - 3.10 (m, 2H), 2.30 (tt, J = 6.4, 2.1 Hz, 4H), 1.97 - 1.77 (m, 2H).

### Example 1: Synthesis of compound 1

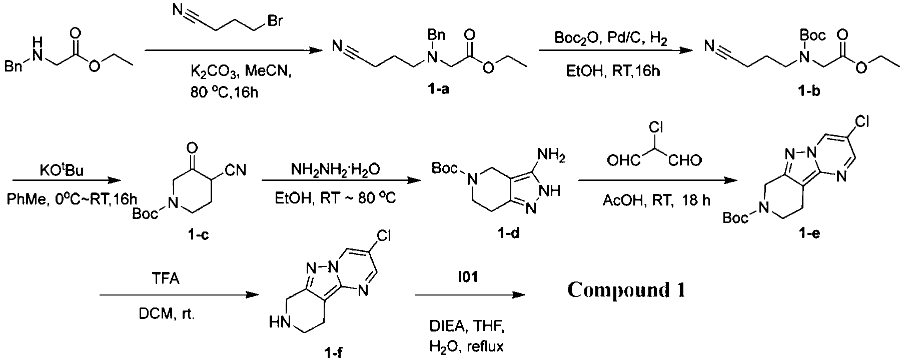

### 1) Ethyl N-benzyl-N-(3-cyanopropyl)glycinate (1-a)

At room temperature, to a solution of ethyl benzylglycinate (5.01 g, 25.87 mmol, 1.0 eq) in acetonitrile (50 mL), 4-bromobutyronitrile (4.59 g, 31.04 mmol, 1.2 eq) and K₂CO₃ (10.72 g, 77.61 mmol, 3.0 eq) were added. After stirring at 80°C for 16 hours, the resulting material was cooled to room temperature, and water (150 mL) was added. After extraction with ethyl acetate (100 mL x 3), the resulting material was dried over anhydrous Na₂SO₄, filtered, concentrated, and purified by column chromatography (PE: EtOAc = 5: 1) to obtain 1-a as a yellow oil product (4.61 g, 68.5%). LCMS: 261.20 [M+H]⁺.

### 2) Ethyl N-(tert-butoxycarbonyl)-N-(3-cyanopropyl)glycinate (1-b)

At room temperature, to a solution of 1-a (4 g, 15.38 mmol, 1.0 eq) and Boc₂O (6.71 g, 30.77 mmol, 2.0 eq) in EtOH (100 mL), 10% Pd/C (0.4 g) was added. After hydrogen absorption at room temperature for 16 hours, a solid was filtered off, followed by concentration and purification by column chromatography (petroleum ether/EtOAc = 3: 1) to obtain 1-b (3.66 g, 88.1%) as a colorless oil product. LCMS: 271.20 [M+H]⁺.

### 3) Tert-butyl 4-cyano-3-piperidinone-1-carboxylate (1-c)

At 0°C, to a solution of 1-b (17 g, 62.9 mmol) in toluene (100 mL), t-BuOK (8.5 g, 75.5 mmol) was added. After stirring at room temperature for 30 minutes, saturated NH₄Cl (200 mL) and n-hexane (200 mL) were added. Then, HCl (2 N) was added to reach pH 6. After extraction with ethyl acetate (200 mL x 2), the resulting material was dried over anhydrous Na₂SO₄, filtered, and concentrated to obtain 1-c (13 g) as a yellow oil product. The crude product was directly used in the next reaction without purification. LCMS: 225.15 [M+H]⁺.

### 4) Tert-butyl 3-amino-2,4,6,7-tetrahydro-5H-pyrazolo[4,3-c]pyridine-5-carboxylate (1-d)

A mixture of 1-c (13 g, 57.9 mmol) and hydrazine hydrate (85%, 6.6 mL, 116 mmol) in EtOH (100 mL) was stirred at 60°C for 3 hours. The resulting material was cooled to room temperature and concentrated. EtOAc (250 mL) was added. The resulting material was washed with saturated Na₂CO₃ (100 mL) and brine (100 mL) successively. The resulting material was dried over anhydrous Na₂SO₄, filtered, concentrated, and purified by column chromatography (DCM/MeOH = 20: 1) to obtain 1-d (10 g, 72%) as a white solid product. LCMS: 239.15 [M+H]⁺.

### 5) Tert-butyl 3-chloro-9,10-dihydropyrido[3',4':3,4]pyrazolo[1,5-a]pyrimidine-8(7H)-carboxylate (1-e)

At room temperature, to a solution of 1-d (150 mg, 0.63 mmol, 1 eq) in AcOH (4 mL), 2-chloromalealdehyde (66 mg, 0.63 mmol, 1 eq) was added and stirred for 16 hours, and a solution of saturated NaHCO₃ (40 mL) was then added. After extraction with ethyl acetate (20 mL x 2), the resulting material was dried over anhydrous Na₂SO₄, filtered, concentrated, and purified by column chromatography (PE: EtOAc = 1: 1) to obtain 1-e (80 mg, 41.2%) as a yellow solid product. LCMS: 309.10 [M+H]⁺.

### 6) 3-Chloro-7,8,9,10-tetrahydropyrido[3',4':3,4]pyrazolo[1,5-a]pyrimidine (1-f)

At 0°C, to a solution of 1-e (80 mg, 0.26 mmol, 1 eq) in DCM (3 mL), TFA (1 mL) was added. After stirring at room temperature for 1 hour, the resulting material was concentrated to obtain 1-f (55 mg, 99%) as a yellow solid product. The crude product was directly used in the next reaction without purification. LCMS: 209.25 [M+H]⁺.

### 7) (R)-2-(3-chloro-9,10-dihydropyrido[3',4':3,4]pyrazolo[1,5-a]pyrimidin-8(7H)-yl)-4-((1-(hydroxymethyl)cyclobutyl)amino)-6,7-dihydrothieno[3,2-d]pyrimidine 5-oxide (1)

At room temperature, to a solution of I01 (66 mg, 0.26 mmol, 1 eq) and 1-f (55 mg, 0.26 mmol, 1.0 eq) in THF (2 mL) and H₂O (0.5 mL), DIEPA (168 mg,1.3 mmol, 5.0 eq) was added. After stirring at 70°C overnight, the resulting material was concentrated. After purification by Pre-TLC (SiO₂, DCM/MeOH = 10/1), 1 (67.3 mg, 56%) as a white solid product was obtained. LCMS: 460.25 [M+H]⁺. ¹H NMR (400 MHz, DMSO-d6) δ 9.42 (d, J = 2.3 Hz, 1H), 8.48 (d, J = 2.3 Hz, 1H), 7.46 (s, 1H), 5.03 (s, 2H), 4.84 (s, 1H), 4.07 (s, 2H), 3.72 (s, 2H), 3.17 (d, J = 13.5 Hz, 2H), 2.99 - 2.89 (m, 1H), 2.81 (s, 3H), 2.31 (dd, J = 21.2, 10.5 Hz, 2H), 2.18 (s, 2H), 1.85 - 1.69 (m, 2H).

The following compounds were synthesized by similar methods.

| Compound | MS (M+H)⁺ | NMR |
|---|---|---|
| 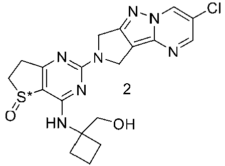 | 446.1 | ¹H NMR (400 MHz, DMSO-d₆) 9.56 (d, J = 5.5 Hz, 1H), 8.58 (s, 1H), 7.47 (s, 1H), 4.87 (d, J = 4.6 Hz, 1H), 4.78 (d, J = 7.2 Hz, 4H), 3.76 (s, 2H), 3.52 - 3.40 (m, 1H), 3.20 (s, 1H), 3.03 - 2.93 (m, 1H), 2.87 (dd, J = 12.8, 6.7 Hz, 1H), 2.39 - 2.29 (m, 2H), 2.21 (s, 2H), 1.78 (s, 2H). |
| 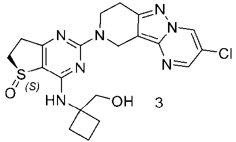 | 460.35 | ¹H NMR (400 MHz, DMSO-d₆) δ 9.41 (s, 1H), 8.51 (s, 1H), 7.44 (s, 1H), 4.99 (s, 2H), 4.83 (s, 1H), 4.11 (s, 2H), 3.71 (s, 2H), 3.21 (s, 2H), 2.89 (s, 4H), 2.32 (d, J = 9.8 Hz, 2H), 2.16 (s, 2H), 1.76 (s, 2H). |
| 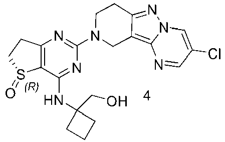 | 460.35 | ¹H NMR (400 MHz, DMSO-d₆) δ 9.41 (s, 1H), 8.51 (s, 1H), 7.44 (s, 1H), 4.99 (s, 2H), 4.83 (s, 1H), 4.11 (s, 2H), 3.72 (s, 2H), 3.19 (s, 2H), 2.89 (s, 4H), 2.32 (dd, J = 20.3, 11.3 Hz, 2H), 2.16 (s, 2H), 1.76 (s, 2H). |

### Example 2: Synthesis of compounds 5 and 6

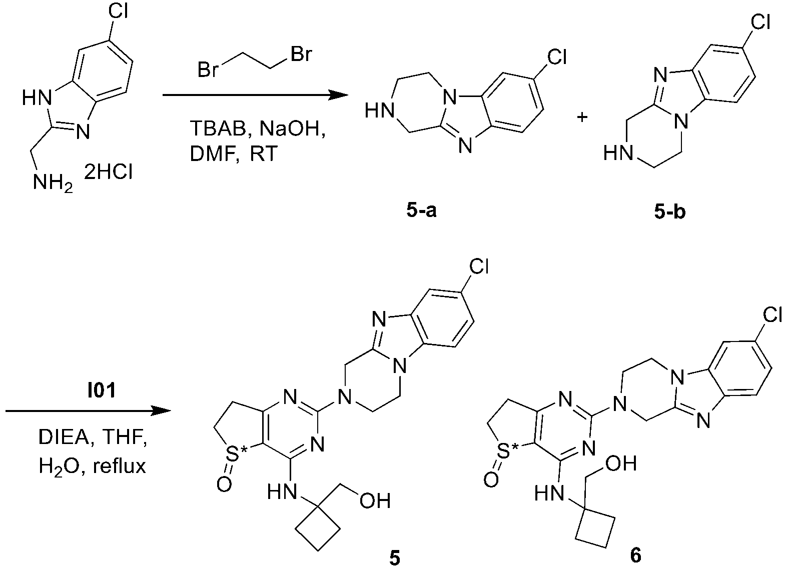

### 1) 7-Chloro-1,2,3,4-tetrahydrobenzo[4,5]imidazo[1,2-a]pyrazine (5-a) and 8-chloro-1,2,3,4-tetrahydrobenzo[4,5]imidazo[1,2-a]pyrazine (5-b)

At room temperature, to a 100 mL three-necked flask, NaOH (15 g, 0.38 mol, 27.0 eq) and water (15 mL) were added and stirred until the solution became clear. (6-Chloro-1H-benzo[d]imidazol-2-yl)methylamine hydrochloride (2.63 g, 14.32 mmol, 1.0 eq) and tetrabutylammonium bromide (185 mg,0.57 mmol, 0.04 eq) were then added successively. After stirring for 1 hour, a solution of 1,2-dibromoethane (5.38 g, 28.64 mmol, 2.0 eq) in 50 mL of DMF was dropwise added. After continued stirring for 4 hours, a solid was filtered off, and a filtrate was concentrated. After purification by column chromatography (DCM: MeOH: aqueous ammonia = 50: 1: 0.05 to 40: 1: 0.05), a mixture of 5-a and 5-b (1.25 g, 50.4%) as a white solid product was obtained. LCMS: 208.10 [M+H]⁺.

### 2) (R)-2-(8-chloro-3,4-dihydrobenzo[4,5]imidazo[1,2-a]pyrazin-2(1H)-yl)-4-((1-(hydroxymethyl)cyclobutyl)amino)-6,7-dihydrothieno[3,2-d]pyrimidine 5-oxide (5) and (R)-2-(7-chloro-3,4-dihydrobenzo[4,5]imidazo[1,2-a]pyrazin-2(1H)-yl)-4-((1-(hydroxymethyl)cyclobutyl)amino)-6,7-dihydrothieno[3,2-d]pyrimidine 5-oxide (6)

At room temperature, to a solution of I01 (40 mg, 0.14 mmol, 1.0 eq) and 5-a/5-b (34.5 mg, 0.17 mmol, 1.2 eq) in THF (4 mL) and H₂O (1 mL), DIEPA (55.mg, 0.42 mmol, 3 eq) was added. After stirring at 65°C for 4 hours, the resulting material was cooled to room temperature and concentrated. After purification by Prep-HPLC (0.1% TFA in ACN/H₂O) and further purification by SFC, 5 (7.94 mg, 12.4%) and 6 (5.36 mg, 8.42%) as a white solid product were obtained. LCMS: 459.10 [M+H]⁺.

5-a: ¹H NMR (400 MHz, Methanol-d₄) δ 7.58 (s, 1H), 7.47 (s, 1H), 7.27 (s, 1H), 5.20 (s, 2H), 4.59 (s, 2H), 4.41 (s, 2H), 4.24 (s, 2H), 3.93 (s, 2H), 3.57 (s, 2H), 3.11 (s, 2H), 2.34 (s, 2H), 1.94 (s, 2H).

5-b: ¹H NMR (400 MHz, Methanol-d₄) δ 7.58 (s, 2H), 7.47 (s, 1H), 7.27 (s, 2H), 5.20 (s, 2H), 4.59 (s, 2H), 4.41 (s, 2H), 4.24 (s, 2H), 3.93 (s, 2H), 3.11 (s, 2H), 2.34 (s, 4H), 1.94 (s, 2H).

### Example 3: Synthesis of compound 7

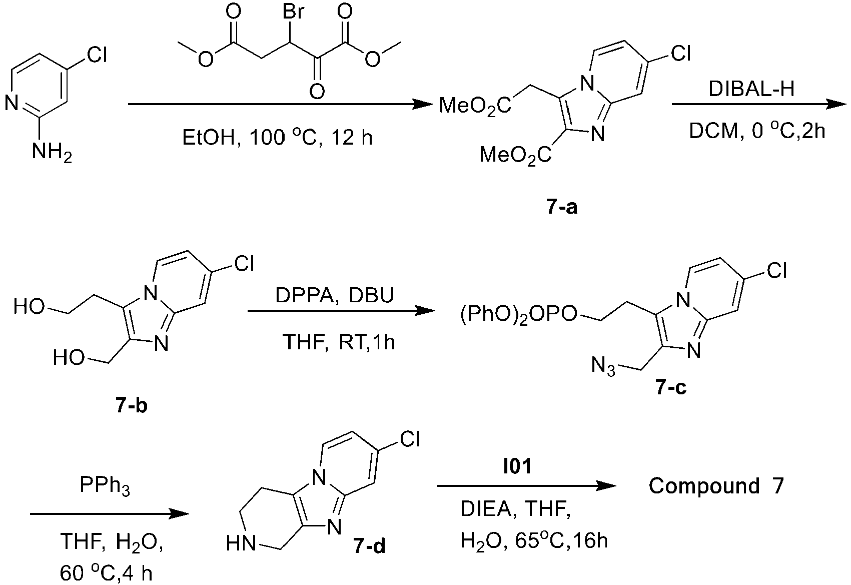

### 1) Methyl 7-chloro-3-(2-methoxy-2-oxoethyl)imidazo[1,2-a]pyridine-2-carboxylate (7-a)

A solution of 4-Chloropyridine-2-amine (1.8 g, 14.06 mmol, 1.0 eq) and 3-bromo-2-ketoglutarate (4.6 g, 18.18 mmol, 1.3 eq) in ethanol (35 mL) was stirred at 100°C for 12 hours. The resulting material was cooled to room temperature and concentrated. After purification by column chromatography (dichloromethane: methanol: aqueous ammonia = 50: 1: 0.05 to 40: 1: 0.05), 7-a (800 mg, 20.2%) as a yellow solid product was obtained. LCMS: 283.13 [M+H]⁺.

### 2) 2-(7-Chloro-2-(hydroxymethyl)imidazo[1,2-a]pyridin-3-yl)ethan-1-ol (7-b)

At 0°C, to a solution of 7-a (500 mg, 1.77 mmol, 1.0 eq) in CH₂Cl₂ (10 mL), 1 M DIBAL-H in CH₂Cl₂ (10.8 mL, 10.8 mmol, 6.0 eq) was dropwise added. After continued stirring for 2 hours, a solution of Rochelle's salt (30 mL) was added. After extraction with dichloromethane (30 mL x 3), the resulting material was dried over anhydrous Na₂SO₄, filtered, concentrated, and purified by column chromatography (DCM: MeOH: aqueous ammonia = 50: 1: 0.05 to 40: 1: 0.05) to obtain 7-b (220 mg, 54.8%) as a yellow oil product. LCMS: 227.05 [M+H]⁺.

### 3) Ethyl diphenyl 2-(2-(azidomethyl)-7-chloroimidazo[1,2-a]pyridin-3-yl)phosphate (7-c)

At 0°C, to a solution of 7-b (200 mg, 0.88 mmol, 1.0 eq) and DBU (269 mg, 1.77 mmol, 2.0 eq) in THF (5 mL), DPPA (487 mg, 1.77 mmol, 2.0 eq) was added. After stirring at room temperature for 1 hour, ethyl acetate (50 mL) was added, and the resulting material was washed with water (50 mL x 2) and saturated brine (50 mL) successively. The resulting material was dried over anhydrous Na₂SO₄, filtered, concentrated, and purified by column chromatography (n-hexane/EtOAc = 2: 1) to obtain 7-c as a yellow oil product (170 mg, 40% yield). LCMS: 484.10 [M+H]⁺.

### 4) 8-Chloro-1,2,3,4-tetrahydroimidazo[1,2-a:4,5-c']dipyridine (7-d)

The compound 7-c (100 mg, 0.21 mmol, 1.0 eq) and PPh₃ (66 mg, 0.25 mmol) in THF/H₂O (2.2 mL, v₁/v₂ = 10: 1) were stirred at 60°C for 4 hours. The resulting material was cooled to room temperature and concentrated. The resulting material was purified by Pre-HPLC (0.1% TFA in H₂O/ACN from 5% to 95%) to obtain 7-d (30 mg, 69.%) as a colorless oil product. LCMS: 208.10 [M+H]⁺.

### 5) (R)-2-(8-chloro-3,4-dihydroimidazo[1,2-a:4,5-c']dipyridin-2(1H)-yl)-4-((1-(hydroxymethyl)cyclobutyl)amino)-6,7-dihydrothieno[3,2-d]pyrimidine 5-oxide (7)

At room temperature, to a solution of 7-d (40 mg, 0.19 mmol, 1.5 eq) and 101 (37 mg, 0.13 mmol, 1.0 eq) in THF (2 mL), DIEPA (50 mg, 0.39 mmol, 3.0 eq) and water (0.5 mL) were added. After stirring at 65°C overnight, the resulting material was concentrated. The resulting material was purified by Pre-HPLC (0.1% TFA in H₂O/ACN from 5% to 95%) to obtain 7 (33.5 mg, 38.4%) as a white solid product. LCMS: 459.10 [M+H]⁺. ¹H NMR 1H NMR (400 MHz, Methanol-d₄) δ 8.62 - 8.58 (m, 1H), 8.04 - 8.01 (m, 1H), 7.52 (dd, J = 7.2, 2.0 Hz, 1H), 5.14 (s, 2H), 4.33 (s, 2H), 3.94 (q, J = 11.4 Hz, 2H), 3.58 (dd, J = 17.2, 8.5 Hz, 1H), 3.42 - 3.32 (m, 1H), 3.16 - 3.01 (m, 5H), 2.41 - 2.32 (m, 4H), 1.92 (q, J = 10.0, 8.7 Hz, 2H).

### Example 4: Synthesis of compound 8

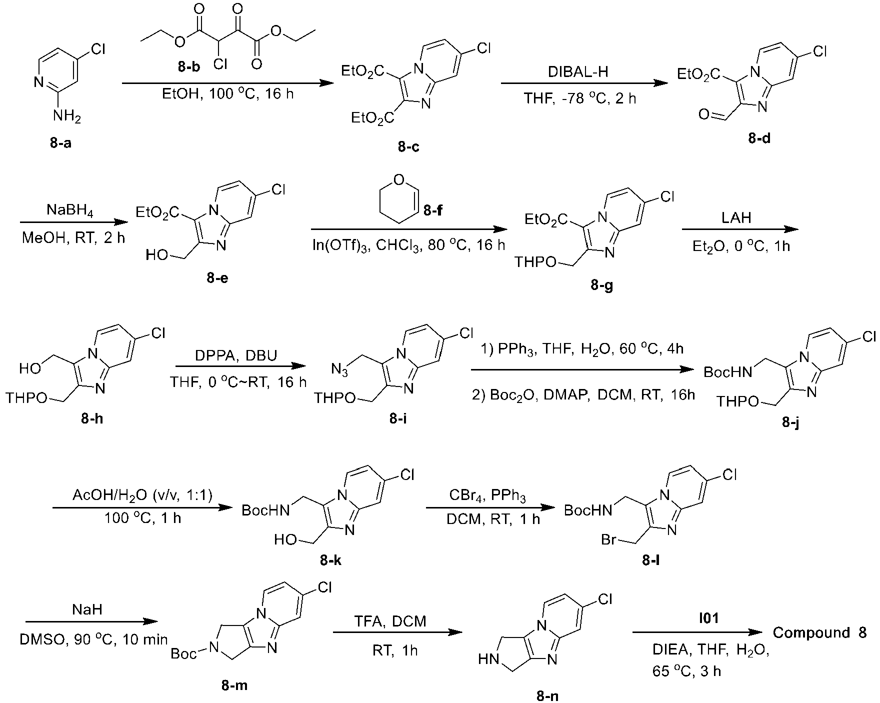

### 1) Diethyl 7-chloroimidazo[1,2-a]pyridine-2,3-dicarboxylate (8-c)

At room temperature, to a solution of 4-chloropyridine-2-amine (8-a, 10 g, 77.78 mmol, 1 eq) in ethanol (100 mL), diethyl 2-chloro-3-oxalacetate (8-b, 8.65 g, 38.89 mmol, 0.5 eq) was added. The resulting material was heated to 100°C, and stirring was continued for 16 hours. The resulting material was cooled to room temperature and concentrated. The resulting material was purified by column chromatography (PE: EtOAc = 5: 1) to obtain 8-c (7.67 g, 33.2%) as a white solid product. LCMS: 297.05 [M+H]⁺.

### 2) Ethyl 7-chloro-2-formylimidazo[1,2-a]pyridine-3-carboxylate (8-d)

At -78°C and under nitrogen protection, to a solution of 8-c (7.4 g, 24.94 mmol, 1 eq) in dry THF (100 mL), DIBAL-H (20.00 mL, 29.93 mmol, 1.2 eq) was dropwise added. After continued stirring for 2 hours, a saturated Rochelle's salt solution (aq. 100 mL) was added. After extraction with ethyl acetate (100 mL x 3), the resulting material was dried over anhydrous Na₂SO₄, filtered, and concentrated to obtain 8-d (4.5 g, 71.4%) as a white solid product. LCMS: 253.00 [M+H]⁺.

### 2) Ethyl 7-chloro-2-(hydroxymethyl)imidazo[1,2-a]pyridine-3-carboxylate (8-e)

At room temperature, to a solution of 8-d (4.5 g, 17.81 mmol, 1 eq) in MeOH (50 mL), NaBH₄ (336.87 mg, 8.91 mmol, 0.5 eq) was added in portions and stirred for 2 hours, 2 M H₂SO₄ (15 mL) was then added. After extraction with dichloromethane (100 mL x 3), the resulting material was dried over anhydrous Na₂SO₄, filtered, concentrated, and purified by column chromatography (DCM: MeOH = 20: 1) to obtain 8-e (4.2 g, 92.6%) as a white solid product. LCMS: 255.00 [M+H]⁺.

### 4) Ethyl 7-chloro-2-(((tetrahydro-2H-pyran-2-yl)oxy)methyl)imidazo[1,2-a]pyridine-3-carboxylate (8-g)

At room temperature, to a solution of 8-e (4.2 g, 16.50 mmol, 1 eq) in CHCl₃ (100 mL), 3,4-dihydro-2H-pyran (8-f, 7.00 g, 84.46 mmol, 5 eq) and In(OTf)₃ (434.00 mg, 0.83 mmol, 0.05 eq) were added. The resulting material was heated to 80°C, and stirring was continued for 16 hours. The resulting material was cooled to room temperature, concentrated, and purified by column chromatography (PE: EtOAc = 5: 1) to obtain 8-g (4.8 g, 85.9%) as a yellow oil product. LCMS: 339.15 [M+H]⁺.

### 5) (7-Chloro-2-(((tetrahydro-2H-pyran-2-yl)oxy)methyl)imidazo[1,2-a]pyridin-3-yl)methanol (8-h)

At 0°C and under nitrogen protection, to a solution of 8-g (4.8 g, 14.17 mmol, 1 eq) in Et₂O (150 mL), LAH (6.00 mL, 14.17 mmol, 1 eq) was dropwise added and stirred for 1 hour, and water (2.4 mL) and a 15% NaOH (0.6 mL) solution were then added. The resulting material was dried over anhydrous Na₂SO₄, filtered, concentrated, and purified by column chromatography (DCM: MeOH = 50: 1) to obtain 8-h (1.6 g, 38.1%) as a white solid product. LCMS: 297.20 [M+H]⁺.

### 6) 3-(Azidomethyl)-7-chloro-2-((tetrahydro-2H-pyran-2-yl)oxy)methyl)imidazo[1,2-a]pyridine (8-i)

At 0°C and under nitrogen protection, to a solution of 8-h (1.6 g, 5.39 mmol, 1 eq) in THF (20 mL), DPPA (1.93 g, 7.00 mmol, 1.3 eq) and DBU (1.07 g, 7.00 mmol, 1.3 eq) were dropwise added. The resulting material was heated to room temperature. After stirring for 16 hours, the reaction solution was directly used for the next reaction. LCMS: 322.20 [M+H]⁺.

### 7) Tert-butyl (7-chloro-2-(((tetrahydro-2H-pyran-2-yl)oxy)methyl)imidazo[1,2-a]pyridin-3-yl)methyl)aminocarboxylate (8-j)

At room temperature, to the solution of the previous step, PPh₃ (2.12 g, 8.09 mmol, 1.5 eq) and water (20 mL) were added successively. The resulting material was heated to 60°C, and stirring was continued for 4 hours. The resulting material was cooled to room temperature, and Boc₂O (2.40 g, 10.78 mmol, 2 eq) and DMAP (33.00 mg, 0.27 mmol, 0.05 eq) were added successively. After stirring for 12 hours, water (30 mL) was added. After extraction with dichloromethane (30 mL x 3), the resulting material was dried over anhydrous Na₂SO₄, filtered, concentrated, and purified by column chromatography (PE: EtOAc = 1: 1) to obtain 8-j (1 g, 81.3%) as a white solid product. LCMS: 396.20 [M+H]⁺.

### 8) Tert-butyl (7-chloro-2-(hydroxymethyl)imidazo[1,2-a]pyridin-3-yl)methyl)aminocarbamate (8-k)

A suspension of 8-j (1 g, 2.52 mmol, 1 eq) in AcOH (10 mL) and H₂O (10 mL) was stirred at 100°C for 1 hour. The resulting material was cooled to room temperature. 4 M NaOH was added to reach pH 7-8. After extraction with ethyl acetate (30 mL x 3), the resulting material was dried over anhydrous Na₂SO₄, filtered, concentrated, and purified by column chromatography (DCM: MeOH = 20: 1) to obtain 8-k (520 mg, 66%) as a white solid product. LCMS: 312.00 [M+H]⁺.

### 9) Tert-butyl (2-(bromomethyl)-7-chloroimidazo[1,2-a]pyridin-3-yl)methyl)aminocarbamate (8-1)

At room temperature, to a solution of 8-k (440 mg, 1.41 mmol, 1 eq) in DCM (10 mL), CBr₄ (562.00 mg, 1.69 mmol, 1.2 eq) and PPh₃ (444.00 mg, 1.69 mmol, 1.2 eq) were added successively. After stirring for 1 hour, the resulting material was concentrated. The resulting material was purified by Pre-TLC (SiO₂, DCM: MeOH = 20: 1) to obtain 8-1 (340 mg, 64.3%) as a white solid product. LCMS: 374.10 [M+H]⁺.

### 10) Tert-butyl 6-chloro-1,3-dihydro-2H-pyrrolo[3',4':4,5]imidazo[1,2-a]pyridine-2-carboxylate (8-m)

At room temperature, to a solution of 8-1 (290 mg, 1.06 mmol, 1 eq) in DMSO (60 mL), NaH (64.00 mg, 1.58 mmol, 1.5 eq) was added. The resulting material was heated to 90°C and stirred for 10 minutes. The resulting material was cooled to room temperature, and water (60 mL) was added. After extraction with ethyl acetate (60 mL x 3), the resulting material was dried over anhydrous Na₂SO₄, filtered, and concentrated. The resulting material was purified by Pre-TLC (SiO₂, PE: EtOAc = 1: 1) to obtain 8-m (13 mg, 5.9%) as a white solid product. LCMS: 294.25 [M+H]⁺.

### 11) 6-Chloro-2,3-dihydro-1H-pyrrolo[3',4':4,5]imidazo[1,2-a]pyridine (8-n)

At room temperature, to a solution of 8-m (17 mg, 0.04 mmol, 1 eq) in DCM (4 mL), TFA (1 mL) was added. After stirring for 1 hour, the resulting material was concentrated to obtain 8-n (9 mg, 80.35%) as a yellow oil product. The crude product was directly used in the next reaction without purification. LCMS: 194.20 [M+H]⁺.

### 12) (R)-2-(6-chloro-1,3-dihydro-2H-pyrrolo[3',4':4,5]imidazo[1,2-a]pyridin-2-yl)-4-((1-(hydroxymethyl)cyclobutyl)amino)-6,7-dihydrothieno[3,2-d]pyrimidine 5-oxide (8)

At room temperature, to the compounds 8-n (9 mg, 0.05 mmol, 1 eq) and I01 (13.38 mg, 0.05 mmol, 1 eq) in THF (4 mL) and H₂O (1 mL), DIEPA (20 mg, 0.15 mmol, 3 eq) was added. After stirring at 65°C for 3 hours, the resulting material was cooled to room temperature and concentrated. The resulting material was purified by Prep-HPLC (0.1% TFA in ACN/H₂O) to obtain 8 (10.9 mg, 52.6%) as a white solid product. LCMS: 445.15 [M+H]⁺. ¹H NMR (400 MHz, DMSO- d6) δ 8.61 (dd, J = 48.6, 7.2 Hz, 1H), 7.83 (dd, J = 11.5, 2.2 Hz, 1H), 7.15 (ddd, J = 7.1, 5.1, 2.2 Hz, 1H), 4.89 (s, 2H), 4.71 (d, J = 20.0 Hz, 2H), 3.80 (s, 2H), 3.49 (m, J = 17.7, 16.4, 8.3 Hz, 2H), 3.28 - 2.88 (m, 4H), 2.36 - 2.20 (m, 4H), 1.78 (dd, J = 18.9, 10.0 Hz, 2H).

### Example 5: Synthesis of compound 9

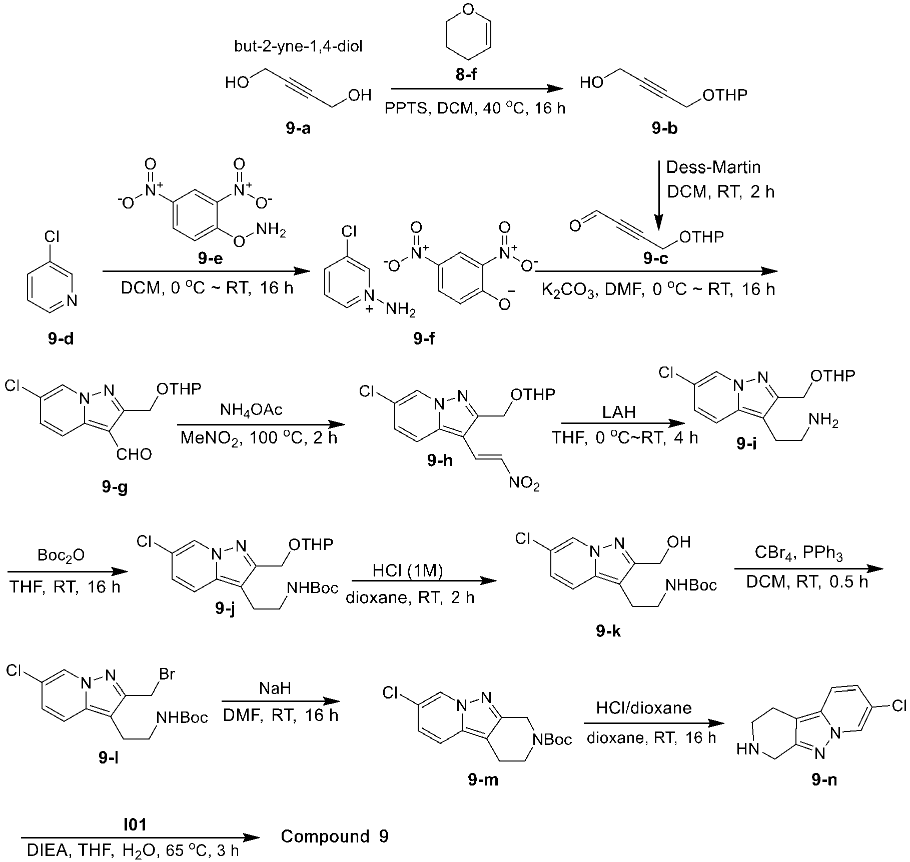

### 1) 4-(Tetrahydro-2H-pyran-2-yl)oxy)2-butyn-1-ol (9-b)

At room temperature, to a solution of 2-butyne-1,4-diol (9-a, 29 g, 336.86 mmol, 1 eq) in DCM (300 mL), 3.4dihydro-2H-pyran (8-f, 28.4 g, 336.86 mmol, 1 eq) and PPTS (8.5 g, 33.69 mmol, 0.1 eq) were added. After stirring at 40°C for 16 hours, water (300 mL) was added. After extraction with ethyl acetate (30 mL x 3), the resulting material was dried over anhydrous Na₂SO₄, filtered, concentrated, and purified by column chromatography (PE: EtOAc = 2: 1) to obtain 9-b (26 g, 45.4%) as a yellow oil product. ¹H NMR (400 MHz, DMSO-d₆) δ 5.16 (t, J = 5.4 Hz, 1H), 4.70 (d, J = 3.7 Hz, 1H), 4.25 - 4.10 (m, 2H), 4.06 (d, J = 4.9 Hz, 2H), 3.67 (ddd, J = 11.5, 8.8, 3.1 Hz, 1H), 3.41 (dd, J = 10.6, 5.3 Hz, 1H), 1.68 - 1.56 (m, 2H), 1.48 - 1.38 (m, 4H).

### 2) 4-(Tetrahydro-2H-pyran-2-yl)oxy)-2-butynal (9-c)

At 0°C, to a solution of 9-b (26 g, 152.75 mmol, 1 eq) in DCM (300 mL), Dess-Martin (97.00 g, 229.13 mmol, 1.5 eq) was added. After stirring at room temperature for 2 hours, water (300 mL) was added. After extraction with dichloromethane (300 mL x 3), the resulting material was dried over anhydrous Na₂SO₄, filtered, concentrated, and purified by column chromatography (PE: EtOAc = 10: 1) to obtain 9-c (21.9 g, 85.2%) as a yellow oil product. ¹H NMR (400 MHz, DMSO- d6) δ 9.21 (s, 1H), 4.72 (s, 1H), 4.53 - 4.40 (m, 2H), 3.74 - 3.65 (m, 1H), 3.44 (d, J = 11.6 Hz, 1H), 1.64 (dd, J = 11.8, 3.8 Hz, 2H), 1.47 (d, J = 10.3 Hz, 4H).

### 3) Amino-3-chloropyridin-1-ium (9-f)

At 0°C, to a solution of 3-chloropyridine (9-d, 10 g, 88.07 mmol, 1 eq) in DCM (300 mL), O-(2,4-dinitrophenyl)hydroxylamine (9-e, 19.30 g, 96.88 mmol, 1.1 eq) was added. After stirring at room temperature for 16 hours, the resulting material was concentrated to obtain 9-f (crude, 22.4 g, 81.4%) as a yellow solid product. ¹H NMR (400 MHz, DMSO- d6) δ 9.02 (s, 1H), 8.70 (d, J = 6.4 Hz, 1H), 8.58 (s, 2H), 8.55 (d, J = 3.2 Hz, 1H), 8.38 (d, J = 8.6 Hz, 1H), 7.98 (dd, J = 8.4, 6.3 Hz, 1H), 7.76 (dd, J = 9.8, 3.2 Hz, 1H), 6.30 (d, J = 9.8 Hz, 1H).

### 4) 6-Chloro-2-(((tetrahydro-2H-pyran-2-yl)oxy)methyl)pyrazolo[1,5-a]pyridine-3-formaldehyde (9-g)

At 0°C, to a solution of 9-f (27.2 g, 86.81 mmol, 1 eq) in DMF (300 mL), 9-c (21.9 g, 130.21 mmol, 1.5 eq) and K₂CO₃ (15.60 g, 112.85 mmol, 1.3 eq) were added successively. After stirring at room temperature for 16 hours, water (300 mL) was added. After extraction with ethyl acetate (300 mL x 3), the resulting material was dried over anhydrous Na₂SO₄, filtered, concentrated, and purified by column chromatography (PE: EtOAc = 10: 1) to obtain 9-g (1.5 g, 5.9%) as a yellow solid product. LCMS: 295.20 [M+H]⁺.

### 5) (E)-6-chloro-3-(2-nitrovinyl)-2-(((tetrahydro-2H-pyran-2-yl)oxy)methyl)pyrazolo[1,5-a]pyridine (9-h)

The compounds 9-g (1.7 g, 5.77 mmol, 1 eq) and NH₄OAc (223.00 mg, 2.88 mmol, 0.5 eq) in MeNO₂ (20 mL) were stirred at 100°C for 2 hours. The resulting material was cooled to room temperature, and a saturated NaHCO₃ solution (20 mL) was added. After extraction with ethyl acetate (30 mL x 3), the resulting material was dried over anhydrous Na₂SO₄, filtered, concentrated, and purified by column chromatography (DCM: EtOAc = 20: 1) to obtain 9-h (1.2 g, 61.6%) as a yellow solid product. LCMS: 338.15 [M+H]⁺.

### 6) 2-(6-Chloro-2-(((tetrahydro-2H-pyran-2-yl)oxy)methyl)pyrazolo[1,5-a]pyridin-3-yl)ethan-1-amine (9-i)

At 0°C, to a solution of 9-h (1.2 g, 3.55 mmol, 1 eq) in THF (20 mL), LAH (5.68 mL, 14.20 mmol, 4 eq) was dropwise added. After stirring at room temperature for 4 hours, a saturated Rochelle's salt solution (30 mL) was added. After extraction with ethyl acetate (30 mL x 3), the resulting material was dried over anhydrous Na₂SO₄, filtered, concentrated, and purified by column chromatography (DCM: EtOAc = 20: 1) to obtain 9-i (900 mg, 81.8%) as a yellow oil product. LCMS: 310.05 [M+H]⁺.

### 7) Tert-butyl (2-(6-chloro-2-(((tetrahydro-2H-pyran-2-yl)oxy)methyl)pyrazolo[1,5-a]pyridin-3-yl)ethyl)aminocarbamate (9-j)

At room temperature, to a solution of 9-i (800 mg, 2.58 mmol, 1 eq) in THF (10 mL), Boc₂O (676.3 mg, 3.10 mmol, 1.2 eq) was added. After stirring for 16 hours, a saturated NaHCO₃ solution (20 mL) was added. After extraction with ethyl acetate (30 mL x 3), the resulting material was dried over anhydrous Na₂SO₄, filtered, concentrated, and purified by column chromatography (PE: EtOAc = 3: 1) to obtain 9-j (270 mg, 25.5%) as a yellow oil product. LCMS: 410.30 [M+H]⁺.

### 8) Tert-butyl (2-(6-chloro-2-(hydroxymethyl)pyrazolo[1,5-a]pyridin-3-yl)ethyl)aminocarbamate (9-k)

At room temperature, to a solution of 9-j (250 mg, 0.61 mmol, 1 eq) in 1,4-dioxane (4 mL), 1 N HCl (2 mL) was added. After continued stirring for 2 hours, 1 N NaOH was added to reach pH 7-8. After extraction with ethyl acetate (10 mL x 3), the resulting material was dried over anhydrous Na₂SO₄, filtered, concentrated, and purified by column chromatography (PE: EtOAc = 1: 1) to obtain 9-k (150 mg, 75.5%) as a yellow solid product. LCMS: 326.25 [M+H]⁺.

### 9) Tert-butyl (2-(2-(bromomethyl)-6-chloropyrazolo[1,5-a]pyridin-3-yl)ethyl)aminocarbamate (9-1)

At room temperature, to a solution of 9k (160 mg, 0.49 mmol, 1 eq) in DCM (4 mL), CBr₄ (245 mg, 0.74 mmol, 1.5 eq) and PPh₃ (168 mg, 0.64 mmol, 1.3 eq) were added successively. After stirring for half an hour, a saturated NaHCO₃ aqueous solution (10 mL) was added. After extraction with ethyl acetate (10 mL x 3), the resulting material was dried over anhydrous Na₂SO₄, filtered, concentrated, and purified by column chromatography (PE: EtOAc = 2: 1) to obtain 9-1 (110 mg, 57.6%) as a white solid product. LCMS: 388.10 [M+H]⁺.

### 10) Tert-butyl7-chloro-3,4-dihydropyrazolo[1,5-a:3,4-c']dipyridine -2(1H)-carboxylate (9-m)

At room temperature, to a solution of 9-l (100 mg, 0.26 mmol, 1 eq) in DMF (15 mL), NaH (16 mg, 0.39 mmol, 1.5 eq) was added. After continued stirring for 16 hours, water (20 mL) was added. After extraction with ethyl acetate (20 mL x 3), the resulting material was dried over anhydrous Na₂SO₄, filtered, concentrated, and purified by Pre-TLC (SiO₂, PE: EtOAc = 4: 1) to obtain 9-m (73 mg, 92.2%) as a white solid product. LCMS: 308.15 [M+H]⁺.

### 11) 7-Chloro-1,2,3,4-tetrahydropyrazolo[1,5-a:3,4-c']dipyridine (9-n)

At room temperature, to a solution of 9-m (70 mg, 0.23 mmol, 1 eq) in *1*,4-dioxane (4 mL), 4 M HCl/1,4-dioxane (4 mL) was dropwise added, and stirring was continued for 16 hours. The resulting material was concentrated to obtain 9-n (40 mg, 84.7%) as a white solid product. The crude product was directly used in the next reaction without purification. LCMS: 208.20 [M+H]⁺.

### 12) (R)-2-(7-Chloro-3,4-dihydropyrazolo[1,5-a:3,4-c']dipyridin-2(1H)-yl)-4-((1-(hydroxymethyl)cyclobutyl)amino)-6,7-dihydrothieno[3,2-d]pyrimidine 5-oxide (9)

At room temperature, to a solution of I01 (50 mg, 0.17 mmol, 1 eq) and 9-n (43.3 mg, 0.21 mmol, 1.2 eq) in THF (4 mL) and H₂O (1 mL), DIEPA (65.91 mg, 0.51 mmol, 3 eq) was added. After stirring at 65°C for 3 hours, the resulting material was concentrated. The resulting material was purified by Pre-TLC (SiO₂, DCM: MeOH = 15: 1) to obtain 9 (62.1 mg, 80.6%) as a white solid product. LCMS: 459.25 [M+H]⁺. ¹H NMR (400 MHz, DMSO- d6) δ 8.89 (d, J = 1.8 Hz, 1H), 7.60 (d, J = 9.4 Hz, 1H), 7.20 (dd, J = 9.5, 1.8 Hz, 1H), 5.01 (s, 2H), 4.06 (t, J = 5.8 Hz, 4H), 3.72 (d, J = 2.7 Hz, 2H), 3.45 (d, J = 8.7 Hz, 1H), 3.24 - 3.18 (m, 1H), 3.03 (d, J = 7.9 Hz, 1H), 2.88 (d, J = 6.4 Hz, 1H), 2.80 (s, 2H), 2.31 (q, J = 10.3 Hz, 2H), 2.21 - 2.16 (m, 2H), 1.76 (t, J = 8.0 Hz, 2H).

The following compounds were synthesized by similar methods.

| Compound | MS (M+H)⁺ | ¹H NMR |
|---|---|---|
| 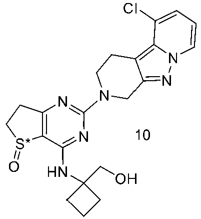 | 459.10 | ¹H NMR (400 MHz, DMSO- d₆) δ 8.57 (d, J = 6.9 Hz, 1H), 7.27 (d, J = 7.4 Hz, 1H), 6.77 (t, J = 7.2 Hz, 1H), 5.02 (s, 2H), 4.36 (s, 2H), 4.06 (d, J = 5.9 Hz, 2H), 3.73 (s, 2H), 3.46 (dd, J = 16.7, 8.2 Hz, 1H), 3.22 (dt, J = 13.6, 8.2 Hz, 1H), 3.03 (dd, J = 26.8, 7.3 Hz, 3H), 2.89 (dd, J = 14.0, 7.1 Hz, 1H), 2.35 - 2.26 (m, 2H), 2.22 - 2.17 (m, 2H), 1.77 (t, J = 8.5 Hz, 2H). |
| 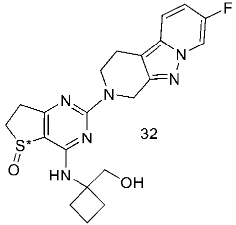 | 443.20 | ¹H NMR (400 MHz, DMSO- d₆) δ 8.88 - 8.84 (m, 1H), 7.63 - 7.59 (m, 1H), 7.25 (s, 1H), 4.99 (s, 2H), 4.06 (s, 2H), 3.72 (s, 2H), 3.49 - 3.36 (m, 2H), 3.18 (d, J = 13.0 Hz, 1H), 3.01 - 2.77 (m, 5H), 2.35 - 2.27 (m, 2H), 2.18 (s, 2H), 1.81 - 1.72 (m, 2H). |

### Example 6: Synthesis of compound 11

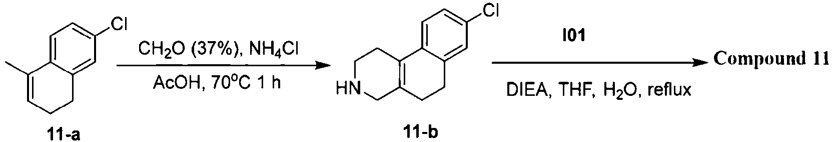

### 1)8-Chloro-1,2,3,4,5,6-hexahydrobenzo[f]isoquinoline (11-b)

At room temperature, to a solution of 7-chloro-4-methyl-1,2-dihydronaphthalene (11-a, 100 mg, 0.56 mmol, 1.0 eq) in acetic acid (4 mL), formaldehyde (100.84 mg, 3.39 mmol, 6.0 eq) was added. After stirring at 70°C for 1 hour, NH₄Cl (89.82 mg, 1.68 mmol, 3.0 equiv.) was added, and stirring was continued at 70°C for 2 hours. The resulting material was cooled to room temperature, and a saturated NaHCO₃ solution (100 mL) was added. After extraction with ethyl acetate (20 mL x 2), the resulting material was dried over anhydrous Na₂SO₄, filtered, concentrated, and purified by column chromatography (PE: EtOAc = 1: 1) to obtain 11-b (40 mg, 32.5%) as a yellow solid product. LCMS: 220.10 [M+H]⁺.

### 2) (R)-2-(8-chloro-1,4,5,6-tetrahydrobenzo[f]isoquinolin-3(2H)-yl)-4-((1-(hydroxymethyl)cyclobutyl)amino)-6,7-dihydrothieno[3,2-d]pyrimidine 5-oxide (11)

At room temperature, to a solution of I01 (60 mg, 0.21 mmol, 1 equiv.) in THF (3 ml) and H₂O (0.6 ml), 11-b (40 mg, 0.19 mmol, 0.9 equiv.) and DIEPA (269.48 mg, 2.09 mmol, 10 equiv.) were added. After stirring at 65°C for 3 hours, the resulting material was concentrated. The resulting material was purified by column chromatography (PE: EtOAc = 1: 1) to obtain 11 (20.7 mg, 20.9%) as a white solid product. LCMS: 471.25 [M+H]⁺. ¹H NMR δ 7.26 - 6.99 (m, 3H), 4.24 (s, 2H), 3.95 (t, J = 5.9 Hz, 2H), 3.71 (s, 2H), 3.45 (s, 1H), 3.28 - 3.16 (m, 1H), 3.02 (d, J = 10.5 Hz, 1H), 2.94 - 2.85 (m, 1H), 2.75 (t, J = 7.9 Hz, 2H), 2.63 (s, 1H), 2.36 - 2.27 (m, 3H), 2.17 (s, 4H), 1.76 (s, 2H).

The following compounds were synthesized by similar methods.

| Compound | MS (M+H)⁺ | NMR |
|---|---|---|
| 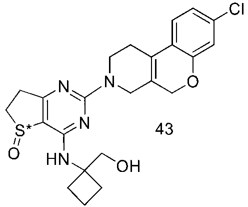 | 473.20 | ¹H NMR (400 MHz, DMSO-d₆) δ 7.42 (s, 1H), 7.10 (d, J = 8.2 Hz, 1H), 6.95 - 6.91 (m, 1H), 6.87 (dd, J = 20.6, 2.1 Hz, 1H), 4.82 (s, 1H), 4.70 (s, 2H), 4.16 (s, 1H), 3.95 (s, 1H), 3.69 (s, 2H), 3.44 - 3.33 (m, 1H), 3.21 - 3.12 (m, 1H), 2.97 - 2.69 (m, 3H), 2.39 - 2.26 (m, 4H), 2.16 (s, 2H), 1.82 - 1.70 (m, 2H). |

### Example 7: Synthesis of compound 12

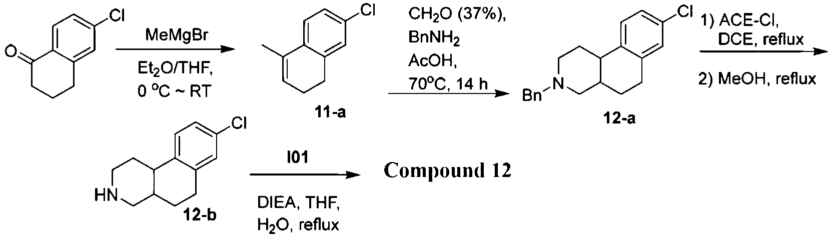

### 1) 7-Chloro-4-methyl-1,2-dihydronaphthalene (11-a)

At 0°C, to a solution of 6-chloro-3,4-dihydronaphthalen-1(2H)-one (5 g, 27.8 mmol, 1.0 eq) in THF (15 mL) and diethyl ether (35 mL), MeMgBr (9.27 mL,3 M, 27.8 mmol, 1.0 eq) was dropwise added. After continued stirring for 2 hours, a saturated NH₄Cl solution (50 mL) was added. After extraction with ethyl acetate (50 mL x 2), the resulting material was dried over anhydrous Na₂SO₄, filtered, concentrated, and purified by column chromatography (PE: EtOAc = 5: 1) to obtain 11-a (2.2 g, 44.5%) as a yellow solid product. LCMS: 179.10 [M+H]⁺.

### 2) 3-Benzyl-8-chloro-1,2,3,4,4,4a,5,6,10b-octahydrobenzo[f]isoquinoline (12-a)

At room temperature, to a solution of 11-a (1.0 g, 5.60 mmol, 1.0 equiv.) in acetic acid (40 ml), formaldehyde (3.06 g, 33.58 mmol, 6.0 equiv.) was added. The resulting material was heated to 70°C and stirred for 2 hours. BnNH₂ (3 g, 5.0 equiv.) was then added. After continued stirring at 70°C for 12 hours, the resulting material was cooled to room temperature, and a saturated NaHCO₃ solution (100 mL) was added. After extraction with ethyl acetate (100 mL x 2), the resulting material was dried over anhydrous Na₂SO₄, filtered, concentrated, and purified by column chromatography (PE: EtOAc = 1: 1) to obtain 12-a (200 mg, 11.5%) as a yellow solid product. LCMS: 312.15 [M+H]⁺.

### 3) 8-Chloro-1,2,3,4,4a,5,6,10b-octahydrobenzo[f]isoquinoline (12-b)

At 0°C, to a solution of 12-a (120 mg, 0.38 mmol, 1.0 equiv.) in 1,2-DCE (2 ml), chloroethyl 1-chloroformate (110.03 mg, 0.77 mmol, 2.0 equiv.) was dropwise added. After stirring at room temperature for 1 hour, the resulting material was concentrated to dryness, and MeOH (10 ml) was then added. After stirring at 65°C for 12 hours, the resulting material was cooled to room temperature, and a saturated NaHCO₃ solution (20 mL) was added. After extraction with ethyl acetate (20 mL x 2), the resulting material was dried over anhydrous Na₂SO₄, filtered, concentrated, and purified by column chromatography (PE: EtOAc = 1: 1) to obtain 12-b (60 mg, 70.3%) as a yellow solid product. LCMS: 222.10 [M+H]⁺.

### 4) (R)-2-((4aS,10bS)-8-chloro-1,4,4a,5,6,10-hexahydrobenzo[f]isoquinolin-3(2H)-yl)-4-((1-(hydroxymethyl)cyclobutyl)amino)-6,7-dihydrothieno[3,2-d]pyrimidine 5-oxide (12)

At room temperature, to a solution of I01 (60 mg, 0.21 mmol, 1 equiv.) in THF (3 ml) and H₂O (0.6 ml), 12-b (46.23 mg, 0.21 mmol, 1.0 equiv.) and DIEPA (269.48 mg, 2.09 mmol, 10 equiv.) were added. After stirring at 65°C for 12 hours, the resulting material was concentrated. The resulting material was purified by column chromatography (PE: EtOAc = 1: 1) to obtain 12 (60 mg, 60.8%) as a white solid product. LCMS: 473.10 [M+H]⁺. ¹H NMR (400 MHz, DMSO-d6) δ 7.63 (s, 1H), 7.20 (q, J = 19.7, 16.5 Hz, 3H), 5.89 (s, 1H), 3.66 (d, J = 19.0 Hz, 4H), 3.41 - 3.33 (m, 1H), 3.31 (s, 1H), 3.16 (s, 1H), 3.02 (s, 3H), 2.82 (dd, J = 13.8, 7.4 Hz, 2H), 2.68 - 2.54 (m, 4H), 2.34 - 2.23 (m, 2H), 2.12 (s, 4H), 1.72 (s, 2H).

### Example 8: Synthesis of compounds 13 and 14

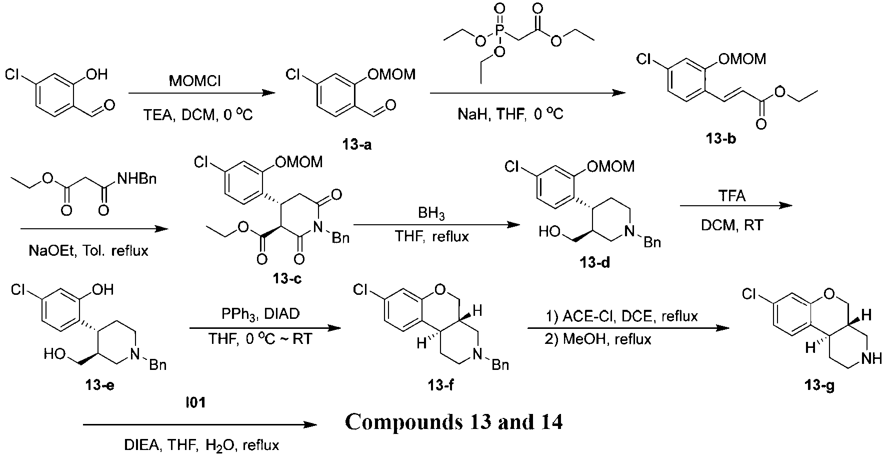

### 1) 4-Chloro-2-(methoxymethoxy)benzaldehyde (13-a)

At 0°C, to a solution of 4-chloro-2-hydroxybenzaldehyde (10 g, 63.87 mmol, 1.0 equiv.) in DCM (100 ml), chloro-(methoxy)methane (5.14 g, 63.87 mmol, 1.0 equiv.) and triethylamine (32.31 g, 319.35 mmol, 5.0 equiv.) were dropwise added successively. After stirring at room temperature for 1 hour, H₂O (150 mL) was added. After extraction with dichloromethane (300 mL x 2), the resulting material was dried over anhydrous Na₂SO₄, filtered, concentrated, and purified by column chromatography (PE: EtOAc = 10: 1) to obtain 13-a (7.02 g, 54.6%) as a yellow oil product. LCMS: 201.05 [M+H]⁺.

### 2) Ethyl (E)-3-(4-chloro-2-(methoxymethoxy)phenyl)acrylate (13-b)

At 0°C, to a solution of triethyl phosphonoacetate (7.02 g, 34.89 mmol, 1.0 equiv.) in THF (100 ml), NaH (837.40 mg, 34.89 mmol, 1 equiv.) was added in portions. After continued stirring for 30 minutes, a solution of 13-a (7.82 g, 34.89 mmol, 1.0 equiv.) in THF (15 mL) was dropwise added. After stirring at 0°C for 1 hour, a saturated NH₄Cl solution (100 ml) was added. After extraction with ethyl acetate (200 mL x 3), the resulting material was dried over anhydrous Na₂SO₄, filtered, concentrated, and purified by column chromatography (PE: EtOAc = 10: 1) to obtain 13-b (8 g, 84.7%) as a yellow oil product. LCMS: 271.20 [M+H]⁺.

### 3) Ethyl (3S,4R)-1-benzyl-4-(4-chloro-2-(methoxymethoxy)phenyl)-2,6-dioxopiperidine-3-carboxylate (13-c)

At room temperature, to a solution of 13-b (1 g, 3.69 mmol, 1.0 equiv.) and ethyl 3-(benzylamino)-3-oxopropanoate (817.31 mg, 3.69 mmol, 1.0 eq.) in THF (30 ml), NaH (177.30 mg, 7.39 mmol, 2.0 equiv.) was added. After stirring at 70°C for 1 hour, the resulting material was cooled to room temperature, and a saturated NH₄Cl solution (30 mL) was added. The resulting material was extracted with ethyl acetate (35 mL x 3), dried over anhydrous Na₂SO₄, filtered, concentrated, and purified by column chromatography (PE: EtOAc = 1: 1) to obtain 13-c (600 mg, 36.4%) as a yellow solid product. LCMS: 446.25 [M+H]⁺.

### 4) ((3S,4R)-1-benzyl-4-(4-chloro-2-(methoxymethoxy)phenyl)piperidin-3-yl)methanol (13-d)

At room temperature, to a solution of 13-c (470 mg, 1.05 mmol, 1.0 eq.) in THF (20 ml), BH₃ (10.54 ml, 10.54 mmol, 10 equiv.) was dropwise added. After stirring at 70°C for 3 hours, the resulting material was cooled to 0°C, and MeOH (30 mL) was dropwise added and then stirred at 60°C for 3 hours. After cooling to room temperature, EtOAc (100 mL) was added, and the resulting material was then washed with a saturated brine (35 mL). The resulting material was dried over anhydrous Na₂SO₄, filtered, concentrated, and purified by column chromatography (PE: EtOAc = 1: 1) to obtain 13-d (220 mg, , 55.5%) as a yellow solid product. LCMS: 376.25 [M+H]⁺.

### 5) 2-((3S,4R)-1-benzyl-3-(hydroxymethyl)piperidin-4-yl)-5-chlorophenol (13-e)

At room temperature, to a solution of 13-d (220 mg, 0.59 mmol, 1.0 equiv.) in DCM (3 ml), TFA (1 ml, 8.77 mmol, 14.98 equiv.) was added. After stirring for 3 hours, the resulting material was concentrated. EtOAc (30 mL) and saturated NaHCO₃ (30 mL) were added. The resulting material was extracted with ethyl acetate (30 mL x 3), dried over anhydrous Na₂SO₄, filtered, concentrated, and purified by column chromatography (PE: EtOAc = 1: 1) to obtain 13-e (190 mg, 97.8%) as a yellow solid product. LCMS: 332.20 [M+H]⁺.

### 6) (4aS,10bR)-3-benzyl-8-chloro-1,3,4,4a,5,10b-hexahydro-2H-chromeno[3,4-c]pyridine (13-f)

At 0°C, to a solution of 13-e (210 mg, 0.63 mmol, 1.0 equiv.) in THF (10 ml), PPh₃ (829.93 mg, 3.16 mmol, 5.0 eq.) and DIAD (639.83 mg, 3.16 mmol, 5.0 equiv.) were added successively. After stirring at room temperature for 16 hours, the resulting material was concentrated. EtOAc (30 mL) was added. The resulting material was washed with water (30 mL x 2) and brine (30 mL) successively. The resulting material was dried over anhydrous Na₂SO₄, filtered, concentrated, and purified by column chromatography (PE: EtOAc = 3: 1) to obtain 13-f (80 mg, 40.3%) as a yellow solid product. LCMS: 314.20 [M+H]⁺.

### 7) (4aS,10bR)-8-chloro-1,3,4,4a,5,10b-hexahydro-2H-chromeno[3,4-c]pyridine (13-g)

At 0°C, to a solution of 13-f (80 mg, 0.25 mmol, 1.0 equiv.) in 1,2-DCE (3 ml), 1-chloroethyl chloroformate (72.89 mg, 0.51 mmol, 2.0 equiv.) was dropwise added. After stirring at room temperature for 1 hour, the resulting material was concentrated, and MeOH (10 ml) was added and stirred overnight. A saturated NaHCO₃ solution (10 mL) was added. The resulting material was extracted with ethyl acetate (20 mL x 3), dried over anhydrous Na₂SO₄, filtered, concentrated, and purified by column chromatography (PE: EtOAc = 1: 1) to obtain 13-g (30 mg, 52.6%) as a yellow solid product. LCMS: 224.20 [M+H]⁺.

### 8) (R)-2-((4aS,10bR)-8-chloro-1,4a,5,10b-tetrahydro-2H-chromeno[3,4-c]pyridin-3(4H)-yl)-4-((1-(hydroxymethyl)cyclobutyl)amino)-6,7-dihydrothieno[3,2-d]pyrimidine 5-oxide (13) and (R)-2-((4aR,10bS)-8-chloro-1,4a,5,10b-tetrahydro-2H-chromeno[3,4-c]pyridin-3(4H)-yl)-4-((1-(hydroxymethyl)cyclobutyl)amino)-6,7-dihydrothieno[3,2-d]pyrimidine 5-oxide (14)

At room temperature, to a solution of I01 (30 mg, 0.10 mmol, 1.0 equiv.) and 13-g (30 mg, 0.13 mmol, 1.3 equiv.) in THF (3 ml) and H₂O (0.6 ml), DIEPA (134.74 mg, 1.04 mmol, 10 equiv.) was added. After stirring at 65°C for 12 hours, the resulting material was concentrated. The resulting material was purified by column chromatography (PE: EtOAc = 1: 1) and further purified by SFC to obtain 13 (17.2 mg, 36.0%) and 14 (15.4 mg, 32.0%) as a white solid product. LCMS: 475.10 [M+H]⁺.

13: ¹H NMR (400 MHz, DMSO-d₆) δ 7.37 (s, 1H), 7.18 (d, J = 8.3 Hz, 1H), 6.86 (dd, J = 8.2, 2.2 Hz, 1H), 6.78 (d, J = 2.2 Hz, 1H), 4.84 (s, 1H), 4.74 (s, 1H), 4.26 (s, 1H), 3.88 (t, J = 11.1 Hz, 1H), 3.68 (d, J = 4.5 Hz, 2H), 3.44 - 3.33 (m, 1H), 3.31 (s, 1H), 3.22 - 3.11 (m, 1H), 3.00 - 2.78 (m, 3H), 2.70 (s, 1H), 2.62 (d, J = 11.2 Hz, 1H), 2.39 - 2.23 (m, 4H), 2.15 (s, 2H), 1.82 - 1.68 (m, 2H), 1.57 (s, 1H).

14: ¹H NMR (400 MHz, DMSO-d₆) δ 7.37 (s, 1H), 7.18 (d, J = 8.3 Hz, 1H), 6.86 (dd, J = 8.3, 2.2 Hz, 1H), 6.78 (d, J = 2.2 Hz, 1H), 4.82 (t, J = 5.5 Hz, 1H), 4.73 (s, 1H), 4.25 (d, J = 10.1 Hz, 1H), 3.88 (t, J = 11.0 Hz, 1H), 3.68 (s, 2H), 3.44 - 3.32 (m, 1H), 3.31 (s, 1H), 3.22 - 3.10 (m, 1H), 3.00 - 2.78 (m, 3H), 2.70 (t, J = 11.4 Hz, 1H), 2.65 - 2.56 (m, 1H), 2.40 - 2.21 (m, 4H), 2.15 (s, 2H), 1.82 - 1.68 (m, 2H), 1.63 - 1.49 (m, 1H).

### Example 9: Synthesis of compound 18:

### (R)-2-(6-chloro-3,4-dihydroisoquinolin-2(1H)-yl)-4-((1-(hydroxymethyl)cyclobutyl)amino)-6,7-dihydrothieno[3,2-d]pyrimidine 5-oxide

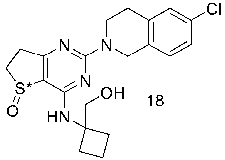

At room temperature, to a solution of I01 (40 mg, 0.14 mmol, 1 eq) and 6-chloro-1,2,3,4-tetrahydroisoquinoline (18-a, 28 mg, 0.17 mmol, 1.2 eq) in THF (4 mL) and H₂O (1 mL), DIEPA (65.91 mg, 0.51 mmol, 3 eq) was added. After stirring at 65°C for 3 hours, the resulting material was concentrated. The resulting material was purified by Pre-TLC (SiO₂, DCM: MeOH = 15: 1) to obtain 18 (50.4 mg, 86.8%) as a white solid product. LCMS: 419.30 [M+H]⁺. ¹H NMR (400 MHz, DMSO- d₆) δ 7.26 (s, 1H), 7.23 (d, J = 1.3 Hz, 2H), 4.81 (s, 2H), 3.89 (d, J = 5.9 Hz, 2H), 3.71 (d, J = 1.8 Hz, 2H), 3.50 - 3.43 (m, 1H), 3.26 - 3.19 (m, 1H), 3.05 - 2.99 (m, 1H), 2.92 - 2.83 (m, 3H), 2.38 - 2.25 (m, 2H), 2.20 (s, 2H), 1.81 - 1.71 (m, 2H).

The following compounds were synthesized by similar methods.

| Compound | MS (M+H)⁺ | NMR |
|---|---|---|
| 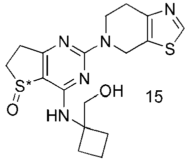 | 392.20 | ¹H NMR (400 MHz, DMSO-d₆) δ 8.94 (s, 1H), 4.98 (s, 2H), 4.04 (d, J = 6.1 Hz, 3H), 3.43 (dd, J = 17.0, 8.3 Hz, 1H), 3.21 (dt, J = 15.7, 8.2 Hz, 1H), 3.18 (m, 1H), 2.98-2.32(m, 7H), 2.28-2.05 (m, 5H), 1.77 - 1.73 (m, 2H). |
| 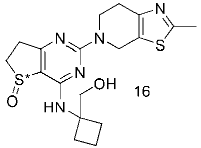 | 406.25 | ¹H NMR (400 MHz, DMSO-d₆) δ4.89 (s, 3H), 4.01 (d, J = 6.1 Hz, 3H), 3.69 (s, 3H), 3.45 (dd, J = 17.2, 8.4 Hz, 1H), 3.22 (m, 1H), 3.01 (dd, J = 18.0, 8.1 Hz, 1H), 2.89 (dd, J = 13.8, 7.2 Hz, 1H), 2.47 (d, J = 11.0 Hz, 3H), 2.29 (s, 3H), 1.79 - 1.70 (m, 3H). |
| 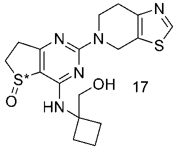 | 392.00 | ¹H NMR (400 MHz, MeOD-d₄) δ 8.15 (s, 1H), 7.46 (s, 1H), 3.95 (m, 4H), 3.77 (m, 1H), 3.59 (m, 3H), 2.46 (m, 3H), 1.97 (m, 4H), 1.21 (m, 3H). |
| 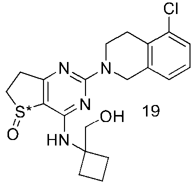 | 419.05 | ¹H NMR (400 MHz, Methanol-d4) δ 7.25 - 7.11 (m, 3H), 4.89 (s, 2H), 4.08 (t, J = 6.0 Hz, 2H), 4.01 - 3.87 (m, 2H), 3.63 - 3.50 (m, 1H), 3.41 - 3.30 (m, 1H), 3.13 - 2.99 (m, 2H), 2.89 (t, J = 6.0 Hz, 2H), 2.33 (m, 4H), 1.98 - 1.83 (m, 2H), 1.35 (m, 2H). |
| 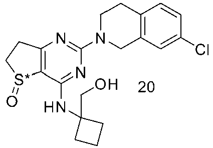 | 419.05 | ¹H NMR (399 MHz, DMSO-d₆) δ 7.41 (s, 1H), 7.32 (s, 1H), 7.23 - 7.13 (m, 1H), 5.30 (t, J = 4.8 Hz, 1H), 4.84 (s, 1H), 3.92 (s, 2H), 3.71 (d, J = 1.7 Hz, 2H), 3.40 (dt, J = 16.2, 7.8 Hz, 1H), 3.18 (dt, J = 13.7, 8.4 Hz, 1H), 2.98 - 2.80 (m, 1H), 2.78 (t, J = 5.9 Hz, 2H), 2.19 (s, 1H), 1.96 (p, J = 6.9, 6.3 Hz, 2H), 1.78 (s, 3H), 1.43 (d, J = 8.2 Hz, 2H), 1.21 (d, J = 3.2 Hz, 1H). |
| 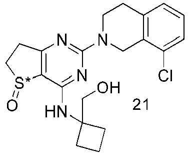 | 419.10 | ¹H NMR (400 MHz, Methanol-d₄) δ 7.29 (d, J = 7.6 Hz, 1H), 7.20 (dd, J = 14.0, 6.5 Hz, 2H), 4.95 (s, 2H), 3.98 (d, J = 3.3 Hz, 3H), 3.79 - 3.66 (m, 1H), 3.45 (dt, J = 15.3, 8.1 Hz, 1H), 3.17 (dd, J = 14.1, 7.0 Hz, 2H), 2.40 (d, J = 6.4 Hz, 4H), 2.01 (s, 1H), 1.96 - 1.87 (m, 1H), 1.29 (d, J = 15.3 Hz, 3H). |
| 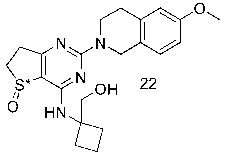 | 415.15 | ¹H NMR (400 MHz, Methanol-d4) δ 7.12 (d, J = 8.9 Hz, 1H), 6.81 (s, 2H), 4.80 (s, 3H), 4.02 - 3.90 (m, 4H), 3.72 (m, 5H), 3.75 (m, 1H), 3.47 (m, 1H), 3.18 (ddd, J = 14.0, 7.4, 1.7 Hz, 1H), 2.96 (s, 2H), 2.45 - 2.34 (m, 4H), 1.97 - 1.87 (m, 2H). |
| 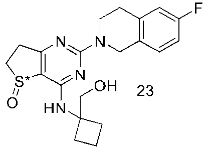 | 403.15 | ¹H NMR (400 MHz, Methanol-d4) δ 7.16 (d, J = 12.4 Hz, 1H), 6.97 (d, J = 8.1 Hz, 2H), 4.03 - 3.83 (m, 4H), 3.90 (d, J = 11.5 Hz, 1H), 3.56 (dt, J = 16.7, 8.1 Hz, 1H), 3.35 (dt, J = 13.8, 8.4 Hz, 2H), 3.12 - 3.00 (m, 2H), 2.87 (t, J = 5.9 Hz, 2H), 2.34 (m, 3H), 2.01-1.97 (m, 2H), 1.91 (m, 4H). |
| 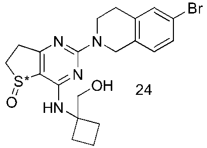 | 464.90 | ¹H NMR (400 MHz, Methanol-d4) δ 7.16 (dd, J = 9.5, 5.5 Hz, 1H), 6.90 (d, J = 8.6 Hz, 2H), 4.03 - 3.87 (m, 3H), 3.56 (dt, J = 16.8, 8.0 Hz, 1H), 3.35 (dt, J = 13.8, 8.4 Hz, 1H), 3.13 - 2.99 (m, 3H), 2.88 (m, 3H), 2.32 (m, 3H), 1.91 (m, 3H), 1.34 (m, 3H). |
| 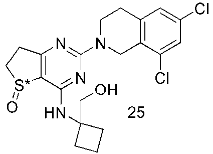 | 453.05 | ¹H NMR (400 MHz, DMSO-d6) δ 7.45 (s, 1H), 7.23 (s, 1H), 4.19 (s, 2H), 4.03 (s, 1H), 3.44 (m, 4H), 3.07 (dd, J = 17.3, 8.0 Hz, 2H), 2.94 (dd, J = 13.6, 7.1 Hz, 2H), 2.42 (dd, J = 21.4, 10.4 Hz, 4H), 1.95 (d, J = 11.2 Hz, 3H), 1.30-1.25 (m, 1H). |
| 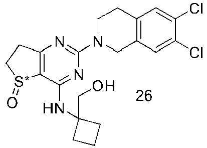 | 453.00 | ¹H NMR (400 MHz, DMSO-d₆) δ 7.55 (s, 1H), 7.44 (s, 1H), 5.28 (d, J = 4.6 Hz, 1H), 4.82 (s, 1H), 3.90 (s, 1H), 3.70 (s, 2H), 3.18 (d, J = 13.4 Hz, 1H), 2.80 (s, 1H), 2.79 (s, 2H), 2.64 (t, J = 2.0 Hz, 1H), 2.32 - 2.25 (m, 2H), 2.19 (s, 2H), 1.96 (q, J = 7.0, 6.5 Hz, 4H), 1.76 (d, J = 9.6 Hz, 1H), 1.42 (s, 1H). |
| 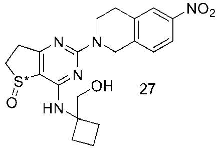 | 430.10 | ¹H NMR (400 MHz, Methanol-d₄) δ 8.13 - 8.06 (m, 2H), 7.45 (d, J = 8.4 Hz, 1H), 5.01 (s, 2H), 4.08 - 3.98 (m, 2H), 3.98 - 3.91 (m, 2H), 3.70 (dt, J = 17.6, 7.7 Hz, 1H), 3.44 (dt, J = 14.0, 8.2 Hz, 1H), 3.17 - 3.10 (m, 1H), 2.39 (td, J = 9.0, 5.0 Hz, 4H), 1.93 (q, J = 9.6, 9.0 Hz, 2H), 1.37 - 1.19 (m, 3H). |
| | | ¹⁹F NMR (376 MHz, Methanol-d₄) δ -77.37. |
| 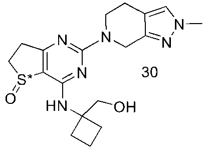 | 389.20 | ¹H NMR (399 MHz, DMSO-d6) δ 7.46 (s, 1H), 4.76 (s, 2H), 3.98 (s, 2H), 3.71 (d, J = 6.3 Hz, 3H), 3.20 - 3.15 (m, 1H), 3.32 (m, 1H),2.97 - 2.79 (m, 1H), 2.63 (d, J = 15.1 Hz, 3H), 2.34 (m, 2H), 2.17 (m, 2H), 2.07 (s, 3H), 1.80 - 1.71 (m, 2H). |
| 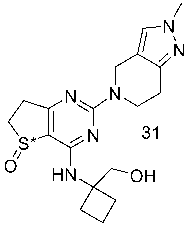 | 389.10 | ¹H NMR (400 MHz, Methanol-d4) δ 7.30 (s, 1H), 4.91 (s, 2H), 4.06 - 3.77 (m, 5H), 3.69 (dt, J = 16.7, 7.8 Hz, 3H), 3.50 - 3.37 (m, 1H), 3.31 - 3.20 (m, 1H), 3.28 - 3.09 (m, 2H), 2.68 (s, 2H), 2.35 (td, J = 8.7, 7.9, 4.7 Hz, 4H), 1.92 (q, J = 8.8 Hz, 2H). |
| 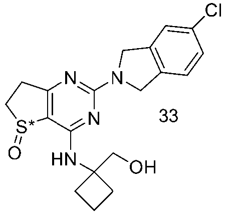 | 405.05 | ¹H NMR (400 MHz, DMSO-d₆) δ 7.50 (d, J = 13.2 Hz, 1H), 7.42 (dd, J = 15.1, 8.2 Hz, 1H), 7.35 (dd, J = 8.2, 2.0 Hz, 1H), 4.78 (t, J = 9.1 Hz, 4H), 3.76 (d, J = 2.5 Hz, 2H), 3.51 (d, J = 8.6 Hz, 1H), 3.27 (dt, J = 15.2, 8.6 Hz, 1H), 3.09 (dd, J = 17.5, 8.1 Hz, 1H), 2.94 (dd, J = 13.8, 7.2 Hz, 1H), 2.67 - 2.52 (m, 1H), 2.37 - 2.19 (m, 5H), 1.83 - 1.70 (m, 2H). |
| 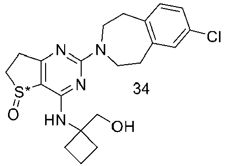 | 433.15 | ¹H NMR (400 MHz, DMSO-d₆) δ 7.25 (dd, J = 7.5, 1.9 Hz, 1H), 7.17 - 7.03 (m, 2H), 3.94 (s, 1H), 3.81 (m, 3H), 3.69 (s, 1H), 3.39 (dt, J = 16.3, 7.8 Hz, 1H), 3.32 (s, 2H), 3.17 (dt, J = 13.6, 8.3 Hz, 1H), 3.08 (s, 2H), 2.98 - 2.77 (m, 4H), 2.30 (m, 3H), 2.16 (s, 2H), 1.76 (q, J = 8.8 Hz, 2H). |
| 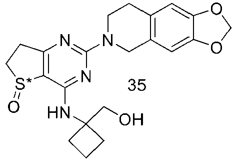 | 429.15 | ¹H NMR (400 MHz, DMSO-d₆) δ 6.75 (d, 2H), 5.92 (s, 2H), 4.71 (s, 2H), 3.85 (t, 2H), 3.71 (m, 2H), 3.23 (m, 1H), 3.03 (m, 1H), 2.90 (m, 1H), 2.73 (s, 2H), 2.29 (m, 3H), 2.18 (m, 2H), 2.05 (s, 1H), 1.76 (m, 2H). |
| 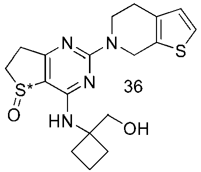 | 391.20 | ¹H NMR (400 MHz, DMSO-d₆) δ 7.33 (d, J = 5.0 Hz, 1H), 6.83 (d, J = 5.0 Hz, 1H), 4.92 (m, 2H), 3.97 (m, 3H), 3.70 (m, 2H), 3.50 - 3.40 (m, 1H), 3.20 (d, J = 13.8 Hz, 1H), 2.99 (d, J = 8.9 Hz, 1H), 2.91 - 2.84 (m, 1H), 2.66 (m, J = 18.1 Hz, 2H), 2.30 (m, 2H), 2.19 (m, 2H), 1.76 (m, J = 9.8 Hz, 2H). |
| 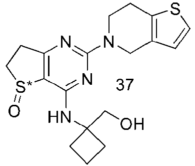 | 391.05 | ¹H NMR (400 MHz, DMSO-d₆) δ 7.28 (d, J = 5.1 Hz, 1H), 6.91 (d, J = 5.2 Hz, 1H), 4.77 (s, 2H), 4.02 (s, 3H), 3.30 (s, 2H), 2.80 - 2.48 (m, 4H), 2.47 (s, 1H), 2.46 (d, J = 11.1 Hz, 1H), 2.16 (s, 2H), 2.05 (s, 2H), 1.75 (s, 2H). |
| 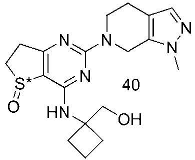 | 389.15 | ¹H NMR (400 MHz, DMSO-d₆) δ 7.21 (s, 1H), 4.84 (s, 2H), 3.71 (d, J = 7.4 Hz, 5H), 3.52 - 3.41 (m, 1H), 3.29 - 3.18 (m, 1H), 3.06 - 2.97 (m, 1H), 2.94 - 2.86 (m, 1H), 2.53 (s, 4H), 2.41 - 2.25 (m, 2H), 2.24 - 2.13 (m, 2H), 1.86 - 1.70 (m, 2H). |
| 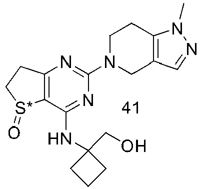 | 389.15 | ¹H NMR (400 MHz, DMSO-d₆) δ 7.42 (s, 1H), 7.22 (s, 1H), 5.73 (s, 1H), 4.68 (m, 1H), 3.98 (m, 1H), 3.69 (s, 1H), 3.62 (s, 3H), 3.30 (m, 1H), 3.10 (m, 2H), 2.66 (m, 2H), 2.40 (m, 1H), 2.16 (m, 1H), 1.76 (d, J = 8.6 Hz, 1H), 1.22 (q, J = 6.6 Hz, 7H). |
| 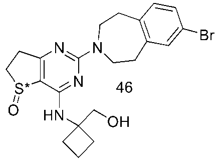 | 479.10 | ¹H NMR (400 MHz, DMSO-d₆) δ 7.38-7.37 (d, J = 2.2 Hz, 1H), 7.29-7.26 (dd, J = 8.0, 2.2 Hz, 1H), 7.12-7.10 (d, J = 8.1 Hz, 1H), 3.91 (m, 3H), 3.71 (m, 4H), 3.44 (m, 1H), 3.22 (m, 1H), 3.04 (m, 1H), 2.85 (m, 4H), 2.30 (dt, J = 19.8, 10.6 Hz, 4H), 2.17 (m, 2H), 1.76 (m, 2H). |
| 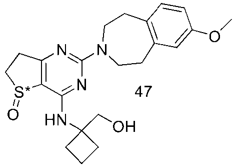 | 429.25 | ¹H NMR (399 MHz, DMSO-d₆) δ 7.04 (d, J = 8.3 Hz, 1H), 6.74 (d, J = 2.7 Hz, 1H), 6.63 (dd, J = 8.2, 2.7 Hz, 1H), 3.89 (s, 2H), 3.80 - 3.69 (m, 6H), 3.59 - 3.43 (m, 2H), 3.29 - 3.16 (m, 1H), 3.06 (dd, J = 16.7, 9.1 Hz, 1H), 2.91 (dd, J = 13.5, 7.0 Hz, 1H), 2.80 (s, 4H), 2.29 (p, J = 10.2, 9.5 Hz, 4H), 2.17 (s, 2H), 1.76 (q, J = 9.1 Hz, 2H). |
| 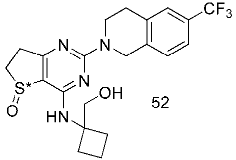 | 453.25 | ¹H NMR (400 MHz, DMSO-d₆) δ 7.57 - 7.49 (m, 2H), 7.43 (d, J = 8.0 Hz, 1H), 4.92 (s, 2H), 3.94 (t, J = 5.9 Hz, 3H), 3.77 - 3.68 (m, 3H), 3.52 - 3.39 (m, 1H), 3.27 - 3.13 (m, 1H), 3.06 - 2.95 (m, 1H), 2.94 - 2.83 (m, 3H), 2.37 - 2.14 (m, 4H), 1.82 - 1.70 (m, 2H). |
| 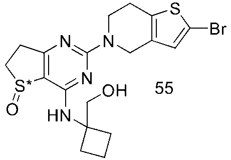 | 471.05 | ¹H NMR (400 MHz, DMSO-d₆) δ 7.06 (s, 1H), 4.71 (s, 2H), 4.00 (t, J = 6.0 Hz, 2H), 3.74 - 3.65 (m, 2H), 3.48 - 3.39 (m, 1H), 3.24 - 3.15 (m, 1H), 2.97 (dd, J = 17.4, 8.0 Hz, 1H), 2.87 (dd, J = 13.8, 7.1 Hz, 1H), 2.74 (s, 2H), 2.32 - 2.20 (m, 2H), 2.21 - 2.13 (m, 2H), 1.75 (q, J = 8.6 Hz, 2H). |
| 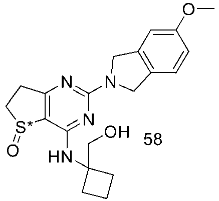 | 401.05 | ¹H NMR (400 MHz, DMSO-d₆) δ 7.30-7.24 (dd, J = 16.9, 8.4 Hz, 1H), 7.00-6.94 (d, 1H), 6.85 (d, J = 8.6 Hz, 1H), 4.75 - 4.69 (m, 5H), 3.76 - 3.73 (m, 4H), 3.08 - 2.88 (m, 4H), 2.38 - 2.22 (m, 5H), 1.78 (m, 3H). |
| 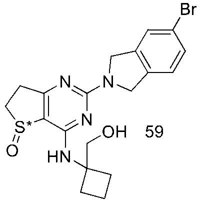 | 450.95 | ¹H NMR (400 MHz, DMSO-d₆) δ 7.63 (d, J = 15.5 Hz, 1H), 7.51 - 7.44 (m, 1H), 7.40 - 7.29 (m, 1H), 4.80 - 4.72 (m, 4H), 3.76 (s, 2H), 3.53 - 3.45 (m, 1H), 3.29 - 3.18 (m, 1H), 3.11 - 2.99 (m, 1H), 2.96 - 2.86 (m, 1H), 2.38 - 2.27 (m, 2H), 2.26 - 2.19 (m, 2H), 1.85 - 1.68 (m, 2H). |
| 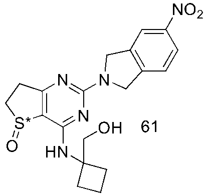 | 416.10 | ¹H NMR (400 MHz, DMSO-d₆) δ 8.30 (d, J = 19.5 Hz, 1H), 8.17 (d, J = 8.4 Hz, 1H), 7.66 (dd, J = 19.2, 8.4 Hz, 1H), 4.91 - 4.85 (m, 4H), 3.77 (s, 2H), 3.54 - 3.43 (m, 1H), 3.30 - 3.19 (m, 1H), 3.09 - 2.99 (m, 1H), 2.96 - 2.87 (m, 1H), 2.39 - 2.28 (m, 2H), 2.26 - 2.18 (m, 2H), 1.77 (d, J = 9.3 Hz, 2H). |
| 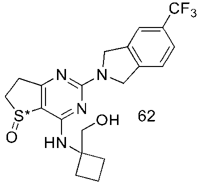 | 439.20 | ¹H NMR (400 MHz, DMSO-d₆) δ 7.80 (d, J = 23.1 Hz, 1H), 7.68 - 7.56 (m, 2H), 4.85 (d, J = 8.8 Hz, 4H), 3.76 (d, J = 2.2 Hz, 2H), 3.54 - 3.44 (m, 1H), 3.29 - 3.19 (m, 1H), 3.09 - 3.00 (m, 1H), 2.95 - 2.87 (m, 1H), 2.39 - 2.19 (m, 4H), 1.87 - 1.70 (m, 2H). |
| 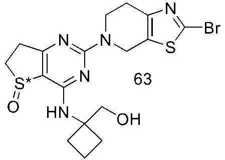 | 471.00 | ¹H NMR (400 MHz, DMSO-d₆) δ 7.75 (s, 1H), 4.88 (s, 2H), 4.01 (m, 4H), 3.69 (s, 2H), 3.40 (dd, J = 16.9, 8.3 Hz, 1H), 3.18 (dt, J = 15.2, 8.3 Hz, 1H), 2.91-2.83 (m, 2H), 2.76 (s, 2H), 2.25 (m, 3H), 1.76 - 1.71 (m, 2H). |
| 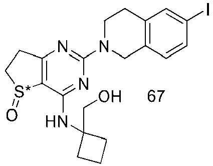 | 511.15 | ¹H NMR (399 MHz, DMSO-d₆) δ 7.56 (d, J = 1.9 Hz, 1H), 7.51 (dd, J = 8.0, 1.8 Hz, 1H), 7.01 (d, J = 8.1 Hz, 1H), 4.78 (s, 2H), 3.88 (t, J = 5.9 Hz, 2H), 3.71 (d, J = 1.9 Hz, 2H), 3.45 (dd, J = 16.7, 8.3 Hz, 1H), 3.27 - 3.16 (m, 1H), 3.01 (dd, J = 17.5, 8.0 Hz, 1H), 2.89 (dd, J = 13.7, 7.1 Hz, 1H), 2.81 (t, J = 5.9 Hz, 2H), 2.30 (dd, J = 20.9, 11.5 Hz, 2H), 2.19 (s, 2H), 1.76 (q, J = 8.8, 7.2 Hz, 2H). |
| 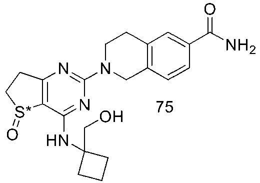 | 428.05 | ¹H NMR (400 MHz, MeOH-d4) δ 7.68 (dd, J = 4.4, 2.5 Hz, 2H), 7.26 (d, J = 8.5 Hz, 1H), 4.95 (s, 2H), 4.07 - 3.91 (m, 4H), 3.56 (dd, J = 17.2, 8.5 Hz, 1H), 3.39 - 3.31 (m, 1H), 3.11 - 3.03 (m, 2H), 2.93 (t, J = 5.9 Hz, 2H), 2.38 - 2.29 (m, 4H), 1.96 - 1.87 (m, 2H). |
| 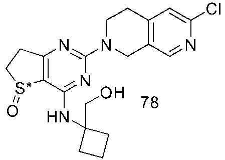 | 420.20 | ¹H NMR (400 MHz, DMSO-d₆) δ 8.28 (s, 1H), 7.89 - 7.64 (m, 1H), 7.35 (s, 1H), 4.87 (s, 2H), 3.92 (t, J = 5.9 Hz, 2H), 3.71 (s, 2H), 3.49 - 3.39 (m, 1H), 3.26 - 3.14 (m, 1H), 3.04 - 2.94 (m, 1H), 2.92 - 2.82 (m, 3H), 2.36 - 2.24 (m, 2H), 2.24 - 2.15 (m, 2H), 1.81 - 1.70 (m, 2H). |
| 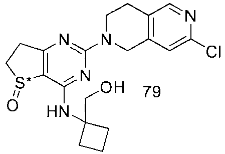 | 420.15 | ¹H NMR (399 MHz, DMSO-d₆) δ 8.20 (s, 1H), 7.95 (s, 1H), 7.45 (s, 1H), 4.87 (s, 2H), 3.96-3.93 (m, 2H), 3.75-3.69 (m, 2H), 3.50-3.42 (m, 1H), 3.26-3.18 (m, 1H), 3.05-2.99 (m, 1H), 2.90-2.87 (m, 1H), 2.83-2.80 (m, 2H), 2.35 - 2.22 (m, 4H), 1.79-1.73 (m, 2H). |

### Example 10: Synthesis of compounds 28 and 29:

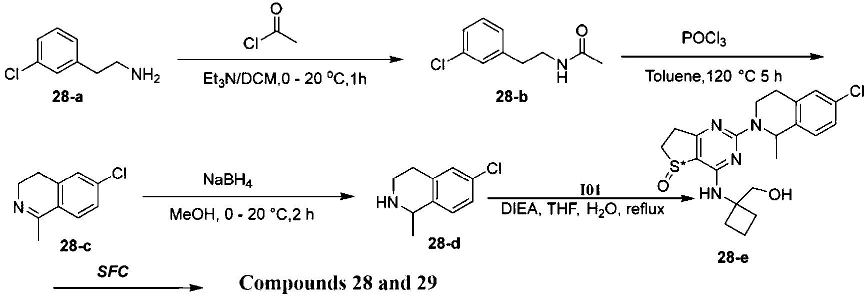

### 1) N-(3-chlorophenethyl)acetamide (28-b)

At 0°C, to a solution of 2-(3-chlorophenyl)ethan-1-amine (28-a, 5 g, 32.258 mmol, 1 eq) in anhydrous DCM (30 mL), TEA (4.88 g, 48.387 mmol, 1.5 eq) and acetylchloride (3.8 g, 48.387 mmol, 1.5 eq) were dropwise added successively. After stirring at room temperature for 1 hours, water (30 mL) was added. After extraction with ethyl acetate (10 mL x 3), the resulting material was dried over anhydrous Na₂SO₄, filtered, and concentrated to obtain 28-b (5.6 g, 88.1%) as a yellow oil product. LCMS: 198.05 [M+H]⁺.

### 2) 6-Chloro-1-methyl-3,4-dihydroisoquinoline (28-c)

At room temperature, 28-b (1.1 g, 5.584 mmol, 1 eq) was added to a POCl₃ solution (10 mL). After stirring at 120°C for 18 hours, the resulting material was cooled to room temperature, and the reaction liquid was poured into ice water (100 mL) and then adjusted to pH-8 with 12 N NaOH. After extraction with ethyl acetate (30 mL x 3), the resulting material was dried over anhydrous Na₂SO₄, filtered, and concentrated to obtain a product of 28-c (1 g, 100%). LCMS: 180.05 [M+H]⁺.

### 3) 6-Chloro-1-methyl-1,2,3,4-tetrahydroisoquinoline (28-d)

At 0°C, to a solution of 28-c (2.48 g, 13.85 mmol, 1eq) in MeOH (20 mL), NaBH₄ (1.05 g, 27.64 mmol, 2 eq) was added in portions. After stirring at room temperature for 2 hours, HCl (1 N) was added, and the resulting material was then adjusted to pH-8 with NaOH (1N). After extraction with dichloromethane (30 mL x 3), the resulting material was dried over anhydrous Na₂SO₄, filtered, and concentrated to obtain a product of 28-d (1.04 g, 41%). LCMS: 182.05 [M+H]⁺.

### 4) (R)-2-((S)-6-chloro-1-methyl-3,4-dihydroisoquinolin-2(1H)-yl)-4-((1-(hydroxymethyl)cyclobutyl)amino)-6,7-dihydrothieno[3,2-d]pyrimidine 5-oxide (28); and (R)-2-(((R)-6-chloro-1-methyl-3,4-dihydroisoquinolin-2(1H)-yl)-4-((1-(hydroxymethyl)cyclobutyl)amino)-6,7-dihydrothieno[3,2-d]pyrimidine 5-oxide (29)

At room temperature, to a solution of I01 (120 mg, 0.418 mmol, 1 eq) in THF/H₂O (4 mL: 1 mL), 8-d (83.2 mg, 0.460 mmol, 1.1 eq) and DIEPA (161.8 mg, 1.254 mmol, 3 eq) were added successively. After stirring at 65°C for 3 hours, the resulting material was concentrated. The resulting material was purified by Pre-HPLC (ACN: 0.1% TFA in H₂O from 5% to 95%) and further separated by SFC to obtain 28 (44.9 mg, 24.9%) and 29 (35.1 mg, 19.4%) as a white solid product. LCMS: 433.25 [M+H]⁺.

28_¹H NMR (400 MHz, DMSO-d₆) δ 7.24 (d, J = 20.0 Hz, 3H), 3.70 (s, 2H), 3.42 (dd, J = 16.9, 8.3 Hz, 2H), 3.16 (s, 1H), 2.96 - 2.75 (m, 4H), 2.38 - 2.24 (m, 3H), 2.17 (s, 2H), 1.76 (m, 2H), 1.38 (s, 3H), 1.21 - 1.10 (m, 1H).

29_ ¹H NMR (400 MHz, DMSO- d₆)) δ 7.28 (d, J = 25.6 Hz, 3H), 3.83 - 3.66 (m, 2H), 3.22 (dt, J = 15.6, 8.5 Hz, 1H), 2.97 (dd, J = 17.1, 8.0 Hz, 1H), 2.91 - 2.73 (m, 4H), 2.44 - 2.26 (m, 3H), 2.14 (d, J = 48.7 Hz, 2H), 1.80 (d, J = 8.0 Hz, 2H), 1.42 (d, J = 6.8 Hz, 3H), 1.24 (m, 1H).

### Example 11: Synthesis of compound 38:

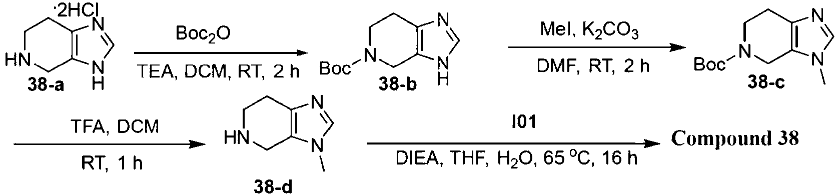

### 1)Tert-butyl 3,4,6,7-tetrahydro-5H-imidazo[4,5-c]pyridine-5-carboxylate (38-b)

At room temperature, to a solution of 4,5,6,7-tetrahydro-3H-imidazo[4,5-C]pyridine dihydrochloride (100 mg, 0.51 mmol, 1 eq) in DCM (5 mL), Boc₂O (114 mg, 0.51 mmol, 1 eq) and TEA (155 mg, 1.53 mmol, 3 eq) were added successively. After stirring for 2 hours, water (10 mL) was added. After extraction with dichloromethane (10 mL x 3), the resulting material was dried over anhydrous Na₂SO₄, filtered, and concentrated to obtain 38-b (100 mg, 55.2%) as a yellow oil product. LCMS: 224.30 [M+H]⁺.

### 2) Tert-butyl 3-methyl-3,4,6,7-tetrahydro-5H-imidazo[4,5-c]pyridine-5-carboxylate (38-c)

At room temperature, to a solution of 38-b (70 mg, 0.31 mmol, 1 eq) in DMF (4 mL), K₂CO₃ (129.00 mg, 0.93 mmol, 3 eq) and MeI (45.00 mg, 0.31 mmol, 1 eq) were added successively. After stirring for 2 hours, water (10 mL) was added. After extraction with ethyl acetate (10 mL x 3), the resulting material was dried over anhydrous Na₂SO₄, filtered, and concentrated to obtain 38-c (45 mg, 60.5%) as a yellow oil product. The crude product was directly used in the next reaction without purification. LCMS: 238.35 [M+H]⁺.

### 3) 3-Methyl-4,5,6,7-tetrahydro-3H-imidazo[4,5-c]pyridine (38-d)

At room temperature, to a solution of 38-c (45 mg, 0.19 mmol, 1 eq) in DCM (4 mL), TFA (1 mL) was added. After stirring for 1 hour, the resulting material was concentrated to obtain 38-d (22 mg, 84.6%) as a yellow oil product. The crude product was directly used in the next reaction without purification. LCMS: 138.35 [M+H]⁺.

### 4) (R)-4-((1-(hydroxymethyl)cyclobutyl)amino)-2-(3-methyl-3,4,6,7-tetrahydro-5H-imidazo[4,5-c]pyridin-5-yl)-6,7-dihydrothieno[3,2-d]pyrimidine-5-oxide (38)

At room temperature, to a solution of I01 (40 mg, 0.14 mmol, 1 eq) and 38-d (23.5 mg, 0.17 mmol, 1.2 eq) in THF (4 mL) and H₂O (1 mL), DIEPA (55.00 mg, 0.42 mmol, 3 eq) was added. After stirring at 65°C for 3 hours, the resulting material was concentrated. The resulting material was purified by Pre-TLC (SiO₂, DCM: MeOH = 15: 1) to obtain 38 (20.7 mg, 38.2%) as a white solid product. LCMS: 389.15 [M+H]⁺. ¹H NMR (400 MHz, DMSO- d₆) δ 8.89 (d, J = 14.2 Hz, 1H), 7.65 (s, 1H), 4.85 (s, 1H), 4.01 (s, 2H), 3.77 (s, 2H), 3.69 (d, J = 4.2 Hz, 5H), 3.29 (dt, J = 79.2, 7.5 Hz, 2H), 2.96 - 2.81 (m, 2H), 2.71 (s, 2H), 2.24 (d, J = 43.0 Hz, 4H), 1.80 - 1.70 (m, 2H).

### Example 12: Synthesis of compound 39:

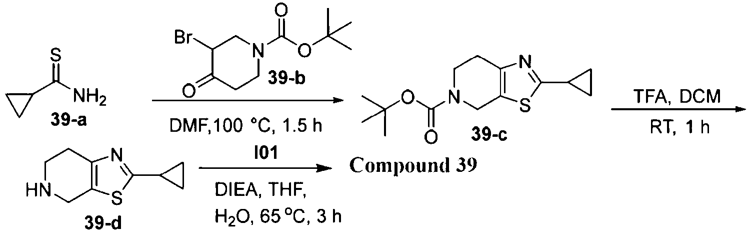

### 1) Tert-butyl 2-cyclopropyl-6,7-dihydrothiazolo[5,4-c]pyridine-5(4H)-carboxylate (39-c)

A solution of cyclopropane thioformamide (39-a, 219 mg, 2.16 mmol, 3 eq) and tert-butyl 3-bromo-4-piperidinone-1-carboxylate (39-b, 200 mg, 0.72 mmol, 1 eq) in DMF (4 mL) was stirred at 100°C for 1.5 hours. The resulting material was cooled to room temperature, and water (10 mL) was added. After extraction with ethyl acetate (10 mL x 3), the resulting material was dried over anhydrous Na₂SO₄, filtered, and concentrated. The resulting material was purified by Pre-TLC (SiO₂, PE: EtOAc = 3: 1) to obtain 39-c (56 mg, 27.8%) as a white solid product. LCMS: 281.10 [M+H]⁺.

### 2) 2-Cyclopropyl-4,5,6,7-tetrahydrothiazolo[5,4-c]pyridine (39-d)

At room temperature, to a solution of 39-c (56 mg, 0.20 mmol, 1 eq) in DCM (4 mL), TFA (1 mL) was added. After stirring for 2 h, the resulting material was concentrated to obtain 39-d (30 mg, 83.3%) as a yellow oil product. The crude product was directly used in the next reaction without purification. LCMS: 181.25 [M+H]⁺.

### 3) (R)-2-(2-cyclopropyl-6,7-dihydrothiazolo[5,4-c]pyridin-5(4H)-yl)-4-((1-(hydroxymethyl)cyclobutyl)amino)-6,7-dihydrothieno[3,2-d]pyrimidine 5-oxide (39)

At room temperature, to a solution of I01 (50 mg, 0.17 mmol, 1 eq) and 39-d (30 mg, 0.17 mmol, 1 eq) in THF (4 mL) and H₂O (1 mL), DIEPA (67.37 mg, 0.51 mmol, 3 eq) was added. After stirring at 65°C for 3 hours, the resulting material was concentrated. The resulting material was purified by Prep-HPLC (0.1% TFA in ACN/H₂O) to obtain 39 (69.7 mg, 92.9%) as a white solid product. LCMS: 432.15 [M+H]⁺. ¹H NMR (400 MHz, DMSO- d6) δ 4.87 (m, 4H), 4.00 (t, J = 5.8 Hz, 2H), 3.69 (s, 2H), 3.51 - 3.38 (m, 1H), 3.27 - 3.14 (m, 1H), 3.06 - 2.85 (m, 2H), 2.71 (t, J = 5.7 Hz, 2H), 2.31 - 2.15 (m, 5H), 1.75 (m, J = 8.9 Hz, 2H), 1.03 (m, J = 8.2, 3.3 Hz, 2H), 0.88 - 0.82 (m, 2H).

### Example 13: Synthesis of compound 44:

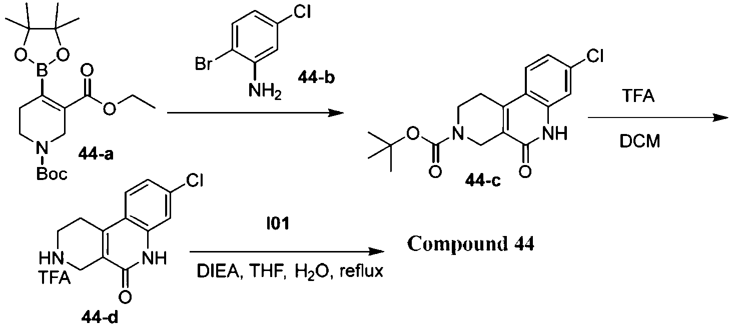

### 1) Tert-butyl 8-chloro-5-oxo-1,4,5,6-tetrahydrobenzo[c][2,7]naphthyridine-3(2H)-carboxylate (44-c)

At room temperature under nitrogen protection, to a solution of tert-butyl 3-ethyl-4-(4,4,5,5-tetramethyl-1,3,2-dioxaboran-2-yl)-5,6-dihydropyridine-1,3(2H)-dicarboxylate (44-a, 200 mg, 0.52 mmol, 1 eq) and 2-bromo-5-chloroaniline (44-b, 108 mg, 0.52 mmol, 1 eq) in THF (4 mL), Na₂CO₃ (132 mg, 1.58 mmol, 3 eq), Pd(dppf)Cl₂ (36 mg,0.05 mmol,0.1 eq) and water (1 mL) were added successively. After heating under reflux for 6 hours, the resulting material was cooled to room temperature, and a solid was filtered off. After extraction with ethyl acetate (10 mL x 3), the resulting material was dried over anhydrous Na₂SO₄, filtered, and concentrated. The resulting material was purified by column chromatography (PE: EA from 15: 1 to 5: 1) to obtain 44-c (50 mg, 28.7%) as a yellow solid product. LCMS: 335.15 [M+H]⁺.

### 2) 8-Chloro-2,3,4,6-tetrahydrobenzo[c][2,7]naphthyridin-5(1H)-one (44-d)

At room temperature, to a solution of 44-c (50 mg, 0.15 mmol, 1 eq) in DCM (2 mL), TFA (1 mL) was added. After stirring for 1 hour, the resulting material was concentrated. The resulting material was purified by Pre-HPLC (ACN: 0.1% TFA in H₂O from 5% to 95%) to obtain 44-d (35 mg, 99.4%) as a white solid product. LCMS: 235.10 [M+H]⁺.

### 3) (R)-8-chloro-3-(4-((1-(hydroxymethyl)cyclobutyl)amino)-5-oxido-6,7-dihydrothieno[3,2-d]pyrimidin-2-yl)-2,3,4,6-tetrahydrobenzo[c][2,7]naphthyridin-5(1H)-one (44)

At room temperature, to a solution of 44-d (35 mg, 0.15 mmol, 1.2 eq) and I01 (36 mg, 0.12 mmol, 1 eq) in THF (4 mL), DIEPA (48 mg, 0.38 mmol, 3 eq) and water (1 mL) were added. After stirring at 65°C for 3 hours, the resulting material was concentrated. The resulting material was purified by Prep-HPLC (0.1% TFA in ACN/H₂O) to obtain 44 (35.7 mg, 48.9%) as a white solid product. LCMS: 486.05 [M+H]⁺. ¹H NMR (400 MHz, DMSO-d₆) δ 7.71 (d, J = 8.7 Hz, 1H), 7.33 (d, J = 2.2 Hz, 1H), 7.23 (dd, J = 8.6, 2.2 Hz, 1H), 4.62 (s, 2H), 4.02 (s, 4H), 3.73 (s, 2H), 3.52 - 3.45 (m, 1H), 3.27 - 3.18 (m, 1H), 3.06 (dd, J = 17.4, 8.0 Hz, 1H), 2.91 (d, J = 21.6 Hz, 3H), 2.41 - 2.25 (m, 3H), 2.19 (s, 2H), 1.77 (d, J = 22.0 Hz, 2H).

The following compounds were synthesized by similar methods.

| Compound | MS (M+H)⁺ | NMR |
|---|---|---|
| 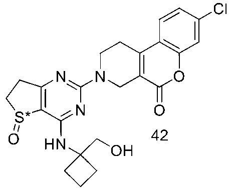 | 487.10 | ¹H NMR (400 MHz, DMSO-d₆) δ 8.11 (s, 1H), 7.98 (d, J = 8.9 Hz, 1H), 7.79 (s, 1H), 4.96 (s, 2H), 4.38 (s, 2H), 4.08 (s, 2H), 3.83 (s, 1H), 3.57 (s, 1H), 3.39 (s, 1H), 3.29 (s, 4H), 2.68 (s, 3H), 2.53 (s, 2H), 2.14 (s, 2H). |

### Example 14: Synthesis of compound 45:

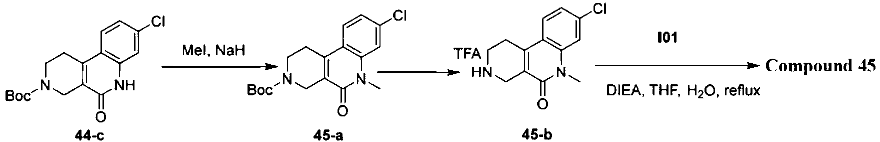

### 1) Tert-butyl 8-chloro-6-methyl-5-oxo-1,4,5,6-tetrahydrobenzo[c][2,7]naphthyridine-3(2H)-carboxylate (45-a)

At 0°C, to a solution of 44-c (50 mg, 0.14 mmol, 1 eq) in DMF (4 mL), NaH (10 mg, 0.02 mmol, 0.1 eq) was added. After stirring for 30 minutes, iodomethane (7.12 mg, 0.30 mmol, 2 eq) was dropwise added. After stirring at room temperature for 2 hours, water (20 mL) was added. After extraction with ethyl acetate (30 mL x 3), the resulting material was dried over anhydrous Na₂SO₄, filtered, and concentrated. The resulting material was purified by column chromatography (PE: EA from 15: 1 to 3: 1) to obtain 45-a (45 mg, 91.8%) as a yellow solid product. LCMS: 349.15 [M+H]⁺.

### 2) 8-Chloro-6-methyl-2,3,4,6-tetrahydrobenzo[c][2,7]naphthyridin-5(1H)-one (45-b)

At room temperature, to a solution of 45-a (36 mg, 0.11 mmol, 1 eq) in DCM (2 mL), TFA (1 mL) was added. After :stirring for 1 hour, the resulting material was concentrated. The resulting material was purified by Pre-HPLC (ACN: 0.1% TFA in H₂O from 5% to 95%) to obtain 45-b (25.7 mg, 89.3%) as a white solid product. LCMS: 249.10 [M+H]⁺.

### 3) (R)-8-chloro-3-(4-((1-(hydroxymethyl)cyclobutyl)amino)-5-oxido-6,7-dihydrothieno[3,2-d]pyrimidin-2-yl)-6-methyl-2,3,4,6-tetrahydrobenzo[c][2,7]naphthyridin-5(1H)-one (45)

At room temperature, to a solution of 44-b (25.7 mg, 0.10 mmol, 1.5 eq) and I01 (20 mg, 0.07 mmol, 1 eq) in THF (4 mL), DIEPA (31 mg, 0.30 mmol, 3 eq) and water (1 mL) were added. After stirring at 65°C for 3 hours, the resulting material was concentrated. The resulting material was purified by Prep-HPLC (0.1% TFA in ACN/H₂O) to obtain 45 (19.7 mg, 39.4%) as a white solid product. LCMS: 500.25 [M+H]⁺. ¹H NMR (399 MHz, DMSO-d₆) δ 7.79 (d, J = 8.6 Hz, 1H), 7.63 (d, J = 2.0 Hz, 1H), 7.34 (dd, J = 8.6, 2.0 Hz, 1H), 4.65 (s, 2H), 4.03 (s, 2H), 3.82 - 3.67 (m, 4H), 3.46 (dt, J = 15.9, 7.8 Hz, 2H), 3.20 (dd, J = 14.0, 8.0 Hz, 1H), 3.04 - 2.85 (m, 4H), 2.46 - 2.27 (m, 4H), 2.18 (s, 2H), 1.79 (dd, J = 19.6, 10.3 Hz, 2H).

### Example 15: Synthesis of compound 48

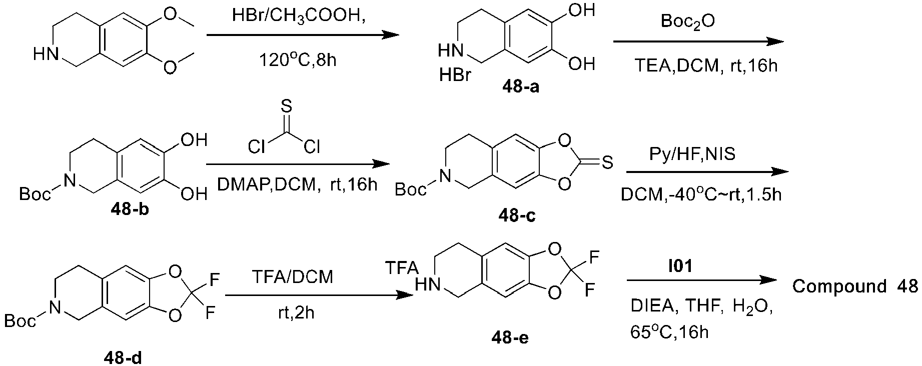

### 1) 1,2,3,4-Tetrahydroisoquinoline-6,7-diol (48-a)

A solution of 6,7-dimethoxy-1,2,3,4-tetrahydroisoquinoline (3.01 g, 10.36 mmol, 1.0 eq) in HBr (22.5 mL, 40% in H₂O) and CH₃COOH (100 mL) was stirred at 120°C for 8 hours. The resulting material was cooled to room temperature and concentrated. The resulting material was purified by column chromatography (100% EtOAc) to obtain 48-a (2.21 g, 86.3%) as a white solid product. LCMS: 166.10 [M+H]⁺.

### 1. Tert-butyl 6,7-dihydroxy-3,4-dihydroisoquinoline-2(1H)-carboxylate (48-b)

At room temperature, to a solution of 48-a (2.21 g, 8.94 mmol, 1.0 eq) and Boc₂O (1.95 g, 8.94 mmol, 1.0 eq) in DCM (30 mL), triethylamine (4.52 g, 44.72 mmol, 5.0 eq) was added. After stirring at room temperature for 16 hours, the resulting material was concentrated. The resulting material was purified by column chromatography (EtOAc/hexanes = 50: 50) to obtain 48-b (1.81 g, 75.89%) as a white solid product. LCMS: 210.15 [M-56+H]⁺.

### 5) Tert-butyl 2-thioxo-7,8-dihydro-[1,3]dioxolo[4,5-g]isoquinoline-6(5H)-carboxylate (48-c)

At 0°C, to a solution of T48-b (1.01 g, 3.77 mmol, 1.0 eq) and DMAP (1.61 g, 13.21 mmol, 3.5 eq) in CH₂Cl₂ (200 mL), thiophosgene (0.57 mL, 7.55 mmol, 2.0 eq) was dropwise added. After stirring at room temperature for 16 hours, the resulting material was concentrated. The resulting material was purified by column chromatography (EtOAc/hexanes = 50: 50) to 48-c (870 mg, 75.1%) as a white solid product. LCMS: 251.90 [M-56+H]⁺.

### 2. Tert-butyl 2,2-difluoro-7,8-dihydro-[1,3]dioxolo[4,5-g]isoquinoline-6(5H)-carboxylate (48-d)

At -40°C, to a solution of 48-c (200 mg, 0.65 mmol, 1.0 eq) in DCM (4 mL), hydrofluoropyridine (70%wt HF; 643 mg, 6.51 mmol, 10 eq) was added. N-iodosuccinimide (438 mg, 1.95 mmol, 3.0 eq) was then added in portions. The reaction solution was heated to 0°C over 30 minutes. After stirring at room temperature for 30 minutes, a solution of NaHSO₃ (0.5 g) in water (3 mL) was added. After stirring for 15 minutes, water (10 mL) was added. After extraction with ethyl acetate (20 mL x 3), the resulting material was dried over anhydrous Na₂SO₄, filtered, and concentrated. The resulting material was purified by Pre-TLC (EtOAc/hexanes = 3: 1) to obtain 48-d (60 mg, 29.4%) as a white solid product. LCMS: 258.05 [M-56+H]⁺.

### 6) 2,2-Difluoro-5,6,7,8-tetrahydro-[1,3]dioxolo[4,5-g]isoquinoline (48-e)

At room temperature, to a solution of 48-d (20 mg, 0.064 mmol, 1.0 eq) in DCM (1 mL), TFA (0.25 mL) was dropwise added. After stirring for 1 hour, the resulting material was concentrated to obtain 48-e (20 mg, 95.7%) as a white solid product. The crude product was directly used in the next reaction without purification. LCMS: 214.10 [M+H]⁺.

### 7) (R)-2-(2,2-difluoro-7,8-dihydro-[1,3]dioxolo[4,5-g]isoquinolin-6(5H)-yl)-4-((1-(hydroxymethyl)cyclobutyl)amino)-6,7-dihydrothieno[3,2-d]pyrimidine 5-oxide (48)

At room temperature, to a solution of 48-e (20 mg, 0.061 mmol, 1.0 eq) and I01 (16 mg, 0.055 mmol, 0.9 eq) in THF (1 mL), DIEPA (24 mg, 0.18 mmol, 3.0 eq) and water (0.25 mL) were added. After stirring at 65°C for 3 hours, the resulting material was concentrated. The resulting material was purified by Prep-HPLC (0.1% TFA in ACN/H₂O) to obtain 48 (18.06 mg, 63.8%) as a white solid product. LCMS: 465.05 [M+H]⁺. ¹H NMR (400 MHz, DMSO-d₆) δ 7.32 (s, 1H), 7.22 (s, 1H), 4.82 (s, 2H), 3.87 (s, 2H), 3.71 (s, 2H), 3.48 - 3.38 (m, 1H), 3.19 (dt, J = 15.6, 8.3 Hz, 1H), 2.97 (d, J = 14.4 Hz, 1H), 2.88 (d, J = 6.8 Hz, 1H), 2.83 (s, 2H), 2.34 - 2.18 (m, 4H), 1.82 - 1.69 (m, 2H).

### Example 16: Synthesis of compound 49

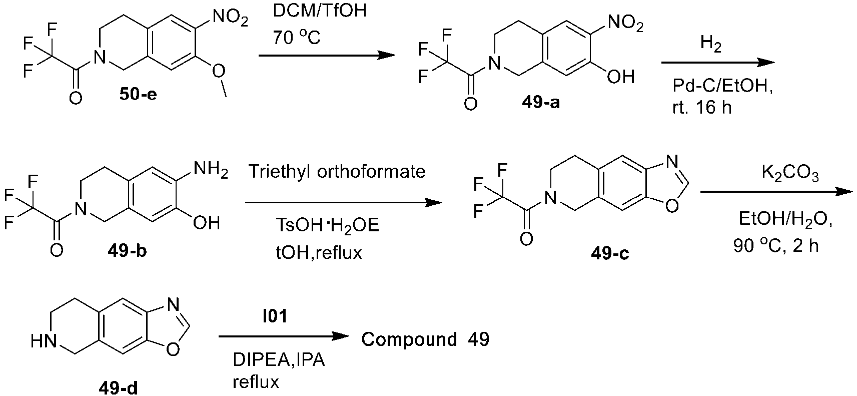

### 1)2,2,2-Trifluoro-1-(7-hydroxy-6-nitro-3,4-dihydroisoquinolin-2(1H)-yl)ethan-1-one (49-a)

A solution of 2,2,2-trifluoro-1-(7-methoxy-6-nitro-3,4-dihydroisoquinolin-2(1H)-yl)ethan-1-one (50-e, 500 mg, 1.65 mol, 1.0 eq) in DCM (5 mL) and TfOH (5 mL) was stirred at 70°C for 1 hour and concentrated. EtOAc (100 mL) was added, and the resulting material was washed with saturated NaHCO₃ (100 mL). The resulting material was dried over anhydrous Na₂SO₄, filtered, and concentrated. The resulting material was purified by column chromatography (EtOAc/hexanes = 1: 1) to obtain 49-a (420 mg, 88%) as a white solid product. LCMS: 291.05 [M+H]⁺.

### 2) 1-(6-Amino-7-hydroxy-3,4-dihydroisoquinolin-2(1H)-yl)-2,2,2-trifluoroethan-1-one (49-b)

At room temperature, to a solution of 49-a (400 mg, 1.37 mol, 1.0 eq) in EtOH (100 mL), Pd/C (10% wt., 40 mg) was added. After hydrogen absorption at room temperature for 16 hours, a solid was filtered off, followed by concentration to obtain 49-b (290 mg, 80.8%) as a white solid product. LCMS: 261.10 [M+H]⁺.

### 3) 1-(7,8-Dihydrooxazolo[4,5-g]isoquinolin-6(5H)-yl)-2,2,2-trifluoroethan-1-one (49-c)

At room temperature, to a solution of 49-b (200 mg, 0.76 mmol, 1.0 eq) in EtOH (10 mL), triethyl orthoformate (680.4 mg, 4.59 mol, 6.0 eq) and TsOH (4 mg, 0.076 mmol, 0.1 eq) were added. After stirring at 80°C for 2 hours, the resulting material was cooled to 0°C, and H₂O (50 mL) was added. After extraction with ethyl acetate (20 mL x 3), the resulting material was dried over anhydrous Na₂SO₄, filtered, and concentrated. The resulting material was purified by column chromatography (EtOAc/hexanes = 1: 1) to 49-c (180 mg, 86.6%) as a white solid product. LCMS: 271.10 [M+H]⁺.

### 4) 5,6,7,8-Tetrahydrooxazolo[4,5-g]isoquinoline (49-d)

At room temperature, to a solution of 49-c (30 mg, 0.11 mmol, 1.0 eq) in EtOH (4 mL) and H₂O (1 mL), K₂CO₃ (46 mg, 0.33 mmol, 3.0 eq) was added. After stirring at 90°C for 2 hours, the resulting material was cooled to room temperature, and a saturated NaHCO₃ solution (30 mL) was added. After extraction with ethyl acetate (15 mL x 3), the resulting material was dried over anhydrous Na₂SO₄, filtered, and concentrated. The resulting material was purified by Pre-TLC (petroleum ether: ethyl acetate = 1: 3) to obtain 49-d (19 mg, 98.2%) as a white solid product. LCMS: 175.10 [M+H]⁺.

### 5) (R)-2-(7,8-dihydrooxazolo[4,5-g]isoquinolin-6(5H)-yl)-4-((1-(hydroxymethyl)cyclobutyl)amino)-6,7-dihydrothieno[3,2-d]pyrimidine 5-oxide (49)

At room temperature, to a solution of 49-d (50.00 mg, 0.24 mmol, 1 eq) and I01 (70.0 mg, 0.24 mmol, 1 eq) in IPA (5 mL), DIEPA (92.88 mg, 0.72 mmol, 3 eq) was added. After heating under reflux for 48 hours, the resulting material was concentrated. The resulting material was purified by Pre-TLC (SiO₂, dichloromethane: methanol = 15: 1) to obtain 49 (2.24 mg, 2.2%) as a white solid product. LCMS: 416.20 [M+H]⁺. ¹H NMR (400 MHz, Methanol-d₄) δ 8.41 (s, 1H), 7.56 (s, 1H), 7.53 (s, 1H), 5.04 (s, 2H), 3.99 (s, 4H), 3.57 (s, 1H), 3.38 - 3.32 (m, 1H), 3.04 (s, 4H), 2.35 (d, J = 9.4 Hz, 4H), 1.92 (s, 2H).

### Example 17: Synthesis of compound 50

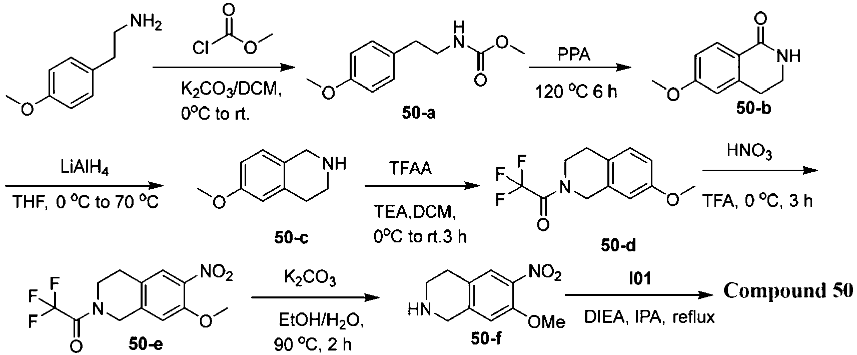

### 1) Methyl (4-methoxyphenethyl)aminoformate (50-a)

At -40°C, to a solution of 2-(4-methoxyphenyl)ethan-1-amine (30 g, 0.2 mol, 1 eq) in DCM (400 mL), K₂CO₃ (82 g, 0.59 mol, 3.0 eq) was added. After stirring for 1 hour, methyl chloroformate (18.8 g, 0.2 mol, 1.0 eq) was dropwise added. After continued stirring for 2 hours, a saturated NaHCO₃ solution (300 mL) was added. After extraction with DCM (300 mL x 3), the resulting material was dried over anhydrous Na₂SO₄, filtered, and concentrated. The resulting material was purified by column chromatography (PE: EtOAc from 10: 1 to 1: 1) to obtain 50-a (72.2 g) as a white solid product. LCMS: 210.15 [M+H]⁺.

### 2) 6-Methoxy-3,4-dihydroisoquinolin-1(2H)-one (50-b)

A solution of 50a (10 g, 0.04 mol, 1.0 eq) in PPA (60 mL) was stirred at 120°C for 1 hour and then cooled to room temperature. A saturated Na₂CO₃ solution (300 mL) was slowly added. After extraction with ethyl acetate (150 mL x 3), the resulting material was dried over anhydrous Na₂SO₄, filtered, and concentrated. The resulting material was purified by column chromatography (PE: EtOAc from 10: 1 to 3: 1) to obtain 50-b (2.0 g, 23.6%) as a white solid product. LCMS: 178.10 [M+H]⁺.

### 3) 6-Methoxy-1,2,3,4-tetrahydroisoquinoline (50-c)

At -40°C, to a solution of 50-b (1.5 g, 8.47 mmol, 1 eq) in THF (10 mL), LiAlH₄ (0.39 g, 10.16 mmol, 1.2 eq) was added. After stirring at 70°C for 2 hours, the resulting material was cooled to room temperature, and H₂O (30 mL) was added. After extraction with ethyl acetate (30 mL x 3), the resulting material was dried over anhydrous Na₂SO₄, filtered, and concentrated. The resulting material was purified by column chromatography (PE: EtOAc from 10: 1 to 3: 1) to obtain 50-c (1 g, 72.4%) as a white solid product. LCMS: 164.10 [M+H]⁺.

### 4) 2,2,2-Trifluoro-1-(7-methoxy-3,4-dihydroisoquinolin-2(1H)-yl)ethan-1-one (50-d)

At 0°C, to a solution of 50-c (3.3 g, 20.24 mmol, 1.0 eq) in DCM (40 mL), TEA (5.1g, 24.29 mmol, 3.0 eq) and TFAA (5.1 g, 24.29 mmol, 1.2 eq) were added successively. After stirring at room temperature for 3 hours, water (100 mL) was added. After extraction with DCM (50 mL x 3), the resulting material was dried over anhydrous Na₂SO₄, filtered, and concentrated. The resulting material was purified by column chromatography (SiO₂, PE: EtOAc = 10: 1) to obtain 50-d (3.5 g, 66.7%) as a white solid product. LCMS: 260.10 [M+H]⁺.

### 5) 2,2,2-Trifluoro-1-(7-methoxy-6-nitro-3,4-dihydroisoquinolin-2(1H)-yl)ethan-1-one (50-e)

At 0°C, to a solution of 50-d (810 mg, 3.1 mmol, 1.0 eq) in TFA (10 mL), HNO₃ (217mg, 3.44 mmol, 1.1 eq) was dropwise added. After stirring for 3 hours, a saturated NaHCO₃ solution (100 mL) was added. After extraction with ethyl acetate (30 mL x 3), the resulting material was dried over anhydrous Na₂SO₄, filtered, and concentrated. The resulting material was purified by column chromatography (PE: EtOAc = 1: 1) to obtain 50-e (720 mg, 75.7%) as a yellow solid product. LCMS: 305.10 [M+H]⁺.

### 6) 7-Methoxy-6-nitro-1,2,3,4-tetrahydroisoquinoline (50-f)

At room temperature, to a solution of 50-e (30 mg, 0.1 mmol, 1.0 eq) in EtOH (4 mL) and H₂O (1 mL), K₂CO₃(14 mg, 0.3 mmol, 3.0 eq) was added. After stirring at 90°C for 2 hours, the resulting material was cooled to room temperature, and H₂O (20 mL) was added. After extraction with ethyl acetate (15 mL x 3), the resulting material was dried over anhydrous Na₂SO₄, filtered, and concentrated. The resulting material was purified by Pre-TLC (PE: EtOAc = 1: 3) to obtain 50-f(16 mg, 77.8%) as a yellow solid product. LCMS: 209.10 [M+H]⁺.

### 7) (R)-4-((1-(hydroxymethyl)cyclobutyl)amino)-2-(7-methoxy-6-nitro-3,4-dihydroisoquinolin-2(1H)-yl)-6,7-dihydrothieno[3,2-d]pyrimidine 5-oxide (50)

At room temperature, to a solution of 50-f (50.00 mg, 0.24 mmol, 1.0 eq) and I01 (70.0 mg, 0.24 mmol, 1.0 eq) in IPA (5 mL), DIEPA (92.88 mg, 0.72 mmol, 3.0 eq) was added. After heating under reflux for 48 hours, the resulting material was concentrated. The resulting material was purified by Pre-TLC (SiO₂, dichloromethane: methanol = 15: 1) to obtain 50 (20.33 mg, 18.2%) as a white solid product. LCMS: 460.25 [M+H]⁺. ¹H NMR (400 MHz, Methanol-d4) δ 7.65 (s, 1H), 7.10 (s, 1H), 4.98 (s, 2H), 4.06 - 3.99 (m, 2H), 3.92 (s, 3H), 3.57 (dt, J = 17.0, 8.1 Hz, 1H), 3.35 (d, J = 13.9 Hz, 1H), 3.29 (s, 2H), 3.10 (s, 2H), 2.85 (s, 2H), 2.32 (s, 4H), 1.90 (s, 2H).

### Example 18: Synthesis of compound 51

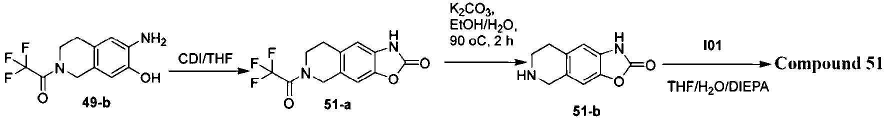

### 1) 6-(2,2,2-Trifluoroacetyl)-5,6,7,8-tetrahydrooxazolo[4,5-g]isoquinolin-2(1H)-one (51-a)

At room temperature, to a solution of 49-b (160 mg, 1.0 mol, 1.0 eq) in THF (5 mL), CDI (180 mg, 3.0 mmol, 3.0 eq) was added. After continued stirring for 2 hours, H₂O (30 mL) was added. After extraction with ethyl acetate (30 mL x 3), the resulting material was dried over anhydrous Na₂SO₄, filtered, and concentrated. The resulting material was purified by column chromatography (PE: EtOAc from 15: 1 to 5: 1) to obtain 51-a (140 mg, 79.2%) as a white solid product. LCMS: 287.10 [M+H]⁺.

### 2) 5,6,7,8-Tetrahydrooxazolo[4,5-g]isoquinolin-2(1H)-one (51-b)

At room temperature, to a solution of 51-b (30 mg , 0.11 mmol, 1.0 eq) in EtOH (4 mL) and H₂O (1 mL), K₂CO₃ (46 mg, 0.33 mmol, 3.0 eq) was added. After stirring at 90°C for 2 hours, the resulting material was cooled to room temperature, and H₂O (30 mL) was added. After extraction with ethyl acetate (30 mL x 3), the resulting material was dried over anhydrous Na₂SO₄, filtered, and concentrated. The resulting material was purified by Pre-TLC (PE: EtOAc = 1: 1) to obtain 51-b (19 mg, 98.2%) as a white solid product. LCMS: 191.10 [M+H]⁺.

### 3) (R)-6-(4-((1-(hydroxymethyl)cyclobutyl)amino)-5-oxido-6,7-dihydrothieno[3,2-d]pyrimidin-2-yl)-5,6,7,8-tetrahydrooxazolo[4,5-g]isoquinolin-2(1H)-one (51)

At room temperature, to a solution of 51-b (50.00 mg, 0.24 mmol, 1.0 eq) and I01 (70.0 mg, 0.24 mmol, 1 eq) in IPA (5 mL), DIPEA (92.88 mg, 0.72 mmol, 3.0 eq) was added. After heating under reflux for 48 hours, the resulting material was concentrated. The resulting material was purified by Pre-TLC (SiO₂, DCM: MeOH = 15: 1) to obtain 51 (45.41 mg, 39.2%) as a white solid product. LCMS: 442.05 [M+H]⁺. H NMR (400 MHz, Methanol-d₄) δ 7.04 (s, 1H), 6.87 (s, 1H), 3.92 (s, 4H), 3.54 (s, 1H), 3.36 (s, 1H), 3.05 (s, 2H), 2.88 (s, 2H), 2.33 (s, 4H), 1.88 (s, 2H).

### Example 19: Synthesis of compound 53

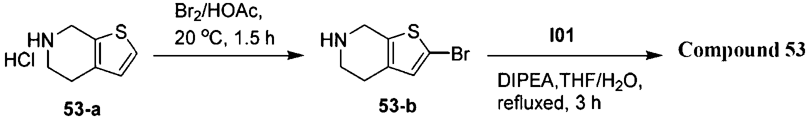

### 1) 2-Bromo-4,5,6,7-tetrahydrothieno[2,3-c]pyridine (53-b)

At room temperature, to a solution of 4,5,6,7-tetrahydrothieno[2,3-c]pyridine hydrogen chloride (50 mg, 0.36 mmol, 1 eq.) in HOAc (2 mL), liquid bromine (63.1 mg, 0.39 mmol, 1.1 eq) was added. After stirring for 1.5 hours, the resulting material was filtered, washed with ethanol (10 mL x 3), and dried to obtain 53-b (50 mg, 63%) as a yellow solid product. LCMS: [M+H⁺] = 218.10

### 2) (R)-2-(2-bromo-4,7-dihydrothieno[2,3-c]pyridin-6(5H)-yl)-4-((1-(hydroxymethyl)cyclobutyl)amino)-6,7-dihydrothieno[3,2-d]pyrimidine 5-oxide (53)

At room temperature, to a solution of I01 (30 mg, 0.10 mmol, 1 eq) and 53-b (34.1 mg, 0.15 mmol, 1.5 equiv.) in THF (2 mL)/H₂O (0.5 mL), DIEPA (40.4 mg, 0.31 mmol, 3 eq) was added. After stirring at 65°C for 3 hours, the resulting material was concentrated. The resulting material was purified by Prep-HPLC (0.1% TFA in ACN/H₂O) to obtain 53 (27.84 mg, 42 %) as a white solid product. LCMS: [M+2]⁺ = 470.45. ¹HNMR: ¹H NMR (399 MHz, DMSO-d₆) δ 7.77 (s, 1H), 6.94 (s, 1H), 4.81 (s, 2H), 3.93 (s, 3H), 3.68 (s, 2H), 3.43 - 3.39 (m, 1H), 3.20-3.14 (m, 1H), 2.99-2.94 (m, 1H), 2.88-2.83 (m, 1H), 2.61 (s, 2H) 2.27-2.16 (m, 4H), 1.75-1.71 (m, 2H).

### Example 20: Synthesis of compound 54

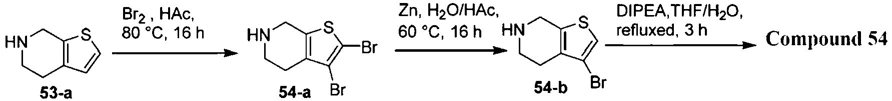

### 1) 2,3-Dibromo-4,5,6,7-tetrahydrothieno[2,3-c]pyridine (54-a)

At room temperature, to a solution of 3-bromo-4,5,6,7-tetrahydrothieno[2,3-c]pyridine (500 mg, 3.59 mmol, 1 eq) in HOAc (20 mL), liquid bromine (126.2 mg, 0.79 mmol, 2.2 equiv.) was added. After stirring at 80°C for 16 hours, the resulting material was cooled to room temperature. After filtration, the filter cake was washed with ethanol (20 mL x 3). The filtrate was concentrated to obtain 54-a (1.0 g, 93%) as a yellow solid product. The crude product was directly used in the next reaction without purification. LCMS: [M+H]⁺ = 297.75

### 2) 3-Bromo-4,5,6,7-tetrahydrothieno[2,3-c]pyridine (54-b)

At room temperature, to a solution of 54-a (100 mg, 0.33 mmol, 1 equiv.) in HOAc (2 mL) and H₂O (2 mL), zinc powder (110 mg, 1.7 mmol, 5 equiv.) was added. After stirring at 60°C for 16 hours, the resulting material was cooled to room temperature. A solid was filtered off. After washing with ethyl acetate (15 mL), water (20 mL) was added. After extraction with ethyl acetate (30 mL x 3), the resulting material was dried over anhydrous Na₂SO₄, filtered, and concentrated. The resulting material was purified by Pre-TLC (DCM: MeOH=10: 1) to obtain 54-b (20 mg, 27%) as a yellow oil product. LCMS: [M+H]⁺ = 219.90.

### 3) (R)-2-(3-bromo-4,7-dihydrothieno[2,3-c]pyridin-6(5H)-yl)-4-((1-(hydroxymethyl)cyclobutyl)amino)-6,7-dihydrothieno[3,2-d]pyrimidine 5-oxide (54)

At room temperature, to a solution of I01 (24 mg, 0.08 mmol, 1 equiv.) and 54-b (18.19 mg, 0.08 mmol, 1 equiv.) in THF (2 mL) /H₂O (0.5 mL), DIPEA (32.3 mg, 0.25 mmol, 3 equiv.) was added. After stirring at 65°C for 3 hours, the resulting material was concentrated. The resulting material was purified by Prep-HPLC (0.1% TFA in ACN/H₂O) to obtain 54 (37.2 mg, 95%) as a white solid product. LCMS: [M+1]⁺ = 470.80. ¹H NMR: ¹H NMR (399 MHz, DMSO-d₆) δ 7.73 (s, 1H), 7.55 (s, 1H), 4.93 (s, 2H), 4.00 (s, 2H), 3.70 (s, 2H), 3.45 - 3.4 (m, 1H), 3.23 - 3.17 (m, 1H), 2.98 - 2.83 (m, 2H), 2.52 (s, 2H), 2.29 - 2.18 (m, 4H), 1.77 - 1.73 (m, 2H).

### Example 21: Synthesis of compound 56

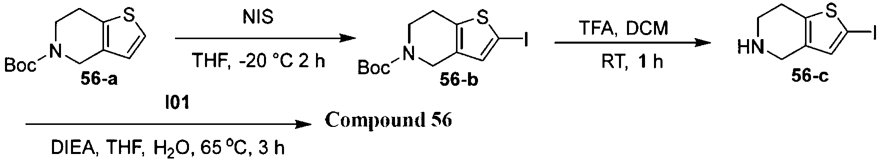

### 1) Tert-butyl 2-iodo-6,7-dihydrothieno[3,2-c]pyridine-5(4H)-carboxylate (56-b)

At -20°C, to a solution of tert-butyl 6,7-dihydrothieno[3,2-c]pyridine-5(4H)-carboxylate (56-a, 200 mg, 0.84 mmol, 1 eq) in THF (5 mL), N-iodosuccinimide (376 mg, 1.67 mmol, 2 eq) was added. After stirring for 2 hours, water (10 mL) was added. After extraction with ethyl acetate (10 mL x 3), the resulting material was dried over anhydrous Na₂SO₄, filtered, and concentrated. The resulting material was purified by column chromatography (PE: EA from 15: 1 to 3: 1) to obtain 56-b (100 mg, 32.8%) as a yellow oil product. LCMS: 366.10 [M+H]⁺.

### 2) 2-Iodo-4,5,6,7-tetrahydrothieno[3,2-c]pyridine (56-c)

At room temperature, to a solution of 56-b (100 mg, 0.27 mmol, 1 eq) in DCM (4 mL), TFA (1 mL) was added. After stirring for 1 hour, the resulting material was concentrated to obtain 56-c (50 mg, 68.9%) as a yellow oil product. The crude product was directly used in the next reaction without purification. LCMS: 266.15 [M+H]⁺.

### 3) (R)-4-((1-(hydroxymethyl)cyclobutyl)amino)-2-(2-iodo-6,7-dihydrothieno[3,2-c]pyridin-5(4H)-yl)-6,7-dihydrothieno[2,2-d]pyrimidine 5-oxide (56)

At room temperature, to a solution of I01 (50 mg, 0.17 mmol, 1 eq) and 56-c (50 mg, 0.19 mmol, 1.1 eq) in THF (4 mL) and H₂O (1 mL), DIEPA (67.37 mg, 0.51 mmol, 3 eq) was added. After stirring at 65°C for 3 hours, the resulting material was concentrated. The resulting material was purified by Prep-HPLC (0.1% TFA in ACN/H₂O) to obtain 56 (16.5 mg, 18.4%) as a white solid product. LCMS: 517.20 [M+H]⁺. ¹H NMR (400 MHz, DMSO- d₆) δ 7.76 (s, 1H), 7.15 (s, 1H), 4.72 (s, 2H), 3.98 (s, 2H), 3.69 (t, J = 2.1 Hz, 2H), 3.41 (dd, J = 17.2, 8.3 Hz, 1H), 3.18 (dd, J = 14.4, 7.6 Hz, 1H), 2.96 (dd, J = 17.2, 8.0 Hz, 1H), 2.86 (dd, J = 13.5, 7.1 Hz, 1H), 2.76 (s, 2H), 2.33 - 2.23 (m, 2H), 2.17 (m, 2H), 1.78 - 1.68 (m, 2H).

### Example 22: Synthesis of compound 57

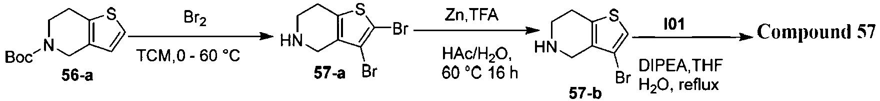

### 1) 2,3-Dibromo-4,5,6,7-tetrahydrothieno[3,2-c]pyridine (57-a)

At 0°C, to a solution of tert-butyl 6,7-dihydrothieno[3,2-c]pyridine-5(4H)-carbamate (56-a, 200 mg, 0.8357 mmol, 1.0 eq) in CHCl₃(5 mL), liquid bromine (267.10 mg, 1.6713 mmol, 2.0 eq) was dropwise added. After stirring at 60°C for 3 hours, the resulting material was cooled to room temperature, and water (20 mL) was added. After extraction with DCM (20 mL x 3), the resulting material was dried over anhydrous Na₂SO₄, filtered, and concentrated. The resulting material was purified by Pre-TLC (SiO₂, PE: EtOAc = 1: 1) to obtain 57-a (180 mg, 87.6%) as a yellow solid product. LCMS: 296.05 [M+H]⁺.

### 2) 3-Bromo-4,5,6,7-tetrahydrothieno[3,2-c]pyridine (57-b)

At room temperature, to a solution of 57-a (150.00 mg, 0.5051 mmol, 1.0 eq) in HOAc (2 mL) and H₂O (2 mL), Zn (66.05 mg, 1.0101 mmol, 2.0 eq) and TFA (172.76 mg, 1.5151 mmol, 3.0 eq) were added successively. After stirring at 60°C for 16 hours, the resulting material was cooled to room temperature. Water (20 mL) was added. After extraction with ethyl acetate (10 mL x 3), the resulting material was dried over anhydrous Na₂SO₄, filtered, and concentrated. The resulting material was purified by Pre-TLC (SiO₂, PE: EtOAc = 1: 1) (PE: EA from 15: 1 to 3: 1) to obtain 57-b (80 mg, 67%) as a yellow solid product. LCMS: 218.10 [M+H]⁺.

### 3) (R)-2-(3-bromo-6,7-dihydrothieno[3,2-c]pyridin-5(4H)-yl)-4-((1-(hydroxymethyl)cyclobutyl)amino)-6,7-dihydrothieno[2,2-d]pyrimidine 5-oxide (57)

At room temperature, to a solution of I01 (80 mg, 0.2777 mmol, 1.0 eq) and 57-b (90.83 mg, 0.4166 mmol, 1.5 eq) in THF/H₂O (4 mL: 1 mL), DIPEA (107.70 mg, 0.8333 mmol, 3.0 eq) was added. After stirring at 65°C for 3 hours, the resulting material was concentrated. The resulting material was purified by Prep-HPLC (0.1% TFA in ACN/H₂O) to obtain 57 (28.5 mg, 60.3%) as a white solid product. LCMS: 469.10 [M+H]⁺. ¹H NMR (400 MHz, DMSO-d₆) δ 7.72 (s, 1H), 7.54 (s, 1H), 4.65 (m, 2H), 4.02 (m, 1H), 3.75 - 3.68 (m, 3H), 3.45 (dt, J = 16.5, 7.8 Hz, 1H), 3.20 (dt, J = 15.1, 8.1 Hz, 1H), 2.98 (dd, J = 17.3, 8.2 Hz, 2H), 2.87 (dd, J = 13.7, 7.2 Hz, 3H), 2.83 (s, 2H), 2.47 (d, J = 9.9 Hz, 1H), 2.36- 2.17 (m, 2H), 1.74 (m, 2H).

### Example 23: Synthesis of compound 60

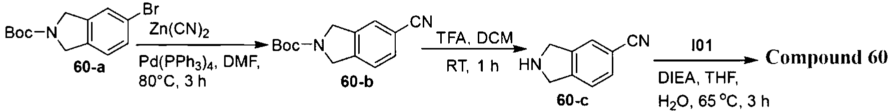

### 3. Tert-butyl 5-cyanoisoindoline-2-carboxylate (60-b)

At room temperature, to a solution of tert-butyl 5-bromoisoindoline-2-carboxylate (60-a, 200 mg, 0.67 mmol, 1 eq) in DMF (5 mL), Zn(CN)₂ (158 mg, 1.34 mmol, 2 eq) and Pd(PPh₃)₄ (155 mg, 0.13 mmol, 0.2 eq) were added successively. After stirring at 80°C for 3 hours, the resulting material was cooled to room temperature, and water (10 mL) was added. After extraction with ethyl acetate (10 mL x 3), the resulting material was dried over anhydrous Na₂SO₄, filtered, and concentrated. The resulting material was purified by Pre-TLC (SiO₂, PE: EtOAc = 10: 1) to obtain 60-b (80 mg, 48.82%) as a white solid product. LCMS: 245.25 [M+H]⁺.

### 2) Isoindoline-5-carbonitrile (60-c)

At room temperature, to a solution of 60-b (80 mg, 0.33 mmol, 1 eq) in DCM (4 mL), TFA (1 mL) was added. After stirring for 1 hour, the resulting material was concentrated to obtain 60-c (40.00 mg, 84.7%) as a yellow oil product. The crude product was directly used in the next reaction without purification. LCMS: 145.30 [M+H]⁺.

### 3) (R)-2-(4-((1-(hydroxymethyl)cyclobutyl)amino)-5-oxido-6,7-dihydrothieno[3,2-d]pyrimidin-2-yl)isoindoline-5-carbonitrile (60)

At room temperature, to a solution of I0 1 (40 mg, 0.14 mmol, 1 eq) and 60-c (25 mg, 0.17 mmol, 1.2 eq) in THF (4 mL) and H₂O (1 mL), DIEPA (55 mg, 0.42 mmol, 3 eq) was added. After stirring at 65°C for 3 hours, the resulting material was concentrated. The resulting material was purified by Prep-HPLC (0.1% TFA in ACN/H₂O) to obtain 60 (47.5 mg, 86.3%) as a white solid product. LCMS: 396.30 [M+H]⁺. ¹H NMR (400 MHz, DMSO- d₆) δ 8.10 (s, 1H), 7.89 (d, J = 4.0 Hz, 1H), 7.77 (dd, J = 7.9, 1.5 Hz, 1H), 7.60 (m, J = 17.8, 7.9 Hz, 1H), 4.84 (t, J = 12.5 Hz, 4H), 3.77 (s, 2H), 3.56 - 3.46 (m, 1H), 3.26 (dt, J = 13.6, 8.2 Hz, 1H), 3.08 (d, J = 16.2 Hz, 1H), 2.93 (dd, J = 13.8, 7.2 Hz, 1H), 2.36 - 2.20 (m, 4H), 1.82 - 1.70 (m, 2H).

### Example 24: Synthesis of compound 64

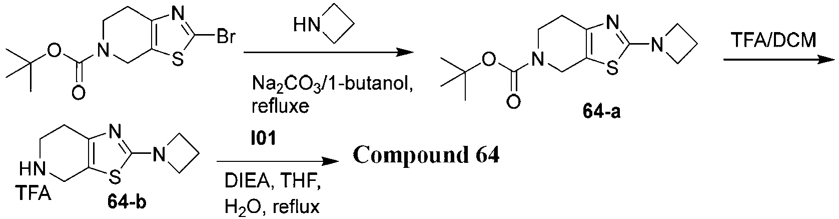

### 1) Tert-butyl 2-(azetidin-1-yl)-6,7-dihydrothiazolo[5,4-c]pyridine-5(4H)-(64-a)

At room temperature, to a solution of tert-butyl 2-bromo-6,7-dihydrothiazolo[5,4]pyridine-5(4H)-carboxylate (500 mg, 1.57 mmol, 1.0 eq) and azetidine (179.3 mg, 3.14 mmol, 2.0 eq) in tert-butanol (5 mL), Na₂CO₃ (499.3 mg, 4.71 mmol, 3.0 eq) was added. After stirring under reflux for 16 hours, the resulting material was cooled to room temperature and concentrated. Water (20 mL) was added. After extraction with ethyl acetate (20 mL x 3), the resulting material was dried over anhydrous Na₂SO₄, filtered, and concentrated. The resulting material was purified by Pre-TLC (SiO₂, PE: EtOAc = 3: 1) to obtain 64-a (280 mg, 76.2%) as a white solid product. LCMS: 296.15 [M+H]⁺.

### 2) 2-(Azetidin-1-yl)-4,5,6,7-tetrahydrothiazolo[5,4-c]pyridine (64-b)

At room temperature, to a solution of 64-a (200 mg, 0.6770 mmol, 1.0 eq) in DCM (4 mL), TFA (1 mL) was added. TFA (1 mL) was added. After stirring for 1 hour, the resulting material was concentrated to obtain 64-b (150.5 mg, 80.5%) as a white solid product. LCMS: 196.10 [M+H]⁺.

### 3) (R)-2-(2-(azetidin-1-yl)-6,7-dihydrothiazolo[5,4-c]pyridin-5(4H)-yl)-4-((1-(hydroxymethyl)cyclobutyl)amino)-6,7dihydrothieno[3,2-d]pyrimidine 5-oxide (64)

At room temperature, to a solution of I01(80.00 mg, 0.2777 mmol, 1.0 eq) and 64-b (81.67 mg, 0.4166 mmol, 1.5 eq) in THF/H₂O (4 mL: 1 mL), DIPEA (107.70 mg, 0.8333 mmol, 3.0 eq) was added. After stirring at 65°C for 4 hours, the resulting material was concentrated. The resulting material was purified by Prep-HPLC (0.1% TFA in ACN/H₂O) to obtain 64 (30.8 mg, 76.5%) as a white solid product. LCMS: 447.30 [M+H]⁺. ¹H NMR (400 MHz, Methanol-d₄) δ 4.33 (t, J = 7.7 Hz, 4H), 4.12 (s, 2H), 3.96 - 3.86 (t, 3H), 3.60 (dt, J = 16.9, 8.0 Hz, 3H), 3.33 (m, 2H), 3.15 - 3.07 (m, 3H), 2.68-2.57 (m, 4H), 2.31 (m, 3H), 1.91-1.86 (m, 2H).

### Example 25: Synthesis of compound 65

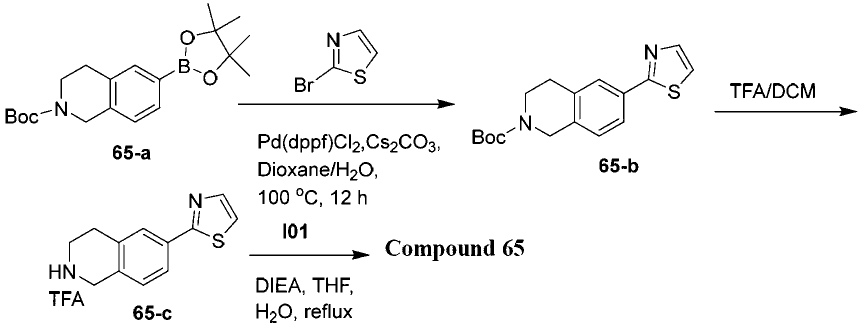

### 1) Tert-butyl 6-(thiazol-2-yl)-3,4-dihydroisoquinoline-2(1H)-carboxylate (65-b)

At room temperature, to a solution of tert-butyl 6-(4,4,5,5-tetramethyl-1,3,2-dioxaboran-2-yl)-3,4-dihydroisoquinoline-2(1H)-carboxylate (65-a, 50 mg, 0.14 mmol, 1 eq), 2-bromothiazole (22.8 mg, 0.14 mmol, 1 eq) and Cs₂CO₃ (136 mg, 0.42 mmol, 3 eq) in H₂O (1 mL)/1,4-dioxane (4 mL), Pd(dppf)Cl₂ (10.00 mg, 0.02 mmol, 0.1 eq) was added. After stirring at 100°C for 16 hours, the resulting material was cooled to room temperature, a solid was filtered off, and water (20 mL) was added. After extraction with ethyl acetate (10 mL x 3), the resulting material was dried over anhydrous Na₂SO₄, filtered, and concentrated. The resulting material was purified by column chromatography (PE: EA from 10: 1 to 4: 1) to obtain 65-b (40 mg, 90.9%) as a yellow solid product. LCMS: 317.15 [M+H]⁺.

### 2) 2-(1,2,3,4-Tetrahydroisoquinolin-6-yl)thiazole (65-c)

At room temperature, to a solution of 65-b (40 mg, 0.13 mmol, 1 eq) in DCM (2 mL), TFA (1 mL) was added. After stirring for 1 hour, the resulting material was concentrated. The resulting material was purified by Pre-HPLC (ACN: 0.1% TFA in H₂O from 5% to 95%) to obtain 65-c (25 mg, 89.3%) as a white solid product. LCMS: 217.10 [M+H]⁺.

### 3) (R)-4-((1-(hydroxymethyl)cyclobutyl)amino)-2-(6-(thiazol-2-yl)-3,4-dihydroisoquinolin-2(1H)-yl)-6,7-dihydrothieno[3,2-d]pyrimidine 5-oxide (65)

At room temperature, to a solution of 65-c (28 mg, 0.13 mmol, 1 eq) and I01 (37 mg, 0.13 mmol, 1 eq) in THF (4 mL), DIEPA (50 mg, 0.39 mmol, 3 eq) and water (1 mL) were added. After stirring at 65°C for 3 hours, the resulting material was concentrated. The resulting material was purified by Prep-HPLC (0.1% TFA in ACN/H₂O) to obtain 65 (34.6 mg, 55.7%) as a white solid product. LCMS: 468.15 [M+H]⁺. ¹H NMR (400 MHz, DMSO-d₆) δ 7.87 (d, J = 3.2 Hz, 1H), 7.78 - 7.72 (m, 3H), 7.32 (d, J = 8.4 Hz, 1H), 4.88 (s, 3H), 3.95 (t, J = 6.0 Hz, 2H), 3.73 (d, J = 2.1 Hz, 2H), 3.45 (d, J = 8.5 Hz, 1H), 3.26 - 3.16 (m, 1H), 3.05 (s, 1H), 2.92 (d, J = 6.3 Hz, 4H), 2.39 - 2.26 (m, 2H), 2.21 (s, 2H), 1.84 - 1.70 (m, 2H).

### Example 26: Synthesis of compound 66

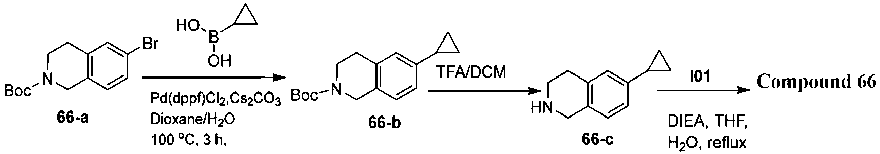

### 1) Tert-butyl 6-cyclopropyl-3,4-dihydroisoquinoline-2(1H)-carboxylate (66-b)

At room temperature, to a mixture of tert-butyl 6-bromo-3,4-dihydroisoquinoline-2(1H)-carboxylate (66-a, 500 mg, 1.60 mmol, 1 eq), cyclopropylboronic acid (138 mg, 1.60 mmol, 1 eq), and Cs₂CO₃ (1.56 g, 4.80 mmol, 3 eq) in H₂O (1 mL)/1,4-dioxane (4 mL), Pd(dppf)Cl₂ (117 mg, 0.16 mmol, 0.1 eq) was added. After stirring at 100°C for 16 hours, the resulting material was cooled to room temperature, a solid was filtered off, and water (20 mL) was added. After extraction with ethyl acetate (30 mL x 3), the resulting material was dried over anhydrous Na₂SO₄, filtered, and concentrated. The resulting material was purified by column chromatography (PE: EA from 15: 1 to 5: 1) to obtain 66-b (200 mg, 45.6%) as a yellow solid product. LCMS: 274.38 [M+H]⁺.

### 2) 6-Cyclopropyl-1,2,3,4-tetrahydroisoquinoline (66-c)

At room temperature, to a solution of 66-b (200 mg, 0.73 mmol, 1 eq) in DCM (2 mL), TFA (1 mL) was added. After stirring for 1 hour, the resulting material was concentrated. The resulting material was purified by Pre-HPLC (ACN: 0.1% TFA in H₂O from 5% to 95%) to obtain 66-c (100 mg, 78.74%) as a white solid product. LCMS: 174.26 [M+H]⁺.

### 3) (R)-2-(6-cyclopropyl-3,4-dihydroisoquinolin-2(1H)-yl)-4-((1-(hydroxymethyl)cyclobutyl)amino)-6,7-dihydrothieno[3,2-d]pyrimidine 5-oxide (66)

At room temperature, to a solution of 66-c (127 mg, 0.46 mmol, 1.5 eq) and I01 (88.00 mg, 0.31 mmol, 1 eq) in THF (4 mL), DIEPA (112.00 mg, 0.93 mmol, 3.0 eq) and water (1 mL) were added. After stirring at 65°C for 3 hours, the resulting material was concentrated. The resulting material was purified by Prep-HPLC (ACN: 0.1% TFA in H₂O from 5% to 95%) to obtain 66 (69.9 mg, 35.9%) as a white solid product. LCMS: 425.20 [M+H]⁺. ¹H NMR (400 MHz, DMSO-d₆) δ 8.19 (s, 1H), 7.03 (d, J = 7.8 Hz, 1H), 6.87 (dd, J = 10.5, 2.4 Hz, 2H), 4.76 (s, 2H), 3.87 (t, J = 6.0 Hz, 2H), 3.72 (s, 2H), 3.49 (dd, J = 17.1, 8.3 Hz, 1H), 3.24 (dt, J = 13.4, 8.2 Hz, 1H), 3.08 (dd, J = 17.5, 8.1 Hz, 1H), 2.92 (dd, J = 13.8, 7.1 Hz, 1H), 2.80 (d, J = 6.1 Hz, 2H), 2.37 - 2.24 (m, 2H), 2.21 (d, J = 11.7 Hz, 2H), 1.85 - 1.69 (m, 3H), 0.86 (dt, J = 8.5, 3.1 Hz, 2H), 0.64 - 0.54 (m, 2H).

### Example 27: Synthesis of compound 68

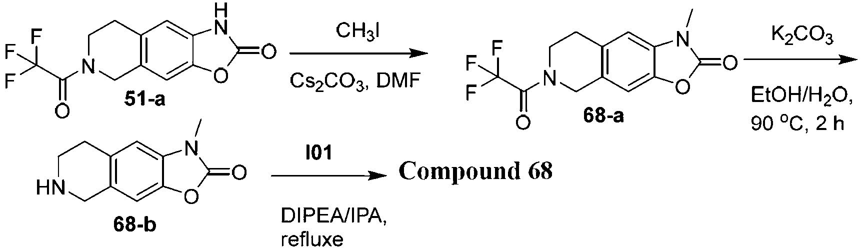

### 1) Methyl-6-(2,2,2-trifluoroacetyl)-5,6,7,8-tetrahydrooxazolo[4,5-g]isoquinolin-2(1H)-one (68-a)

At room temperature, to a solution of 51-a (80 mg, 0.28 mol, 1.0 eq) in DMF (5 mL), Cs₂CO₃ (114 mg, 0.84 mmol, 3.0 eq) and CH₃I (1 mL) were added. After stirring at 50°C for 2 hours, the resulting material was cooled to room temperature, and H₂O (30 mL) was added. After extraction with ethyl acetate (30 mL x 3), the resulting material was dried over anhydrous Na₂SO₄, filtered, and concentrated. The resulting material was purified by column chromatography (PE: EtOAc = 5: 1) to obtain 68-a (60 mg, 71.2%) as a white solid product. LCMS: 301.10 [M+H]⁺.

### 2) 1-Methyl-5,6,7,8-tetrahydrooxazolo[4,5-g]isoquinolin-2(1H)-one (68-b)

At room temperature, to a solution of 68-a (30 mg, 0.1 mmol, 1.0 eq) in EtOH (4 mL) and H₂O (1 mL), K₂CO₃ (41 mg, 0.3 mmol, 3.0 eq) was added. After stirring at 90°C for 2 hours, the resulting material was cooled to room temperature, and H₂O (30 mL) was added. After extraction with ethyl acetate (30 mL x 3), the resulting material was dried over anhydrous Na₂SO₄, filtered, and concentrated. The resulting material was purified by Pre-TLC (PE: EtOAc = 1: 1) to obtain 68-b (19 mg, 92.9%) as a white solid product. LCMS: 205.10 [M+H]⁺.

### 3) (R)-6-(4-((1-(hydroxymethyl)cyclobutyl)amino)-5-oxido-6,7-dihydrothieno[3,2-d]pyrimidin-2-yl)-1-methyl-5,6,7,8-tetrahydrooxazolo[4,5-g]isoquinolin-2(1H)-one (68)

At room temperature, to a solution of 68-b (50.00 mg, 0.24 mmol, 1.0 eq) and I01 (70.0 mg, 0.24 mmol, 1.0 eq) in IPA (5 mL), DIPEA (92.88 mg, 0.72 mmol, 3.0 eq) was added. After heating under reflux for 48 hours, the resulting material was concentrated. The resulting material was purified by Pre-TLC (SiO₂, DCM: MeOH = 15: 1) to obtain 68 (44.2 mg, 39.6%) as a white solid product. LCMS: 456.50 [M+H]⁺. ¹H NMR (400 MHz, Methanol-d₄) δ 7.13 (s, 1H), 7.04 (s, 1H), 3.95 (d, J = 14.5 Hz, 4H), 3.71 (s, 1H), 3.48 (s, 1H), 3.36 (s, 3H), 3.32 (dd, J = 8.4, 1.6 Hz, 1H), 3.28 (s, 2H), 3.17 (ddd, J = 13.9, 7.4, 1.6 Hz, 1H), 3.04 (d, J = 6.6 Hz, 2H), 2.36 (s, 4H), 1.92 (s, 2H).

### Example 28: Synthesis of compound 69

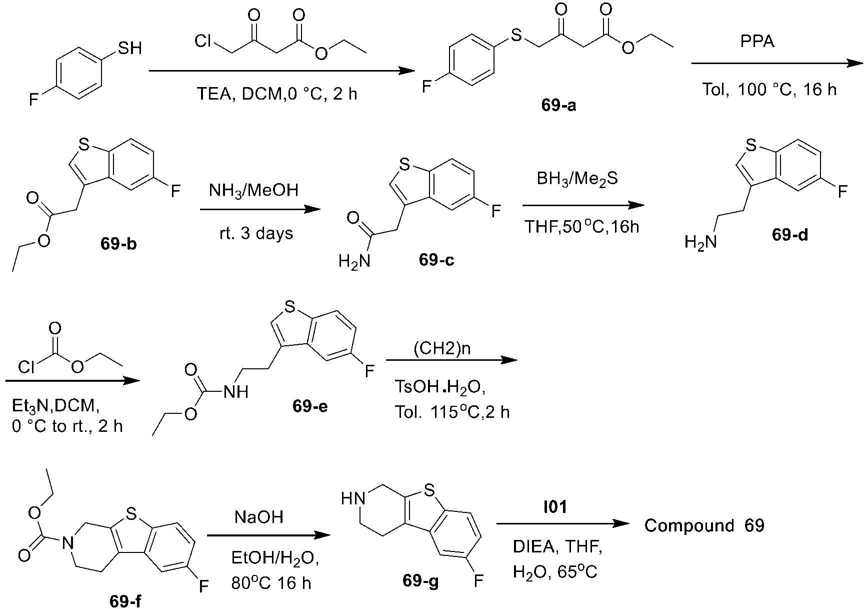

### 1) Ethyl 4-((4-fluorophenyl)thio)-3-acetoacetate (69-a)

At 0°C, to a solution of 4-fluorothiophenol (10 g, 78.12 mmol, 1.0 eq) and ethyl 4-chloro-3-acetoacetate (13 g, 78.12 mmol) in DCM (400 mL), triethylamine (12 g, 117.18 mmol, 1.5 eq) was dropwise added. After continued stirring for 2 hours, the resulting material was poured into water (300 mL). After extraction with DCM (200 mL x 3), the resulting material was dried over anhydrous Na₂SO₄, filtered, and concentrated. The resulting material was purified by column chromatography (hexanes/EtOAc = 10: 1) to obtain 69-a (18 g, 90%) as a yellow oil product. LCMS: 257.10 [M+H]⁺.

### 2) Ethyl 2-(5-fluorobenzo[b]thien-3-yl)acetate (69-b)

At 100°C, to a solution of PPA (21.6 g) in toluene (20 mL), a solution of 69-a (5 g, 19.51 mmol, 1.0 eq) in toluene (5 mL) was added. After stirring for 16 hours, the resulting material was cooled to room temperature and poured into ice water (100 mL), and the pH was adjusted to pH 8 by adding K₂CO₃. After extraction with ethyl acetate (50 mL x 3), the resulting material was dried over anhydrous Na₂SO₄, filtered, and concentrated. The resulting material was purified by column chromatography (PE: EtOAc = 10: 1) to obtain 69-b (2.21 g, 47.5%) as a yellow oil product. LCMS: 239.10 [M+H]⁺.

### 3) 2-(5-Fluorobenzo[b]thien-3-yl)acetamide (69-c)

At room temperature, a solution of 69-b (2.21 g, 9.27 mmol, 1.0 eq) in ammonia in MeOH (7 M, 40 mL, 278 mmol) was stirred for 3 days and then concentrated. The resulting material was purified by column chromatography (DCM/MeOH = 10: 1) to obtain 69-c (1.4 g, 72.1%) as a white solid product. LCMS: 210.05 [M+H]⁺.

### 4) 2-(5-Fluorobenzo[b]thien-3-yl)ethan-1-amine (69-d)

At room temperature, to a solution of 69-c (500 mg, 2.26 mmol, 1.0 eq) in THF (10 mL), 1M borane/Me₂S Complex in THF (5.64 mL, 5.64 mmol, 2.5 eq) was dropwise added. After stirring at 50°C for 16 hours, the resulting material was cooled to 0°C, and methanol (20 mL) was dropwise added slowly. The resulting material was then refluxed for 3 hours. The resulting material was cooled to room temperature and concentrated. 2N NaOH (15 mL) was added. After extraction with ethyl acetate (100 mL x 3), the resulting material was dried over anhydrous Na₂SO₄, filtered, and concentrated. The resulting material was purified by column chromatography (DCM/MeOH = 20: 1) to obtain 69-d (130 mg, 27.8%) as a colorless oil product. LCMS: 196.10 [M+H]⁺.

### 4. Ethyl 2-(5-fluorobenzo[b]thien-3-yl)ethyl)carbamate (69-e)

At 0°C, to a solution of 69-d (100 mg, 0.51 mmol, 1.0 eq) and Et₃N (78 mg, 0.77 mmol, 1.5 eq) in DCM (3 mL), ethyl chloroformate (61 mg, 0.56 mmol, 1.1 eq) was dropwise added. After stirring at room temperature for 2 hours, saturated NH₄Cl (30 mL) was added. After extraction with DCM (20 mL x 3), the resulting material was dried over anhydrous Na₂SO₄, filtered, and concentrated. The resulting material was purified by Pre-TLC (hexanes/ethyl acetate = 10: 1) to obtain 69-e (109 mg, 79.6%) as a colorless oil product. LCMS: 268.10 [M+H]⁺.

### 8) Ethyl 6-fluoro-3,4-dihydrobenzo[4,5]thieno[2,3-c]pyridine-2(1H)-carboxylate (69-f)

The compound 69-e (100 mg, 0.39 mmol, 1.0 eq), paraformaldehyde (22 mg, 0.75 mmol, 2.0 eq), p-toluenesulfonic acid monohydrate (4 mg, 0.019 mmol, 0.05 eq) in toluene (2 mL) were stirred at 115°C under reflux for 2 hours. The resulting material was cooled to room temperature and concentrated. The resulting material was purified by Pre-TLC (hexanes/ethyl acetate = 10: 1) to obtain 69-f(70 mg, 67%) as a colorless oil product. LCMS: 280.10 [M+H]⁺.

### 9) 6-Fluoro-1,2,3,4-tetrahydrobenzo[4,5]thieno[2,3-c]pyridine (69-g)

At room temperature, to a solution of69-f(50 mg, 0.18 mmol, 1.0 eq) in methanol (2 mL) and water (0.5 mL), NaOH (36 mg, 0.89 mmol, 5.0 eq) was added. After stirring at 80°C for 16 hours, the resulting material was cooled to room temperature and concentrated. The resulting material was purified by Pre-HPLC (0.1% TFA in water/ACN from 5% to 95%) to obtain 69-g (20 mg, 18.3%) as a white solid product. LCMS: 208.10 [M+H]⁺.

### 10) (R)-2-(6-fluoro-3,4-dihydrobenzo[4,5]thieno[2,3-c]pyridin-2(1H)-yl)-4-((1-(hydroxymethyl)cyclobutyl)amino)-6,7-dihydrothieno[3,2-d]pyrimidine 5-oxide (69)

At room temperature, to a solution of 69-g (20 mg, 0.097 mmol, 1.0 eq) and I01 (25 mg, 0.087 mmol, 0.9 eq) in THF (1 mL), DIEPA (37 mg, 0.29 mmol, 3.0 eq) and water (0.25 mL) were added. After stirring at 65°C for 16 hours, the resulting material was concentrated. The resulting material was purified by Prep-HPLC (0.1% TFA in ACN/H₂O) to obtain 69 (37.24 mg, 83.8%) as a yellow solid product. LCMS: 459.35 [M+H]⁺. ¹H NMR (400 MHz, DMSO-d₆) δ 7.93 (dd, J = 8.8, 4.9 Hz, 1H), 7.48 (dd, J = 9.9, 2.5 Hz, 1H), 7.17 (td, J = 8.9, 2.5 Hz, 1H), 5.03 (s, 2H), 4.10 (s, 3H), 3.72 (s, 2H), 3.43 (dt, J = 17.1, 8.1 Hz, 1H), 3.19 (dt, J = 15.1, 8.3 Hz, 1H), 3.00 - 2.91 (m, 1H), 2.89 - 2.84 (m, 1H), 2.81 (s, 2H), 2.32 (d, J = 24.7 Hz, 2H), 2.24 - 2.17 (m, 2H), 1.78 (d, J = 9.0 Hz, 2H).

### Example 29: Synthesis of compound 70

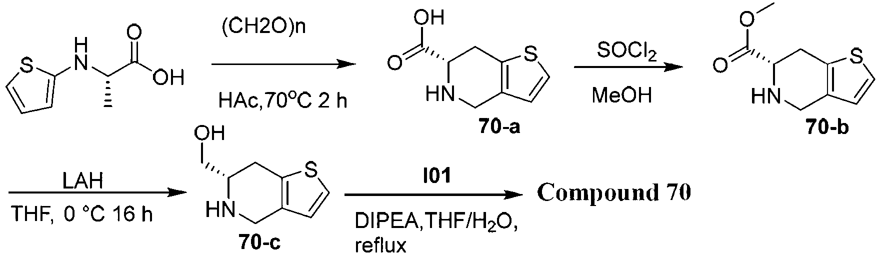

### 1) (S)-4,5,6,7-tetrahydrothieno[3,2-c]pyridine-6-carboxylic acid (70-a)

At room temperature, to a solution of thien-2-yl-L-alanine (1 g, 5.84 mmol, 1.0 equiv.) in HOAc(30 ml), paraformaldehyde (1052.2 mg, 35.04 mmol, 6.0 equiv.) was added. After stirring at 70°C for 2 hours, the resulting material was cooled to room temperature, and saturated NaHCO₃ (20 mL) was added. After extraction with ethyl acetate (30 mL x 3), the resulting material was dried over anhydrous Na₂SO₄, filtered, and concentrated. The resulting material was purified by column chromatography (DCM/MeOH = 20: 1) to obtain 70-a (300 mg, 28%) as a colorless solid product. LCMS: 184.05 [M+H]⁺.

### 3) Methyl (S)-4,5,6,7-tetrahydrothieno[3,2-c]pyridine-6-carboxylate (70-b)

At 0°C, to a solution of 70-a (100 mg, 0.55 mmol, 1 equiv.) in MeOH (2 ml), SOCl₂ (194.8 mg, 1.64 mmol, 3 equiv.) was added. After stirring at 60°C for 5 hours, the resulting material was concentrated to obtain 70-b (100 mg, 92.9%) as a yellow solid product. The crude product was directly used in the next reaction without purification. LCMS: 198.05 [M+H]⁺.

### 4) (S)-(4,5,6,7-tetrahydrothieno[3,2-c]pyridin-6-yl)methanol (70-c)

At 0°C, to a solution of 70-b (100 mg, 0.51 mmol, 1.0 equiv.) in THF (2 mL), LiAlH₄ (0.052 ml, 1.52 mmol, 3.0 equiv.) was added. After continued stirring for 5 hours, water (2 mL) was added, and the resulting material was concentrated. The resulting material was purified by Prep-HPLC (0.1% TFA in ACN/H₂O) to obtain 70-c (40 mg, 0.24 mmol, 46.6%) as a yellow solid product. LCMS: 170.10 [M+H]⁺.

### 5) (R)-2-((S)-6-(hydroxymethyl)-6,7-dihydrothieno[3,2-c]pyridin-5(4H)-yl)-4-((1-(hydroxymethyl)cyclobutyl)amino)-6,7-dihydrothieno[3,2-d]pyrimidine 5-oxide (70)

At room temperature, to a solution of I01 (10 mg, 0.035 mmol, 1 equiv.) and 70-c (5.88 mg, 0.035 mmol, 1.0 equiv.) in THF (3 ml) and H₂O (0.6 ml), DIEPA (89.82 mg, 0.70 mmol, 20 equiv.) was added. After stirring at 65°C for 12 hours, the resulting material was concentrated. The resulting material was purified by Pre-TLC (MeOH: DCM = 1: 10) (0.1% TFA in ACN/H₂O) to obtain 70 (2.30 mg, 17.16%) as a white solid product. LCMS: 421.15 [M+H]⁺. ¹H NMR (400 MHz, DMSO-d₆) δ 7.39 - 7.24 (m, 2H), 6.90 (d, J = 5.0 Hz, 1H), 5.32 (d, J = 18.0 Hz, 1H), 5.20 (s, 1H), 4.80 (s, 2H), 4.07 (d, J = 17.3 Hz, 1H), 3.71 (s, 2H), 3.44 - 3.36 (m, 1H), 3.26 (s, 2H), 3.19 (s, 1H), 3.02 - 2.79 (m, 4H), 2.37 - 2.27 (m, 2H), 2.16 (s, 2H), 1.83 - 1.68 (m, 2H).

### Example 30: Synthesis of compound 71

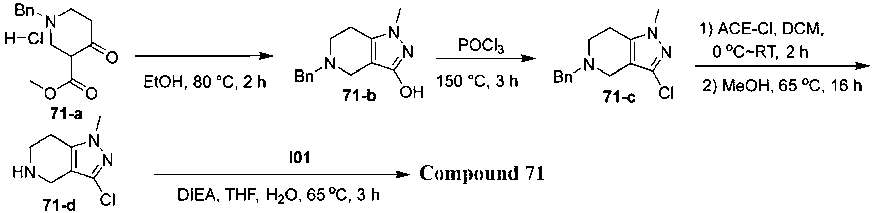

### 1) 5-Benzyl-1-methyl-4,5,6,7-tetrahydro-1H-pyrazolo[4,3-c]pyridin-3-ol (71-b)

The compounds methyl 1-benzyl-4-piperidinone-3-carboxylate hydrochloride (71-a, 2 g, 6.72 mmol, 1 eq) and methylhydrazine sulfate (4.85 g, 33.58 mmol, 5 eq) in EtOH (20 mL) were stirred at 80°C for 2 hours. The resulting material was cooled to room temperature and concentrated. The resulting material was purified by Prep-HPLC (0.1% TFA in ACN/H₂O) to obtain 71-b (1.8 g, 91.5%) as a yellow oil product. LCMS: 244.30 [M+H]⁺.

### 2) 5-Benzyl-3-chloro-1-methyl-4,5,6,7-tetrahydro-1H-pyrazolo[4,3-c]pyridine (71-c)

71-b (1.8 g, 7.39 mmol, 1 eq) was dissolved in POCl₃ (20 mL) and then stirred at 150°C for 3 hours. The resulting material was cooled to room temperature and concentrated. 1 N NaOH (aq. 50 mL) was added. After extraction with ethyl acetate (50 mL x 3), the resulting material was dried over anhydrous Na₂SO₄, filtered, and concentrated. The resulting material was purified by column chromatography (DCM: MeOH = 20: 1) to obtain 71-c (110 mg, 5.7%) as a yellow oil product. LCMS: 262.25 [M+H]⁺.

### 3) 3-Chloro-1-methyl-4,5,6,7-tetrahydro-1H-pyrazolo[4,3-c]pyridine (71-d)

At 0°C, to a solution of 71-c (110 mg, 0.42 mmol, 1 eq) in DCM (5 mL), 1-chloroethyl chloroformate (120 mg, 0.84 mmol, 2 eq) was added. After stirring at room temperature for 2 hours, the resulting material was concentrated. MeOH (5 mL) was then added. After stirring at 65°C for 16 hours, the resulting material was concentrated to obtain 71-d (50 mg, 69.3%) as a yellow solid product. The crude product was directly used in the next reaction without purification. LCMS: 172.25 [M+H]⁺.

### 4) (R)-2-(3-chloro-1-methyl-1,4,6,7-tetrahydro-5H-pyrazolo[4,3-c]pyridin-5-yl)-4-((1-(hydroxymethyl)cyclobutyl)amino)-6,7-dihydrothieno[3,2-d]pyrimidine -5-oxide (71)

At room temperature, to a solution of I01 (50 mg, 0.17 mmol, 1 eq) and 71-d (45 mg, 0.26 mmol, 1.5 eq) in THF (4 mL) and H₂O (1 mL), DIEPA (66 mg, 0.51 mmol, 3 eq) was added. After stirring at 65°C for 3 hours, the resulting material was concentrated. The resulting material was purified by Prep-HPLC (0.1% TFA in ACN/H₂O) to obtain 71 (41.9 mg, 57%) as a yellow solid product. LCMS: 423.20 [M+H]⁺. ¹H NMR (400 MHz, DMSO- d₆) δ 4.64 (s, 2H), 3.96 (d, J = 7.0 Hz, 2H), 3.69 (s, 3H), 3.66 (s, 2H), 3.42 (dt, J = 16.5, 7.7 Hz, 2H), 3.26 - 3.08 (m, 2H), 2.99 - 2.84 (m, 2H), 2.61 (m, J = 6.0 Hz, 2H), 2.35 - 2.24 (m, 2H), 2.14 (m, 2H), 1.75 (q, J = 9.1 Hz, 2H).

### Example 31: Synthesis of compound 72

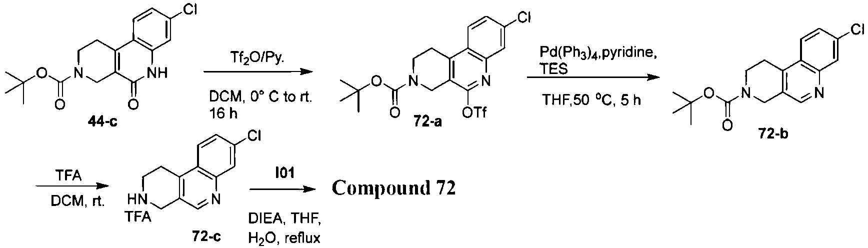

### 1) Tert-butyl 8-chloro-5-((trifluoromethyl)sulfonyl)oxy)-1,4-dihydrobenzo[c][2,7]naphthyridine-3(2H)-carboxylate (72-a)

At 0°C, to a solution of 44-c (330 mg, 0.99 mmol, 1 equiv.) in DCM (10 ml), pyridine (1169.50 mg, 14.79 mmol, 15 equiv.) and Tf₂O (834.29 mg, 2.96 mmol, 3 equiv.) were added successively. After stirring at room temperature for 16 hours, a saturated NH₄Cl solution (20 mL) was added. After extraction with DCM (20 mL x 3), the resulting material was dried over anhydrous Na₂SO₄, filtered, and concentrated. The resulting material was purified by column chromatography (PE: EtOAc from 20: 1 to 5: 1) to obtain 72-a (300 mg, 65.2%) as a yellow solid product. LCMS: 467.10 [M+H]⁺.

### 2) Tert-butyl 8-chloro-1,4-dihydrobenzo[c][2,7]naphthyridine-3(2H)-carboxylate (72-b)

At room temperature, to a solution of 72-a (40 mg, 0.086 mmol, 1 equiv.) in THF (2 ml), pyridine (101.66 mg, 1.29 mmol, 15 equiv.), triethylsilane (149.44 mg, 1.29 mmol, 15 equiv.), and Pd(PPh₃)₄ (19.80 mg, 0.017 mmol, 0.2 equiv.) were added successively. After stirring at 50°C for 5 hours, a solid was filtered off. A filtrate was concentrated. The resulting material was purified by column chromatography (PE: EtOAc from 20: 1 to 5: 1)) to obtain 72-b (20 mg,73.2%) as a yellow solid product. LCMS: 319.15 [M+H]⁺.

### 3) 8-Chloro-1,2,3,4-tetrahydrobenzo[c][2,7]naphthyridine (72-c)

At 0°C, to a solution of 72-b (25 mg, 0.078 mmol, 1 equiv.) in DCM (1.5 ml), TFA (0.5 ml, 5.10 mmol, 65.04 equiv.) was added. After stirring for 1 hour, the resulting material was concentrated to obtain 72-c (69.8 mg, 81.8%) as a white solid product. The crude product was directly used in the next reaction without purification. LCMS: 219.10 [M+H]⁺.

### 4) (R)-2-(8-chloro-1,4-dihydrobenzo[c][2,7]naphthyridin-3(2H)-yl)-4-((1-(hydroxymethyl)cyclobutyl)amino)-6,7-dihydrothieno[3,2-d]pyrimidine 5-oxide (72)

At room temperature, to a solution of I01 (15 mg, 0.052 mmol, 1 equiv.) and 72-c (11.40 mg, 0.052 mmol, 1 equiv.) in THF (3 ml) and H₂O (0.6 ml), DIEPA (67.37 mg, 0.52 mmol, 10 equiv.) was added. After stirring at 65°C for 12 hours, the resulting material was concentrated. The resulting material was purified by Pre-TLC (MeOH: DCM = 1: 10) to obtain 72 (10 mg, 40.8%) as a yellow solid product. LCMS: 470.05 [M+H]⁺. ¹H NMR (400 MHz, DMSO-d₆) 8.80 (s, 1H), 8.10 - 7.99 (m, 2H), 7.63 (dd, J = 9.0, 2.3 Hz, 1H), 7.48 (s, 1H), 5.03 (s, 2H), 4.84 (s, 1H), 4.11 (s, 2H), 3.73 (s, 2H), 3.46 - 3.36 (m, 1H), 3.28 (s, 2H), 3.18 (d, J = 6.0 Hz, 2H), 2.94 (dd, J = 17.0, 8.3 Hz, 1H), 2.84 (dd, J = 13.8, 7.3 Hz, 1H), 2.30 (s, 1H), 2.21 (s, 2H), 1.84 - 1.72 (m, 2H).

### Example 32: Synthesis of compound 73

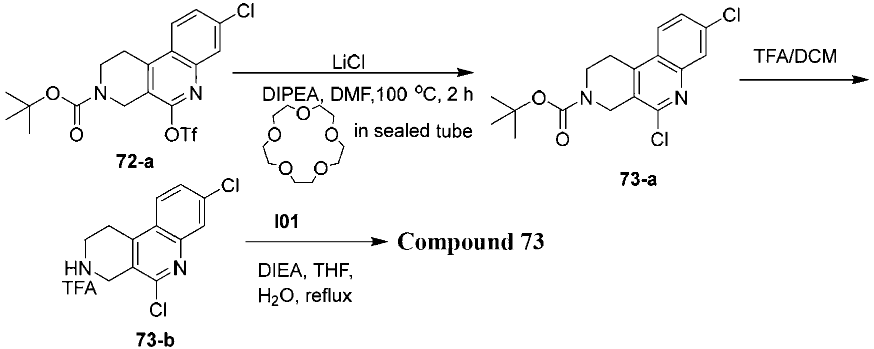

### 1) Tert-butyl 5,8-dichloro-1,4-dihydrobenzo[c][2,7]naphthyridine-3(2H)-carboxylate (73-a)

A solution of 72-a (30 mg, 0.064 mmol, 1 equiv.), DIPEA (132.88 mg, 1.03 mmol, 16 equiv.), LiCl (54.48 mg, 1.29 mmol, 20 equiv.), and 1,4,7,10,13-pentaoxacyclopentadecane (141.54 mg, 0.64 mmol, 10 equiv.) in DMF (1.5 ml) in a sealed tube was stirred at 100°C for 2 hours. The resulting material was cooled to room temperature and concentrated. The resulting material was purified by Pre-TLC (PE: EtOAc = 1: 1) to obtain 73-a (15 mg, 66.1%) as a yellow solid product. LCMS: 353.10 [M+H]⁺.

### 2) 5,8-Dichloro-1,2,3,4-tetrahydrobenzo[c][2,7]naphthyridine (73-b)

At 0°C, to a solution of 73-a (15 mg, 0.042 mmol, 1 equiv.) in DCM (1.5 ml), TFA (0.50 ml) was added. After stirring for 1 hour, the resulting material was concentrated to obtain 72-b (69.8 mg, 81.8%) as a yellow solid product. The crude product was directly used in the next reaction without purification. LCMS: 253.05 [M+H]⁺.

### 3) (R)-2-(5,8-dichloro-1,4-dihydrobenzo[c][2,7]naphthyridin-3(2H)-yl)-4-((1-(hydroxymethyl)cyclobutyl)amino)-6,7-dihydrothieno[3,2-d]pyrimidine 5-oxide (73)

At room temperature, to a solution of I01 (15 mg, 0.052 mmol, 1 equiv.) in THF (3 ml) and H₂O (0.6 ml), 73-b (10.00 mg, 0.040 mmol, 0.8 equiv.) and DIEPA (67.37 mg, 0.52 mmol, 10 equiv.) were added. After stirring at 65°C for 12 hours, the resulting material was concentrated. The resulting material was purified by Pre-TLC (MeOH: DCM = 1: 10) to obtain 73 (8 mg, 30.4%) as a white solid product. LCMS: 504.20 [M+H]⁺. ¹H NMR (400 MHz, DMSO-d₆) δ 8.09 (d, J = 9.0 Hz, 1H), 8.00 (d, J = 2.2 Hz, 1H), 7.68 (dd, J = 9.0, 2.2 Hz, 1H), 7.48 (s, 1H), 4.98 (s, 2H), 4.81 (s, 1H), 4.11 (s, 2H), 3.74 (s, 2H), 3.49 - 3.37 (m, 1H), 3.23 (s, 3H), 3.00 - 2.90 (m, 1H), 2.85 (dd, J = 13.6, 7.2 Hz, 1H), 2.34 (dd, J = 21.4, 11.1 Hz, 2H), 2.20 (s, 2H), 1.85 - 1.72 (m, 2H).

### Example 33: Synthesis of compound 74

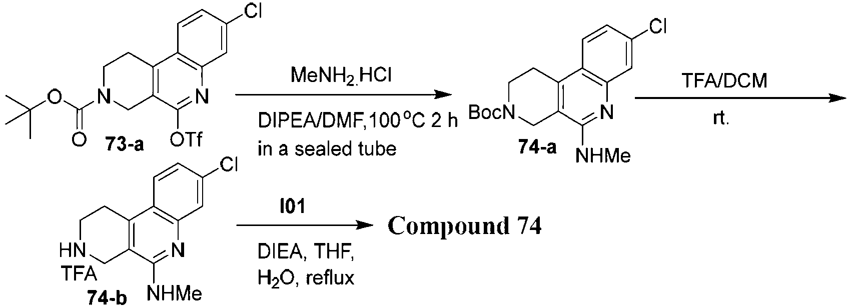

### 1) Tert-butyl 8-chloro-5-(methylamino)-1,4-dihydrobenzo[c][2,7]naphthyridine-3(2H)-carboxylate (74-a)

In a sealed tube, 73-a (40 mg, 0.086 mmol, 1 equiv.), MeNH₂ HCl (46.28 mg, 0.69 mmol, 8 equiv.), DIPEA (177.17 mg, 1.37 mmol, 16 equiv.), and DMF (2 mL) were added successively. After stirring at 100°C for 2 hours, the resulting material was cooled to room temperature and concentrated. The resulting material was purified by Pre-TLC (PE: EtOAc = 1: 1) to obtain 74-a (25 mg, 83.9%) as a yellow solid product. LCMS: 348.15 [M+H]⁺.

### 2) 8-Chloro-N-methyl-1,2,3,4-tetrahydrobenzo[c][2,7]naphthyridin-5-amine (74-b)

At 0°C, to a solution of 74-a (25 mg, 0.072 mmol, 1 equiv.) in DCM (1.5 ml), TFA (0.5 ml) was added. After stirring for 1 hour, the resulting material was concentrated to obtain 74-b (17.2 mg, 97.2%) as a white solid product. The crude product was directly used in the next reaction without purification. LCMS: 248.10 [M+H]⁺.

### 3) (R)-2-(8-chloro-5-(methylamino)-1,4-dihydrobenzo[c][2,7]naphthyridin-3(2H)-yl)-4-((1-(hydroxymethyl)cyclobutyl)amino)-6,7-dihydrothieno[3,2-d]pyrimidine 5-oxide (74)

At room temperature, to a solution of 101 (20 mg, 0.070 mmol, 1.0 equiv.) in THF (3 ml) and H₂O (0.6 ml), 74-b (17.22 mg, 0.070 mmol, 1.0 equiv.) and DIEPA (89.83 mg, 0.70 mmol, 10 equiv.) were added. After stirring at 65°C for 12 hours, the resulting material was concentrated. The resulting material was purified by Pre-TLC (MeOH: DCM = 1: 10) to obtain 74 (10 mg, 28.8%) as a white solid product. LCMS: 499.25 [M+H]⁺. ¹H NMR (399 MHz, DMSO-d₆) δ 7.70 (d, J = 8.8 Hz, 1H), 7.49 (d, J = 2.2 Hz, 1H), 7.41 (s, 1H), 7.18 - 7.10 (m, 1H), 6.75 (s, 1H), 4.80 (d, J = 20.1 Hz, 1H), 4.69 (d, J = 17.3 Hz, 1H), 4.16 - 4.07 (m, 1H), 4.01 (s, 1H), 3.74 (s, 2H), 3.40 (dt, J = 16.3, 7.8 Hz, 1H), 3.26 (s, 1H), 3.17 (dt, J = 15.4, 8.3 Hz, 1H), 3.01 - 2.90 (m, 5H), 2.83 (dd, J = 13.6, 7.2 Hz, 1H), 2.31 (d, J = 14.3 Hz, 2H), 2.20 (s, 2H), 1.79 (s, 2H).

### Example 34: Synthesis of compound 76

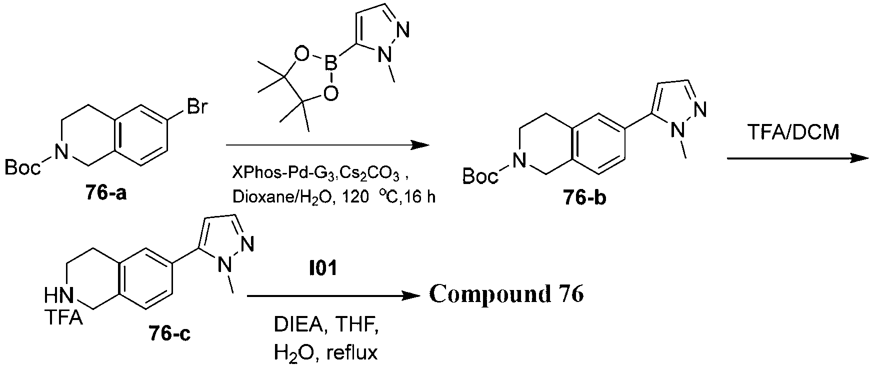

### 1) Tert-butyl 6-(1-methyl-1H-pyrazol-5-yl)-3,4-dihydroisoquinoline-2(1H)-carboxylate (76-b)

At room temperature, to a mixture of tert-butyl 6-bromo-3,4-dihydroisoquinoline-2(1H)-carboxylate (76-a, 100 mg, 0.32 mmol, 1 eq), methyl-5-(4,4,5,5-tetramethyl-1,3,2-dioxaboran-2-yl)-1H-pyrazole (66.7 mg, 0.32 mmol, 1 eq), and Cs₂CO₃ (312 mg, 0.96 mmol, 3 eq) in H₂O (1 mL)/1,4-dioxane (4 mL), XPhos-Pd-G₃ (28.00 mg, 0.03 mmol, 0.1 eq) was added. After stirring at 120°C for 16 hours, the resulting material was cooled to room temperature, a solid was filtered off, and water (20 mL) was added. After extraction with ethyl acetate (30 mL x 3), the resulting material was dried over anhydrous Na₂SO₄, filtered, and concentrated. The resulting material was purified by column chromatography (PE: EA from 10: 1 to 4: 1) to obtain 76-b (80 mg, 79.62%) as a yellow solid product. LCMS: 314.20 [M+H]⁺.

### 2) 6-(1-Methyl-1H-pyrazol-5-yl)-1,2,3,4-tetrahydroisoquinoline (76-c)

At room temperature, to a solution of 76-b (80 mg, 0.26 mmol, 1 eq) in DCM (2 mL), TFA (1 mL) was added. After stirring for 1 hour, the resulting material was concentrated. The resulting material was purified by Pre-HPLC (ACN: 0.1% TFA in H₂O from 5% to 95%) to obtain 76-c (69.8 mg, 81.8%) as a white solid product. LCMS: 214.15 [M+H]⁺.

### 3) (R)-4-((1-(hydroxymethyl)cyclobutyl)amino)-2-(6-(1-methyl-1H-pyrazol-5-yl)-3,4-dihydroisoquinolin-2(1H)-yl)-6,7-dihydrothieno[3,2-d]pyrimidine 5-oxide (76)

At room temperature, to a solution of 76-c (69.8 mg, 0.33 mmol, 1 eq) and I01 (94 mg, 0.33 mmol, 1 eq) in THF (4 mL), DIEPA (127.70 mg, 0.99 mmol, 3.0 eq) and water (1 mL) were added. After stirring at 65°C for 3 hours, the resulting material was concentrated. The resulting material was purified by Prep-HPLC (0.1% TFA in ACN/H₂O) to obtain 76 (65.7 mg, 42.8%) as a yellow solid product. LCMS: 465.20 [M+H]⁺. ¹H NMR (400 MHz, DMSO-d₆) δ7.42 (d, J = 1.9 Hz, 1H), 7.33 (d, J = 4.8 Hz, 3H), 6.33 (d, J = 1.9 Hz, 1H), 4.89 (s, 2H), 3.95 (t, J = 5.9 Hz, 2H), 3.81 (s, 3H), 3.74 (s, 2H), 3.51 (dt, J = 16.5, 7.7 Hz, 1H), 3.26 (dt, J = 15.0, 8.2 Hz, 1H), 3.09 (dd, J = 17.6, 8.2 Hz, 1H), 2.93 (d, J = 6.3 Hz, 3H), 2.38 - 2.14 (m, 4H), 1.85 - 1.70 (m, 2H).

The following compounds were synthesized by similar methods.

| Compound | MS (M+H)⁺ | NMR |
|---|---|---|
| 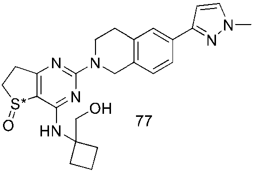 | 465.25 | ¹H NMR (400 MHz, DMSO) δ 7.67 (d, J = 2.3 Hz, 1H), 7.58 (d, J = 4.5 Hz, 2H), 7.19 (d, J = 8.3 Hz, 1H), 6.62 (d, J = 2.2 Hz, 1H), 4.84 (s, 2H), 3.93 (t, J = 6.0 Hz, 2H), 3.83 (s, 3H), 3.73 (d, J = 1.9 Hz, 2H), 3.47 (dd, J = 17.0, 8.3 Hz, 1H), 3.22 (dd, J = 14.9, 7.3 Hz, H), 3.03 (dd, J = 16.9, 8.2 Hz, 1H), 2.88 (t, J = 6.3 Hz, 3H), 2.45 - 2.13 (m, 6H), 1.82 - 1.71 (m, 2H). |

### Example 35: Synthesis of compound 80

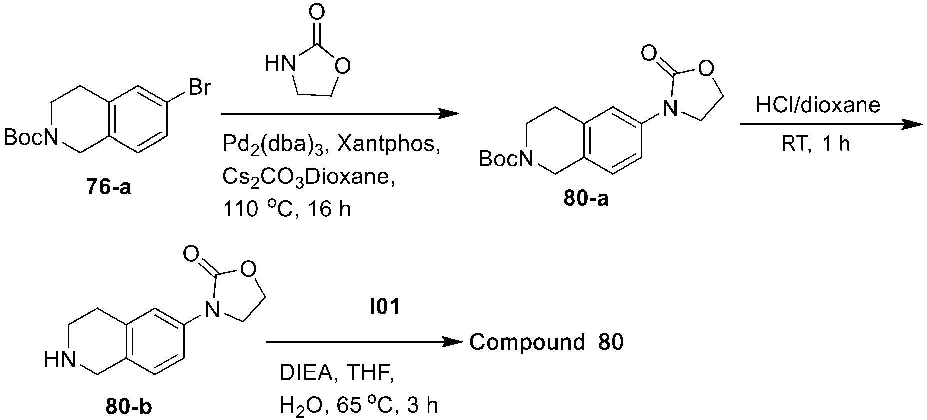

### 1) Tert-butyl 6-(2-oxooxazolidin-3-yl)-3,4-dihydroisoquinoline-2(1H)-carboxylate (80-a)

At room temperature, to a solution of tert-butyl 6-bromo-3,4-dihydroisoquinoline-2(1H)-carbamate (76-a, 200 mg, 0.64 mmol, 1 eq) in 1,4-dioxane (20 mL), 2-oxazolidinone (112 mg, 1.28 mmol, 2 eq), Pd₂(dba)₃ (59 mg, 0.06 mmol, 0.1 eq), Xantphos (37 mg, 0.06 mmol, 0.1 eq), and Cs₂CO₃ (1.30 g, 3.84 mmol, 6 eq) were added successively. After stirring at 110°C for 16 hours, the resulting material was cooled to room temperature, a solid was filtered off, and water (20 mL) was added. After extraction with ethyl acetate (30 mL x 3), the resulting material was dried over anhydrous Na₂SO₄, filtered, and concentrated. The resulting material was purified by Pre-TLC (SiO₂, petroleum ether: ethyl acetate = 1: 1) to obtain 80-a (200 mg, 98.1%) as a white solid product. LCMS: 319.15 [M+H]⁺.

### 2) Tert-butyl 6-bromo-3,4-dihydroisoquinoline-2(1H)-carboxylate (80-b)

At room temperature, a solution of 80-a (200 mg, 0.63 mmol, 1 eq) in 4 M HCl/1,4-dioxane (4 mL) was stirred for 1 hour. The resulting material was concentrated to obtain 80-b (120 mg, 87.5%) as a yellow solid product. The crude product was directly used in the next reaction without purification. LCMS: 219.15 [M+H]⁺.

### 3) (R)-3-(2-(4-((1-(hydroxymethyl)cyclobutyl)amino)-5-oxido-6,7-dihydrothieno[3,2-d]pyrimidin-2-yl)-1,2,3,4-tetrahydroisoquinolin-6-yl)oxazolidin-2-one (80)

At room temperature, to a solution of I01 (40 mg, 0.14 mmol, 1 eq) and 80-b (36.40 mg, 0.17 mmol, 1.2 eq) in THF (4 mL) and H₂O (1 mL), DIEPA (55.00 mg, 0.42 mmol, 3 eq) was added. After stirring at 65°C for 3 hours, the resulting material was concentrated. The resulting material was purified by Prep-HPLC (0.1% TFA in ACN/H₂O) to obtain 80 (64 mg, 98%) as a yellow solid product. LCMS: 470.25 [M+H]⁺. ¹H NMR (400 MHz, DMSO- d₆) δ 7.40 (dd, J = 8.4, 2.4 Hz, 1H), 7.34 (d, J = 2.4 Hz, 1H), 7.20 (d, J = 8.5 Hz, 1H), 4.80 (s, 2H), 4.39 (dd, J = 9.1, 6.9 Hz, 2H), 4.00 (dd, J = 9.0, 7.0 Hz, 2H), 3.90 (t, J = 5.9 Hz, 2H), 3.76 - 3.68 (m, 2H), 3.46 (dd, J = 16.9, 8.2 Hz, 1H), 3.21 (dd, J = 15.0, 7.1 Hz, 1H), 3.03 (dd, J = 17.4, 8.1 Hz, 1H), 2.87 (dt, J = 19.7, 6.3 Hz, 3H), 2.35 - 2.16 (m, 4H), 1.77 (m, J = 8.8 Hz, 2H).

### Example 36: Synthesis of compound 104

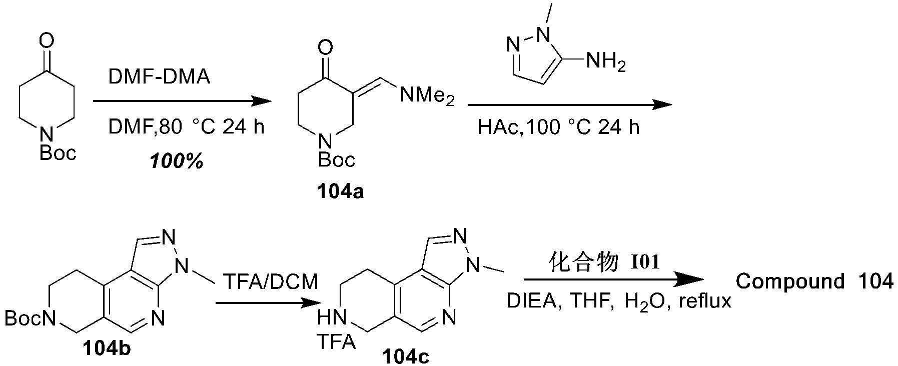

### 1) Synthesis of compound 104a

At room temperature, to a solution of tert-butyl 4-oxopiperidine-1-carbamate (2.00 g, 10.04 mmol, 1 eq) in DMF (2 mL), N,N-dimethylformamide dimethyl acetal (1.20 g, 10.04 mmol, 1 eq) was added. After stirring at 80°C for 24 hours, the resulting material was cooled to room temperature, a solid was filtered off, and water (20 mL) was added. After extraction with ethyl acetate (30 mL x 2), the resulting material was dried over anhydrous Na₂SO₄, filtered, and concentrated. The resulting material was purified by column chromatography (PE: EA from 15: 1 to 3: 1) to obtain 104a (1.56 g, 61.11%) as a yellow solid product. LCMS: 255.15 [M+H]⁺.

### 2) Synthesis of compound 104b

At room temperature, to a solution of 104a (1.56 g, 6.13 mmol, 1 eq) in acetic acid (50 mL), 2-methylpyrazol-3-amine (595.72 mg, 6.13 mmol, 1 equiv.) was added. After stirring at 100°C for 24 hours, the resulting material was cooled to room temperature and concentrated. The resulting material was purified by Pre-HPLC (ACN: 0.1% TFA in H₂O from 5% to 95%) to obtain 104b (750.00 mg, 42.40%) as a white solid product. LCMS: 289.15 [M+H]⁺.

### 3) Synthesis of compound 104c

At room temperature, to a solution of 104b (200.00 mg, 0.69 mmol, 1 eq) in DCM (2 mL), TFA (1 mL) was added. After stirring at room temperature for 1 hours, the resulting material was concentrated. The resulting material was purified by Pre-HPLC (ACN: 0.1% TFA in H₂O from 5% to 95%) to obtain 104c (50.00 mg, 38.29%) as a white solid product. LCMS: 189.22 [M+H]⁺.

### 4) Synthesis of (R)-4-((1-(hydroxymethyl)cyclobutyl)amino)-2-(3-methyl-3,6,8,9-tetrahydro-7H-pyrazolo[3,4-c][2,7]naphthyridin-7-yl)-6,7-dihydrothieno[3,2-d]pyrimidine 5-oxide (104)

At room temperature, to a solution of 104c (43.72 mg, 0.21 mmol, 1 eq) and I01 (60.00 mg, 0.21 mmol, 1 eq) in THF (4 mL), N,N-diisopropylethylamine (80.84 mg, 0.63 mmol, 3 eq) and water (1 mL) were added. After stirring at 65°C for 16 hours, the resulting material was concentrated. The resulting material was purified by Pre-HPLC (ACN: 0.1% TFA in H₂O from 5% to 95%) to obtain Compound 104 (51.00 mg, 53.06%). LCMS: 440.15 [M+H]⁺. ¹H NMR (400 MHz, DMSO-d₆) δ 8.08 (s, 1H), 8.04 (s, 1H), 4.98 (s, 2H), 4.00 (d, J = 15.0 Hz, 6H), 3.74 (s, 2H), 3.47 (dd, J = 17.0, 8.2 Hz, 1H), 3.26 - 3.19 (m, 1H), 3.12 (s, 2H), 3.09 - 3.02 (m, 1H), 2.90 (dd, J = 13.5, 7.1 Hz, 1H), 2.38 - 2.17 (m, 5H), 1.82 - 1.75 (m, 2H).

The following compounds were synthesized by similar methods.

| Compound | MS (M+H)⁺ | NMR |
|---|---|---|
| 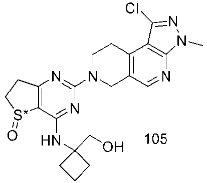 | 474.15 | ¹H NMR (400 MHz, CD₃OD) δ 7.93 (s, 1H), 5.08 (s, 2H), 4.16 - 4.11 (m, 2H), 4.01 (s, 3H), 3.99 - 3.90 (m, 2H), 3.63 - 3.52 (m, 1H), 3.42 - 3.33 (m, 1H), 3.20 - 3.16 (m, 2H), 3.11 - 3.02 (m, 2H), 2.39 - 2.33 (m, 4H), 1.98 - 1.88 (m, 2H). |

### Example 37: Synthesis of compound 113

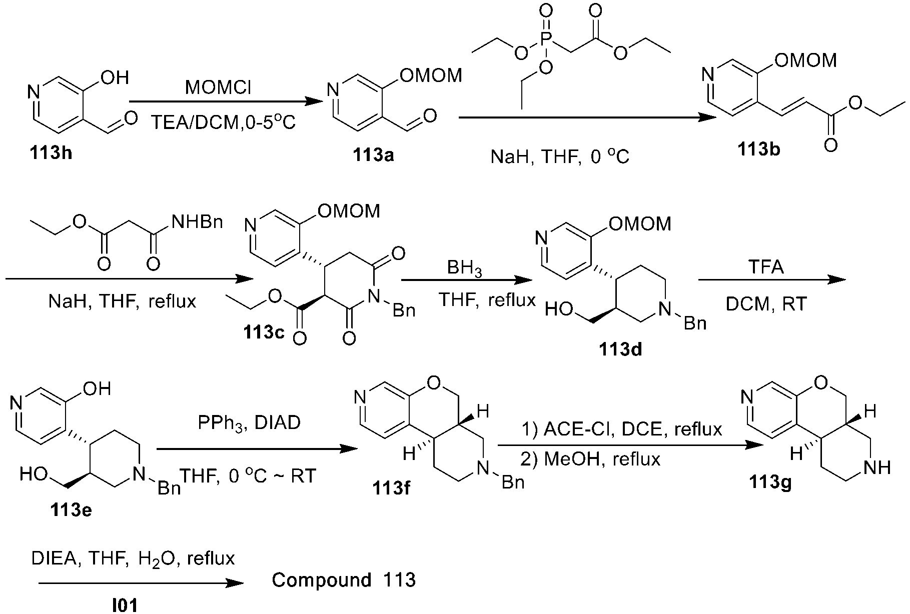

### 1) Synthesis of compound 113a

At 0°C and under nitrogen protection, to a solution of Compound 113h (10 g, 81.30 mmol, 1.0 equiv.) in DCM (100 ml), chloro-(methoxy)methane (6.6 g, 81.30 mmol, 1.0 equiv.) and triethylamine (41.05 g, 406.5 mmol, 5.0 equiv.) were added successively. After continued stirring at 0°C for 1 hour, water (150 mL) was added. After extraction with DCM (200 mL x 2), the resulting material was dried over anhydrous Na₂SO₄, filtered, and concentrated. The resulting material was purified by column chromatography (PE: EtOAc = 10: 1) to obtain 113a (7.02 g, 42.03 mmol, 51.73%) as a yellow oil product. LCMS: 168.05 [M+H]⁺.

### 2) Synthesis of compound 113b

At 0°C and under nitrogen protection, to a solution of ethyl 2-(diethoxyphosphoryl)acetate (10 g, 81.30 mmol, 1.0 equiv.) in THF (100 ml), NaH (1.7 g, 42.03 mmol, 1 equiv.) was added in portions. After continued stirring at 0°C for 30 minutes, a solution of 113a (9.4 g, 42.03 mmol, 1.0 equiv.) in THF (15 ml) was dropwise added. After continued stirring at 0°C for 1 hour, a saturated NH₄Cl aqueous solution (100 mL) was added. After extraction with EtOAc (150 mL x 3), the resulting material was dried over anhydrous Na₂SO₄, filtered, and concentrated. The resulting material was purified by column chromatography (PE: EtOAc = 10: 1) to obtain 113b (8 g, 33.75 mmol, 80.29%) as a yellow oil product. LCMS: 238.10 [M+H]⁺.

### 3) Synthesis of compound 113c

At room temperature, to a solution of 113b (1 g, 4.21 mmol, 1.0 equiv.) and ethyl 3-(benzylamino)-3-oxopropanoate (930.41 mg, 4.21 mmol, 1.0 eq.) in THF (30 mL), NaH (336.8 mg, 8.42 mmol, 2.0 equiv.) was added. After stirring at 70°C for 1 hour, the resulting material was cooled to room temperature, and saturated NH₄Cl (30 mL) was added. After extraction with EtOAc (35 mL x 3), the resulting material was dried over anhydrous Na₂SO₄, filtered, and concentrated. The resulting material was purified by column chromatography (PE: EtOAc = 1: 1) to obtain 113c (600 mg, 1.45 mmol, 34.52%) as a yellow solid product. LCMS: 413.15 [M+H]⁺.

### 4) Synthesis of compound 113d

At room temperature, to a solution of 113c (470 mg, 1.14 mmol, 1.0 eq.) in THF (20 mL), borane (11.44 ml, 11.44 mmol, 10 equiv.) was dropwise added. After continued stirring at 70°C for 3 hours, the resulting material was cooled to 0°C. The reaction was quenched with methanol (30 mL). The resulting material was then stirred at 60°C for 3 hours and cooled to room temperature. Water (50 mL) was added. After extraction with EtOAc (30 mL x 3), the resulting material was dried over anhydrous Na₂SO₄, filtered, and concentrated. The resulting material was purified by column chromatography (PE: EtOAc = 1: 1) to obtain 113d (100 mg, 0.29 mmol, 25.64%) as a yellow solid product. LCMS: 343.20 [M+H]⁺.

### 5) Synthesis of compound 113e

At room temperature, to a solution of 113d (220 mg, 0.64 mmol, 1.0 equiv.) in DCM (3 mL), TFA (1 ml, 9.58 mmol, 14.98 equiv.) was added. After continued stirring for 3 hours, the resulting material was concentrated. EtOAc (30 mL) and saturated NaHCO₃ (20 mL) were added. After extraction with EtOAc (30 mL x 3), the resulting material was dried over anhydrous Na₂SO₄, filtered, and concentrated. The resulting material was purified by column chromatography (PE: EtOAc = 1: 1) to obtain 113e (190 mg, 0.63mmol, 98.43%) as a yellow solid product. LCMS: 299.17 [M+H]⁺.

### 6) Synthesis of compound 113f

At 0°C and under nitrogen protection, to a solution of 113e (210 mg, 0.70 mmol, 1.0 equiv.) in THF (10 mL), PPh₃ (917 mg, 3.5 mmol, 5.0 eq.) and DIAD (707 mg, 3.5 mmol, 5.0 equiv.) were added successively. After stirring at room temperature for 16 hours, EtOAc (30 mL), water (30 mL x 2), and saturated brine (30 mL) were added. After extraction with EtOAc (30 mL x 3), the resulting material was dried over anhydrous Na₂SO₄, filtered, and concentrated. The resulting material was purified by column chromatography (PE: EtOAc = 3: 1) to obtain 113f (80 mg, 0.28 mmol, 40.00%) as a yellow solid product. LCMS: 281.16 [M+H]⁺.

### 7) Synthesis of compound 113g

At 0°C and under nitrogen protection, to a solution of 113f (80 mg, 0.28 mmol, 1.0 equiv.) in 1,2-DCE (3 mL), ACE-Cl (73.89 mg, 0.56 mmol, 2.0 equiv.) was added. After stirring at room temperature for 1 hours, the resulting material was concentrated. MeOH (10 mL) was added, and the resulting material was heated under reflux for 12 hours. The resulting material was cooled to room temperature, and saturated NaHCO₃ (10 mL) was added. After extraction with EtOAc (20 mL x 3), the resulting material was dried over anhydrous Na₂SO₄, filtered, and concentrated. The resulting material was purified by column chromatography (PE: EtOAc =1: 1) to obtain 113g (30 mg, 0.15 mmol, 53.6%) as a yellow solid product. LCMS: 191.10 [M+H]⁺.

### 8) Synthesis of (R)-4-((1-(hydroxymethyl)cyclobutyl)amino)-2-((6aR,10aS)-6a,9,10,10-tetrahydro-6H-pyrano[2,3-c:5,4-c']dipyridin-8(7H)-yl)-6,7-dihydrothieno[3,2-d]pyrimidine 5-oxide (113)

At room temperature, to a solution of I01(100 mg, 0.34 mmol, 1.0 equiv.) and 113g (86.06 mg, 0.45 mmol, 1.3 equiv.) in THF (3 ml) and H₂O (0.6 ml), DIEA (438.6 mg, 3.4 mmol, 10 equiv.) was added. After stirring at 65°C for 12 hours, the resulting material was cooled to room temperature. Water (10 mL) was added. After extraction with EtOAc (20 mL x 3), the resulting material was dried over anhydrous Na₂SO₄, filtered, and concentrated. The resulting material was purified by column chromatography (MeOH: DCM = 1: 10) to obtain a mixed product. After further purification by SFC, Compound 113 (42.69 mg, 0.096 mmol, 28.45%) was obtained. LCMS: 442.30 [M+H]⁺. ¹H NMR (400 MHz, DMSO-d₆) δ 8.05 - 7.99 (m, 2H), 7.37 (s, 1H), 7.18 (d, J = 4.9 Hz, 1H), 4.85 (s, 1H), 4.83 - 4.70 (m, 2H), 4.30 (s, 1H), 3.91 (d, J = 11.2 Hz, 1H), 3.69 (d, J = 5.7 Hz, 2H), 3.36 (dd, J = 16.6, 7.9 Hz, 1H), 3.16 (d, J = 13.8 Hz, 1H), 2.90 (ddd, J = 30.1, 23.8, 12.8 Hz, 4H), 2.79 (d, J = 2.9 Hz, 1H), 2.62 (d, J = 12.3 Hz, 1H), 2.40 (d, J = 12.5 Hz, 1H), 2.29 (dd, J = 24.6, 11.2 Hz, 2H), 2.15 (s, 2H), 1.82 - 1.69 (m, 2H), 1.64 (s, 1H).

The following compounds were synthesized by similar methods.

| Compound | MS (M+H)⁺ | NMR |
|---|---|---|
| 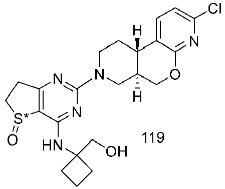 | 476.30 | ¹H NMR (400 MHz, DMSO-d₆) δ 7.64 (d, J = 7.9 Hz, 1H), 7.38 (s, 1H), 6.99 (d, J = 7.8 Hz, 1H), 4.81 (t, J = 5.7 Hz, 3H), 4.38 (s, 1H), 4.05 (t, J = 11.2 Hz, 1H), 3.76 - 3.64 (m, 2H), 3.43 - 3.33 (m, 1H), 3.17 (dd, J = 14.0, 7.8 Hz, 1H), 2.93 (q, J = 10.3, 7.4 Hz, 2H), 2.86 - 2.73 (m, 2H), 2.62 (t, J = 12.0 Hz, 1H), 2.31 (m, J = 33.8, 11.5, 9.2 Hz, 3H), 2.15 (s, 2H), 1.80 - 1.69 (m, 2H), 1.60 (d, J = 11.8 Hz, 1H), 1.18 (d, J = 13.3 Hz, 1H). |

### Example 38: Synthesis of compound 114

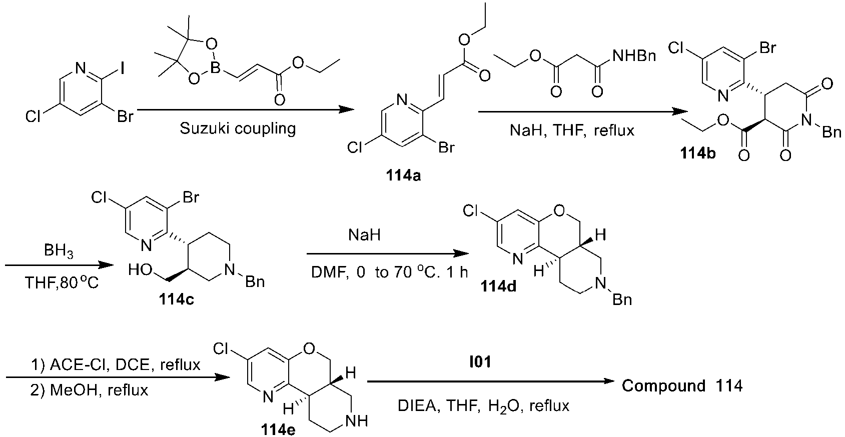

### 1) Synthesis of compound 114a

At room temperature, to a solution of 3-bromo-5-chloro-2-iodopyridine (2 g, 6.28 mmol, 1.0 eq) and ethyl (E)-3-(4,4,5,5-tetramethyl-1,3,2-dioxaboran-2-yl)acrylate (1.42g, 6.28 mmol, 1.0 eq) in THF (20 mL), K₂CO₃ (2.59 g, 18.84 mmol, 3.0 eq), [1,1'-bis(diphenylphosphino)ferrocene]palladium(II) dichloride (453.22 mg, 0.62 mmol,0.1 eq), and water (5 mL) were added successively. Under nitrogen, the resulting material was heated under reflux for 6 h. The resulting material was cooled to room temperature, and a solid was filtered off. After extraction with EtOAc (20 mL x 3), the resulting material was dried over anhydrous Na₂SO₄, filtered, and concentrated. The resulting material was purified by column chromatography (PE: EA from 15: 1 to 5: 1) to obtain 114a (1.7 g, 6.89 mmol, 93.34%) as a white solid product. LCMS: 290.54 [M+H]⁺.

### 2) Synthesis of compound 114b

At room temperature, to a solution of 114a (2 g, 6.89 mmol, 1.0 equiv.) in THF (30 ml), ethyl 3-(benzylamino)-3-oxopropanoate (1.52 g, 6.89 mmol, 1.0 eq.) and NaH (551.2 mg, 13.78 mmol, 2.0 equiv.) were added. The resulting material was heated to 70°C and stirred for 1 hour. The resulting material was cooled to room temperature, and saturated NH₄Cl (30 mL) was added. After extraction with EtOAc (30 mL x 3), the resulting material was dried over anhydrous Na₂SO₄, filtered, and concentrated. The resulting material was purified by column chromatography (PE: EtOAc =1: 1) to obtain 114b (2 g, 4.30 mmol, 62.40%) as a yellow solid product. LCMS: 465.01 [M+H]⁺.

### 3) Synthesis of compound 114c

At 0°C and under nitrogen protection, to a solution of 114b (2 g, 4.30 mmol, 1.0 eq.) in anhydrous THF (20 ml, 100.0%), borane (4.3 ml, 43.0 mmol, 10 equiv.) was dropwise added. The resulting material was heated to 80°C and stirred for 3 hours. After cooling to 0°C, MeOH (30 mL) was added carefully and then stirred at 60°C for 3 hours. Water (30 mL) was added. After extraction with EtOAc (30 mL x 3), the resulting material was dried over anhydrous Na₂SO₄, filtered, and concentrated. The resulting material was purified by column chromatography (PE: EtOAc = 1: 1) to obtain 114c (200 mg, 0.50 mmol, 11.7%) as a yellow solid product. LCMS: 395.05 [M+H]⁺.

### 4) Synthesis of compound 114d

At 0°C and under nitrogen protection, to a solution of 114c (200 mg, 0.50 mmol, 1.0 equiv.) in THF (30 ml), NaH (38.3 mg, 1.0 mmol, 2.0 equiv. 60% wt) was added. After stirring at 70°C for 1 hour, the resulting material was cooled to 0°C, and a saturated NH4Cl solution (30 mL) was added. After extraction with EtOAc (35 mL x 3), the resulting material was dried over anhydrous Na₂SO₄, filtered, and concentrated. The resulting material was purified by column chromatography (PE: EtOAc =1: 1) to obtain 114d (100 mg, 0.31 mmol, 63.69%) as a yellow solid product. LCMS: 315.15 [M+H]⁺.

### 5) Synthesis of compound 114e

At 0°C and under nitrogen protection, to a solution of 114d (100 mg, 0.31 mmol, 1.0 equiv.) 1 1,2-DCE (3 ml), ACE-Cl (87.2 mg, 0.61 mmol, 2.0 equiv.) was dropwise added. After stirring at room temperature for 1 hour, the resulting material was concentrated, and MeOH (10 ml) was then added. After heating under reflux for 12 hours, the resulting material was cooled to room temperature. Saturated NaHCO₃ (10 mL) was added. After extraction with EtOAc (20 mL x 3), the resulting material was dried over anhydrous Na₂SO₄, filtered, and concentrated. The resulting material was purified by column chromatography (PE: EtOAc =1: 1) to obtain 114e (60 mg, 0.26 mmol, 83.87%) as a yellow solid product. LCMS: 225.10 [M+H]⁺.

### 6) Synthesis of (R)-2-((6aS,10aR)-3-chloro-6a,9,10,10-tetrahydro-6H-pyrano[3,2-b:5,4-c']dipyridin-8(7H)-yl)-4-((1-(hydroxymethyl)cyclobutyl)amino)-6,7-dihydrothieno[3,2-d]pyrimidine 5-oxide (114)

At room temperature, to a solution of I01 (60 mg, 0.20 mmol, 1.0 equiv.) and 114e (60.93 mg, 0.27 mmol, 1.3 equiv.) in THF (3 ml) and H₂O (0.6 ml), DIEA (438.6 mg, 3.4 mmol, 10 equiv.) was added. After stirring at 65°C for 12 hours, the resulting material was cooled to room temperature, and water (20 mL) was added. After extraction with EtOAc (20 mL x 3), the resulting material was dried over anhydrous Na₂SO₄, filtered, and concentrated. The resulting material was purified by column chromatography (PE: EtOAc = 1: 1) to obtain Compound 114 (15.59 mg, 0.032 mmol, 16.4%). LCMS: 476.00 [M+H]⁺. ¹H NMR (399 MHz, Methanol-d4) δ 8.03 (d, J = 2.2 Hz, 1H), 7.22 (d, J = 2.2 Hz, 1H), 4.35 (dd, J = 10.6, 3.8 Hz, 1H), 4.03 - 3.87 (m, 4H), 3.55 (dd, J = 17.3, 8.6 Hz, 1H), 3.41 - 3.32 (m, 1H), 3.12 - 3.00 (m, 4H), 2.89 - 2.84 (m, 1H), 2.71 (d, J = 11.8 Hz, 1H), 2.63 (d, J = 11.4 Hz, 1H), 2.39 - 2.30 (m, 4H), 1.93 - 1.84 (m, 2H), 1.41 - 1.32 (m, 2H).

The following compounds were synthesized by similar methods.

| Compound | MS (M+H)⁺ | NMR |
|---|---|---|
| 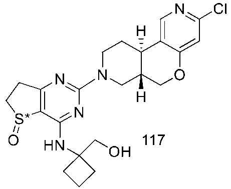 | 476.00 | ¹H NMR (400 MHz, DMSO-d₆) δ 7.34 (s, 1H), 7.23 - 7.17 (m, 2H), 4.88 (d, J = 12.0 Hz, 1H), 4.80 (d, J = 5.9 Hz, 2H), 4.30 (d, J = 10.5 Hz, 1H), 3.93 (t, J = 11.1 Hz, 1H), 3.74 - 3.64 (m, 2H), 3.37 (dt, J = 16.4, 7.9 Hz, 1H), 3.17 (dt, J = 13.4, 8.4 Hz, 1H), 2.97 - 2.80 (m, 4H), 2.64 (t, J = 12.0 Hz, 1H), 2.42 (s, 1H), 2.30 (dd, J = 23.1, 11.3 Hz, 2H), 2.15 (s, 2H), 1.77 (d, J = 9.2 Hz, 1H), 1.72 (t, J = 9.0 Hz, 2H), 1.28 - 1.20 (m, 1H). |

### Example 39: Synthesis of compound 118

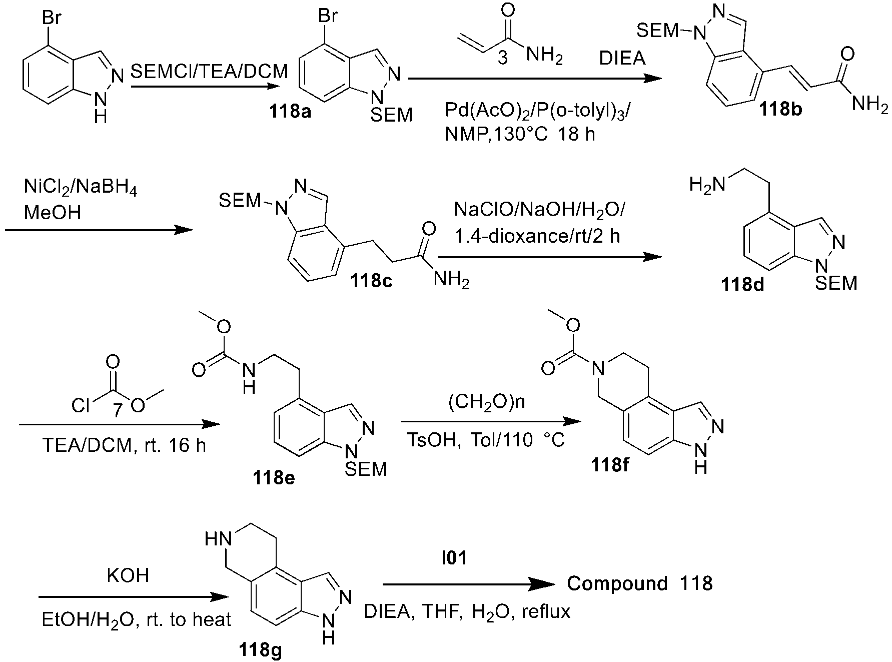

### 1) Synthesis of compound 118a

At 0°C and under nitrogen protection, to a solution of 4-bromo-1H-indazole (10 g, 51.28 mmol, 1.0 equiv.) in DCM (100 ml), SEMCl (8.54 g, 51.28 mmol, 1.0 equiv.) and triethylamine (25.89 g, 256.40 mmol, 5.0 equiv.) were dropwise added successively. After stirring at room temperature for 1 hour, water (150 mL) was added. After extraction with DCM (300 mL x 2), the resulting material was dried over anhydrous Na₂SO₄, filtered, and concentrated. The resulting material was purified by column chromatography (PE: EtOAc = 10: 1) to obtain 118a (7.02 g, 21.46 mmol, 41.86%) as a yellow oil product. LCMS: 327.29 [M+H]⁺.

### 2) Synthesis of compound 118b

At room temperature, to a solution of 118a (2 g, 6.11 mmol, 1 eq) and acrylamide (434.25 mg, 6.11 mmol, 1 eq) in NMP (20 mL), DIPEA (2.36 g, 18.33 mmol, 3 eq), P(o-tolyl)₃ (185.44 mg, 0.6 mmol, 0.1 eq) and Pd(AcO)₂ (137.86 mg, 0.61 mmol,0.1 eq) were added. After stirring at 130°C for 18 hours, the resulting material was cooled to room temperature, and a solid was filtered off. Water (20 mL) was added. After extraction with EtOAc (30 mL x 2), the resulting material was dried over anhydrous Na₂SO₄, filtered, and concentrated. The resulting material was purified by column chromatography (PE: EA from 15: 1 to 5: 1) to obtain 118b (1.7 g, 5.36 mmol, 87.8%) as a yellow solid product. LCMS: 318.15 [M+H]⁺.

### 3) Synthesis of compound 118

At room temperature, to a solution of 118b (1 g, 3.15 mmol, 1.0 equiv.) in MeOH (30 ml), NaBH₄ (119.87 mg, 3.15 mmol, 1.0 eq.) and NiCl₂ (40.3 mg, 0.31 mmol, 0.1 equiv.) were added. After stirring for 1 hour, a saturated NH₄Cl solution (30 mL) was added. After extraction with EtOAC (35 mL x 2), the resulting material was dried over anhydrous Na₂SO₄, filtered, and concentrated. The resulting material was purified by column chromatography (PE: EtOAc = 1: 1) to obtain 118c (1 g, 3.12 mmol, 99.20%) as a yellow solid product. LCMS: 320.17 [M+H]⁺.

### 4) Synthesis of compound 118d

At room temperature, to a solution of 118c (470 mg, 1.46 mmol, 1.0 eq.) in 1,4-dioxance (2 ml, 80.0%) and H₂O (1 ml, 20.0%), NaClO (1 ml, 4.38 mmol, 3 equiv.) and NaOH (175.2 mg, 4.38 mmol, 3.0 eq) were added. After stirring at room temperature for 3 hours and extraction with EtOAC (30 mL x 3), the resulting material was dried over anhydrous Na₂SO₄, filtered, and concentrated. The resulting material was purified by column chromatography (PE: EtOAc = 1: 1) to obtain 118d (220 mg, 0.75 mmol, 51.78%) as a yellow solid product. LCMS: 292.18 [M+H]⁺.

### 5) Synthesis of compound 118e

At 0°C and under nitrogen protection, to a solution of 118d (220 mg, 0.75 mmol, 1.0 equiv.) in DCM (5 ml), ClC(O)OCH₃ (105.75 mg, 1.12 mmol, 1.5 equiv.) and triethylamine (378.75 mg, 3.75 mmol, 5.0 equiv.) were added successively. After stirring at room temperature for 1 hour, water (150 mL) was added. After extraction with DCM (300 mL x 2), the resulting material was dried over anhydrous Na₂SO₄, filtered, and concentrated. The resulting material was purified by column chromatography (PE: EtOAc = 10: 1) to obtain 118e (200 mg, 0.57 mmol, 76.00%) as a yellow oil product. LCMS: 350.18 [M+H]⁺.

### 6) Synthesis of compound 118f

At room temperature, to a solution of 118e (200 mg, 0.57 mmol, 1.0 equiv.) in Tol (10 ml), (CH₂O)n (153.9 mg, 1.71 mmol, 3.0 eq.) and TsOH (9.80 mg, 0.057 mmol, 0.1 equiv.) were added successively. After stirring at 110°C for 16 hours, the resulting material was cooled to room temperature and concentrated. EtOAc (30 mL), water (30 mL x 2), and saturated brine (30 mL) were added. After extraction with EtOAc (30 mL x 3), the resulting material was dried over anhydrous Na₂SO₄, filtered, and concentrated. The resulting material was purified by column chromatography (PE: EtOAc = 1: 1) to obtain 118f (100 mg, 0.43 mmol, 75.62%) as a yellow solid product. LCMS: 232.10 [M+H]⁺.

### 7) Synthesis of compound 118g

At 0°C and under nitrogen protection, to a solution of 118f(100 mg, 0.43 mmol, 1.0 equiv.) in EtOH (4 ml) and H₂O (1 ml), KOH (48.16 mg, 0.86 mmol, 2.0 equiv.) was added. After heating under reflux for 12 hours, the resulting material was cooled to room temperature. EtOAc (30 mL) was added, and the resulting material was washed with saturated NH₄Cl (30 mL x 2) and brine (30 mL), respectively. The resulting material was dried over anhydrous Na₂SO₄, filtered, and concentrated. The resulting material was purified by column chromatography (DCM: MeOH = 10: 1) to obtain 118g (60 mg, 0.34 mmol, 80.65%) as a yellow solid product. LCMS: 174.10 [M+H]⁺.

### 8) Synthesis of (R)-4-((1-(hydroxymethyl)cyclobutyl)amino)-2-(3,6,8,9-tetrahydro-7H-pyrazolo[4,3-f]isoquinolin-7-yl)-6,7-dihydrothieno[3,2-d]pyrimidine 5-oxide (118)

At room temperature, to a solution of I01 (200 mg, 0.69 mmol, 1.0 equiv.) and 118g (155.18 mg, 0.89 mmol, 1.3 equiv.) in THF (3 ml) and H₂O (0.6 ml), DIEA (890.1 mg, 6.9 mmol, 10 equiv.) was added. After stirring at 65°C for 12 hours, the resulting material was cooled to room temperature, and an aqueous solution (20 mL) was added. After extraction with EtOAC (20 mL x 3), the resulting material was dried over anhydrous Na₂SO₄, filtered, and concentrated. The resulting material was purified by column chromatography (MeOH: DCM = 1: 10) to obtain Compound 118 (54.48 mg, 12.84 mmol, 18.62% yield). LCMS: 425.10 [M+H]⁺. ¹H NMR (400 MHz, Methanol-d4) δ 8.03 (s, 1H), 7.37 (d, J = 8.6 Hz, 1H), 7.17 (d, J = 8.6 Hz, 1H), 4.98 (s, 2H), 4.16 (d, J = 5.5 Hz, 2H), 4.03 - 3.91 (m, 2H), 3.64 - 3.52 (m, 1H), 3.41 - 3.32 (m, 1H), 3.13 - 3.04 (m, 4H), 2.45 - 2.32 (m, 4H), 1.92 (d, J = 8.6 Hz, 2H).

### Example 40: Synthesis of compound 135

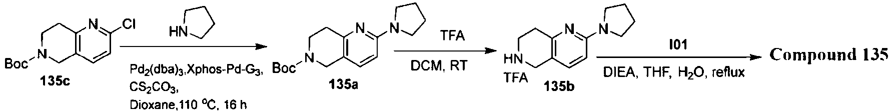

### 1) Synthesis of compound 135a

At room temperature, to a solution of 135c (100.00 mg, 0.37 mmol, 1 eq) in 1,4-dioxane (5 mL), pyrrolidine (26.49 mg, 0.37 mmol, 1 eq), XPhos-Pd-G₃ (31.3 mg, 0.037mmol, 0.1 eq), Pd₂(dba)₃ (67.71 mg, 0.074 mmol, 0.2 eq), and Cs₂CO₃ (630.75 mg, 1.11 mmol, 3.0 eq) were added successively. After stirring at 110°C for 16 hours, the resulting material was cooled to room temperature, a solid was filtered off, and water (15 mL) was added. After extraction with EtOAc (20 mL x 3), the resulting material was dried over anhydrous Na₂SO₄, filtered, and concentrated. The resulting material was purified by Pre-TLC (SiO₂, petroleum ether: ethyl acetate = 3: 1) to obtain 135a (45.00 mg,0.14 mmol, 39.98%) as a yellow oil product. LCMS: 304.19 [M+H]⁺.

### 2) Synthesis of compound 135b

At room temperature, to a solution of 135a (45.00 mg, 0.14 mmol, 1 eq) in DCM (4 mL), TFA (1 mL) was added. After stirring for 1 hour, the resulting material was concentrated to obtain 135b (20.00 mg, 0.098 mmol, 70.02%) as a yellow oil product. The crude product was directly used in the next reaction without purification. LCMS: 204.14 [M+H]⁺.

### 3) Synthesis of (R)-4-((1-(hydroxymethyl)cyclobutyl)amino)-2-(2-(pyrrolidin-1-yl)-7,8-dihydro-1,6-naphthyridin-6(5H)-yl)-6,7-dihydrothieno[3,2-d]pyrimidine 5-oxide (135)

At room temperature, to a solution of 135b (44.66 mg 0.22 mmol, 1.0 eq) and I01 (64.00 mg, 0.22 mmol, 1 eq) in THF (5 mL), N,N-diisopropylethylamine (85.14 mg 0.66 mmol, 3.0 eq) was added. After stirring at 80°C for 4 hours, the resulting material was concentrated. The resulting material was purified by Pre-HPLC (ACN: 0.1% TFA in H₂O from 5% to 95%) to obtain Compound 135 (37.05 mg). LCMS: 455.22 [M+H]⁺. ¹H NMR (400 MHz, DMSO-d₆) δ 7.86 (d, J = 9.3 Hz, 1H), 7.65 (s, 1H), 6.93 (d, J = 9.3 Hz, 1H), 4.71 (s, 2H), 4.00 (s, 2H), 3.71 (s, 2H), 3.50 (s, 4H), 3.39 (s, 1H), 3.20 (d, J = 13.8 Hz, 1H), 2.99 - 2.85 (m, 4H), 2.26 (d, J = 40.1 Hz, 4H), 1.97 (d, J = 6.7 Hz, 4H), 1.81 - 1.70 (m, 2H).

### Example 41: Synthesis of compound 169

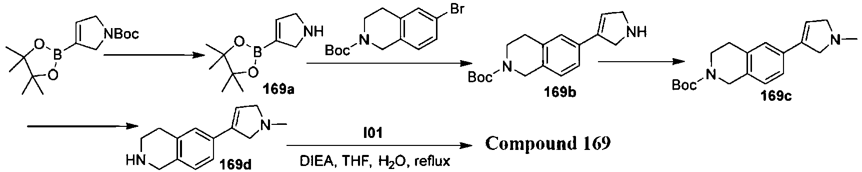

### 1) Synthesis of compound 169a

At room temperature, to a solution of tert-butyl 3-(4,4,5,5-tetramethyl-1,3,2-dioxaboran-2-yl)-2,5-dihydro-1H-pyrrole-1-carboxylate (500.00 mg, 1.69 mmol, 1 eq) in DCM (4 mL), TFA (1 mL) was added. After stirring for 1 hour, the resulting material was concentrated to obtain 169a (180.00 mg,1.59 mmol, 95.25%) as a colorless oil product. The crude product was directly used in the next reaction without purification. LCMS: 196.2 [M+H]⁺.

### 2) Synthesis of compound 169b

At room temperature, to a solution of 169a (180 mg,1.59 mmol,1.0 eq) and tert-butyl 6-bromo-3,4-dihydroisoquinolin-2(1H)-carboxylate (495.39 mg, 1.59 mmol, 1.0 eq) in THF (10 mL), K₂CO₃ (658.26 mg, 4.77 mmol, 3.0 eq), 1,1'-bis(diphenylphosphino)ferrocene]palladium(II) dichloride (116.38 mg, 0.159 mmol, 0.1 eq) and water (5 mL) were added successively. After heating under reflux for 6 hours, the resulting material was cooled to room temperature, and a solid was filtered off. Water (20 mL) was added. After extraction with EtOAc (30 mL x 3), the resulting material was dried over anhydrous Na₂SO₄, filtered, and concentrated. The resulting material was purified by column chromatography (MeOH: DCM = 1: 10) to obtain 169b (200 mg, 0.66 mmol, 41.92%) as a yellow solid product. LCMS: 301.18 [M+H]⁺.

### 3) Synthesis of compound 169c

At room temperature, to a solution of 169b (200 mg, 0.66 mmol, 1.0 equiv.) in anhydrous DCE (3 ml), HCHO (19.8 mg, 0.66 mmol, 1.0 eq.) and AcOH (3.96 mg, 0.066 mmol, 0.1 equiv.) were added successively. After continued stirring for 1 hour, the resulting material was cooled to 0°C. NaBH₄ (50.16 mg, 1.32 mmol, 2.0 equiv.) was added in portions. After stirring at room temperature for 2 hours, a saturated NH₄Cl solution (30 mL) was added. After extraction with EtOAc (35 mL x 3), the resulting material was dried over anhydrous Na₂SO₄, filtered, and concentrated. The resulting material was purified by column chromatography (PE: EtOAc = 1: 1) to obtain 169c (200 mg, 0.63 mmol, 96.50%) as a yellow solid product. LCMS: 315.20 [M+H]⁺.

### 4) Synthesis of compound 169d

At room temperature, to a solution of 169c (200.00 mg, 0.63 mmol, 1 eq) in DCM (4 mL), TFA (1 mL) was added. After stirring at room temperature for 1 hours, the resulting material was concentrated to obtain 169d (100.00 mg, 74.17% yield) as a yellow oil product. The crude product was directly used in the next reaction without purification. LCMS: 215.15 [M+H]⁺.

### 5) Synthesis of (R)-4-((1-(hydroxymethyl)cyclobutyl)amino)-2-(6-(1-methyl-2,5-dihydro-1H-pyrrol-3-yl)-3,4-dihydroisoquinolin-2(1H)-yl)-6,7-dihydrothieno[3,2-d]pyrimidine 5-oxide (169)

At room temperature, to a solution of I01 (181.44 mg, 0.63 mmol, 1.0 equiv.) and 169d (30 mg, 0.63 mmol, 1.0 equiv.) in THF (3 ml) and H₂O (0.6 ml), DIEA (812.70 mg, 6.30 mmol, 10 equiv.) was added. After stirring at 65°C for 12 hours, the resulting material was cooled to room temperature, and water (20 mL) was added. After extraction with EtOAc (20 mL x 3), the resulting material was dried over anhydrous Na₂SO₄, filtered, and concentrated. The resulting material was purified by Pre-HPLC (ACN: 0.1% TFA in H₂O from 5% to 95%) to obtain Compound 169 (22.18 mg, 36.0 %). LCMS: 466.10 [M+H]⁺. ¹H NMR (400 MHz, Methanol-d₄) δ 7.25 (d, J = 8.0 Hz, 1H), 7.19 (s, 1H), 7.13 (d, J = 8.0 Hz, 1H), 6.17 (s, 1H), 4.05 - 3.89 (m, 4H), 3.82 (d, J = 4.3 Hz, 2H), 3.64 (s, 2H), 3.56 (dt, J = 16.9, 8.1 Hz, 1H), 3.36 (dd, J = 14.3, 8.4 Hz, 1H), 3.29 (d, J = 1.7 Hz, 2H), 3.13 - 2.99 (m, 2H), 2.87 (t, J = 5.9 Hz, 2H), 2.55 (s, 3H), 2.40 - 2.27 (m, 4H), 1.91 (q, J = 9.1 Hz, 2H).

### Example 42: Synthesis of compound 180

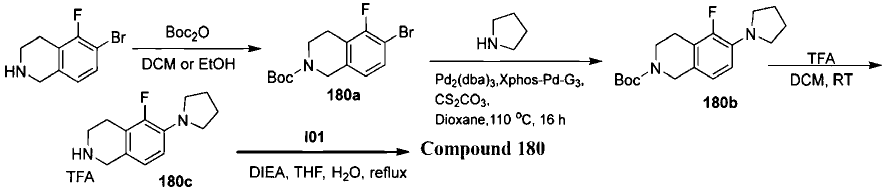

### 1) Synthesis of compound 180a

At room temperature, to a solution of 6-bromo-5-fluoro-1,2,3,4-tetrahydroisoquinoline (100.00 mg, 0.43 mmol, 1 eq) in DCM (5 mL), Boc₂O (94.86 mg, 0.43 mmol, 1 eq) and TEA (131.94 mg, 1.30 mmol, 3 eq) were added successively. After continued stirring for 2 hours, water (10 mL) was added. After extraction with DCM (10 mL x 3), the resulting material was dried over anhydrous Na₂SO₄, filtered, and concentrated to obtain 180a (crude, 100.00 mg, 69.92%) as a yellow oil product. LCMS: 330.20 [M+H]⁺.

### 2) Synthesis of compound 180b

At room temperature, to a solution of 180a (100.00 mg, 0.30 mmol, 1 eq) in 1,4-dioxane (5 mL), pyrrolidine (21.61 mg, 0.30 mmol, 1 eq), XPhos-Pd-G₃ (25.75 mg, 0.03mmol, 0.1 eq), Pd₂(dba)₃(55.62 mg, 0.06 mmol, 0.2 eq), and Cs₂CO₃ (296.32 mg, 0.91 mmol, 3.0 eq) were added successively. After stirring at 110°C for 16 hours, the resulting material was cooled to room temperature, a solid was filtered off, and water (20 mL) was added. After extraction with EtOAc (20 mL x 3), the resulting material was dried over anhydrous Na₂SO₄, filtered, and concentrated. The resulting material was purified by Pre-TLC (SiO₂, petroleum ether: ethyl acetate = 3: 1) to obtain 180b (45.00 mg, 46.21%) as a yellow oil product. LCMS: 321.20 [M+H]⁺.

### 3) Synthesis of compound 180c

At room temperature, to a solution of 180b (45.00 mg, 0.14 mmol, 1 eq) in DCM (4 mL), TFA (1 mL) was added. After continued stirring for 1 hour, the resulting material was concentrated to obtain 180c (20.00 mg, 64.66%) as a yellow oil product. The crude product was directly used in the next reaction without purification. LCMS: 221.15 [M+H]⁺.

### 4) Synthesis of (R)-2-(5-fluoro-6-(pyrrolidin-1-yl)-3,4-dihydroisoquinolin-2(1H)-yl)-4-((1-(hydroxymethyl)cyclobutyl)amino)-6,7-dihydrothieno[3,2-d]pyrimidine 5-oxide (180)

At room temperature, to a solution of I01(26.15 mg, 0.09 mmol, 1 eq) and 180c (20.00 mg, 0.09 mmol, 1 eq) in THF (4 mL) and H₂O (1 mL), DIEA (35.20 mg, 0.24 mmol, 3 eq) was added. After stirring at 65°C for 3 hours, the resulting material was concentrated. The resulting material was purified by Prep-HPLC (0.1% TFA in ACN/H₂O) to obtain compound 180 (2.17 mg, 5.06%). LCMS: 472.10 [M+H]⁺. ¹H NMR (400 MHz, Methanol-d4) δ 6.85 (d, J = 8.5 Hz, 1H), 6.69 (t, J = 8.6 Hz, 1H), 4.00 - 3.91 (m, 4H), 3.60 (d, J = 7.6 Hz, 1H), 3.33 (s, 6H), 3.23 - 3.05 (m, 4H), 2.87 (s, 3H), 2.37 (dd, J = 12.3, 8.4 Hz, 4H), 1.94 (d, J = 6.8 Hz, 6H).

### Example 43: Synthesis of compound 181

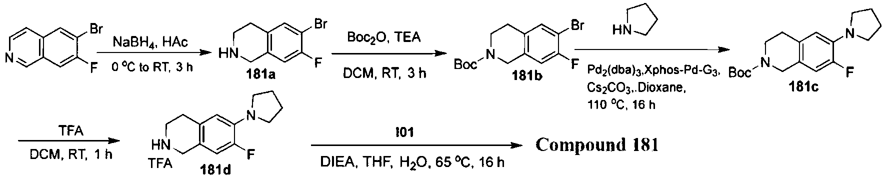

### 1) Synthesis of compound 181a

At 0°C and under nitrogen protection, to a solution of 6-bromo-7-fluoroisoquinoline (200.00 mg, 0.88 mmol, 1 eq) in HOAc (4 mL), NaBH₄ (66.94 mg, 1.77 mmol, 2 eq) was added. After stirring at room temperature for 3 hours, a saturated Rochelle's reagent (20 mL) was added. After extraction with EtOAc (20 mL x 3), the resulting material was dried over anhydrous Na₂SO₄, filtered, and concentrated to obtain 181a (150 mg, 73.68%) as a yellow oil product. LCMS: 231.95 [M+H]⁺.

### 2) Synthesis of compound 181b

At room temperature, to a solution of 181a (150.00 mg, 0.65 mmol, 1 eq) in DCM (5 mL), Boc₂O (214.00 mg, 0.98 mmol, 1.5 eq) and TEA (198.00 mg, 1.95 mmol, 3 eq) were added successively. After continued stirring for 3 hours, water (10 mL) was added. After extraction with DCM (10 mL x 3), the resulting material was dried over anhydrous Na₂SO₄, filtered, and concentrated to obtain 181b (crude, 160.00 mg, 74.32%) as a yellow oil product. LCMS: 331.95 [M+H]⁺.

### 3) Synthesis of compound 181c

At room temperature, to a solution of 181b (160.00 mg, 0.49 mmol, 1 eq) in 1,4-dioxane (5 mL), pyrrolidine (35.00 mg, 0.49 mmol, 1 eq), XPhos-Pd-G₃ (42.00 mg, 0.049 mmol, 0.1 eq), Pd₂(dba)₃(45.00 mg, 0.049 mmol, 0.1 eq), and Cs₂CO₃ (473.00 mg, 1.46 mmol, 3.0 eq) were added successively. After stirring at 110°C for 16 hours, the resulting material was cooled to room temperature, and water (10 mL) was added. After extraction with EtOAc (10 mL x 3), the resulting material was dried over anhydrous Na₂SO₄, filtered, and concentrated. The resulting material was purified by Pre-TLC (SiO₂, petroleum ether: ethyl acetate = 3: 1) to obtain 181c (50.00 mg, 32.49% yield) as a yellow oil product. LCMS: 321.10 [M+H]⁺.

### 4) Synthesis of compound 181d

At room temperature, to a solution of 181c (50.00 mg, 0.16 mmol, 1 eq) in DCM (4 mL), TFA (1 mL) was added. After continued stirring for 1 hour, the resulting material was concentrated to obtain 181d (30.00 mg, 87.28%) as a yellow oil product. The crude product was directly used in the next reaction without purification. LCMS: 221.20 [M+H]⁺.

### 5) Synthesis of (R)-2-(7-fluoro-6-(pyrrolidin-1-yl)-3,4-dihydroisoquinolin-2(1H)-yl)-4-((1-(hydroxymethyl)cyclobutyl)amino)-6,7-dihydrothieno[3,2-d]pyrimidine 5-oxide (181)

At room temperature, to a solution of I01 (40.00 mg, 0.14 mmol, 1 eq) and 181d (30.00 mg, 0.14 mmol, 1 eq) in THF (4 mL) and H₂O (1 mL), DIEA (53.00 mg, 0.41 mmol, 3 eq) was added. After stirring at 65°C for 16 hours, the resulting material was concentrated. The resulting material was purified by Prep-HPLC (0.1% TFA in ACN/H₂O) to obtain Compound 181 (33.89 mg, 52.77%). LCMS: 472.10 [M+H]⁺. ¹H NMR (400 MHz, DMSO-d₆) δ 6.91 (d, J = 14.3 Hz, 1H), 6.51 (d, J = 9.1 Hz, 1H), 4.69 (s, 2H), 3.72 (s, 2H), 3.85 (d, J = 5.7 Hz, 2H), 3.72 (s, 2H), 3.53 - 3.42 (m, 2H), 3.23 (s, 6H), 3.03 (d, J = 16.3 Hz, 1H), 2.89 (dd, J = 13.7, 7.3 Hz, 1H), 2.74 (s, 2H), 2.32 - 2.18 (m, 4H), 1.85 (d, J = 6.0 Hz, 4H), 1.76 (dd, J = 11.6, 7.1 Hz, 2H).

The following compounds were synthesized by similar methods.

| Compound | MS (M+H)⁺ | NMR |
|---|---|---|
| 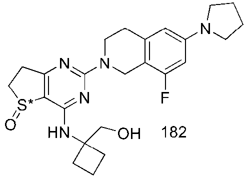 | 472.05 | ¹H NMR 1H NMR (399 MHz, Methanol-d4) δ 6.28 - 6.18 (m, 2H), 4.81 (s, 2H), 3.96 (d, J = 12.8 Hz, 4H), 3.78 (dt, J = 18.1, 7.6 Hz, 1H), 3.50 (dt, J = 14.6, 8.0 Hz, 1H), 3.42 - 3.32 (m, 1H), 3.28 - 3.16 (m, 6H), 2.95 (s, 2H), 2.48 - 2.35 (m, 4H), 2.02 (q, J = 3.3 Hz, 4H), 2.01 - 1.88 (m, 3H). |
| 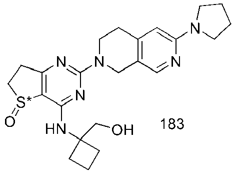 | 455.35 | ¹H NMR (400 MHz, Methanol-d4) δ 7.79 (s, 1H), 6.94 (s, 1H), 3.97 (dt, J = 28.5, 7.0 Hz, 5H), 3.69 - 3.57 (m, 2H), 3.54 (s, 5H), 3.14 - 3.00 (m, 4H), 2.36 (d, J = 11.0 Hz, 4H), 2.13 (s, 3H), 2.02 (s, 1H), 1.94 - 1.85 (m, 3H), 1.28 (s, 1H). |

### Example 44: Synthesis of compound 184

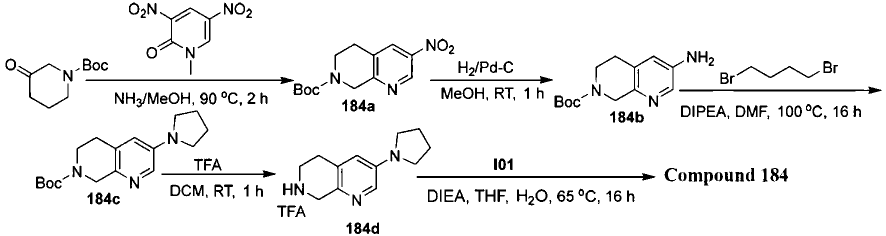

### 1) Synthesis of compound 184a

At room temperature, tert-butyl 3-oxopiperidine-1-carbamate (1.50 g, 7.53 mmol, 1 eq), 1-methyl-3,5-dinitropyridin-2(1H)-one (1.95 g, 9.79 mmol, 1.3 eq), and 7M NH₃/MeOH (15 mL) were placed in a sealed tube. After stirring at 90°C for 2 hours, the resulting material was cooled to room temperature and concentrated. The resulting material was purified by Prep-HPLC (0.1 % TFA in ACN/H₂O) to obtain 184a (90 mg, 6.0%) as a yellow solid product. LCMS: 280.25 [M+H]⁺.

### 2) Synthesis of compound 184b

At room temperature, to a solution of 184a (60.00 mg, 0.21 mmol, 1 eq) in MeOH (4 mL), Pd/C (10%, 6 mg) was added, followed by deoxygenation three times. After hydrogen absorption with a balloon at room temperature for 1 hour, a solid was filtered off, followed by concentration to obtain 184b (40.00 mg, 74.69%) as a yellow solid product. LCMS: 250.15 [M+H]⁺.

### 3) Synthesis of compound 184c

At room temperature, to a solution of 184b (40.00 mg, 0.16 mmol, 1 eq) in DMF(3 mL), 1,4-dibromobutane (51.96 mg, 0.24 mmol, 1.5 eq) and DIPEA (61.92 mg, 0.48 mmol, 3.0 eq) were added. After stirring at 100°C for 16 hours, the resulting material was cooled to room temperature, and water (10 mL) was added. After extraction with EtOAc (10 mL x 3), the resulting material was dried over anhydrous Na₂SO₄, filtered, and concentrated. The resulting material was purified by Pre-TLC (SiO₂, petroleum ether: ethyl acetate = 3: 1) to obtain 184c (10.00 mg, 20.54%) as a yellow oil product. LCMS: 304.15 [M+H]⁺.

### 4) Synthesis of compound 184d

At room temperature, to a solution of 184c (13.00 mg, 0.04 mmol, 1 eq) in DCM (2 mL), TFA (0.5 mL) was added. After continued stirring for 1 hour, the resulting material was concentrated to obtain 184d (8.00 mg, 91.84%) as a yellow oil product. The crude product was directly used in the next reaction without purification. LCMS: 204.20 [M+H]⁺.

### 5) Synthesis of (R)-4-((1-(hydroxymethyl)cyclobutyl)amino)-2-(3-(pyrrolidin-1-yl)-5,8-dihydro-1,7-naphthyridin-7(6H)-yl)-6,7-dihydrothieno[3,2-d]pyrimidine 5-oxide (184)

At room temperature, to a solution of I01 (11.00 mg, 0.04 mmol, 1 eq) and 184d (8.00 mg, 0.04 mmol, 1 eq) in THF (2 mL) and H₂O (0.5 mL), DIEA (16.00 mg, 0.12 mmol, 3 eq) was added. After stirring at 65°C for 16 hours, the resulting material was concentrated. The resulting material was purified by Prep-HPLC (0.1% TFA in ACN/H₂O) to obtain Compound 184 (4.65 mg, 26.00%). LCMS: 455.15 [M+H]⁺. ¹H NMR (400 MHz, DMSO-d₆) δ 7.73 (d, J = 2.7 Hz, 1H), 7.35 (s, 1H), 4.84 (t, J = 5.7 Hz, 1H), 4.73 (s, 1H), 3.92 (s, 2H), 3.72 (t, J = 4.1 Hz, 2H), 3.40 (d, J = 8.7 Hz, 2H), 3.18 (d, J = 6.6 Hz, 6H), 2.94 - 2.82 (m, 2H), 2.76 (s, 2H), 2.32 (dd, J = 21.0, 10.5 Hz, 2H), 2.19 - 2.12 (m, 2H), 1.91 (t, J = 3.3 Hz, 4H), 1.76 (q, J = 9.1 Hz, 2H).

### Example 45: Synthesis of compound 218

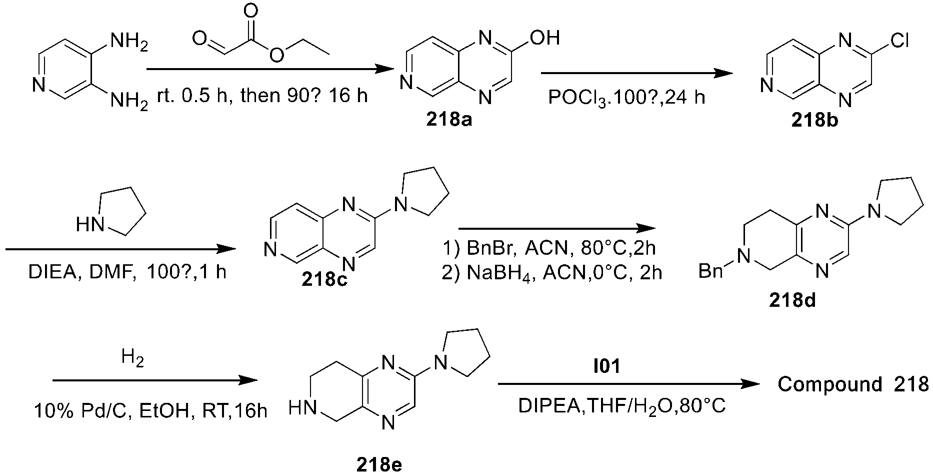

### 1) Synthesis of compound 218a

At room temperature, to a solution of pyridine-3,4-diamine (5.00 g, 42.02 mmol, 1 eq) in ethanol (75 mL), ethyl glyoxylate (9.2 g, 46.22 mmol, 1.1 eq) was added. After stirring at room temperature for 30 minutes, the resulting material was stirred at 90°C for 18 hours. The resulting material was cooled to room temperature, filtered, washed with ethanol, and dried to obtain 218a (3.1 g, 50.16%) as a yellow solid product. The crude product was directly used in the next reaction without purification. LCMS: 148.45 [M+H]⁺.

### 2) Synthesis of compound 218b

Under nitrogen protection, a solution of 218a (1.00 g, 6.80 mmol, 1 eq) in POCl₃ (10 mL) was stirred at 100°C for 24 hours. The resulting material was cooled to room temperature and concentrated to obtain 218b (1.00 g, 89.29%) as a black white solid product. The crude product was directly used in the next reaction without purification. LCMS: 166.05 [M+H]⁺.

### 3) Synthesis of compound 218c

At room temperature, to a solution of 218b (1.00 g, 6.06 mmol, 1 eq) in DMF (20 mL), DIPEA (1.56 g, 12.12 mmol, 2 eq) and pyrrolidine (860.60 mg, 12.12 mmol, 2 eq) were added. The resulting material was stirred at 100°C for 1 hour. After cooling to room temperature, ethyl acetate (30 mL) was added, followed by washing with a saturated brine (50 mL). The resulting material was dried over anhydrous Na₂SO₄, filtered, and concentrated. The resulting material was purified by column chromatography (PE: EtOAc = 1: 1) to obtain 218c (130 mg, 10.74%) as a yellow solid product. LCMS: 201.15 [M+H]⁺.

### 4) Synthesis of compound 218d

At room temperature, to a solution of 218c (130.00 mg, 0.65 mmol, 1 eq) in acetonitrile (5 mL), benzyl bromide (114.48 mg, 0.67 mmol, 1.03 eq) was added. After stirring at 80°C for 2 hours, the resulting material was cooled to room temperature, filtered, and dried to obtain 6-benzyl-2-(pyrrolidin-1-yl)-5,6,7,8-tetrahydropyrido[3,4-b]pyrazine (120 mg). 6-Benzyl-2-(pyrrolidin-1-yl)-5,6,7,8-tetrahydropyrido[3,4-b]pyrazine (120 mg) was dissolved in acetonitrile (5 mL), and at 0°C, sodium borohydride (123.5 mg, 3.35 mmol, 5 eq) was added. After continued stirring at 0°C for 2 hours, a saturated NaHCO₃ solution (10 mL) was added. After extraction with EtOAc (10 mL x 3), the resulting material was dried over anhydrous Na₂SO₄, filtered, and concentrated. The resulting material was purified by column chromatography (PE: EtOAc = 1: 1) to obtain 218d (50 mg, 26.16%) as a yellow solid product. LCMS: 295.15 [M+H]⁺.

### 5) Synthesis of compound 218e

At room temperature, a solution of 218d (50.00 mg, 0.17 mmol, 1 eq) and 10% Pd/C (20 mg) in ethanol (2 mL) was hydrogenated with a balloon overnight. After filtration to remove a solid, the resulting material was concentrated to obtain 218e (30 mg, 86.45%) as a yellow solid product. LCMS: 205.15 [M+H]⁺.

### 6) Synthesis of (R)-4-((1-(hydroxymethyl)cyclobutyl)amino)-2-(2-(pyrrolidin-1-yl)-7,8-dihydropyrido[3,4-b]pyrazin-6(5H)-yl)-6,7-dihydrothieno[3,2-d]pyrimidine 5-oxide (218)

At room temperature, to a solution of 218e (30 mg, 0.15 mmol, 1 eq) and I01 (42.35 mg, 0.15 mmol, 1 eq) in THF (2 mL), DIPEA (37.94 mg, 0.29 mmol, 2 eq) and water (1 mL) were added. After stirring at 80°C for 4 hours, the resulting material was concentrated. The resulting material was purified by Prep-HPLC (0.1% TFA in ACN/H₂O) to obtain Compound 218 (11.26 mg, 16.80%). LCMS: 456.25 [M+H]⁺. ¹H NMR (400 MHz, DMSO-d₆) δ 8.05 (s, 1H), 7.80 (s, 1H), 5.05 (s, 1H), 4.77 (s, 2H), 4.03 (t, J = 6.0 Hz, 2H), 3.80 - 3.69 (m, 2H), 3.57 - 3.45 (m, 1H), 3.44 - 3.34 (m, 4H), 3.30 - 3.20 (m, 1H), 3.06 (dd, J = 17.2, 8.0 Hz, 1H), 2.93 (dd, J = 13.6, 7.2 Hz, 1H), 2.83 - 2.76 (m, 2H), 2.40 - 2.25 (m, 2H), 2.25 - 2.15 (m, 2H), 1.97 - 1.88 (m, 4H), 1.85 - 1.72 (m, 2H).

Referring to the preparation methods of Examples 1-45, the following compounds were synthesized:

| Compound | MS (M+H)⁺ | NMR |
|---|---|---|
| 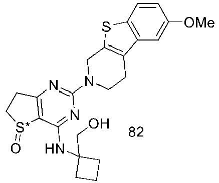 | 471.15 | ¹H NMR (400 MHz, DMSO-d₆) δ 7.76 (d, 1H), 7.14 (s, 1H), 6.93 (d, 1H), 5.01 (s, 1H), 4.84 (s, 1H), 4.11 (s, 2H), 3.78 (s, 3H), 3.72 (s, 2H), 3.28 - 3.26 (m, 1H), 3.22 - 3.11 (m, 1H), 2.87 - 2.78 (m, 3H), 2.40 - 2.12 (m, 4H), 1.85 - 1.70 (m, 2H), 1.26 - 1.18 (m, 1H). |
| 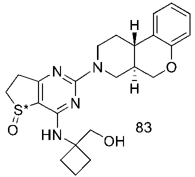 | 441.15 | ¹H NMR (400 MHz, DMSO-d₆) δ 7.34 (s, 1H), 7.16 (d, J = 7.7 Hz, 1H), 7.05 (t, J = 7.6 Hz, 1H), 6.81 (t, J = 7.4 Hz, 1H), 6.69 (dd, J = 8.2, 1.2 Hz, 1H), 4.82 (t, J = 5.6 Hz, 2H), 4.74 (s, 1H), 4.21 (d, J = 10.4 Hz, 1H), 3.85 (t, J = 10.9 Hz, 1H), 3.69 (d, J = 5.1 Hz, 2H), 3.43 - 3.32 (m, 1H), 3.18 - 3.13 (m, 1H), 3.04 - 2.78 (m, 4H), 2.75 - 2.58 (m, 2H), 2.40 (d, J = 13.7 Hz, 1H), 2.30 (dd, J = 23.5, 11.2 Hz, 2H), 2.19 - 2.12 (m, 2H), 1.81 - 1.70 (m, 2H), 1.59 (d, J = 11.9 Hz, 1H). |
| 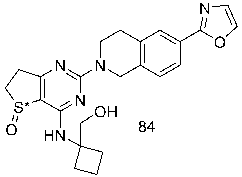 | 452.25 | ¹H NMR (400 MHz, DMSO-d₆) δ 8.17 (s, 1H), 7.80 - 7.76 (m, 2H), 7.38 - 7.32 (m, 2H), 4.90 (s, 3H), 3.95 (t, J = 5.9 Hz, 2H), 3.75 - 3.71 (m, 2H), 3.51 - 3.42 (m, 1H), 3.25 - 3.18 (m, 1H), 3.06 - 2.99 (m, 1H), 2.94 - 2.86 (m, 3H), 2.42 - 2.29 (m, 2H), 2.27 - 2.10 (m, 3H), 1.81 - 1.73 (m, 2H). |
| 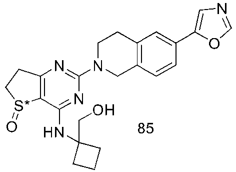 | 452.15 | ¹H NMR (400 MHz, DMSO-d₆) δ 8.40 (s, 1H), 8.24 (s, 1H), 7.63 (s, 1H), 7.57 - 7.49 (m, 2H), 7.30 (d, J = 8.3 Hz, 1H), 4.86 (s, 2H), 4.00 - 3.88 (m, 2H), 3.73 (s, 2H), 3.58 - 3.45 (m, 1H), 3.33 - 3.20 (m, 1H), 3.17 - 3.03 (m, 1H), 2.99 - 2.85 (m, 3H), 2.38 - 2.16 (m, 4H), 1.85 - 1.70 (m, 2H). |
| 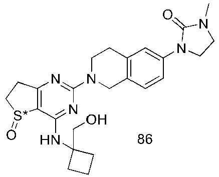 | 483.25 | ¹H NMR (400 MHz, DMSO-d₆) δ 7.42 (dd, J = 8.2, 2.3 Hz, 1H), 7.31 (s, 1H), 7.11 (d, J = 8.5 Hz, 1H), 4.77 (s, 2H), 3.89 (t, J = 6.0 Hz, 2H), 3.75 - 3.69 (m, 4H), 3.46 (dd, J = 17.1, 8.2 Hz, 1H), 3.38 (dd, J = 9.1, 6.7 Hz, 2H), 3.24 (dd, J = 14.2, 7.8 Hz, 1H), 3.04 (dd, J = 17.2, 8.0 Hz, 1H), 2.89 (dd, J = 13.8, 7.1 Hz, 1H), 2.82 (s, 2H), 2.71 (s, 3H), 2.35 - 2.18 (m, 4H), 1.76 (t, J = 9.4 Hz, 2H). |
| 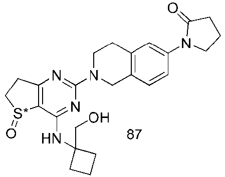 | 468.25 | ¹H NMR (400 MHz, DMSO-d₆) δ 7.50 - 7.34 (m, 2H), 7.17 (d, J = 8.5 Hz, 1H), 4.81 (d, J = 14.3 Hz, 2H), 3.91 (s, 1H), 3.81 - 3.68 (m, 4H), 2.97 - 2.79 (m, 3H), 2.42 (d, J = 8.1 Hz, 11H), 2.17 (s, 1H), 2.00 (dq, J = 15.8, 7.8 Hz, 2H), 1.80 - 1.70 (m, 1H), 1.20 (s, 1H). |
| 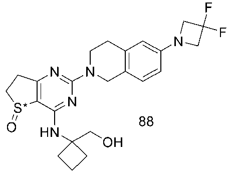 | 476.20 | ¹H NMR (400 MHz, Methanol-d₄) δ 7.08 (d, J = 8.2 Hz, 1H), 6.49 - 6.41 (m, 2H), 4.78 (s, 3H), 4.17 (t, J = 11.9 Hz, 5H), 3.94 (d, J = 14.2 Hz, 4H), 3.74 (dt, J = 16.5, 7.5 Hz, 1H), 3.47 (dt, J = 14.8, 8.1 Hz, 1H), 3.18 (dd, J = 14.0, 7.3 Hz, 1H), 2.94 (s, 2H), 2.39 (d, J = 7.1 Hz, 3H), 1.93 (q, J = 8.5 Hz, 2H), 1.29 (d, J = 14.2 Hz, 2H). |
| 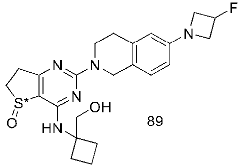 | 458.30 | ¹H NMR (400 MHz, DMSO-d₆) δ 7.00 (d, J = 8.2 Hz, 1H), 6.35 - 6.28 (m, 2H), 5.43 (d, J = 56.8 Hz, 1H), 4.71 (s, 3H), 4.11 - 4.03 (m, 2H), 3.85 - 3.79 (m, 3H), 3.76 - 3.71 (m, 3H), 3.51 - 3.44 (m, 1H), 3.27 - 3.20 (m, 1H), 3.10 - 3.02 (m, 1H), 2.94 - 2.88 (m, 1H), 2.78 (s, 2H), 2.34 - 2.15 (m, 5H), 1.79 - 1.72 (m, 2H). |
| 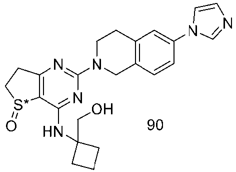 | 451.35 | ¹H NMR (400 MHz, DMSO-d₆) δ 9.58 (s, 1H), 8.22 (s, 1H), 7.88 - 7.81 (m, 1H), 7.65 - 7.55 (m, 3H), 7.48 (d, J = 8.3 Hz, 1H), 4.92 (s, 2H), 3.97 (s, 3H), 3.72 (s, 2H), 3.46 - 3.38 (m, 1H), 3.22 - 3.14 (m, 1H), 2.97 - 2.83 (m, 4H), 2.36 - 2.26 (m, 2H), 2.23 - 2.16 (m, 2H), 1.81 - 1.69 (m, 2H). |
| 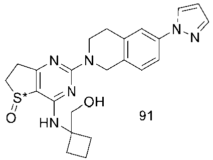 | 451.30 | ¹H NMR (400 MHz, Methanol-d₄) δ 8.17 (d, J = 2.5 Hz, 1H), 7.68 (d, J = 1.8 Hz, 1H), 7.54 (d, J = 6.3 Hz, 2H), 7.29 (d, J = 8.9 Hz, 1H), 6.49 (t, J = 2.2 Hz, 1H), 4.93 (s, 2H), 4.09 - 3.96 (m, 3H), 3.92 (d, J = 11.3 Hz, 1H), 3.57 (dt, J = 16.8, 8.0 Hz, 1H), 3.35 (dt, J = 13.8, 8.4 Hz, 3H), 3.13 - 3.03 (m, 2H), 3.03 - 2.92 (m, 2H), 2.38 (d, J = 10.5 Hz, 1H), 2.33 (s, 2H), 1.97 - 1.83 (m, 2H), 1.29 (d, J = 13.7 Hz, 1H). |
| 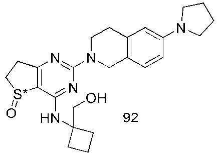 | 454.45 | ¹H NMR (400 MHz, DMSO-d₆) δ 8.42 (s, 1H), 6.96 (d, J = 8.2 Hz, 1H), 6.43 - 6.35 (m, 2H), 4.69 (s, 2H), 3.85 - 3.81 (m, 2H), 3.75 - 3.70 (m, 2H), 3.57 - 3.50 (m, 1H), 3.30 - 3.23 (m, 1H), 3.15 (d, J = 6.0 Hz, 5H), 2.98 - 2.92 (m, 1H), 2.79 (d, J = 6.5 Hz, 2H), 2.30 - 2.15 (m, 4H), 1.89 (s, 4H), 1.79 - 1.72 (m, 2H), 1.27 (d, J = 63.6 Hz, 1H). |
| 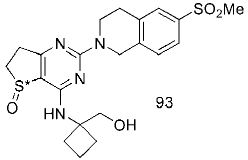 | 463.15 | ¹H NMR (400 MHz, DMSO-d₆) δ 7.79 - 7.66 (m, 2H), 7.48 (d, J = 8.0 Hz, 1H), 4.94 (s, 1H), 3.94 (d, J = 5.9 Hz, 4H), 3.72 (s, 3H), 3.44 (dt, J = 16.5, 7.8 Hz, 1H), 3.14 (s, 4H), 2.93 (tq, J = 21.0, 7.6 Hz, 4H), 2.30 (dd, J = 21.2, 10.8 Hz, 2H), 2.20 (s, 1H), 1.75 (dd, J = 17.5, 8.8 Hz, 2H), 1.20 (s, 1H). |
| 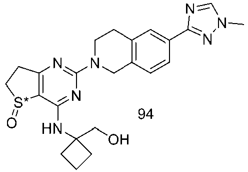 | 466.35 | ¹H NMR (400 MHz, DMSO-d₆) δ 8.47 (s, 1H), 7.78 (d, J = 5.8 Hz, 2H), 7.27 (d, J = 8.2 Hz, 1H), 4.86 (s, 2H), 3.93 (d, J = 6.0 Hz, 2H), 3.87 (s, 3H), 3.76 - 3.69 (m, 3H), 3.50 - 3.44 (m, 1H), 3.21 (dd, J = 13.6, 8.3 Hz, 1H), 3.07 - 3.01 (m, 1H), 2.90 (s, 2H), 2.38 - 2.16 (m, 6H), 1.78 (d, J = 8.4 Hz, 2H). |
| 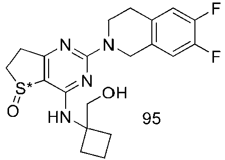 | 421.30 | ¹H NMR (400 MHz, DMSO-d₆) δ 7.34 (dd, J = 11.2, 8.3 Hz, 1H), 7.23 (dd, J = 11.3, 8.4 Hz, 1H), 4.80 (s, 2H), 3.89 (t, J = 5.9 Hz, 3H), 3.42 (dd, J = 17.0, 8.3 Hz, 4H), 3.20 (dt, J = 13.3, 8.2 Hz, 1H), 2.97 (dd, J = 17.4, 8.1 Hz, 1H), 2.83 (dt, J = 34.7, 6.6 Hz, 3H), 2.36 - 2.23 (m, 2H), 2.20 (s, 1H), 1.77 (s, 1H), 1.74 (d, J = 9.0 Hz, 1H), 1.20 (s, 1H). |
| 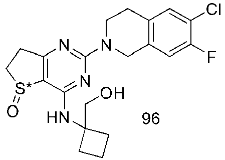 | 437.10 | ¹H NMR (400 MHz, DMSO-d₆) δ 7.35 (dd, J = 16.2, 8.7 Hz, 2H), 5.28 (s, 1H), 4.82 (s, 2H), 3.90 (s, 2H), 3.70 (d, J = 5.8 Hz, 2H), 3.43 - 3.34 (m, 1H), 2.77 (s, 2H), 2.18 (s, 4H), 1.94 (s, 2H), 1.75 (d, J = 9.5 Hz, 2H), 1.41 (s, 1H), 0.81 (s, 1H). |
| 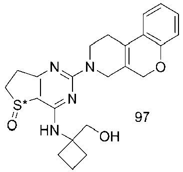 | 439.20 | ¹H NMR (400 MHz, DMSO-d₆) δ 8.00 (s, 1H), 7.59 (d, J = 8.0 Hz, 1H), 7.09 (dd, J = 12.0, 7.0 Hz, 2H), 6.89 (t, J = 7.5 Hz, 1H), 6.78 (dd, J = 15.0, 8.0 Hz, 1H), 6.37 (s, 1H), 4.83 (s, 1H), 4.65 (s, 1H), 4.41 - 4.34 (m, 1H), 3.96 (s, 2H), 3.72 (s, 2H), 3.61 (t, J = 10.9 Hz, 1H), 3.47 (dt, J = 15.6, 7.6 Hz, 1H), 3.31 - 3.18 (m, 1H), 3.05 (dt, J = 17.7, 8.7 Hz, 1H), 2.90 (dd, J = 13.7, 7.2 Hz, 1H), 2.80 - 2.65 (m, 1H), 2.37 - 2.16 (m, 5H), 1.77 (q, J = 8.9 Hz, 2H). |
| 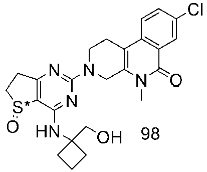 | 500.20 | ¹H NMR (400 MHz, DMSO-d₆) δ 8.17 (s, 1H), 7.76 (d, 1H), 7.71 (d, 1H), 4.88 (s, 2H), 4.02 (s, 2H), 3.72 (s, 2H), 3.54 - 3.54 (m, 3H), 3.24 - 3.13 (m, 2H), 3.03 - 2.92 (m, 2H), 2.91 - 2.82 (m, 2H), 2.78 (t, 2H), 2.36 - 2.28 (m, 2H), 2.23 - 2.15 (m, 2H), 1.82 - 1.72 (m, 2H). |
| 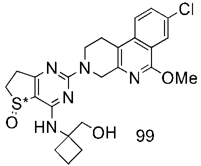 | 500.35 | ¹H NMR (400 MHz, DMSO-d₆) δ 8.10 (s, 1H), 7.92 (d, 1H), 7.79 (d, J = 9.0, 2.3 Hz, 1H), 4.88 (s, 2H), 4.11 (s, 2H), 4.02 (s, 3H), 3.75 (s, 2H), 3.51 - 3.42 (m, 1H), 3.28 - 3.16 (m, 1H), 3.08 - 2.97 (m, 3H), 2.94 - 2.83 (m, 1H), 2.41 - 2.28 (m, 2H), 2.27 - 2.12 (m, 2H), 1.90 - 1.75 (m, 2H). |
| 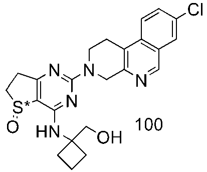 | 470.15 | ¹H NMR (400 MHz, DMSO-d₆) δ 9.17 (s, 1H), 8.25 (s, 1H), 8.04 (d, 1H), 7.83 (d, 1H), 5.03 (s, 2H), 4.14 (s, 2H), 3.75 (s, 2H), 3.52 - 3.42 (m, 1H), 3.25 - 3.16 (m, 3H), 3.04 - 2.97 (m, 1H), 2.91 - 2.86 (m, 1H), 2.37 - 2.19 (m, 4H), 1.85 - 1.75 (m, 2H). |
| 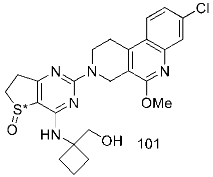 | 500.15 | ¹H NMR (400 MHz, DMSO-d₆) δ 7.92 (d, J = 8.8 Hz, 1H), 7.77 (d, J = 2.2 Hz, 1H), 7.45 (dd, J = 8.7, 2.2 Hz, 1H), 4.83 (s, 4H), 4.09 (s, 2H), 4.02 (s, 3H), 3.74 (s, 2H), 3.45 (dd, J = 17.0, 8.3 Hz, 1H), 3.25 - 3.17 (m, 1H), 3.12 (s, 2H), 3.01 (dd, J = 17.2, 8.0 Hz, 1H), 2.88 (dd, J = 13.5, 7.3 Hz, 1H), 2.28 (d, J = 76.0 Hz, 4H), 1.77 (dd, J = 20.0, 10.6 Hz, 2H). |
| 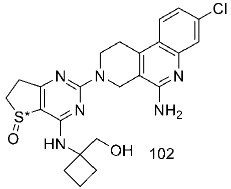 | 485.35 | ¹H NMR (400 MHz, DMSO-d₆) δ 8.76 (s, 1H), 7.97 (d, J = 8.8 Hz, 1H), 7.70 (d, J = 2.1 Hz, 1H), 7.61 (s, 1H), 7.51 (dd, J = 8.8, 2.1 Hz, 1H), 4.83 (d, J = 17.0 Hz, 4H), 4.15 - 4.01 (m, 2H), 3.74 (s, 2H), 3.42 (dt, J = 16.4, 7.9 Hz, 1H), 3.22 - 3.08 (m, 3H), 2.99 - 2.84 (m, 2H), 2.26 (d, J = 31.2 Hz, 4H), 1.78 (s, 2H). |
| 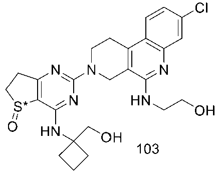 | 529.35 | ¹H NMR (400 MHz, DMSO-d₆) δ 7.94 (s, 2H), 7.62 (s, 1H), 7.51 - 7.44 (m, 1H), 4.82 (q, J = 17.9 Hz, 5H), 4.15 - 4.01 (m, 2H), 3.72 (d, J = 14.7 Hz, 6H), 3.42 (dt, J = 16.4, 7.8 Hz, 1H), 3.23 - 3.15 (m, 1H), 3.09 (s, 2H), 2.97 - 2.83 (m, 2H), 2.26 (d, J = 34.3 Hz, 4H), 1.78 (s, 2H). |
| 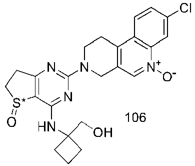 | 486.15 | ¹H NMR (400 MHz, DMSO-d₆) δ 8.71 (s, 1H), 8.51 (d, J = 2.2 Hz, 1H), 8.12 (d, J = 9.0 Hz, 1H), 7.77 (dd, J = 9.0, 2.2 Hz, 1H), 4.93 (s, 4H), 4.10 (t, J = 6.0 Hz, 2H), 3.74 (s, 2H), 3.47 - 3.40 (m, 1H), 3.22 - 3.16 (m, 1H), 3.10 (d, J = 6.0 Hz, 2H), 3.01 - 2.95 (m, 1H), 2.89 - 2.83 (m, 1H), 2.24 (s, 4H), 1.81 - 1.74 (m, 2H). |
| 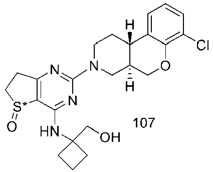 | 475.15 | ¹H NMR (400 MHz, DMSO-d₆) δ 7.39 (s, 1H), 7.18 (dd, J = 24.1, 7.8 Hz, 2H), 6.82 (t, J = 7.8 Hz, 1H), 4.83 (t, J = 5.7 Hz, 2H), 4.76 (s, 1H), 4.39 - 4.31 (m, 1H), 3.93 (t, J = 11.0 Hz, 1H), 3.69 (d, J = 5.7 Hz, 2H), 3.45 - 3.34 (m, 2H), 3.16 (dt, J = 13.4, 8.3 Hz, 1H), 3.00 - 2.73 (m, 4H), 2.61 (t, J = 12.1 Hz, 1H), 2.40 (d, J = 12.7 Hz, 1H), 2.27 (dt, J = 21.6, 10.0 Hz, 2H), 2.15 (d, J = 6.3 Hz, 2H), 1.75 (q, J = 8.8 Hz, 2H), 1.61 (d, J = 11.7 Hz, 1H). |
| 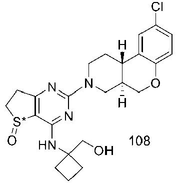 | 475.30 | ¹H NMR (400 MHz, DMSO-d₆) δ 7.39 (s, 1H), 7.19 (d, J = 2.6 Hz, 1H), 7.09 (dd, J = 8.8, 2.5 Hz, 1H), 6.73 (d, J = 8.7 Hz, 1H), 4.83 (t, J = 5.7 Hz, 2H), 4.74 (s, 1H), 4.23 (d, J = 10.4 Hz, 1H), 3.85 (t, J = 11.0 Hz, 1H), 3.69 (d, J = 5.8 Hz, 2H), 3.38 (q, J = 8.1 Hz, 2H), 3.16 (dt, J = 13.5, 8.3 Hz, 1H), 3.00 - 2.70 (m, 4H), 2.60 (t, J = 12.1 Hz, 1H), 2.40 (d, J = 12.8 Hz, 1H), 2.28 (dt, J = 21.4, 10.2 Hz, 2H), 2.14 (s, 2H), 1.82 - 1.68 (m, 2H), 1.57 (d, J = 11.6 Hz, 1H). |
| 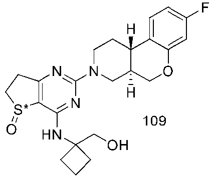 | 459.30 | ¹H NMR (400 MHz, DMSO-d₆) δ 7.38 (s, 1H), 7.18 (t, J = 7.8 Hz, 1H), 6.69 - 6.60 (m, 1H), 6.55 (dd, J = 10.6, 2.7 Hz, 1H), 4.82 (t, J = 5.8 Hz, 2H), 4.73 (s, 1H), 4.37 - 4.20 (m, 1H), 3.87 (t, J = 11.0 Hz, 1H), 3.69 (d, J = 5.7 Hz, 2H), 3.38 (dd, J = 15.5, 7.5 Hz, 1H), 3.16 (dt, J = 16.1, 8.4 Hz, 1H), 2.99 - 2.86 (m, 2H), 2.82 (dd, J = 13.6, 7.3 Hz, 1H), 2.64 (dt, J = 32.4, 11.9 Hz, 2H), 2.32 (dd, J = 23.9, 13.3 Hz, 2H), 2.15 (s, 2H), 1.81 - 1.66 (m, 2H), 1.57 (d, J = 9.9 Hz, 1H), 1.22 - 0.96 (m, 2H). |
| 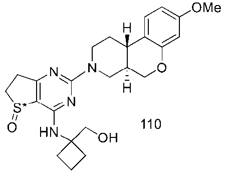 | 471.30 | ¹H NMR (400 MHz, DMSO-d₆) δ 7.35 (s, 1H), 7.05 (d, J = 8.6 Hz, 1H), 6.40 (dd, J = 8.5, 2.7 Hz, 1H), 6.27 (d, J = 2.6 Hz, 1H), 4.83 (q, J = 9.4, 5.6 Hz, 2H), 4.21 (s, 1H), 3.82 (t, J = 10.9 Hz, 1H), 3.66 (d, J = 19.4 Hz, 5H), 3.39 (dd, J = 16.9, 8.2 Hz, 1H), 3.23 - 3.10 (m, 2H), 2.99 - 2.77 (m, 3H), 2.61 (q, J = 13.0, 12.5 Hz, 2H), 2.31 (dt, J = 19.9, 11.5 Hz, 4H), 1.79 - 1.69 (m, 2H), 1.54 (d, J = 12.1 Hz, 1H), 1.20 (s, 1H), 1.13 (d, J = 13.2 Hz, 1H). |
| 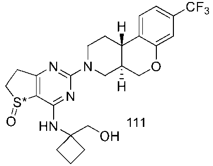 | 509.35 | ¹H NMR (400 MHz, DMSO-d₆) δ 7.40 (d, J = 8.0 Hz, 1H), 7.36 (s, 1H), 7.15 (d, J = 8.0 Hz, 1H), 7.01 (s, 1H), 4.88 (d, J = 12.4 Hz, 1H), 4.81 (t, J = 5.8 Hz, 2H), 4.30 (d, J = 10.2 Hz, 1H), 3.92 (d, J = 10.9 Hz, 1H), 3.69 (d, J = 5.5 Hz, 2H), 3.36 (dd, J = 16.8, 8.1 Hz, 1H), 3.18 (dd, J = 13.9, 8.1 Hz, 1H), 3.04 - 2.89 (m, 2H), 2.83 (d, J = 14.8 Hz, 2H), 2.64 (t, J = 12.1 Hz, 1H), 2.30 (dd, J = 24.0, 11.4 Hz, 2H), 2.15 (s, 2H), 1.80 - 1.69 (m, 2H), 1.63 (d, J = 11.2 Hz, 1H), 1.22 (d, J = 12.6 Hz, 2H). |
| 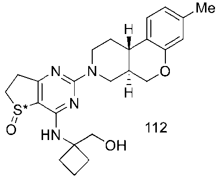 | 455.15 | ¹H NMR (400 MHz, DMSO-d₆) δ 7.33 (d, J = 9.8 Hz, 1H), 7.03 (d, J = 7.8 Hz, 1H), 6.62 (d, J = 7.7 Hz, 1H), 6.51 (s, 1H), 4.90 - 4.78 (m, 2H), 4.19 (d, J = 10.2 Hz, 1H), 3.88 - 3.77 (m, 1H), 3.72 - 3.65 (m, 2H), 3.45 - 3.33 (m, 2H), 3.23 - 3.10 (m, 1H), 3.01 - 2.80 (m, 3H), 2.71 - 2.48 (m, 2H), 2.42 - 2.24 (m, 3H), 2.16 (s, 5H), 1.83 - 1.71 (m, 2H), 1.55 (d, J = 11.7 Hz, 1H), 1.20 - 1.06 (m, 1H). |
| 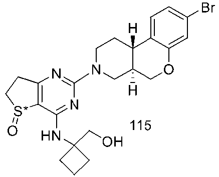 | 520.80 | ¹H NMR (400 MHz, DMSO-d₆) δ 7.35 (s, 1H), 7.12 (d, 1H), 6.99 (d, 1H), 6.91 (s, 1H), 4.86 - 4.73 (m, 2H), 4.35 - 4.21 (m, 1H), 3.88 (t, J = 10.8 Hz, 1H), 3.69 (s, 2H), 3.45 - 3.33 (m, 1H), 3.21 - 3.13 (m, 1H), 2.98 - 2.80 (m, 3H), 2.74 - 2.57 (m, 2H), 2.40 - 2.24 (m, 3H), 2.18 - 2.11 (m, 2H), 1.78 - 1.54 (m, 3H), 1.25 - 1.10 (m, 1H). |
| 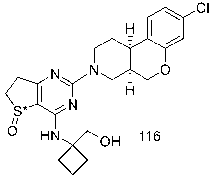 | 475.35 | ¹H NMR (400 MHz, Methanol-d₄) δ 7.13 (d, J = 8.3 Hz, 1H), 6.83 (dd, J = 8.3, 2.2 Hz, 1H), 6.73 (d, J = 2.1 Hz, 1H), 4.40 (d, J = 13.8 Hz, 1H), 4.27 (s, 1H), 4.15 (dd, J = 11.2, 3.5 Hz, 1H), 3.98 - 3.88 (m, 3H), 3.66 - 3.50 (m, 2H), 3.40 - 3.32 (m, 2H), 3.05 (dt, J = 14.5, 8.9 Hz, 4H), 2.31 (td, J = 13.1, 12.1, 7.1 Hz, 6H), 1.93 - 1.84 (m, 4H). |
| 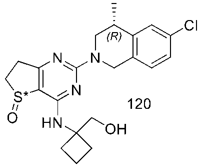 | 433.10 | ¹H NMR (400 MHz, DMSO-d₆) δ 7.34 (d, J = 11.0 Hz, 2H), 7.25 (s, 1H), 4.85 (t, J = 5.7 Hz, 1H), 3.74 (d, J = 5.7 Hz, 2H), 3.30 - 3.16 (m, 4H), 3.09 - 2.86 (m, 3H), 2.37 (s, 2H), 2.21 (s, 2H), 1.99 (q, J = 6.9, 6.4 Hz, 1H), 1.80 (q, J = 9.0 Hz, 2H), 1.31 - 1.15 (m, 5H). |
| 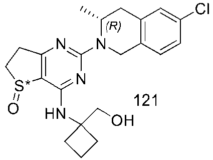 | 433.10 | ¹H NMR (400 MHz, Methanol-d4) δ 7.22 - 7.13 (m, 3H), 5.21 (s, 1H), 5.13 (d, J = 17.5 Hz, 1H), 4.57 (s, 1H), 4.42 (d, J = 17.5 Hz, 1H), 3.94 (q, J = 11.3 Hz, 2H), 3.57 (dt, J = 16.9, 8.1 Hz, 1H), 3.36 (dt, J = 16.1, 8.4 Hz, 2H), 3.06 (dt, J = 15.0, 8.4 Hz, 3H), 2.76 - 2.66 (m, 1H), 2.33 (dd, J = 16.1, 7.3 Hz, 3H), 1.92 - 1.84 (m, 1H), 1.29 (d, J = 16.8 Hz, 1H), 1.07 (d, J = 6.7 Hz, 3H), 0.08 (s, 1H). |
| 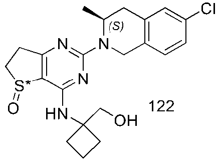 | 433.05 | ¹H NMR (400 MHz, Methanol-d4) δ 7.22 - 7.13 (m, 3H), 5.21 (s, 1H), 5.13 (d, J = 17.5 Hz, 1H), 4.57 (s, 1H), 4.42 (d, J = 17.5 Hz, 1H), 3.94 (q, J = 11.3 Hz, 2H), 3.57 (dt, J = 16.9, 8.1 Hz, 1H), 3.36 (dt, J = 16.1, 8.4 Hz, 2H), 3.06 (dt, J = 15.0, 8.4 Hz, 3H), 2.76 - 2.66 (m, 1H), 2.33 (dd, J = 16.1, 7.3 Hz, 3H), 1.92 - 1.84 (m, 1H), 1.29 (d, J = 16.8 Hz, 1H), 1.07 (d, J = 6.7 Hz, 3H), 0.08 (s, 1H). |
| 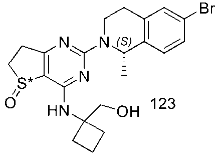 | 478.95 | ¹H NMR (400 MHz, Methanol-d4) δ 7.32 (dq, J = 4.8, 2.2 Hz, 2H), 7.12 (d, J = 8.8 Hz, 1H), 4.70 (s, 1H), 4.00 (d, J = 11.3 Hz, 1H), 3.91 (d, J = 11.3 Hz, 1H), 3.57 (dt, J = 17.4, 8.0 Hz, 1H), 3.45 - 3.30 (m, 4H), 3.14 - 3.00 (m, 2H), 2.94 - 2.83 (m, 2H), 2.47 - 2.25 (m, 4H), 2.01 - 1.81 (m, 2H), 1.47 (d, J = 6.8 Hz, 3H), 1.35 - 1.26 (m, 1H). |
| 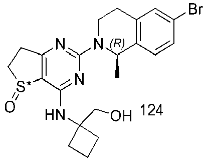 | 479.05 | ¹H NMR (400 MHz, Methanol-d4) δ 7.32 (dq, J = 4.8, 2.2 Hz, 2H), 7.12 (d, J = 8.8 Hz, 1H), 4.70 (s, 1H), 4.00 (d, J = 11.3 Hz, 1H), 3.91 (d, J = 11.3 Hz, 1H), 3.57 (dt, J = 17.4, 8.0 Hz, 1H), 3.45 - 3.30 (m, 4H), 3.14 - 3.00 (m, 2H), 2.94 - 2.83 (m, 2H), 2.47 - 2.25 (m, 4H), 2.01 - 1.81 (m, 2H), 1.47 (d, J = 6.8 Hz, 3H), 1.35 - 1.26 (m, 1H). |
| 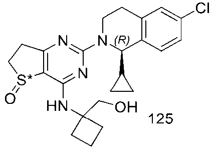 | 459.15 | ¹H NMR (399 MHz, DMSO-d₆) δ 7.36 (d, J = 7.3 Hz, 1H), 7.28 (s, 1H), 7.21 (d, J = 10.2 Hz, 2H), 5.23 - 5.12 (m, 1H), 4.86 - 4.75 (m, 1H), 4.76 - 4.47 (m, 1H), 3.68 (dd, J = 42.4, 5.7 Hz, 2H), 3.50 (s, 1H), 3.20 - 3.13 (m, 1H), 2.96 - 2.85 (m, 1H), 2.81 (d, J = 14.4 Hz, 3H), 2.46 - 2.04 (m, 5H), 1.76 (s, 2H), 1.30 (s, 1H), 0.79 - 0.55 (m, 2H), 0.39 (s, 2H). |
| 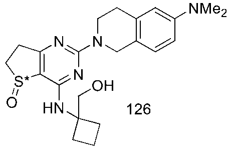 | 428.35 | ¹H NMR (400 MHz, DMSO-d₆) δ 8.06 (s, 1H), 7.12 - 7.08 (m, 1H), 6.88 - 6.74 (m, 2H), 4.75 (s, 2H), 3.87 (d, J = 6.0 Hz, 3H), 3.73 (s, 3H), 3.66 - 3.45 (m, 2H), 3.44 - 3.13 (m, 2H), 3.11 - 3.02 (m, 1H), 2.94 (s, 1H), 2.83 (s, 3H), 2.37 - 2.25 (m, 2H), 2.21 (s, 2H), 1.82 - 1.72 (m, 2H). |
| 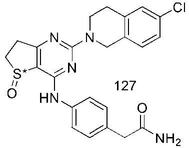 | 468.05 | ¹H NMR (400 MHz, DMSO-d₆) δ 9.55 (s, 1H), 7.62 (d, 2H), 7.25 - 7.20 (m, 5H), 4.84 (s, 2H), 3.94 (s, 2H), 3.53 - 3.46 (m, 3H), 3.27 - 3.20 (m, 2H), 3.04 (q, 1H), 2.93 (q, 1H), 2.84 (t, 2H). |
| 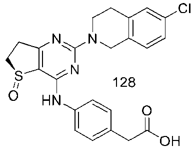 | 469.00 | ¹H NMR (400 MHz, DMSO-d₆) δ 9.55 (s, 1H), 7.62 (d, 2H), 7.22 (t, 5H), 4.84 (s, 2H), 3.93 (s, 2H), 3.54 - 3.46 (m, 3H), 3.27 - 3.20 (m, 2H), 3.05 (q, 1H), 2.94 (q, 1H), 2.84 (t, 2H). |
| 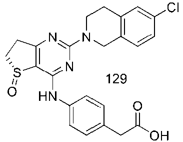 | 469.05 | ¹H NMR (400 MHz, DMSO-d₆) δ 9.55 (s, 1H), 7.62 (d, 2H), 7.27 - 7.19 (m, 5H), 4.84 (s, 2H), 3.93 (s, 2H), 3.54 - 3.46 (m, 3H), 3.27 - 3.20 (m, 2H), 3.08 - 3.00 (m, 1H), 2.99 - 2.89 (m, 1H), 2.84 (t, 2H). |
| 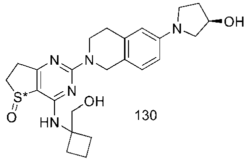 | 470.35 | ¹H NMR (400 MHz, Methanol-d4) δ 7.01 (d, J = 8.3 Hz, 1H), 6.52 - 6.42 (m, 2H), 4.75 (s, 3H), 4.51 (s, 1H), 3.96 (d, J = 2.3 Hz, 3H), 3.90 (s, 1H), 3.80 - 3.66 (m, 1H), 3.50 - 3.40 (m, 4H), 3.22 - 3.12 (m, 3H), 2.92 (s, 2H), 2.38 (s, 5H), 2.18 - 2.09 (m, 1H), 1.93 (d, J = 8.7 Hz, 2H), 1.29 (d, J = 16.1 Hz, 2H). |
| 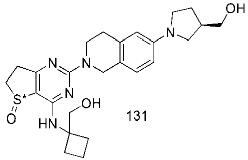 | 484.15 | ¹H NMR (400 MHz, DMSO-d₆) δ 6.97 (d, J = 8.3 Hz, 1H), 6.43 - 6.32 (m, 2H), 4.70 (s, 2H), 3.53 (dt, J = 17.5, 7.7 Hz, 3H), 3.45 - 3.05 (m, 9H), 3.01 - 2.89 (m, 2H), 2.81 (t, J = 6.0 Hz, 2H), 2.43 - 2.34 (m, 2H), 2.34 - 2.22 (m, 3H), 2.20 (dd, J = 8.8, 3.9 Hz, 1H), 1.98 (ddd, J = 12.6, 8.6, 5.2 Hz, 1H), 1.79 (dd, J = 9.2, 4.4 Hz, 2H), 1.71 (tdd, J = 14.9, 10.2, 6.1 Hz, 2H), 1.21 (d, J = 3.5 Hz, 1H). |
| 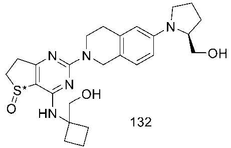 | 484.20 | ¹H NMR (400 MHz, DMSO-d₆) δ 7.27 (s, 1H), 6.42 (d, J = 8.5 Hz, 1H), 6.35 (s, 1H), 4.82 (s, 1H), 4.69 (s, 2H), 3.86 (s, 2H), 3.71 (d, J = 5.8 Hz, 2H), 3.58 (s, 1H), 3.42 (d, J = 2.7 Hz, 1H), 3.14 - 3.12 (m, 1H), 2.94 (s, 3H), 2.85 (s, 1H), 2.73 (s, 2H), 2.29 (s, 3H), 2.16 (s, 2H), 2.04 (s, 1H), 1.95 (s, 2H), 1.88 (s, 2H), 1.77 (s, 3H). |
| 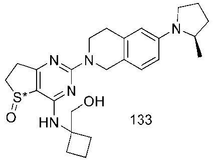 | 468.35 | ¹H NMR (400 MHz, DMSO-d₆) δ 7.28 (s, 1H), 6.94 (d, J = 8.5 Hz, 1H), 6.37 (d, J = 8.4 Hz, 1H), 6.29 (s, 1H), 4.82 (t, J = 5.7 Hz, 1H), 4.69 (s, 2H), 3.82 (d, J = 30.4 Hz, 3H), 3.71 (d, J = 5.8 Hz, 2H), 3.37 (dd, J = 16.7, 8.2 Hz, 1H), 3.19 - 3.13 (m, 1H), 3.02 (d, J = 8.1 Hz, 1H), 2.95 - 2.89 (m, 1H), 2.87 - 2.80 (m, 1H), 2.73 (s, 2H), 2.33 (dd, J = 21.0, 10.7 Hz, 2H), 2.16 (s, 2H), 1.91 (d, J = 35.9 Hz, 3H), 1.76 (d, J = 8.6 Hz, 2H), 1.61 (s, 1H), 1.20 (s, 1H), 1.04 (d, J = 6.1 Hz, 3H). |
| 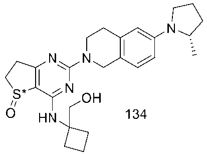 | 468.20 | ¹H NMR (400 MHz, Methanol-d4) δ 7.30 (d, J = 8.8 Hz, 1H), 7.08 (s, 2H), 4.90 (s, 2H), 3.98 (dd, J = 11.5, 3.5 Hz, 5H), 3.71 (dt, J = 17.7, 7.7 Hz, 2H), 3.57 - 3.40 (m, 2H), 3.30 - 3.12 (m, 3H), 3.00 (t, J = 6.0 Hz, 2H), 2.46 - 2.29 (m, 5H), 2.19 (hept, J = 6.6 Hz, 2H), 2.00 - 1.80 (m, 3H), 1.35 - 1.20 (m, 4H). |
| 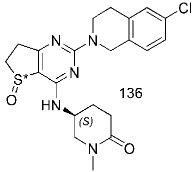 | 446.05 | ¹H NMR (400 MHz, DMSO-d₆) δ 7.80 (d, J = 7.1 Hz, 1H), 7.26 - 7.19 (m, 3H), 4.84 (s, 2H), 4.50 (s, 1H), 3.94 (s, 2H), 3.49 - 3.39 (m, 2H), 3.22 (d, J = 10.3 Hz, 2H), 2.99 - 2.92 (m, 1H), 2.85 (dd, J = 14.1, 6.3 Hz, 3H), 2.78 (s, 3H), 2.31 (s, 2H), 1.91 (d, J = 7.0 Hz, 2H). |
| 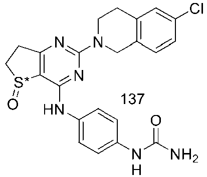 | 469.25 | ¹H NMR (400 MHz, DMSO-d₆) δ 9.42 (s, 1H), 8.48 (s, 1H), 7.46 (d, 2H), 7.35 (d, 2H), 7.23 (d, 3H), 5.80 (s, 2H), 4.83 (s, 1H), 3.91 (s, 1H), 3.48 (q, 1H), 3.26 - 3.17 (m, 2H), 3.02 (q, 1H), 2.91 (q, 1H), 2.83 (s, 2H), 1.20 (s, 1H). |
| 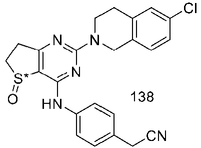 | 450.25 | ¹H NMR (400 MHz, DMSO-d₆) δ 9.63 (s, 1H), 7.70 (d, 2H), 7.31 (d, 2H), 7.24 (d, 3H), 4.84 (s, 1H), 3.96 (d, 5H), 3.50 (q, 1H), 3.29 - 3.21 (m, 2H), 3.05 (q, 1H), 2.94 (q, 1H), 2.84 (t, 2H). |
| 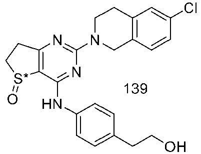 | 455.00 | ¹H NMR (400 MHz, DMSO-d₆) δ 9.48 (s, 1H), 7.57 (d, 2H), 7.20 (q, 5H), 4.84 (s, 2H), 3.93 (s, 2H), 3.59 (t, 2H), 3.51 - 3.47 (m, 2H), 3.25 (q, 1H), 3.08 - 2.99 (m, 1H), 2.93 (q, 1H), 2.84 (t, 2H), 2.69 (t, 2H). |
| 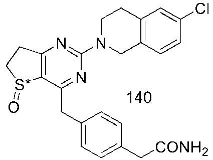 | 471.00 | ¹H NMR (400 MHz, DMSO-d₆) δ 9.71 (s, 1H), 9.26 (s, 1H), 7.89 (s, 1H), 7.63 (s, 1H), 6.88 (s, 1H), 4.49 (s, 2H), 4.03 (d, J = 5.6 Hz, 2H), 3.83 (s, 3H), 3.51 - 3.46 (m, 1H), 3.22 (d, J = 7.1 Hz, 1H), 3.05 - 2.98 (m, 1H), 2.93 - 2.89 (m, 1H), 2.73 (s, 2H). |
| 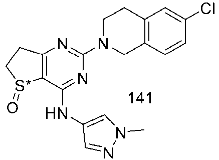 | 414.95 | ¹H NMR (400 MHz, Methanol-d4) δ 7.94 (s, 1H), 7.73 (s, 1H), 7.25 (d, J = 6.6 Hz, 2H), 4.98 (s, 1H), 4.41 (s,1H), 4.10 (t, J = 6.0 Hz, 1H), 3.94 (s, 2H), 2.97 (t, J = 5.9 Hz, 1H), 2.63 (d, J = 2.0 Hz, 5H), 2.07 (s, 1H), 1.31 (d, J = 3.8 Hz, 3H). |
| 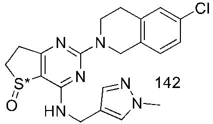 | 429.25 | ¹H NMR (400 MHz, DMSO-d₆) δ 8.64 (s, 1H), 7.55 (s, 1H), 7.34 (s, 1H), 7.28 - 7.23 (m, 3H), 4.88 (s, 2H), 4.42 (d, J = 5.7 Hz, 2H), 3.96 (s, 2H), 3.72 (s, 3H), 3.49 (dt, J = 16.7, 7.8 Hz, 1H), 3.28 (dt, J = 13.5, 8.2 Hz, 1H), 3.14 - 3.03 (m, 1H), 2.89 (dt, J = 20.7, 5.9 Hz, 3H). |
| 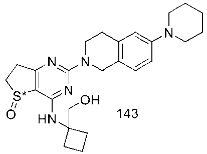 | 468.40 | ¹H NMR (400 MHz, DMSO-d₆) δ 7.81 (s, 1H), 7.30 (s, 3H), 4.84 (s, 2H), 3.92 (t, J = 5.8 Hz, 2H), 3.72 (s, 2H), 3.57 - 3.29 (m, 6H), 3.28 - 3.19 (m, 1H), 3.01 (dd, J = 17.2, 8.1 Hz, 1H), 2.87 (q, J = 6.9, 5.8 Hz, 3H), 2.36 - 2.27 (m, 2H), 2.20 (tt, J = 8.6, 4.1 Hz, 2H), 1.79 (p, J = 5.6 Hz, 6H), 1.59 (d, J = 8.3 Hz, 2H). |
| 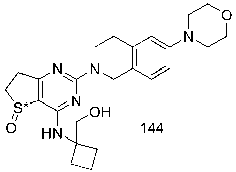 | 470.35 | ¹H NMR (400 MHz, DMSO-d₆) δ 8.49 (s, 1H), 7.05 (d, J = 8.4 Hz, 1H), 6.83 (dd, J = 8.5, 2.5 Hz, 1H), 6.78 (d, J = 2.5 Hz, 1H), 4.73 (s, 2H), 3.86 (t, J = 6.0 Hz, 2H), 3.74 (d, J = 2.3 Hz, 2H), 3.70 (dd, J = 6.1, 3.5 Hz, 5H), 3.60 - 3.51 (m, 1H), 3.34 - 3.25 (m, 1H), 3.15 (dd, J = 17.5, 8.1 Hz, 1H), 3.06 (t, J = 4.8 Hz, 5H), 2.99 - 2.93 (m, 1H), 2.83 (t, J = 5.9 Hz, 2H), 2.35 - 2.26 (m, 2H), 2.23 (d, J = 7.3 Hz, 2H), 1.81 - 1.74 (m, 2H). |
| 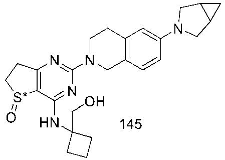 | 466.35 | ¹H NMR (399 MHz, Methanol-d4) δ 6.94 (d, 1H), 6.44 (dd, 1H), 6.36 (s, 1H), 4.76 (s, 1H), 4.57 (s, 1H), 4.03 - 3.89 (m, 4H), 3.57 (q, 1H), 3.50 (d, 2H), 3.37 (dt, 1H), 3.16 - 3.02 (m, 4H), 2.82 (t, 2H), 2.45 - 2.29 (m, 4H), 2.03 (s, 1H), 1.98 - 1.86 (m, 2H), 1.68 - 1.61 (m, 2H), 1.35 - 1.26 (m, 1H), 0.69 (q, 1H), 0.31 (q, 1H). |
| 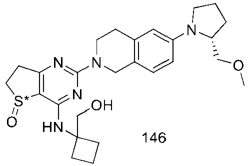 | 466.35 | ¹H NMR (400 MHz, Methanol-d4) δ 7.14 (d, J = 8.2 Hz, 1H), 6.84 - 6.75 (m, 2H), 4.81 (s, 4H), 3.94 (d, J = 17.7 Hz,5H), 3.82 - 3.68 (m, 1H), 3.58 - 3.42 (m, 3H), 3.34 (s, 3H), 3.29 (d, J = 10.3 Hz, 5H), 2.97 (s, 2H), 2.38 (d, J = 8.4 Hz, 2H), 2.07 (s, 4H), 1.92 (dd, J = 10.1, 7.0 Hz, 2H), 1.29 (d, J = 15.4 Hz, 1H). |
| 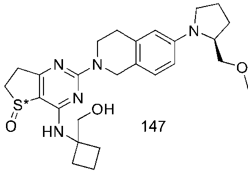 | 498.30 | 1H NMR (400 MHz, Methanol-d4) δ 7.17 (d, J = 8.2 Hz, 1H), 6.84 (d, J = 11.2 Hz, 2H), 4.83 (s, 3H), 3.96 (d, J = 16.0 Hz, 4H), 3.77 (dt, J = 17.9, 7.6 Hz, 1H), 3.61 - 3.44 (m, 3H), 3.42 - 3.31 (m, 5H), 3.31 - 3.15 (m, 6H), 2.40 (dd, J = 9.5, 5.7 Hz, 4H), 2.10 (d, J = 4.4 Hz, 4H), 1.99 - 1.89 (m, 2H). |
| 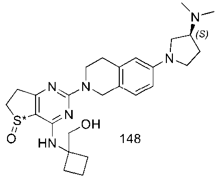 | 497.15 | ¹H NMR (400 MHz, Methanol-d4) δ 7.06 (d, J = 8.4 Hz, 1H), 6.61 - 6.50 (m, 2H), 4.77 (s, 2H), 4.08 - 3.89 (m, 5H), 3.79 - 3.40 (m, 5H), 3.34 - 3.27 (m, 5H), 3.25 - 3.11 (m, 1H), 2.95 (s, 8H), 2.60 - 2.47 (m, 1H), 2.37 (s, 3H), 2.33 - 2.19 (m, 1H), 1.93 (d, J = 8.9 Hz, 2H). |
| 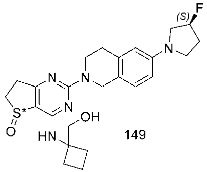 | 472.15 | ¹H NMR (400 MHz, Methanol-d4) δ 7.04 (d, J = 8.3 Hz, 1H), 6.54 - 6.44 (m, 2H), 4.75 (s, 2H), 3.93 (d, J = 29.3 Hz, 4H), 3.79 (dt, J = 18.0, 7.5 Hz, 2H), 3.63 - 3.30 (m, 7H), 3.27 - 3.16 (m, 1H), 2.96 (s, 2H), 2.41 (q, J = 8.9, 8.2 Hz, 5H), 2.32 - 2.21 (m, 1H), 1.93 (q, J = 8.4 Hz, 2H), 1.33 - 1.24 (m, 1H). |
| 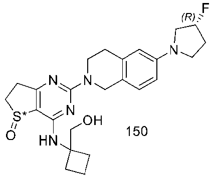 | 472.15 | ¹H NMR (400 MHz, DMSO-d₆) δ 6.99 (d, 1H), 6.45 - 6.40 (m, 1H), 6.37 (d, 1H), 5.40 (d, 1H), 4.70 (s, 2H), 3.85 (s, 2H), 3.74 - 3.72 (m, 2H), 3.42 (d, 2H), 3.33 - 3.23 (m, 4H), 3.05 (d, 1H), 2.90 (dd, 1H), 2.79 (s, 2H), 2.32 (dd, 2H), 2.20 (s, 4H), 1.77 (s, 2H). |
| 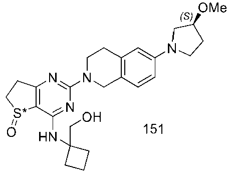 | 484.20 | ¹H NMR (400 MHz, DMSO-d₆) δ 6.96 (d, 1H), 6.39 (d, 1H), 6.33 (s, 1H), 4.69 (s, 2H), 4.03 (s, 1H), 3.84 (t, 2H), 3.73 (s, 2H), 3.36 (q, 2H), 3.26 - 3.15 (m, 9H), 3.05 (q, 1H), 2.91 (q, 1H), 2.78 (t, 2H), 2.36 - 2.28 (m, 2H), 2.23 - 2.17 (m, 2H), 2.04 - 1.98 (m, 2H), 1.80 - 1.73 (m, 2H). |
| 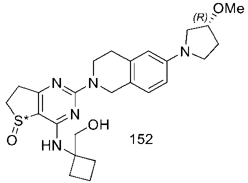 | 484.15 | ¹H NMR (400 MHz, DMSO-d₆) δ 6.96 (d, 1H), 6.38 (d, 1H), 6.33 (s, 1H), 4.69 (s, 2H), 4.03 (s, 1H), 3.84 (t, 2H), 3.73 (s, 2H), 3.36 (q, 2H), 3.28 - 3.14 (m, 8H), 3.07 - 3.00 (m, 1H), 2.90 (q, 1H), 2.79 (t, 2H), 2.31 (q, 2H), 2.26 - 2.14 (m, 2H), 2.06 - 1.96 (m, 2H), 1.84 - 1.71 (m, 2H). |
| 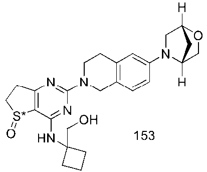 | 482.35 | ¹H NMR (399 MHz, Methanol-d4) δ 7.05 (d, J = 8.3 Hz, 1H), 6.58 - 6.50 (m, 2H), 4.77 (s, 2H), 4.63 (d, J = 2.2 Hz, 1H), 4.50 (d, J = 2.1 Hz, 1H), 3.98 (s, 4H), 3.81 (d, J = 3.8 Hz, 2H), 3.80 - 3.72 (m, 1H), 3.57 - 3.46 (m, 2H), 3.42 - 3.34 (m, 1H), 3.21 (ddd, J = 13.8, 7.5, 1.7 Hz, 1H), 3.09 - 3.04 (m, 1H), 2.95 (s, 2H), 2.42 (q, J = 8.6, 7.7 Hz, 4H), 2.04 - 1.89 (m, 4H). |
| 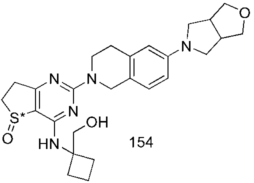 | 496.15 | ¹H NMR (400 MHz, Methanol-d4) δ 7.05 (d, J = 8.3 Hz, 1H), 6.67 - 6.57 (m, 2H), 4.76 (s, 2H), 3.97 - 3.90 (m, 5H), 3.80 - 3.75 (m, 1H), 3.65 (dd, J = 9.0, 3.2 Hz, 2H), 3.51 - 3.34 (m, 4H), 3.20 (ddd, J = 12.4, 8.0, 4.5 Hz, 4H), 3.10 - 3.01 (m, 2H), 2.95 (s, 2H), 2.38 (d, J = 8.5 Hz, 4H), 1.97 - 1.90 (m, 2H). |
| 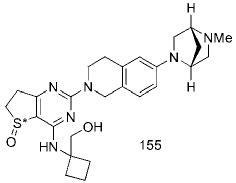 | 495.40 | ¹H NMR (400 MHz, Methanol-d4) δ 7.08 (d, J = 8.3 Hz, 1H), 6.61 - 6.51 (m, 2H), 4.77 (s, 2H), 4.64 (s, 1H), 4.33 (s, 1H), 4.00 - 3.83 (m, 4H), 3.80 - 3.72 (m, 2H), 3.69 (d, J = 7.7 Hz, 1H), 3.50 - 3.41 (m, 1H), 3.36 - 3.29 (m, 2H), 3.18 - 3.14 (m, 1H), 2.94 (s, 3H), 2.83 (s, 1H), 2.46 - 2.20 (m, 6H), 1.92 (q, J = 9.0 Hz, 2H). |
| 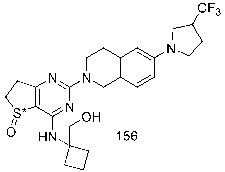 | 522.20 | ¹H NMR (400 MHz, Methanol-d4) δ 7.04 (d, J = 8.4 Hz, 1H), 6.57 - 6.44 (m, 2H), 4.76 (s, 2H), 3.96 (s, 4H), 3.75 (d, J = 17.8 Hz, 1H), 3.59 - 3.44 (m, 2H), 3.41 - 3.30 (m, 4H), 3.23 - 3.13 (m, 2H), 2.94 (s, 2H), 2.38 (d, J = 8.9 Hz, 4H), 2.30 (d, J = 7.7 Hz, 1H), 2.16 (s, 1H), 1.93 (d, J = 8.3 Hz, 2H). |
| 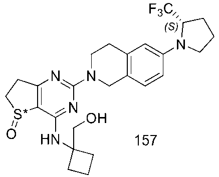 | 522.45 | ¹H NMR (400 MHz, DMSO-d₆) δ 7.01 (d, J = 8.4 Hz, 1H), 6.64 (d, J = 8.5 Hz, 1H), 6.59 (s, 1H), 4.72 (s, 2H), 4.52 (s, 3H), 3.86 (dt, J = 18.3, 6.3 Hz, 2H), 3.73 (d, J = 2.0 Hz, 2H), 3.58 - 3.46 (m, 2H), 3.23 (dd, J = 14.3, 7.6 Hz, 1H), 3.10 - 3.02 (m, 2H), 2.91 (dd, J = 13.8, 7.1 Hz, 1H), 2.80 (s, 2H), 2.31 (dd, J = 20.4, 10.2 Hz, 2H), 2.20 (s, 2H), 2.02 (d, J = 19.8 Hz, 4H), 1.81 - 1.72 (m, 2H). |
| 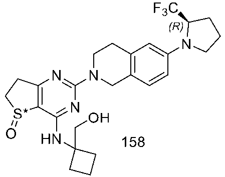 | 522.30 | ¹H NMR (400 MHz, DMSO-d₆) δ 7.01 (d, J = 8.4 Hz, 1H), 6.64 (dd, J = 8.3, 2.5 Hz, 1H), 6.59 (s, 1H), 4.72 (s, 2H), 4.46 (d, J = 48.1 Hz, 2H), 3.91 - 3.81 (m, 3H), 3.73 (d, J = 2.0 Hz, 2H), 3.56 - 3.44 (m, 2H), 3.23 (dd, J = 15.0, 6.9 Hz, 1H), 3.10 - 3.02 (m, 2H), 2.90 (dd, J = 13.6, 7.1 Hz, 1H), 2.79 (d, J = 6.2 Hz, 2H), 2.30 (dt, J = 20.4, 10.2 Hz, 2H), 2.20 (s, 2H), 2.02 (d, J = 19.8 Hz, 4H), 1.82 - 1.70 (m, 2H). |
| 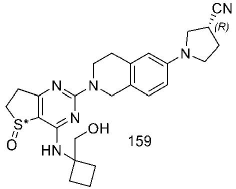 | 479.15 | ¹H NMR (400 MHz, Methanol-d4) δ 7.01 (d, J = 8.4 Hz, 1H), 6.50 (dd, J = 8.3, 2.5 Hz, 1H), 6.43 (d, J = 2.5 Hz, 1H), 4.79 (s, 2H), 4.01 - 3.92 (m, 4H), 3.58 (ddd, J = 9.6, 7.9, 5.4 Hz, 2H), 3.51 - 3.41 (m, 3H), 3.39 - 3.32 (m, 2H), 3.11 - 3.02 (m, 2H), 2.85 (t, J = 5.9 Hz, 2H), 2.42 - 2.26 (m, 6H), 1.97 - 1.86 (m, 2H). |
| 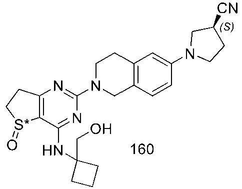 | 479.15 | ¹H NMR (400 MHz, Methanol-d4) δ 7.01 (d, J = 8.4 Hz, 1H), 6.50 (dd, J = 8.3, 2.6 Hz, 1H), 6.43 (d, J = 2.4 Hz, 1H), 4.79 (s, 2H), 4.01 - 3.92 (m, 4H), 3.58 (ddd, J = 9.4, 7.8, 4.9 Hz, 2H), 3.51 - 3.41 (m, 3H), 3.36 (ddt, J = 11.1, 6.2, 3.1 Hz, 2H), 3.10 - 3.02 (m, 2H), 2.85 (t, J = 6.0 Hz, 2H), 2.42 - 2.28 (m, 6H), 1.96 - 1.86 (m, 2H). |
| 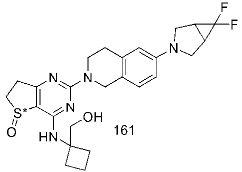 | 502.20 | ¹H NMR (400 MHz, Methanol-d4) δ 7.02 (d, J = 8.4 Hz, 1H), 6.47 - 6.37 (m, 2H), 4.75 (s, 2H), 3.96 (d, J = 1.9 Hz, 3H), 3.73 (dd, J = 16.6, 8.7 Hz, 1H), 3.61 (d, J = 9.7 Hz, 2H), 3.55 - 3.43 (m, 3H), 3.38 - 3.30 (m, 1H), 3.18 (dd, J = 14.2, 6.9 Hz, 1H), 2.93 (s, 2H), 2.50 (d, J = 12.4 Hz, 2H), 2.37 (d, J = 9.0 Hz, 4H), 2.01 (s, 1H), 1.93 (q, J = 8.9 Hz, 2H), 1.31 (s, 2H). |
| 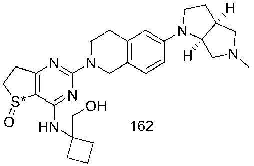 | 509.25 | ¹H NMR (400 MHz, DMSO-d₆) δ 7.67 (s, 1H), 7.02 (d, J = 8.3 Hz, 1H), 6.46 (d, J = 8.3 Hz, 1H), 6.39 (t, J = 3.5 Hz, 1H), 4.72 (s, 2H), 3.85 (s, 1H), 3.72 (s, 2H), 3.58 (d, J = 4.1 Hz, 1H), 3.38 (s, 2H), 3.21 (dt, J = 15.8, 7.8 Hz, 6H), 2.99 (dt, J = 17.7, 8.7 Hz, 3H), 2.87 (dd, J = 13.6, 7.3 Hz, 2H), 2.78 (d, J = 4.1 Hz, 6H), 2.33 - 2.27 (m, 2H), 2.18 (t, J = 4.5 Hz, 2H), 1.87 (t, J = 6.5 Hz, 1H), 1.78 - 1.73 (m, 2H). |
| 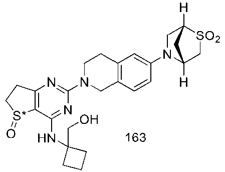 | 530.15 | ¹H NMR (400 MHz, DMSO-d₆) δ 7.29 (s, 1H), 7.01 (d, J = 8.2 Hz, 1H), 6.47 - 6.44 (m, 1H), 6.40 (d, J = 2.5 Hz, 1H), 4.82 (s, 1H), 4.72 (s, 1H), 4.65 (s, 1H), 3.98 (s, 1H), 3.89 (s, 1H), 3.71 (d, J = 5.0 Hz, 2H), 3.64 - 3.60 (m, 1H), 3.55 (d, J = 10.4 Hz, 1H), 3.41 - 3.35 (m, 2H), 3.14 (dd, J = 25.1, 11.5 Hz, 6H), 2.93 - 2.88 (m, 1H), 2.82 (d, J = 6.4 Hz, 1H), 2.75 (d, J = 6.1 Hz, 2H), 2.35 - 2.29 (m, 2H), 2.17 (s, 2H), 1.76 (d, J = 8.9 Hz, 2H). |
| 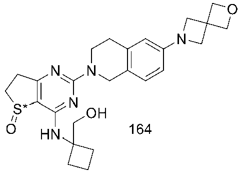 | 482.35 | ¹H NMR (400 MHz, DMSO-d₆) δ 8.10 (s, 1H), 6.97 (t, J = 6.8 Hz, 1H), 6.31 - 6.22 (m, 2H), 4.70 (s, 2H), 4.67 (s, 2H), 4.55 (s, 1H), 3.89 (s, 2H), 3.84 (s, 2H), 3.72 (s, 2H), 3.57 (d, J = 2.4 Hz, 1H), 3.49 (s, 1H), 3.36 (d, J = 23.4 Hz, 1H), 3.24 (s, 1H), 3.12 - 3.04 (m, 1H), 2.94 - 2.88 (m, 1H), 2.78 (s, 2H), 2.36 - 2.27 (m, 2H), 2.20 (s, 2H), 1.80 - 1.72 (m, 2H). |
| 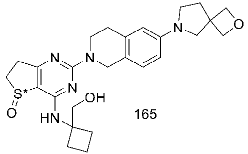 | 496.25 | ¹H NMR (400 MHz, Methanol-d4) δ 7.02 (d, J = 8.3 Hz, 1H), 6.53 - 6.42 (m, 2H), 4.77 (s, 3H), 4.76 - 4.62 (m, 5H), 4.03 - 3.91 (m, 4H), 3.54 (s, 2H), 3.21 (dd, J = 25.7, 7.9 Hz, 2H), 2.92 (s, 2H), 2.44 - 2.35 (m, 4H), 2.32 (t, J = 6.9 Hz, 2H), 1.31 (d, J = 16.6 Hz, 6H). |
| 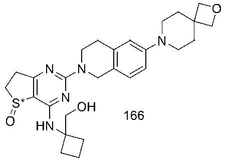 | 510.25 | ¹H NMR (400 MHz, Methanol-d4) δ 7.64 - 7.54 (m, 2H), 7.43 (dd, J = 12.7, 8.4 Hz, 1H), 4.96 (d, J = 8.0 Hz, 2H), 4.36 - 4.27 (m, 2H), 4.04 (t, J = 5.9 Hz, 2H), 3.94 (d, J = 7.0 Hz, 4H), 3.83 (d, J = 16.0 Hz, 4H), 3.64 (dd, J = 19.1, 11.4 Hz, 3H), 3.41 (dt, J = 10.7, 8.2 Hz, 1H), 3.26 - 3.07 (m, 2H), 3.03 (t, J = 5.6 Hz, 2H), 2.42 - 2.32 (m, 6H), 2.00 (s, 1H), 1.97 - 1.89 (m, 3H). |
| 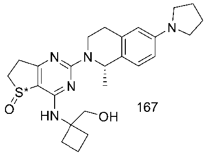 | 468.35 | ¹H NMR (399 MHz, DMSO-d₆) δ 7.25 (d, J = 13.1 Hz, 1H), 6.99 (d, J = 8.3 Hz, 1H), 6.40 (s, 1H), 6.27 (s, 1H), 5.60 (d, J = 55.7 Hz, 1H), 4.84 (s, 1H), 4.52 (d, J = 63.8 Hz, 1H), 3.75 - 3.72 (m, 2H), 3.46 - 3.39 (m, 1H), 3.16 (d, J = 6.9 Hz, 5H), 2.95 - 2.82 (m, 2H), 2.73 (s, 2H), 2.34 (d, J = 21.2 Hz, 2H), 2.18 (s, 2H), 2.07 (s, 1H), 1.91 (d, J = 6.7 Hz, 4H), 1.80 (s, 2H), 1.35 (s, 3H). |
| 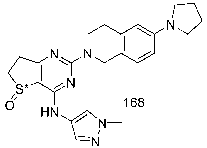 | 450.30 | ¹H NMR (400 MHz, DMSO-d₆) δ 9.66 (s, 1H), 7.89 (s, 1H), 7.64 (s, 1H), 7.01 (d, J = 8.3 Hz, 1H), 6.39 (d, J = 8.4 Hz, 1H), 6.33 (s, 1H), 4.76 (s, 2H), 3.93 (s, 2H), 3.82 (s, 3H), 3.52 - 3.42 (m, 1H), 3.23 (s, 1H), 3.16 (s, 4H), 3.00 (d, J = 9.1 Hz, 1H), 2.90 (d, J = 6.5 Hz, 1H), 2.80 (s, 2H), 1.90 (s, 4H). |
| 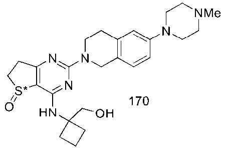 | 483.40 | ¹H NMR (400 MHz, DMSO-d₆) δ 9.87 (s, 1H), 7.70 (s, 1H), 7.07 (d, J = 8.4 Hz, 1H), 6.84 (dd, J = 8.4, 2.5 Hz, 1H), 6.78 (d, J = 2.5 Hz, 1H), 4.74 (s, 2H), 3.88 (s, 2H), 3.77 (d, J = 13.5 Hz, 2H), 3.72 (s, 2H), 3.48 (s, 2H), 3.44 - 3.39 (m, 1H), 3.23 - 3.17 (m, 1H), 3.10 (d, J = 10.8 Hz, 2H), 3.02 - 2.95 (m, 1H), 2.91 (d, J = 8.2 Hz, 1H), 2.88 - 2.84 (m, 2H), 2.83 - 2.81 (m, 3H), 2.78 (d, J = 5.9 Hz, 2H), 2.32 (ddd, J = 20.7, 9.7, 3.0 Hz, 2H), 2.19 (td, J = 8.9, 8.3, 4.5 Hz, 2H), 1.80 - 1.72 (m, 2H). |
| 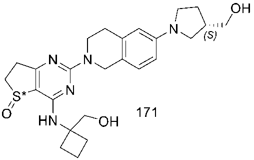 | 484.15 | ¹H NMR (400 MHz, Methanol-d4) δ 7.04 (d, J = 8.3 Hz, 1H), 6.58 - 6.48 (m, 2H), 4.77 (d, J = 11.6 Hz, 5H), 3.96 (s, 2H), 3.90 (s, 1H), 3.75 (dd, J = 17.7, 7.9 Hz, 1H), 3.65 - 3.30 (m, 6H), 3.24 - 3.06 (m, 3H), 2.94 (s, 2H), 2.57 - 2.48 (m, 1H), 2.39 (s, 3H), 1.93 (d, J = 9.2 Hz, 4H), 1.29 (d, J = 16.5 Hz, 2H). |
| 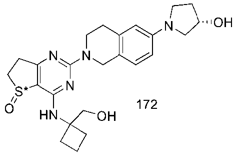 | 470.20 | ¹H NMR (400 MHz, Methanol-d4) δ 7.04 (d, J = 8.3 Hz, 1H), 6.56 - 6.46 (m, 2H), 4.75 (s, 2H), 4.52 (s, 1H), 3.96 (s, 4H), 3.85 - 3.71 (m, 2H), 3.56 - 3.29 (m, 6H), 3.21 (t, J = 9.1 Hz, 3H), 2.95 (s, 2H), 2.40 (q, J = 8.7, 8.2 Hz, 5H), 2.02 (s, 1H), 1.92 (t, J = 8.5 Hz, 2H). |
| 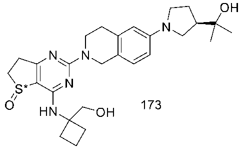 | 512.50 | ¹H NMR (400 MHz, Methanol-d4) δ 7.09 (d, J = 8.2 Hz, 1H), 6.70 - 6.62 (m, 2H), 4.78 (s, 3H), 3.93 (d, J = 25.6 Hz, 4H), 3.77 (dt, J = 17.8, 7.5 Hz, 1H), 3.56 - 3.30 (m, 7H), 3.26 - 3.15 (m, 2H), 2.97 (s, 2H), 2.52 - 2.39 (m, 3H), 2.09 - 1.95 (m, 2H), 1.95 - 1.88 (m, 2H), 1.29 (d, J = 16.7 Hz, 2H), 1.24 (s, 6H). |
| 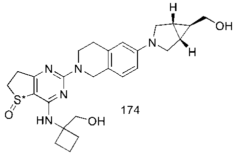 | 496.15 | ¹H NMR (400 MHz, DMSO-d₆) δ 6.93 (d, 1H), 6.37 (d, 1H), 6.31 (s, 1H), 4.68 (s, 2H), 3.84 (t, 2H), 3.72 (s, 2H), 3.42 (d, 2H), 3.28 (d, 2H), 3.22 - 3.15 (m, 1H), 3.08 (d, 2H), 3.02 - 2.95 (m, 2H), 2.86 (d, 1H), 2.75 (t, 2H), 2.38 - 2.26 (m, 3H), 2.18 (s, 2H), 1.83 - 1.69 (m, 3H), 1.53 (s, 2H), 0.83 (s, 1H). |
| 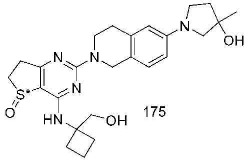 | 484.40 | ¹H NMR (400 MHz, DMSO-d₆) δ 8.20 (s, 1H), 6.95 (d, J = 8.3 Hz, 1H), 6.33 (dd, J = 8.4, 2.5 Hz, 1H), 6.28 (d, J = 2.4 Hz, 1H), 4.68 (s, 2H), 3.83 (d, J = 6.1 Hz, 3H), 3.73 (d, J = 2.4 Hz, 3H), 3.55 - 3.48 (m, 1H), 3.31 - 3.19 (m, 3H), 3.14 - 3.07 (m, 3H), 2.95 - 2.89 (m, 1H), 2.79 (s, 2H), 2.31 (dd, J = 21.5, 10.9 Hz, 2H), 2.21 (s, 2H), 1.91 - 1.81 (m, 2H), 1.79 - 1.71 (m, 2H), 1.30 (s, 3H). |
| 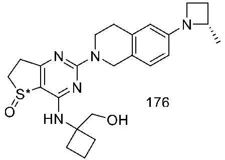 | 454.10 | ¹H NMR (400 MHz, DMSO-d₆) δ 7.00 (d, J = 8.3 Hz, 1H), 6.42 - 6.35 (m, 2H), 4.71 (s, 2H), 4.04 (d, J = 7.7 Hz, 1H), 3.84 (s, 2H), 3.73 (s, 2H), 3.48 (s, 4H), 3.24 (dd, J = 14.7, 7.5 Hz, 2H), 3.08 (d, J = 11.3 Hz, 1H), 2.92 (d, J = 6.9 Hz, 1H), 2.79 (s, 2H), 2.33 - 2.18 (m, 6H), 1.97 (t, J = 9.2 Hz, 1H), 1.79 - 1.73 (m, 2H), 1.36 (d, J = 6.0 Hz, 2H). |
| 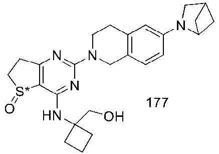 | 466.20 | ¹H NMR (400 MHz, DMSO-d₆) δ 7.11 (d, J = 8.2 Hz, 1H), 6.85 (d, J = 10.3 Hz, 2H), 4.76 (s, 1H), 4.46 (d, J = 6.5 Hz, 1H), 3.87 (d, J = 6.0 Hz, 2H), 3.72 (s, 2H), 3.50 - 3.43 (m, 4H), 3.40 (s, 2H), 3.25 - 3.21 (m, 1H), 3.03 (d, J = 9.2 Hz, 1H), 2.89 (t, J = 5.9 Hz, 2H), 2.82 (d, J = 6.1 Hz, 2H), 2.32 - 2.18 (m, 4H), 1.97 (s, 2H), 1.80 - 1.73 (m, 2H), 1.37 (d, J = 4.9 Hz, 2H). |
| 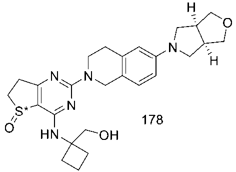 | 496.25 | ¹H NMR (400 MHz, DMSO-d₆) δ 6.99 (d, J = 8.3 Hz, 1H), 6.50 (d, J = 8.4 Hz, 1H), 6.45 (s, 1H), 4.71 (s, 2H), 3.85 (s, 3H), 3.57 - 3.48 (m, 6H), 3.31 - 3.25 (m, 4H), 3.10 (dd, J = 9.9, 2.8 Hz, 4H), 2.95 (t, J = 6.3 Hz, 3H), 2.81 (t, J = 6.2 Hz, 2H), 2.32 - 2.21 (m, 4H), 1.80 - 1.74 (m, 2H). |
| 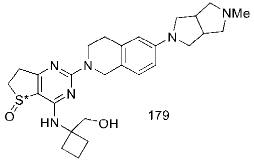 | 509.30 | ¹H NMR (400 MHz, DMSO-d₆) δ 7.60 (s, 1H), 7.02 (d, J = 8.3 Hz, 1H), 6.57 - 6.48 (m, 2H), 4.72 (s, 2H), 3.87 (s, 2H), 3.72 (s, 2H), 3.30 - 3.19 (m, 9H), 3.03 - 2.94 (m, 4H), 2.88 (d, J = 7.1 Hz, 1H), 2.83 (d, J = 4.8 Hz, 2H), 2.77 (s, 4H), 2.35 - 2.28 (m, 2H), 2.21 - 2.16 (m, 2H), 1.75 (dd, J = 15.4, 6.2 Hz, 2H). |
| 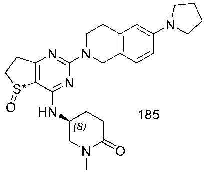 | 481.25 | ¹H NMR (400 MHz, DMSO-d₆) δ 7.75 (d, J = 7.0 Hz, 1H), 6.97 (d, J = 8.3 Hz, 1H), 6.37 (dd, J = 8.2, 2.4 Hz, 1H), 6.30 (d, J = 2.4 Hz, 1H), 4.72 (s, 2H), 4.51 - 4.45 (m, 1H), 3.90 (s, 2H), 3.43 (dd, J = 17.7, 9.1 Hz, 2H), 3.25 - 3.18 (m, 2H), 3.14 (d, J = 6.6 Hz, 4H), 2.97 - 2.85 (m, 2H), 2.76 (d, J = 9.5 Hz, 5H), 2.31 (t, J = 6.6 Hz, 2H), 1.89 (p, J = 3.1 Hz, 6H). |
| 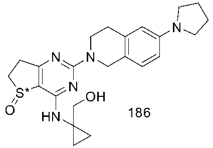 | 440.05 | ¹H NMR (399 MHz, DMSO-d₆) δ 8.63 (s, 1H), 7.00 (d, J = 8.3 Hz, 1H), 6.44 (dd, J = 8.3, 2.5 Hz, 1H), 6.40 (d, J = 2.4 Hz, 1H), 4.76 (s, 2H), 3.66 (d, J = 11.1 Hz, 2H), 3.60 - 3.47 (m, 3H), 3.34 - 3.24 (m, 1H), 3.23 - 3.09 (m, 6H), 2.98 - 2.93 (m, 1H), 2.84 (t, J = 6.1 Hz, 2H), 1.97 - 1.90 (m, 4H), 0.87 (s, 2H), 0.79 - 0.67 (m, 2H). |
| 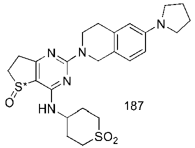 | 502.35 | ¹H NMR (400 MHz, DMSO-d₆) δ 7.74 (d, 1H), 7.02 (s, 1H), 6.37 (d, 1H), 6.30 (s, 1H), 4.73 (s, 2H), 4.36 (s, 1H), 3.91 (s, 2H), 3.47 - 3.39 (m, 2H), 3.23 - 3.05 (m, 8H), 2.99 - 2.80 (m, 2H), 2.75 (s, 2H), 2.16 - 2.07 (m, 4H), 1.89 (t, 4H). |
| 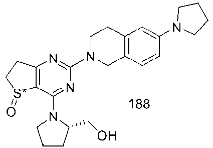 | 454.15 | ¹H NMR (400 MHz, Methanol-d4) δ 6.97 (d, J = 8.4 Hz, 1H), 6.47 - 6.40 (m, 1H), 6.35 (d, J = 2.2 Hz, 1H), 4.83 - 4.71 (m, 3H), 4.02 - 3.86 (m, 3H), 3.80 - 3.71 (m, 2H), 3.60 (dt, J = 17.0, 8.2 Hz, 2H), 3.26 - 3.14 (m, 6H), 3.14 - 3.06 (m, 2H), 2.82 (t, J = 6.0 Hz, 2H), 2.20 - 2.12 (m, 2H), 1.98 (p, J = 3.3 Hz, 5H), 1.27 (s, 1H). |
| 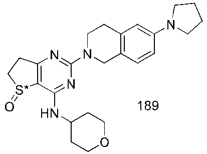 | 454.10 | ¹H NMR (400 MHz, DMSO-d₆) δ 7.52 (d, J = 7.4 Hz, 1H), 6.96 (d, J = 8.3 Hz, 1H), 6.37 (dd, J = 8.4, 2.5 Hz, 1H), 6.29 (d, J = 2.4 Hz, 1H), 4.71 (s, 2H), 4.18 (s, 1H), 3.87 (t, J = 8.7 Hz, 4H), 3.41 (d, J = 7.9 Hz, 2H), 3.19 - 3.12 (m, 6H), 2.95 - 2.83 (m, 2H), 2.74 (t, J = 6.1 Hz, 2H), 1.91 - 1.87 (m, 4H), 1.77 (d, J = 12.6 Hz, 2H), 1.59 (td, J = 11.7, 4.1 Hz, 2H). |
| 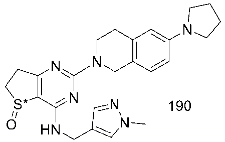 | 464.35 | ¹H NMR (399 MHz, DMSO-d₆) δ 8.80 (s, 1H), 7.57 (s, 1H), 7.36 (s, 1H), 7.04 (d, J = 7.9 Hz, 1H), 6.44 (dd, J = 8.3, 2.5 Hz, 1H), 6.39 (d, J = 2.4 Hz, 1H), 4.78 (s, 2H), 4.45 (d, J = 5.8 Hz, 2H), 3.55 - 3.51 (m, 3H), 3.36 - 3.30 (m, 2H), 3.19 (s, 3H), 3.12 (dd, J = 17.3, 8.2 Hz, 2H), 3.00 - 2.91 (m, 2H), 2.84 (t, J = 6.1 Hz, 3H), 1.92 (q, J = 3.3 Hz, 4H). |
| 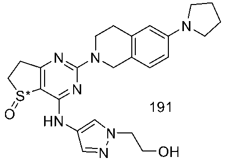 | 480.40 | ¹H NMR (400 MHz, DMSO-d₆) δ 9.69 (s, 1H), 8.04 (d, 1H), 7.63 (s, 1H), 7.02 (s, 1H), 6.38 (d, 1H), 6.32 (s, 1H), 4.96 (s, 1H), 4.76 (s, 2H), 4.12 (s, 2H), 3.93 (s, 2H), 3.78 - 3.67 (m, 2H), 3.47 (q, 1H), 3.26 - 3.20 (m, 1H), 3.15 (t, 4H), 3.00 (q, 1H), 2.90 (q, 1H), 2.79 (s, 2H), 1.90 (t, 4H). |
| 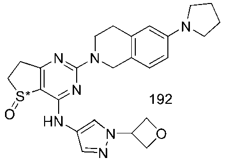 | 492.35 | ¹H NMR (400 MHz, DMSO-d₆) δ 9.76 (s, 1H), 8.16 (d, 1H), 7.79 (s, 1H), 7.02 (s, 1H), 6.43 - 6.31 (m, 2H), 5.57 (p, 1H), 4.94 (s, 2H), 4.87 (t, 2H), 4.77 (s, 2H), 3.94 (s, 2H), 3.48 (q, 1H), 3.25 - 3.21 (m, 1H), 3.15 (t, 4H), 3.01 (q, 1H), 2.91 (q, 1H), 2.81 (s, 1H), 1.90 (t, 4H). |
| 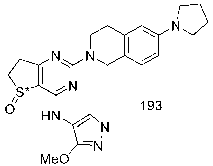 | 480.15 | ¹H NMR (400 MHz, DMSO-d₆) δ 8.67 (s, 1H), 7.57 (s, 1H), 6.94 (s, 1H), 6.37 (d, J = 8.5 Hz, 1H), 6.29 (s, 1H), 4.69 (s, 2H), 3.86 (s, 2H), 3.73 (s, 3H), 3.67 (s, 3H), 3.14 (d, J = 6.3 Hz, 6H), 2.98 - 2.82 (m, 2H), 2.73 (s, 2H), 1.89 (d, J = 3.5 Hz, 4H). |
| 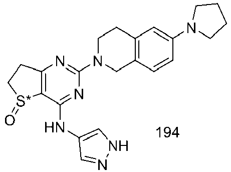 | 436.35 | ¹H NMR (400 MHz, DMSO-d₆) δ 10.13 (s, 1H), 7.88 (s, 2H), 7.01 (d, J = 8.3 Hz, 1H), 6.47 - 6.38 (m, 2H), 4.76 (s, 2H), 3.64 - 3.52 (m, 3H), 3.34 - 3.28 (m, 1H), 3.24 - 3.08 (m, 6H), 3.00 - 2.94 (m, 1H), 2.84 (t, J = 5.9 Hz, 2H), 1.93 - 1.85 (m, 4H). |
| 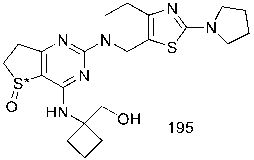 | 461.10 | ¹H NMR (400 MHz, Methanol-d4) δ 4.82 (s, 2H), 4.14 (d, J = 6.2 Hz, 2H), 3.91 (q, J = 11.4 Hz, 2H), 3.58 (q, J = 9.0, 6.9 Hz, 5H), 3.38 (d, J = 14.1 Hz, 1H), 3.17 - 3.04 (m, 2H), 2.76 - 2.68 (m, 2H), 2.37 - 2.29 (m, 4H), 2.20 - 2.14 (m, 4H), 1.96 - 1.85 (m, 2H). |
| 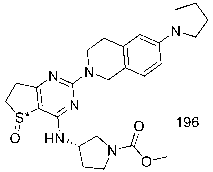 | 497.35 | ¹H NMR (399 MHz, DMSO-d₆) δ 7.88 (d, J = 6.0 Hz, 1H), 6.99 (d, J = 8.3 Hz, 1H), 6.39 (d, J = 8.4 Hz, 1H), 6.33 (s, 1H), 4.74 (s, 2H), 4.60 (s, 1H), 3.92 (s, 1H), 3.65 (d, J = 8.9 Hz, 1H), 3.57 (d, J = 5.0 Hz, 2H), 3.43 (dt, J = 16.4, 7.9 Hz, 2H), 3.22 (d, J = 8.4 Hz, 1H), 3.16 (d, J = 6.7 Hz, 4H), 2.97 (dd, J = 17.1, 8.2 Hz, 2H), 2.87 (d, J = 6.4 Hz, 1H), 2.77 (t, J = 5.9 Hz, 2H), 2.14 (d, J = 6.7 Hz, 1H), 2.03 (d, J = 6.5 Hz, 1H), 1.91 (d, J = 6.7 Hz, 4H), 1.22 (s, 2H), 0.84 (s, 1H). |
| 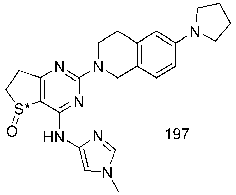 | 450.35 | ¹H NMR (400 MHz, DMSO-d₆) δ 10.22 (s, 1H), 8.58 (s, 1H), 7.48 (d, J = 16.9 Hz, 2H), 7.01 (d, J = 8.3 Hz, 1H), 6.39 (d, J = 8.5 Hz, 1H), 6.33 (d, J = 2.4 Hz, 1H), 4.72 (d, J = 17.4 Hz, 2H), 3.54 - 3.47 (m, 2H), 3.32 - 3.25 (m, 2H), 3.16 (s, 3H), 3.07 (dd, J = 17.2, 8.1 Hz, 2H), 2.98 - 2.93 (m, 2H), 2.79 (s, 2H), 1.90 (d, J = 3.4 Hz, 4H). |
| 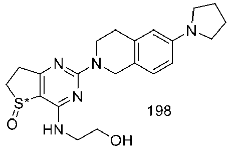 | 414.05 | ¹H NMR (400 MHz, DMSO-d₆) δ 8.47 (s, 2H), 7.50 (s, 1H), 6.97 (d, J = 8.5 Hz, 1H), 6.37 (d, J = 8.3 Hz, 1H), 6.30 (s, 1H), 4.72 (s, 2H), 3.90 (t, J = 5.9 Hz, 2H), 3.55 (d, J = 6.9 Hz, 2H), 3.45 - 3.38 (m, 2H), 3.24 - 3.19 (m, 1H), 3.15 (s, 2H), 3.09 (s, 2H), 2.93 (d, J = 8.9 Hz, 1H), 2.86 - 2.81 (m, 1H), 2.75 (s, 2H), 1.89 (d, J = 6.5 Hz, 4H). |
| 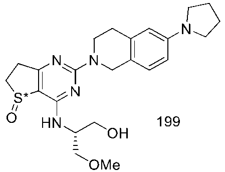 | 458.10 | ¹H NMR (400 MHz, Methanol-d4) δ 6.98 (d, J = 8.3 Hz, 1H), 6.46 (dd, J = 8.2, 2.5 Hz, 1H), 6.38 (d, J = 2.5 Hz, 1H), 4.81 (s, 3H), 4.63 - 4.55 (m, 1H), 4.01 (t, J = 5.9 Hz, 2H), 3.73 (s, 2H), 3.66 - 3.58 (m, 3H), 3.46 - 3.33 (m, 5H), 3.16 - 3.02 (m, 2H), 2.85 (t, J = 5.9 Hz, 2H), 2.01 (td, J = 8.2, 6.7, 5.0 Hz, 5H), 1.31 (d, J = 16.6 Hz, 3H). |
| 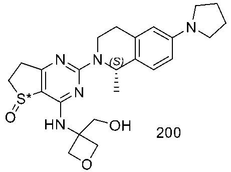 | 470.45 | ¹H NMR (400 MHz, DMSO-d₆) δ 8.10 (d, J = 7.2 Hz, 1H), 6.98 (t, J = 10.8 Hz, 1H), 6.37 (t, J = 10.1 Hz, 1H), 6.24 (s, 1H), 5.47 (d, J = 127.8 Hz, 1H), 5.15 (t, J = 5.8 Hz, 1H), 4.65 - 4.53 (m, 5H), 3.78 (d, J = 4.7 Hz, 2H), 3.47 - 3.35 (m, 2H), 3.14 (s, 5H), 2.93 - 2.82 (m, 2H), 2.69 (s, 2H), 1.88 (s, 4H), 1.33 - 1.24 (m, 3H). |
| 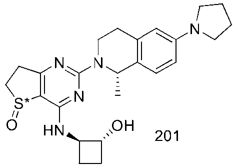 | 454.05 | ¹H NMR (400 MHz, DMSO-d₆) δ 7.03 (s, 1H), 6.44 (d, J = 8.1 Hz, 1H), 6.33 (s, 1H), 5.59 (s, 1H), 4.25 (d, J = 82.0 Hz, 3H), 3.57 - 3.51 (m, 2H), 3.42 (s, 2H), 3.26 (s, 1H), 3.19 (s, 4H), 3.10 (s, 1H), 2.95 (s, 1H), 2.80 (s, 2H), 1.96 (d, J = 30.9 Hz, 6H), 1.46 (s, 2H), 1.40 (d, J = 6.5 Hz, 3H). |
| 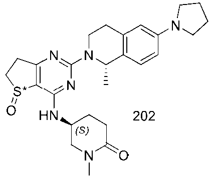 | 495.10 | ¹H NMR (400 MHz, Methanol-d4) δ 7.14 (d, J = 8.4 Hz, 1H), 6.75 (d, J = 8.5 Hz, 1H), 6.68 (s, 1H), 4.67 (s, 1H), 3.77 - 3.54 (m, 4H), 3.40 (d, J = 9.9 Hz, 7H), 3.16 (dd, J = 14.3, 7.2 Hz, 1H), 2.95 (s, 6H), 2.50 (t, J = 6.7 Hz, 2H), 2.08 (q, J = 6.3 Hz, 6H), 1.52 (d, J = 6.7 Hz, 3H). |
| 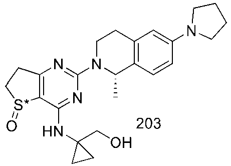 | 454.35 | ¹H NMR (400 MHz, DMSO-d₆) δ 8.66 (d, J = 94.2 Hz, 1H), 6.99 (d, J = 8.4 Hz, 1H), 6.43 (d, J = 8.4 Hz, 1H), 6.33 (s, 1H), 5.56 (s, 1H), 4.40 (d, J = 130.1 Hz, 1H), 3.71 - 3.38 (m, 4H), 3.16 (s, 6H), 3.09 - 2.66 (m, 4H), 1.89 (d, J = 6.2 Hz, 4H), 1.38 (s, 3H), 0.83 (s, 2H), 0.71 (s, 2H). |
| 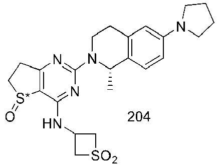 | 488.15 | ¹H NMR (400 MHz, DMSO-d₆) δ 8.38 (s, 1H), 7.04 (dd 1H), 6.39 (t 1H), 6.26 (s, 1H), 5.60 (d 1H), 4.66 - 4.52 (m, 4H), 4.51 - 4.40 (m, 1H), 4.40 - 4.30 (m, 2H), 3.44 (q, 1H), 3.15 (s, 5H), 3.00 - 2.85 (m, 2H), 2.73 (s, 2H), 1.89 (s, 4H), 1.33 (t, 3H). |
| 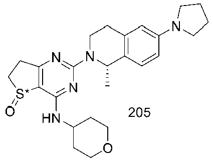 | 468.30 | ¹H NMR (400 MHz, DMSO-d₆) δ 7.53 (s, 1H), 6.99 (d, J = 8.4 Hz, 1H), 6.38 (d, J = 8.3 Hz, 1H), 6.25 (d, J = 2.4 Hz, 1H), 5.59 (d, J = 51.9 Hz, 1H), 4.53 (s, 1H), 4.17 (s, 1H), 3.87 (s, 2H), 3.46 - 3.32 (m, 4H), 3.14 (d, J = 6.7 Hz, 5H), 2.97 - 2.81 (m, 2H), 2.71 (d, J = 6.4 Hz, 2H), 1.92 - 1.86 (m, 4H), 1.82 - 1.70 (m, 2H), 1.59 (dd, J = 20.7, 8.9 Hz, 2H), 1.33 (d, J = 6.6 Hz, 3H). |
| 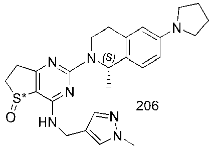 | 478.10 | ¹H NMR (400 MHz, DMSO-d₆) δ 7.99 (s, 1H), 7.51 (s, 1H), 7.31 (s, 1H), 7.01 (s, 1H), 6.39 (s, 1H), 6.26 (s, 1H), 5.64 (s, 1H), 4.59 (s, 1H), 4.43 - 4.30 (m, 2H), 3.74 - 3.70 (m, 3H), 3.39 (d, J = 8.9 Hz, 1H), 3.15 (t, J = 5.3 Hz, 6H), 2.90 (s, 1H), 2.82 (dd, J = 13.7, 7.3 Hz, 1H), 2.72 (s, 2H), 1.89 (d, J = 5.9 Hz, 4H), 1.33 (s, 3H). |
| 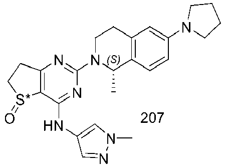 | 464.05 | ¹H NMR (400 MHz, Methanol-d4) δ 7.87 (s, 1H), 7.73 (s, 1H), 6.99 (d, J = 8.4 Hz, 1H), 6.46 (d, J = 8.5 Hz, 1H), 6.35 (s, 1H), 3.90 (s, 4H), 3.23 (s, 4H), 3.13 (d, J = 16.6 Hz, 2H), 2.85 (s, 2H), 1.99 (d, J = 6.3 Hz, 4H), 1.47 (d, J = 6.4 Hz, 3H), 1.29 (d, J = 17.1 Hz, 5H). |
| 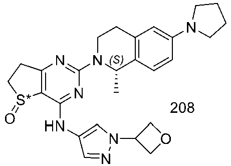 | 506.45 | ¹H NMR (400 MHz, DMSO-d₆) δ 8.29 (s, 1H), 8.07 (s, 1H), 7.78 (d, 1H), 7.08 - 7.00 (m, 1H), 6.45 - 6.35 (m, 1H), 6.29 (s, 1H), 5.71 - 5.52 (m, 2H), 5.00 (s, 1H), 4.91 (s, 1H), 4.85 (s, 2H), 3.57 - 3.39 (m, 2H), 3.16 (s, 5H), 3.04 - 2.88 (m, 3H), 2.79 (d, 2H), 1.90 (s, 4H), 1.40 (d, 3H). |
| 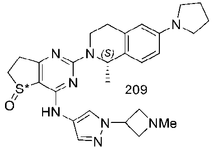 | 519.25 | ¹H NMR (400 MHz, DMSO-d₆) δ 9.77 (d, J = 17.7 Hz, 1H), 7.69 (d, J = 18.1 Hz, 1H), 7.03 (t, J = 9.9 Hz, 1H), 6.34 (d, J = 44.0 Hz, 2H), 4.97 (t, J = 6.3 Hz, 1H), 3.77 (s, 2H), 3.51 - 3.44 (m, 4H), 3.15 (s, 6H), 2.97 (d, J = 40.0 Hz, 3H), 2.79 (d, J = 26.0 Hz, 3H), 2.38 (s, 3H), 1.89 (s, 4H), 1.40 (d, J = 24.7 Hz, 3H). |
| 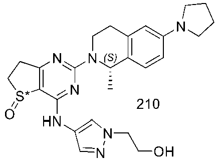 | 494.35 | ¹H NMR (400 MHz, DMSO-d₆) δ 8.03 (d, 1H), 7.63 (s, 1H), 7.03 (d, 1H), 6.41 (s, 1H), 6.30 (s, 1H), 5.61 (d, 1H), 4.49 (d, 1H), 4.12 (s, 2H), 3.71 (s, 2H), 3.30 - 3.23 (m, 2H), 3.17 (s, 4H), 3.08 - 2.99 (m, 2H), 2.97 - 2.89 (m, 2H), 2.79 (s, 2H), 1.89 (t, 4H), 1.40 (d, 3H). |
| 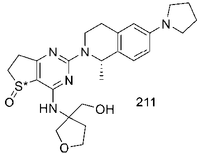 | 484.25 | ¹H NMR (400 MHz, DMSO-d₆) δ 7.89 - 7.63 (m, 1H), 7.01 (s, 1H), 6.42 (s, 1H), 6.31 (s, 1H), 5.53 (d 1H), 4.41 (s, 2H), 3.95 (s, 4H), 3.53 - 3.42 (m, 2H), 3.41 - 3.36 (m, 1H), 3.16 (s, 5H), 3.06 - 2.97 (m, 1H), 2.94 - 2.89 (m, 1H), 2.77 (s, 2H), 2.39 - 2.27 (m, 1H), 2.15 - 2.07 (m, 1H), 1.89 (t, 4H), 1.36 (s, 3H). |
| 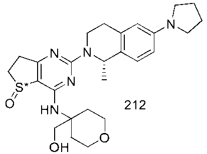 | 498.10 | ¹H NMR (400 MHz, DMSO-d₆) δ 6.98 (d, J = 8.2 Hz, 1H), 6.70 (d, J = 42.6 Hz, 1H), 6.42 (s, 1H), 6.31 (s, 1H), 5.48 (d, J = 54.3 Hz, 1H), δ 4.34 (d, J = 39.0 Hz, 1H), 3.74 (d, J = 12.3 Hz, 2H), 3.61 (s, 2H), 3.46 (dd, J = 16.5, 8.5 Hz, 4H), 3.30 (s, 2H), 3.15 (d, J = 6.4 Hz, 4H), 3.04 (s, 1H), 2.91 (s, 1H), 2.77 (s, 2H), 2.35 (d, J = 21.9 Hz, 2H), 1.91 (d, J = 6.0 Hz, 4H), 1.62 (d, J = 13.6 Hz, 2H), 1.36 (d, J = 12.7 Hz, 3H). |
| 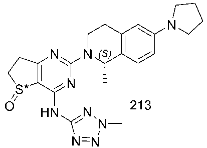 | 466.10 | ¹H NMR (400 MHz, DMSO-d₆) δ 10.69 (d, J = 24.2 Hz, 1H), 6.97 (dd, J = 42.8, 8.2 Hz, 1H), 6.42 (d, J = 8.5 Hz, 1H), 6.29 (d, J = 11.3 Hz, 1H), 5.64 (s, 1H), 5.39 (d, J = 7.0 Hz, 1H), 4.49 (d, J = 20.9 Hz, 1H), 4.34 (d, J = 8.0 Hz, 3H), 3.50 (s, 1H), 3.29 (dd, J = 32.5, 10.4 Hz, 2H), 3.16 (s, 3H), 3.08 (d, J = 11.3 Hz, 1H), 2.94 (dd, J = 13.5, 6.9 Hz, 1H), 2.69 (d, J = 28.2 Hz, 2H), 1.89 (s, 4H), 1.36 - 1.22 (m, 3H). |
| 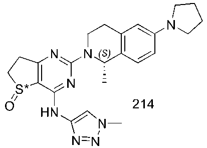 | 465.40 | ¹H NMR (400 MHz, DMSO-d₆) δ 10.40 (s, 1H), 8.08 (s, 1H), 7.27 - 6.95 (m, 1H), 6.39 (s, 1H), 6.28 (s, 1H), 5.66 (s, 1H), 4.32 (s, 1H), 4.09 (d, J = 15.3 Hz, 3H), 3.54 - 3.44 (m, 2H), 3.16 (s, 4H), 3.04 (s, 1H), 2.93 - 2.86 (m, 1H), 2.78 (d, J = 23.2 Hz, 2H), 1.90 (s, 4H), 1.37 (s, 3H). |
| 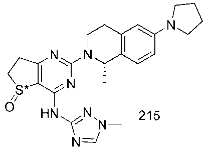 | 464.45 | ¹H NMR (400 MHz, Methanol-d4) δ 7.51 (s, 1H), 6.96 (s, 1H), 6.85 (s, 1H), 6.45 (d, J = 8.6 Hz, 1H), 6.31 (s, 1H), 6.25 (d, J = 2.1 Hz, 1H), 4.61 (s, 1H), 3.71 (s, 4H), 3.63 (s, 1H), 3.21 (d, J = 15.4 Hz, 6H), 2.79 (s, 1H), 2.73 (s, 1H), 1.98 (d, J = 6.4 Hz, 6H), 1.30 (s, 3H). |
| 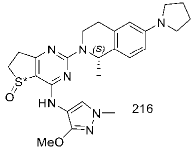 | 494.20 | ¹H NMR (400 MHz, DMSO-d₆) δ 8.66 (s, 1H), 7.55 (s, 1H), 6.94 (d, J = 36.9 Hz, 1H), 6.38 (d, J = 8.7 Hz, 1H), 6.25 (s, 1H), 3.70 (d, J = 25.2 Hz, 7H), 3.42 (s, 2H), 3.24 (s, 2H), 3.14 (s, 4H), 2.89 (dd, J = 46.9, 12.9 Hz, 2H), 2.70 (s, 2H), 1.89 (t, J = 6.2 Hz, 4H), 1.31 (s, 3H). |
| 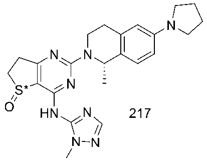 | 465.15 | ¹H NMR (400 MHz, Methanol-d4) δ 8.00 (s, 1H), 6.77 (s, 2H), 3.77 (s, 3H), 3.71 - 3.64 (m, 1H), 3.44 (s, 6H), 3.28 (s, 2H), 3.22 - 3.10 (m, 2H), 2.86 (s, 2H), 2.10 (s, 4H), 1.42 (s, 3H). |
| 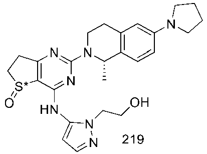 | 494.15 | ¹H NMR (400MHz, Chloroform-d) δ 9.38 - 9.22 (m, 1H), 7.54 - 7.44 (m, 1H), 7.03 (s, 1H), 6.48 (d, J = 17.6 Hz, 2H), 6.33 (s, 1H), 5.83 - 5.56 (m, 1H), 4.76 - 4.42 (m, 2H), 4.30 - 4.24 (m, 2H), 3.99 (t, J = 4.4 Hz, 2H), 3.75 - 3.63 (m, 1H), 3.52 - 3.41 (m, 1H), 3.40 - 3.33 (m, 1H), 3.27 (s, 4H), 3.15 - 3.04 (m, 2H), 2.95 - 2.76 (m, 2H), 2.00 (d, J = 4.0 Hz, 4H), 1.50 - 1.43 (m, 3H). |
| 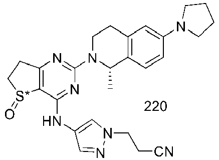 | 503.15 | ¹H NMR (400 MHz, DMSO-d₆) δ 9.72 (d, J = 12.6 Hz, 1H), 8.01 (d, J = 7.6 Hz, 1H), 7.73 (d, J = 7.4 Hz, 1H), 7.04 (dd, J = 32.4, 8.5 Hz, 1H), 6.38 (t, J = 9.9 Hz, 1H), 6.27 (s, 1H), 5.59 (dd, J = 56.7, 6.6 Hz, 1H), 4.62 - 4.36 (m, 3H), 3.51 - 3.34 (m, 2H), 3.21 (t, J = 8.5 Hz, 1H), 3.14 (s, 4H), 3.03 (d, J = 6.9 Hz, 3H), 2.90 (dd, J = 13.6, 7.2 Hz, 1H), 2.77 (d, J = 21.8 Hz, 2H), 1.88 (d, J = 6.2 Hz, 4H), 1.38 (dd, J = 19.8, 6.5 Hz, 3H). |
| 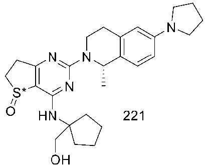 | 482.20 | ¹H NMR (400 MHz, DMSO-d₆) δ 6.96 (s, 1H), 6.41 (d, J = 16.4 Hz, 2H), 6.26 (s, 1H), 5.60 (d, J = 50.5 Hz, 1H), 4.87 (s, 1H), 4.53 (d, J = 58.2 Hz, 1H), 3.63 (s, 2H), 3.39 - 3.31 (m, 1H), 3.27 - 3.19 (m, 2H), 3.15 (s, 4H), 2.97 - 2.90 (m, 1H), 2.81 (s, 1H), 2.72 (s, 2H), 2.11 (s, 2H), 1.89 (s, 4H), 1.77 (s, 2H), 1.66 (s, 2H), 1.55 (s, 2H), 1.34 (d, J = 6.6 Hz, 3H). |
| 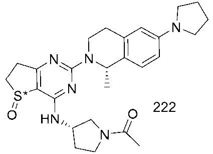 | 495.30 | ¹H NMR (400 MHz, Methanol-d4) δ 6.97 (s, 1H), 6.45 (d, J = 8.7 Hz, 1H), 6.32 (d, J = 2.5 Hz, 1H), 5.69 (s, 1H), 4.72 (s, 1H), 4.59 (s, 1H), 3.87 (ddd, J = 38.6, 11.8, 6.6 Hz, 1H), 3.66 - 3.56 (m, 2H), 3.50 (s, 1H), 3.46 (s, 1H), 3.22 (s, 5H), 3.07 (dd, J = 15.6, 9.8 Hz, 3H), 2.82 (d, J = 15.3 Hz, 2H), 2.03 - 1.95 (m, 7H), 1.43 (d, J = 6.5 Hz, 4H), 1.27 (s, 2H). |
| 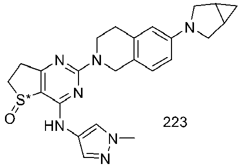 | 462.45 | ¹H NMR (400 MHz, DMSO-d₆) δ 9.66 (s, 1H), 7.87 (s, 1H), 7.63 (s, 1H), 6.99 (d, J = 8.4 Hz, 1H), 6.39 (d, J = 7.0 Hz, 1H), 6.33 (s, 1H), 4.74 (s, 2H), 3.90 (d, J = 7.1 Hz, 2H), 3.81 (s, 3H), 3.48 - 3.39 (m, 3H), 3.19 (d, J = 8.3 Hz, 1H), 3.07 (d, J = 8.9 Hz, 2H), 3.03 - 2.96 (m, 1H), 2.92 - 2.87 (m, 1H), 2.78 (s, 2H), 1.62 (s, 2H), 0.68 - 0.60 (m, 1H), 0.20 (d, J = 4.3 Hz, 1H). |
| 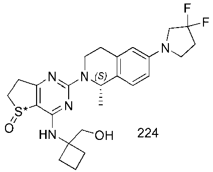 | 504.30 | ¹H NMR (400 MHz, DMSO-d₆) δ 7.31 (d, J = 6.8 Hz, 1H), 7.03 (t, J = 11.0 Hz, 1H), 6.46 (d, J = 8.4 Hz, 1H), 6.35 (d, J = 2.4 Hz, 1H), 5.59 (d, J = 63.4 Hz, 1H), 4.83 (t, J = 5.7 Hz, 1H), 4.52 (d, J = 65.2 Hz, 1H), 3.70 (d, J = 5.4 Hz, 2H), 3.60 (t, J = 13.5 Hz, 2H), 3.38 (t, J = 7.3 Hz, 3H), 3.25 - 3.11 (m, 2H), 2.94 - 2.79 (m, 2H), 2.71 (s, 2H), 2.52 - 2.47 (m, 1H), 2.42 (d, J = 7.2 Hz, 1H), 2.36 - 2.25 (m, 2H), 2.15 (s, 2H), 1.76 (s, 2H), 1.33 (d, J = 6.6 Hz, 3H). |

### Example 46: In vitro activity assay

### A. PDE4B2 enzyme experiment and PDE4D2 enzyme experiment

All compounds were dissolved in DMSO into 10 mM or 20 mM stock solutions. The compound to be tested was diluted with DMSO in a 3-fold gradient. 20 nL/well of the compound or a DMSO control was transferred to a 384-well plate by using Echo 550 instrument. The plate was sealed with a membrane and centrifuged for 1 minute. A 2x enzyme solution was prepared with an assay buffer, and 2 uL/well of the 2x enzyme solution was added, followed by equilibration at room temperature for 10 minutes. A 2x Cyclic-3',5'-AMP substrate solution was prepared with an assay buffer, and 2 uL/well of the 2x substrate solution was added, followed by incubation at room temperature for 60 minutes. 4 uL of AMP-glo reagent was added, followed by incubation at room temperature for 60 minutes. 8 **ul** of an AMP detection solution was added, followed by incubation at room temperature for 60 minutes. An RLU value was read on Envision 2105 plate reader. The RLU value represents the concentration of the product AMP. Inhibition rate % ⁼ (1 - (RLU_{positive control} - RLU_{compound})/(RLUpₒₛᵢₜᵢᵥₑ control - RLU_{negative} control)) * 100. The IC₅₀ value was then caulculated by Graphpad 8.0 software using Y = Bottom + (Top - Bottom)/(1 + 10^((LogIC₅₀ - X) * HillSlope)).

The experimental results are shown in Tables 1 and 2:

**Table 1**

| Compound | PDE4B2 IC₅₀ (nM) | PDE4D2 IC₅₀ Binding (nM) | Compound | PDE4B2 IC₅₀ (nM) | PDE4D2 IC₅₀ Binding (nM) |
|---|---|---|---|---|---|
| 1 | 2.67 | 7.72 | 104 | 1.669 | 2.939 |
| 2 | 19.35 | 60.07 | 107 | 1.144 | 2.95 |
| 3 | 925.7 | | 108 | 1.412 | 4.168 |
| 4 | 2.71 | 7.247 | 116 | 3.009 | 12.4 |
| 5 | 0.45 | | 118 | 0.414 | 0.748 |
| 6 | 0.41 | 0.473 | 119 | 1.374 | 2.795 |
| 7 | 0.175 | | 120 | 0.98 | |
| 9 | 0.094 | 0.224 | 121 | 13.48 | 48.21 |
| 10 | 2.31 | | 122 | 0.838 | 2.04 |
| 11 | 1.2 | | 124 | 0.98 | 2.924 |
| 12 | 15.37 | | 125 | 0.968 | 1.983 |
| 13 | 6.39 | | 126 | 0.9507 | 1.643 |
| 14 | 0.21 | 1.08 | 127 | 0.164 | 0.424 |
| 15 | 20.43 | 28.63 | 128 | 5.434 | 18.14 |
| 16 | 10.65 | 17.34 | 129 | 0.202 | 0.705 |
| 17 | 470.7 | | 130 | 0.297 | 0.5897 |
| 18 | 1.22 | 3.212 | 131 | 0.147 | 0.3332 |
| 19 | 7.17 | | 132 | 0.368 | 0.6511 |
| 20 | 1.85 | | 133 | 0.515 | 1.555 |
| 21 | 2.11 | | 134 | 0.098 | 0.2404 |
| 22 | 0.97 | 1.463 | 135 | 1.753 | 3.382 |
| 23 | 2.84 | | 136 | 0.04068 | 0.08246 |
| 24 | 0.38 | 0.92 | 140 | 0.131 | 0.361 |
| 25 | 0.92 | 2.185 | 142 | 0.837 | 2.268 |
| 26 | 0.77 | 2.509 | 143 | 0.113 | 0.2516 |
| 27 | 0.55 | 0.88 | 144 | 0.440 | 0.6966 |
| 28 | 0.68 | | 145 | 0.245 | 0.985 |
| 29 | 0.54 | 1.787 | 148 | 0.700 | 1.105 |
| 31 | 7.417 | | 149 | 0.430 | 1.081 |
| 32 | 0.2006 | 0.405 | 150 | 0.418 | 0.694 |
| 34 | 1.96 | | 151 | 0.298 | 0.655 |
| 35 | 1.32 | 3.891 | 152 | 0.426 | 0.748 |
| 37 | 2.99 | | 153 | 0.182 | 0.520 |
| 39 | 2.848 | | 154 | 0.138 | 0.279 |
| 43 | 0.384 | 0.8757 | 155 | 0.692 | |
| 44 | 0.38 | | 156 | 1.260 | 1.757 |
| 49 | 2.642 | 4.811 | 158 | 0.273 | 0.653 |
| 50 | 0.313 | 0.695 | 159 | 0.224 | 0.389 |
| 51 | 0.596 | 1.179 | 160 | 0.224 | 0.748 |
| 52 | 0.4879 | 0.8264 | 161 | 0.6702 | 0.8241 |
| 53 | 0.589 | 1.218 | 162 | 0.5888 | 0.5837 |
| 56 | 0.9982 | 1.953 | 163 | 0.170 | 0.300 |
| 59 | 2.124 | 4.75 | 165 | 0.3514 | 0.3981 |
| 63 | 4.206 | | 166 | 0.4572 | 0.4529 |
| 64 | 1.779 | 3.462 | 167 | 0.087 | 0.993 |
| 65 | 0.0546 | 0.09314 | 168 | 0.108 | 0.5609 |
| 66 | 0.568 | 1.128 | 170 | 0.729 | 1.292 |
| 67 | 0.332 | 0.655 | 173 | 0.386 | 0.391 |
| 68 | 0.4765 | 0.7702 | 174 | 0.1965 | 0.2306 |
| 69 | 0.156 | 0.2765 | 175 | 0.299 | 0.603 |
| 70 | 160.2 | | 176 | 0.6066 | 0.7156 |
| 71 | 5.457 | | 177 | 0.5081 | 0.5016 |
| 72 | 0.229 | 0.5869 | 179 | 0.5023 | 0.4596 |
| 73 | 0.297 | 0.958 | 180 | 4.5550 | 6.3840 |
| 74 | 0.284 | 1.089 | 183 | 0.4128 | |
| 75 | 0.81 | | 185 | 0.02513 | 0.02634 |
| 76 | 0.056 | 0.089 | 186 | 0.0646 | 0.1546 |
| 77 | 0.050 | 0.049 | 187 | 0.4499 | 0.116 |
| 78 | 1.819 | 4.02 | 188 | 0.387 | 0.7295 |
| 79 | 9.188 | | 189 | 0.066 | 0.145 |
| 80 | 0.391 | 0.5601 | 190 | 0.1683 | 0.5723 |
| 82 | 0.366 | 0.764 | 191 | 0.182 | 0.851 |
| 83 | 0.6207 | 1.067 | 192 | 0.1398 | 0.4499 |
| 85 | 0.085 | 0.1566 | 193 | 0.4175 | 1.275 |
| 86 | 0.2404 | 0.481 | 194 | 0.3334 | 1.081 |
| 89 | 0.7045 | 1.63 | 195 | 0.956 | 1.118 |
| 90 | 0.190 | 0.3846 | 196 | 0.0693 | 0.2442 |
| 91 | 0.142 | 0.233 | 197 | 13.29 | 23.85 |
| 92 | 0.161 | 0.776 | 198 | 0.608 | 1.795 |
| 93 | 0.3439 | 0.6031 | 200 | 0.117 | 0.119 |
| 94 | 0.07115 | 0.1251 | 201 | 0.194 | 0.5963 |
| 98 | 0.889 | 2.044 | 208 | 0.220 | 0.766 |
| 99 | 1.238 | 3.139 | 210 | 0.263 | 1.205 |
| 100 | 0.4257 | 1.154 | 212 | 0.945 | 1.247 |
| 101 | 0.6555 | 1.964 | 216 | 0.2268 | 0.750 |
| 103 | 0.338 | 0.873 | 218 | 0.9145 | 1.883 |

**Table 2**

| Compound | PDE4B2 Enzyme Inhibition% @100 nM | **PDE4B2** Enzyme Inhibition% @10 nM | **PDE4B2** Enzyme Inhibition% @1 nM |
|---|---|---|---|
| 8 | 94.08 | 45.03 | |
| 33 | 98.64 | 72.33 | |
| 36 | 99.04 | 74.54 | |
| 38 | 84.73 | 35.42 | |
| 40 | 97.34 | 57.73 | |
| 41 | 78.77 | 20.43 | |
| 42 | 100.25 | 97.67 | |
| 45 | 100.11 | 99.81 | |
| 46 | 100.22 | 98.91 | |
| 47 | 100.06 | 98.41 | |
| 48 | 100.68 | 87.03 | |
| 54 | 96.584 | 58.893 | 9.276 |
| 55 | 99.90 | 93.26 | |
| 57 | 99.971 | 90.548 | 38.869 |
| 58 | 97.48 | 68.74 | |
| 60 | 99.35 | 65.75 | |
| 61 | 98.20 | 70.90 | |
| 62 | 100.57 | 83.85 | |

The above data show that the compounds of the present invention have stronger inhibitory activity on PDE4B2 enzyme.

### B. PDE4B2 cell experiment

All compounds were dissolved in DMSO into 10 mM or 20 mM stock solutions. The compound to be tested was diluted with DMSO with a 3-fold gradient. Firstly, 20 nL/well of Foskolin (10 mM) was transferred to a 384 cell culture plate by using Echo instrument. 40 nL/well of the compound or a DMSO control was transferred to a 384-well plate by using Echo 550 instrument. Flpin-293-PDE4B2 cells were digested, resuspended in an experimental buffer, and inoculated into a 384 cell culture plate, with an inoculation density of 20000 cells per well and an inoculation volume of 20 µl per well. The plate was incubated at 37°C for 30 minutes. Eu-cAMP tracer and Ulight-anti-cAMP were frozen and thawed and diluted in a lysis buffer. 10 of µl Eu-cAMP tracer was added to an experimental well, and 10 µl of Ulight-anti-cAMP was added to the experimental well. The reaction plate was centrifuged at 200 g at room temperature for 30 s and then left to stand at 25°C for 1 h, and data was then collected by Envision. Inhibition % = 100 - (Signal compound - SignalAve positive control)/(SignalAve negative control - SignalAve positive control) × 100. Graphpad 8.0 software was then used to obtain an IC₅₀ value by calculation using Y = Bottom + (Top - Bottom)/(1 + 10^((LogIC₅₀ - X) * HillSlope)).

The experimental results are as shown in Tables 3 and 4.

**Table 3**

| Compound | PDE4B2 IC₅₀ Cell (nM) | Compound | PDE4B2 IC₅₀ Cell (nM) |
|---|---|---|---|
| 1 | 22.1 | 25 | 23.7 |
| 2 | 418 | 26 | 19.67 |
| 4 | 29.6 | 27 | 8.11 |
| 5 | 13.8 | 28 | 36.3 |
| 6 | 8.2 | 29 | 20.9 |
| 7 | 6.65 | 35 | 46.1 |
| 9 | 2.83 | 37 | 89.5 |
| 10 | 73.79 | 39 | 28.7 |
| 13 | 562.40 | 42 | 25.14 |
| 14 | 16.5 | 44 | 21.72 |
| 15 | 150.13 | 45 | 3.99 |
| 16 | 78.05 | 47 | 6.28 |
| 18 | 17.6 | 73 | 9.156 |
| 19 | 204.80 | 74 | 8.978 |
| 22 | 14.78 | 101 | 13.21 |
| 23 | 29.1 | 167 | 20.7 |
| 24 | 10.36 | | |

**Table 4**

| Compound | Cell Inhibition% @100 nM | Cell Inhibition% @10 nM | Cell Inhibition% @1 nM |
|---|---|---|---|
| 8 | 15.10 | 3.50 | |
| 11 | 69.06 | 13.50 | |
| 12 | 7.46 | 15.96 | |
| 20 | 69.92 | 14.18 | |
| 21 | 79.24 | 18.76 | |
| 30 | 48.00 | 11.30 | |
| 32 | 97.65 | 74.22 | 23.54 |
| 33 | 33.2 | 9 | |
| 34 | 62 | 14 | |
| 36 | 66.60 | 18.10 | |
| 38 | 38.24 | 20.32 | |
| 40 | 46.20 | 12.00 | |
| 43 | 100.08 | 81.51 | |
| 46 | 99.05 | 69.61 | |
| 48 | 76.2 | 25.7 | |
| 49 | 81.25 | 29.88 | 11.19 |
| 50 | 93.64 | 49.44 | 21.51 |
| 51 | 70.08 | 38.39 | 17.07 |
| 52 | 99.55 | 81.81 | |
| 53 | 95.8 | 63.7 | |
| 55 | 93.43 | 52.78 | |
| 56 | 90.2 | 40.6 | |
| 57 | 71.62 | 14.91 | 11.76 |
| 58 | 57.11 | 15.88 | |
| 59 | 85.82 | 42.65 | |
| 60 | 65.9 | 21.2 | |
| 61 | 55.2 | 17.7 | |
| 62 | 69.1 | 29.5 | |
| 63 | 76.6 | 31.1 | |
| 64 | 87.6 | 43.6 | |
| 65 | 100.0 | 96.8 | |
| 66 | 94.36 | 38.70 | 14.81 |
| 67 | 96.42 | 47.36 | 14.20 |
| 68 | 96.14 | 56.28 | 8.99 |
| 69 | 100.11 | 81.23 | 17.16 |
| 70 | 3.78 | 1.71 | 1.75 |
| 71 | 66.08 | 9.57 | -3.96 |
| 72 | 99.27 | 70.09 | 13.31 |
| 75 | 51.36 | 24.88 | 4.93 |
| 76 | 100.72 | 97.95 | 48.20 |
| 77 | 100.73 | 99.37 | 61.02 |
| 78 | 86.33 | 32.09 | 2.20 |
| 79 | 59.37 | 11.19 | 1.99 |
| 80 | 94.73 | 58.99 | 15.78 |
| 82 | 99.87 | 77.47 | 19.08 |
| 83 | 97.34 | 47.82 | 5.79 |
| 84 | 100.72 | 96.96 | 32.85 |
| 85 | 101.1 | 97.0 | 41.7 |
| 86 | 97.77 | 63.13 | 8.69 |
| 87 | 89.06 | 31.33 | 2.90 |
| 88 | 93.47 | 28.76 | 3.67 |
| 89 | 95.32 | 37.42 | 3.47 |
| 90 | 97.23 | 65.79 | 14.99 |
| 91 | 99.96 | 86.01 | 19.25 |
| 92 | 98.17 | 43.69 | 3.35 |
| 93 | 94.24 | 61.02 | 14.02 |
| 94 | 100.44 | 94.09 | 34.83 |
| 95 | 66.7 | 6.8 | -4.2 |
| 96 | 84.8 | 15.7 | -2.7 |
| 97 | 86.54 | 17.36 | 1.66 |
| 98 | 95.76 | 46.69 | -1.71 |
| 99 | 96.53 | 43.68 | 4.18 |
| 100 | 97.30 | 47.68 | 6.00 |
| 102 | 95.46 | 35.86 | -0.08 |
| 103 | 95.41 | 38.89 | 2.83 |
| 104 | 84.49 | 28.60 | 1.81 |
| 105 | 93.48 | 42.53 | 7.53 |
| 106 | 67.52 | 21.18 | 0.41 |
| 107 | 98.12 | 52.48 | 2.82 |
| 108 | 95.13 | 37.35 | -0.82 |
| 109 | 97.85 | 65.40 | 17.70 |
| 110 | 100.03 | 76.13 | 18.98 |
| 111 | 81.06 | 22.21 | 8.07 |
| 112 | 99.44 | 54.08 | 12.30 |
| 113 | 71.88 | 22.83 | 5.01 |
| 114 | 54.55 | 8.74 | -1.54 |
| 115 | 97.73 | 39.69 | 7.72 |
| 116 | 73.36 | 16.91 | 2.17 |
| 117 | 61.63 | 17.32 | 5.32 |
| 118 | 89.05 | 41.67 | 11.06 |
| 119 | 95.96 | 55.15 | 14.01 |
| 120 | 23.1 | 7.4 | 4.1 |
| 121 | 29.4 | 10.0 | 0.4 |
| 122 | 96.8 | 49.9 | 5.4 |
| 123 | 86.8 | 29.6 | 3.3 |
| 124 | 97.3 | 42.6 | 8.7 |
| 125 | 90.64 | 21.77 | 2.85 |
| 126 | 99.95 | 77.39 | 19.78 |
| 127 | 100.08 | 72.99 | 9.78 |
| 128 | 25.39 | 6.68 | -0.97 |
| 129 | 72.19 | 39.59 | 6.93 |
| 130 | 100.0 | 57.9 | 11.8 |
| 131 | 98.70 | 76.01 | 16.63 |
| 132 | 101.8 | 78.6 | 16.2 |
| 133 | 94.2 | 33.0 | 10.5 |
| 134 | 100.73 | 86.83 | 19.25 |
| 135 | 84.1 | 24.7 | 8.0 |
| 136 | 101.49 | 98.30 | 48.14 |
| 137 | 83.76 | 21.79 | 4.94 |
| 138 | 89.63 | 27.94 | 4.95 |
| 139 | 101.8 | 78.6 | 16.2 |
| 140 | 100.35 | 86.98 | 17.65 |
| 141 | 95.98 | 49.17 | 11.37 |
| 142 | 94.42 | 34.76 | 6.79 |
| 143 | 100.09 | 72.45 | 13.95 |
| 144 | 98.60 | 51.38 | 7.35 |
| 145 | 95.87 | 36.01 | 4.25 |
| 146 | 101.10 | 85.08 | 14.76 |
| 147 | 94.37 | 32.23 | 8.67 |
| 148 | 92.10 | 37.34 | 3.99 |
| 149 | 95.99 | 38.27 | 5.99 |
| 150 | 97.54 | 49.58 | 11.84 |
| 151 | 99.12 | 56.52 | 7.66 |
| 152 | 99.17 | 66.02 | 16.13 |
| 153 | 99.86 | 76.84 | 10.23 |
| 154 | 99.73 | 91.04 | 26.48 |
| 155 | 76.06 | 46.84 | 11.97 |
| 156 | 91.74 | 25.72 | 1.42 |
| 157 | 83.09 | 23.21 | 8.27 |
| 158 | 99.33 | 61.64 | 14.79 |
| 159 | 99.58 | 73.37 | 16.82 |
| 160 | 99.84 | 75.05 | 15.06 |
| 161 | 96.35 | 37.27 | 3.10 |
| 162 | 93.80 | 52.84 | 10.72 |
| 163 | 96.69 | 56.74 | 11.89 |
| 164 | 61.09 | 19.97 | 6.23 |
| 165 | 99.87 | 76.78 | 10.31 |
| 166 | 98.24 | 61.65 | 9.23 |
| 168 | 98.56 | 46.86 | 6.54 |
| 169 | 96.71 | 48.59 | 4.67 |
| 170 | 86.94 | 26.70 | 1.97 |
| 171 | 94.30 | 36.47 | 4.06 |
| 172 | 96.61 | 45.95 | 3.77 |
| 173 | 99.48 | 65.74 | 8.43 |
| 175 | 98.43 | 59.38 | 6.90 |
| 176 | 97.78 | 51.48 | 6.10 |
| 177 | 98.63 | 53.27 | 7.25 |
| 178 | 98.93 | 68.04 | 8.53 |
| 179 | 92.23 | 51.54 | 9.95 |
| 181 | 98.78 | 46.63 | 3.18 |
| 182 | 97.95 | 40.61 | 3.68 |
| 183 | 99.86 | 78.50 | 12.77 |
| 184 | 100.20 | 94.34 | 36.50 |
| 185 | 101.22 | 100.37 | 75.29 |
| 186 | 100.52 | 93.16 | 24.68 |
| 187 | 100.92 | 93.80 | 25.27 |
| 188 | 96.63 | 39.56 | 8.72 |
| 189 | 100.57 | 93.47 | 24.43 |
| 190 | 96.69 | 47.12 | 3.93 |
| 191 | 94.30 | 35.17 | 8.90 |
| 192 | 99.24 | 60.29 | 15.40 |
| 193 | 95.99 | 38.21 | 4.88 |
| 194 | 92.91 | 33.61 | 4.28 |
| 196 | 100.39 | 89.46 | 23.72 |
| 198 | 97.66 | 67.42 | 18.10 |
| 199 | 61.57 | 8.04 | -0.51 |
| 200 | 99.94 | 74.41 | 14.74 |
| 201 | 99.89 | 72.33 | 7.33 |
| 202 | 100.88 | 99.43 | 52.55 |
| 203 | 99.92 | 84.14 | 14.78 |
| 204 | 100.28 | 87.19 | 23.10 |
| 205 | 100.74 | 92.41 | 28.48 |
| 206 | 100.28 | 89.72 | 26.91 |
| 207 | 100.22 | 94.41 | 30.80 |
| 209 | 61.66 | 9.75 | 4.48 |
| 210 | 95.42 | 32.70 | 8.70 |
| 211 | 98.44 | 50.07 | 12.51 |
| 212 | 97.52 | 34.62 | 3.24 |
| 213 | 89.03 | 29.57 | 9.25 |
| 214 | 64.12 | 15.08 | 1.80 |
| 215 | 100.01 | 84.32 | 20.07 |
| 216 | 98.20 | 50.40 | 11.65 |
| 217 | 84.53 | 25.01 | 2.48 |
| 218 | 84.2 | 23.6 | 7.4 |
| 219 | 66.28 | 32.62 | 6.98 |
| 220 | 100.24 | 73.36 | 13.80 |
| 221 | 99.68 | 71.53 | 11.26 |
| 222 | 100.94 | 96.19 | 41.45 |
| 223 | 99.24 | 73.73 | 11.28 |
| 224 | 98.89 | 44.44 | 10.65 |

The above data show that the compounds of the present invention have stronger inhibitory activity on PDE4B2 cells.

### Example 47: LPS induced PBMC TNFa release cell experiment

Human PBMCs were purchased from TPCS, with article number PB025C-W. The PBMCs were inoculated into a 96-well plate. All the compounds were dissolved with DMSO to form 10 mM stock solutions. A positive compound and the compound to be tested were diluted 3 folds with DMSO and then diluted with a culture medium. The resulting dilutions were added to a 96-well plate, such that the highest concentration of the compound in the test wells was 3000 nM or 1000 nM. LPS was purchased from Sigma (article number L2880). Teh LPS was diluted with the culture medium and then added to the 96-well plate. The cell well plate was incubated in an incubator at 37°C at 5% CO₂ for 24 hours. After centrifugation, the supernatant was collected and detected by Elisa kit (the kit was purchased from R&D, article number VAL105), and OD450 data were collected on Envision instrument. Inhibition % = 100 - (Signal compound - SignalAve positive control)/(SignalAve negative control - SignalAve positive control) × 100. Graphpad 8.0 software was then used to obtain an IC₅₀ value by calculation using Y = Bottom + (Top - Bottom)/(1 + 10^((LogIC50 - X) * HillSlope)).

The experiment shows that the compounds of the present invention have excellent inhibitory activity on TNFα secreted in human PBMCs and can better inhibit the secretion of inflammatory factor TNFα in human PBMCs, thereby showing good anti-inflammatory effect.

The examples of the present patent are provided by way of illustration rather than limitation. Those skilled in the art will readily recognize that various non-critical parameters can be changed or modified to produce substantially similar results.

Although the technical solution of the present invention has been described and recited in detail, it should be understood that for those skilled in the art, these are only illustrative, and it is apparent for those skilled in the art to modify the above examples or use equivalent alternatives. These modifications or improvements without departing from the spirit of the present invention all fall within the scope of protection of the present invention.

## Claims

1. A compound represented by formula (I), a stereoisomer thereof, or a pharmaceutically acceptable salt thereof: 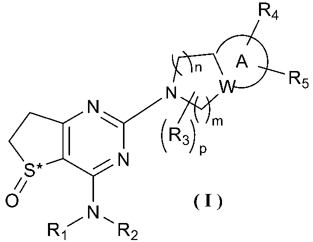 wherein:
S* represents a chiral sulfur atom and has R configuration or S configuration;
W is selected from N, CH, or C;
ring A is a monocyclic group or a bicyclic group, wherein the monocyclic group is selected from 5- to 8-membered heteroaryl or C₆₋₁₀ aryl, and the bicyclic group is selected from 5- to 6-membered heteroaryl-fused 5- to 6-membered heteroaryl, 5- to 6-membered heteroaryl-fused phenyl, 5- to 6-membered cycloalkyl-fused phenyl, 5- to 6-membered cycloalkyl-fused 5- to 6-membered heteroaryl, 5- to 6-membered heterocyclyl-fused phenyl, 5- to 6-membered heterocyclyl-fused 5- to 6-membered heteroaryl, benzo-fused 5- to 6-membered heterocyclyl, or benzo-fused 5- to 6-membered heteroaryl;
R₁ is selected from hydrogen or C₁₋₆ alkyl;
R₂ is selected from C₁₋₆ alkyl, C₃₋₈ cycloalkyl, 3- to 8-membered heterocyclyl, C₆₋₁₀ aryl, or 5- to 10-membered heteroaryl, wherein the C₁₋₆ alkyl, C₃₋₈ cycloalkyl, 3- to 8-membered heterocyclyl, C₆₋₁₀ aryl, and 5- to 10-membered heteroaryl are optionally further substituted with one or more groups selected from deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, 3- to 6-membered heterocyclyl, 5-to 6-membered heteroaryl, 5- to 6-membered heteroaryl-substituted C₁₋₆ alkyl, R^{2.1}-substituted C₁₋₆ alkyl, C₁₋₆ alkyl-substituted 5- to 6-membered heteroaryl, C₁₋₆ alkyl-substituted 3- to 6-membered heterocyclyl, -C(O)-R^{2.1}, -S(O)₂-R^{2.2}, -C(O)O-R^{2.1}, -C(O)NR^{2.3}R^{2.4}, -SR^{2.5}, -SOR^{2.2}, -OR^{2.5}, or -NR²⁻³R^{2.4};
or R₁ and R₂ are connected to form 3- to 6-membered heterocyclyl or 5- to 6-membered heteroaryl, wherein the 3- to 6-membered heterocyclyl and the 5- to 6-membered heteroaryl are optionally further substituted with one or more groups selected from hydrogen, deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, or 3- to 6-membered heterocyclyl;
each R₃ is independently selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, or 3- to 6-membered heterocyclyl;
or any R₃, together with carbon atoms on a ring, forms C₃₋₈ cycloalkyl or 3- to 8-membered heterocyclyl;
R₄ is selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, 7- to 9-membered heterocyclyl, 5- to 8-membered heteroaryl, -C(O)-R^{4.1}, -S(O)₂-R^{4.2}, -C(O)O-R^{4.1}, -C(O)NR^{4.3}R^{4.4}, -SOR^{4.2}, -OR^{4.5}, -SR^{4.5}, or -NR^{4.3}R^{4.4}, wherein the C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, 7- to 9-membered heterocyclyl, and 5- to 8-membered heteroaryl are capable of being optionally further substituted with one or more groups selected from deuterium, halogen, cyano, hydroxyl, oxo, C₁₋₆ alkyl, C₁₋₆ haloalkyl, C₁₋₆ hydroxyalkyl, C₁₋₆ alkoxy, C₁₋₆ alkoxy C₁₋₆ alkyl, -NR^{4.3}R^{4.4}, C₃₋₆ cycloalkyl, or 3- to 6-membered heterocyclyl;
R₅ is selected from hydrogen, deuterium, halogen, amino, nitro, cyano, hydroxyl, oxo, C₁₋₆ alkyl, C₁₋₆ haloalkyl, C₁₋₆ deuteroalkyl, C₁₋₆ hydroxyalkyl, C₁₋₆ alkoxy, C₃₋₆ cycloalkyl, 3- to 6-membered heterocyclyl, -C(O)-R^{5.1}, -S(O)₂-R^{5.2}, -C(O)O-R^{5.1}, -C(O)NR^{5.3}R^{5.4}, -SOR^{5.2}, -OR^{5.5}, -SR^{5.5}, or -NR^{5.3}R^{5.4};
or R₄ is connected to R₅ to form 5- to 6-membered heteroaryl or C₆₋₁₀ aryl, wherein the 5-to 6-membered heteroaryl and C₆₋₁₀ aryl are capable of being optionally further substituted with one or more groups selected from deuterium, halogen, cyano, hydroxyl, oxo, C₁₋₆ alkyl, C₁₋₆ hydroxyalkyl, C₁₋₆ alkoxy, C₃₋₆ cycloalkyl, or 3- to 6-membered heterocyclyl;
R^{2.1}, R^{2.2} , R^{2.3}, R^{2.4}, R^{2.5}, R^{4.1}, R^{4.2}, R^{4.3}, R^{4.4}, R^{4.5}, R^{5.1}, R^{5.2} , R^{5.3}, R^{5.4}, and R^{5.5} are each independently selected from hydrogen, deuterium, halogen, amino, hydroxyl, cyano, carboxyl, C₁₋₆ alkyl, C₁₋₆ hydroxyalkyl, C₁₋₆ haloalkyl, C₁₋₆ alkoxy, -C(O)NH₂, C₃₋₆ cycloalkyl, or 3- to 6-membered heterocyclyl;
m and n are each independently selected from 1, 2, or 3; and
p is selected from 1 or 2.

2. The compound, the stereoisomer thereof, or the pharmaceutically acceptable salt thereof according to claim 1, wherein the compound is further as represented by formula (II-A), formula (II-B), formula (II-C), or formula (II-D):

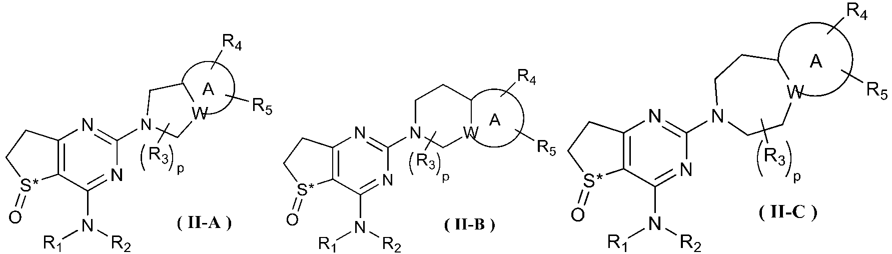

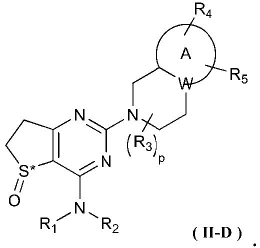

3. The compound, the stereoisomer thereof, or the pharmaceutically acceptable salt thereof according to claim 1 or 2, wherein
ring A is a monocyclic group selected from 5-membered heteroaryl, 6-membered heteroaryl, or phenyl; preferably, the ring A is selected from thiazolyl, oxazolyl, oxadiazolyl, thiadiazolyl, phenyl, pyrazolyl, thienyl, furyl, imidazolyl, triazolyl, tetrazolyl, pyridinyl, pyrimidinyl, pyrazinyl, or pyridazinyl.

4. The compound, the stereoisomer thereof, or the pharmaceutically acceptable salt thereof according to claim 1 or 2, wherein ring A is a bicyclic group selected from 5-membered heteroaryl-fused 6-membered heteroaryl, 5-membered heteroaryl-fused phenyl, 6-membered cycloalkyl-fused phenyl, 6-membered heterocyclyl-fused phenyl, benzo-fused 5-membered heterocyclyl, benzo-fused 5-membered heteroaryl, 6-membered heteroaryl-fused 5-membered heteroaryl, 6-membered heterocyclyl-fused 6-membered heteroaryl, or 6-membered heteroaryl-fused phenyl; and
preferably, ring A is selected from pyrazolopyrimidinyl, imidazophenyl, imidazopyridinyl, pyrazolopyridinyl, cyclohexyl-fused phenyl, oxacyclohexyl-fused phenyl, benzodioxazolyl, 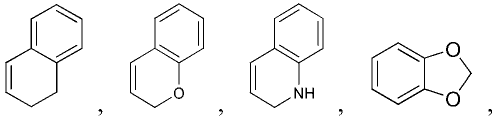 benzoxazolyl, 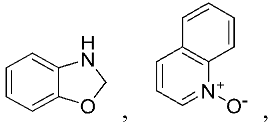 oxacyclohexyl-fused pyridinyl, pyridone-fused phenyl, pyridopyrazolyl, benzopyrazolyl, thienophenyl, or pyridophenyl.

5. The compound, the stereoisomer thereof, or the pharmaceutically acceptable salt thereof according to claim 1, wherein the general formula (I) is further as represented by formula (III-A), or formula (III-B):

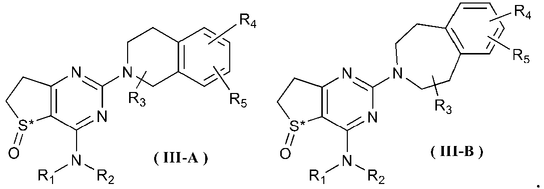

6. The compound, the stereoisomer thereof, or the pharmaceutically acceptable salt thereof according to claim 5, wherein the formula (III-A) is further as represented by formula (IV): 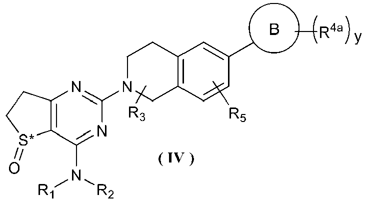 wherein ring B is C₃₋₆ cycloalkyl, 3- to 6-membered heterocyclyl, 7- to 9-membered heterocyclyl, phenyl, or 5- to 6-membered heteroaryl; each R^{4a} is independently selected from deuterium, halogen, amino, hydroxyl, cyano, carboxyl, oxo, C₁₋₃ alkyl, C₁₋₃ hydroxyalkyl, C₁₋₃ haloalkyl, C₁₋₃ alkoxy, C₁₋₃ alkoxyalkyl, -N(CH₃)₂, -C(O)NH₂, C₃₋₆ cycloalkyl, or 3- to 6-membered heterocyclyl; and y is 0, 1, or 2.

7. The compound, the stereoisomer thereof, or the pharmaceutically acceptable salt thereof according to claim 6, wherein the ring B is selected from cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, pyrazolyl, oxazolyl, thiazolyl,

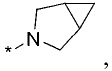

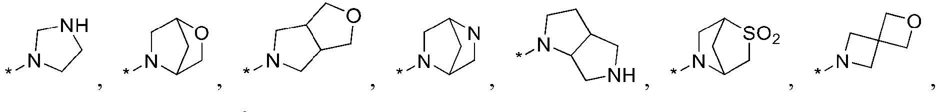

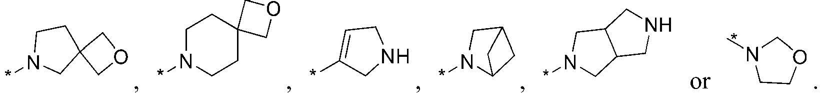

8. The compound, the stereoisomer thereof, or the pharmaceutically acceptable salt thereof according to any one of claims 5-7, wherein the R₁ is selected from hydrogen, and the R₂ is selected from C₁₋₃ alkyl, C₃₋₆ cycloalkyl, 3- to 6-membered heterocyclyl, phenyl, or 5- to 6-membered heteroaryl, wherein the C₁₋₃ alkyl, C₃₋₆ cycloalkyl, 3- to 6-membered heterocyclyl, phenyl, and 5- to 6-membered heteroaryl are optionally further substituted with one or more groups selected from deuterium, halogen, amino, nitro, oxo, cyano, hydroxyl, C₁₋₆ alkyl, C₁₋₆ hydroxyalkyl, C₁₋₆ alkoxy, C₃₋₆ cycloalkyl, 3- to 6-membered heterocyclyl, 5- to 6-membered heteroaryl, 5- to 6-membered heteroaryl-substituted C₁₋₆ alkyl, R^{2.1}-substituted C₁₋₆ alkyl, C₁₋₆ alkyl-substituted 5- to 6-membered heteroaryl, C₁₋₆ alkyl-substituted 3- to 6-membered heterocyclyl, -C(O)-R^{2.1}, -S(O)₂-R^{2.2}, -C(O)O-R^{2.1}, -C(O)NR^{2.3}R^{2.4}, -SR^{2.5}, -SOR^{2.2}, -OR^{2.5}, or -NR^{2.3}R^{2.4}.

9. The compound, the stereoisomer thereof, or the pharmaceutically acceptable salt thereof according to claim 6, wherein each R^{4a} is independently selected from deuterium, fluoro, chloro, amino, hydroxyl, cyano, carboxyl, oxo, methyl, ethyl, -CH₂OCH₃, -N(CH₃)₂, methoxy, ethoxy, trifluoromethyl, hydroxymethyl, or hydroxyethyl.

10. The compound, the stereoisomer thereof, or the pharmaceutically acceptable salt thereof according to any one of claims 1-6, wherein R₁ is selected from hydrogen or C₁₋₆ alkyl;
or R₂ is as represented by formula (III): 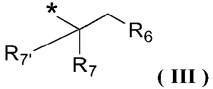 wherein:
R₆ is selected from hydroxyl, cyano, amino, or halogen;
R₇ and R_{7'} are each independently selected from hydrogen and C₁₋₆ alkyl, wherein the C₁₋₆ alkyl is optionally further substituted with one or more groups selected from deuterium, halogen, amino, hydroxyl, cyano, oxo, C₁₋₃ alkyl, C₁₋₃ haloalkyl, C₁₋₃ hydroxyalkyl, C₁₋₃ alkoxy, -C(O)-R^{2.1}, -S(O)₂-R^{2.2}, -C(O)O-R^{2.1}, -C(O)NR^{2.3}R^{2.4}, -SR^{2.5}, -SOR^{2.2}, -OR^{2.5}, or -NR^{2.3}R^{2.4};
preferably, R₇ and R_{7'} are each independently selected from hydrogen, methyl, ethyl, propyl, isopropyl, or butyl;
or R₂ is selected from methyl, ethyl, cyclopropyl, cyclobutyl, cyclopentyl, cyclohexyl, oxacyclohexyl, cycloheptyl, phenyl, pyridinyl, pyrazolyl, triazolyl, tetrazolyl, oxetanyl, azetidinyl, thietanyl, tetrahydropyranyl, tetrahydrofuryl, tetrahydrothiopyran, pyrrolidinyl, tetrahydropyrrolidinyl, tetrahydrothienyl, piperidinyl, piperazinyl, morpholinyl, thiomorpholinyl, azepanyl, oxaspiroheptyl, benzopyridinyl, pyridopyridinyl, benzimidazolyl, benzopyrimidinyl, or naphthyl, wherein the methyl, ethyl, cyclopropyl, cyclobutyl, cyclopentyl, cyclohexyl, oxacyclohexyl, cycloheptyl, phenyl, pyridinyl, pyrazolyl, triazolyl, tetrazolyl, oxetanyl, azetidinyl, thietanyl, tetrahydropyranyl, tetrahydrofuryl, tetrahydrothiopyran, pyrrolidinyl, tetrahydropyrrolidinyl, tetrahydrothienyl, piperidinyl, piperazinyl, morpholinyl, thiomorpholinyl, azepanyl, oxaspiroheptyl, benzopyridinyl, pyridopyridinyl, benzimidazolyl, benzopyrimidinyl, and naphthyl are capable of being optionally further substituted with one or more groups selected from deuterium, fluoro, chloro, bromo, amino, hydroxyl, mercapto, cyano, oxo, methyl, ethyl, propyl, isopropyl, butyl, isobutyl, cyclopropyl, -CF₃, -CHF₂, -CH₂F, methoxy, ethoxy, propoxy, oxiranyl, oxetanyl, aziridinyl, azetidinyl, -(CH₂)₂CN, -C(O)-R^{2.1}, - S(O)₂-R^{2.2}, -C(O)O-R^{2.1}, -C(O)NR^{2.3}R^{2.4}, -SR^{2.5}, -SOR^{2.2}, -OR^{2.5}, or -NR^{2.3}R^{2.4};
or each R₃ is independently selected from hydrogen, deuterium, halogen, amino, nitro, cyano, hydroxyl, oxo, C₁₋₃ alkyl, C₁₋₃ haloalkyl, C₁₋₃ deuteroalkyl, C₁₋₃ hydroxyalkyl, C₁₋₃ alkoxy, C₃₋₆ cycloalkyl, or 3- to 6-membered heterocyclyl;
or R₄ is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, -C(O)-R^{4.1}, -S(O)₂-R^{4.2}, -C(O)O-R^{4.1}, -C(O)NR^{4.3}R^{4.4}, -SOR^{4.2}, -OR^{4.5}, -SR^{4.5}, -NR^{4.3}R^{4.4}, C₃₋₆ cycloalkyl, 3- to 6-membered heterocyclyl, 7- to 9-membered heterocyclyl, phenyl, or 5- to 6-membered heteroaryl, wherein the C₃₋₆ cycloalkyl, 3- to 6-membered heterocyclyl, 7- to 9-membered heterocyclyl, phenyl, and 5- to 6-membered heteroaryl are capable of being optionally further substituted with one or more substituents selected from deuterium, halogen, amino, nitro, cyano, hydroxyl, oxo, C₁₋₃ alkyl, C₁₋₃ hydroxyalkyl, C₁₋₃ alkoxy, -NR^{4.3}R^{4.4}, C₁₋₃ alkoxy C₁₋₃ alkyl, or C₁₋₃ haloalkyl;
or R₅ is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, -C(O)-R^{5.1}, -S(O)₂-R^{5.2}, -C(O)O-R^{5.1}, -C(O)NR^{5.3}R^{5.4}, -SOR^{5.2}, -OR^{5.5}, -SR^{5.5}, -NR^{5.3}R^{5.4}, C₃₋₆ cycloalkyl, 3- to 6-membered heterocyclyl, phenyl, or 5- to 6-membered heteroaryl, wherein the C₃₋₆ cycloalkyl, 3- to 6-membered heterocyclyl, phenyl, and 5- to 6-membered heteroaryl are capable of being optionally further substituted with one or more substituents selected from deuterium, halogen, amino, nitro, cyano, hydroxyl, oxo, C₁₋₃ alkyl, C₁₋₃ hydroxyalkyl, C₁₋₃ alkoxy, or C₁₋₃ haloalkyl;
the R^{2.1}, R^{2.2} , R^{2.3}, R^{2.4} , R^{2.5}, R^{4.1}, R^{4.2} , R^{4.3}, R^{4.4} , R^{4.5}, R^{5.1}, R^{5.2} , R^{5.3}, R^{5.4}, and R^{5.5} are each independently selected from hydrogen, deuterium, halogen, amino, hydroxyl, cyano, carboxyl, C₁₋₃ alkyl, C₁₋₃ hydroxyalkyl, C₁₋₃ haloalkyl, C₁₋₃ alkoxy, -C(O)NH₂, C₃₋₈ cycloalkyl, or 3- to 8-membered heterocyclyl;
preferably, the R^{2.1}, R^{2.2} , R^{2.3}, R^{2.4} , R^{2.5}, R^{4.1}, R^{4.2} , R^{4.3}, R^{4.4} , R^{4.5}, R^{5.1}, R^{5.2} , R^{5.3}, R^{5.4}, and R^{5.5} are each independently selected from hydrogen, deuterium, fluoro, chloro, bromo, amino, hydroxyl, cyano, carboxyl, methyl, ethyl, propyl, isopropyl, hydroxymethyl, hydroxyethyl, -CF₃, -CHF₂, -CH₂F, methoxy, ethoxy, propoxy, -C(O)NH₂, cyclopropyl, cyclobutyl, cyclopentyl, oxiranyl, oxetanyl, aziridinyl, azetidinyl, tetrahydropyrrolyl, or tetrahydrofuryl.

11. The compound, the stereoisomer thereof, or the pharmaceutically acceptable salt thereof according to any one of claims 1-5 or 8, wherein the R₄ is selected from hydrogen, deuterium, halogen, amino, nitro, cyano, hydroxyl, oxo, C₁₋₃ alkyl, C₁₋₃ hydroxyalkyl, C₁₋₃ alkoxy, C₁₋₃ haloalkyl, cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, or pyrazolyl, wherein the C₁₋₃ alkyl, C₁₋₃ hydroxyalkyl, C₁₋₃ alkoxy, C₁₋₃ haloalkyl, cyclopropyl, cyclobutyl, cyclopentyl, oxiranyl, oxetanyl, aziridinyl, azetidinyl, pyrrolidinyl, triazolyl, piperidinyl, morpholinyl, piperazinyl, thiazolyl, imidazolyl, and pyrazolyl are optionally substituted with one or more substituents selected from deuterium, halogen, amino, nitro, cyano, hydroxyl, fluoro, chloro, oxo, methyl, ethyl, methoxy, ethoxy, trifluoromethyl, hydroxymethyl, hydroxyethyl, trifluoroethyl, -CH₂OCH₃, or -N(CH₃)₂.

12. The compound, the stereoisomer thereof, or the pharmaceutically acceptable salt thereof according to any one of claims 1-5, 8, or 10, wherein the R₄ is selected from hydrogen, deuterium, fluoro, chloro, bromo, iodo, nitro, cyano, hydroxyl, amino, oxo, methyl, ethyl, propyl, isopropyl, methoxy, ethoxy, propoxy, isopropoxy, cyclopropyl, cyclobutyl, oxiranyl, oxetanyl, aziridinyl, azetidinyl, -CF₃, -CHF₂, -CH₂F, -NMe₂, -SO₂Me, -SO₂Et, -CONH₂, - CONHMe, -CO₂Me, 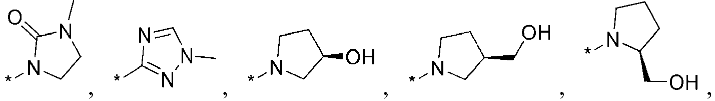 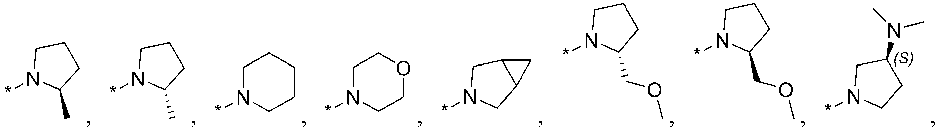 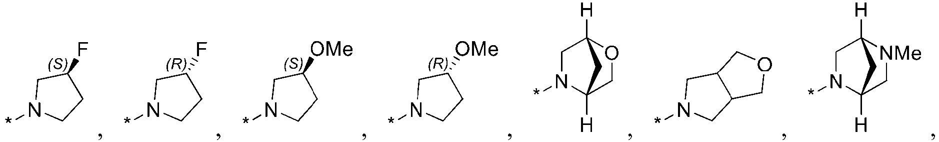 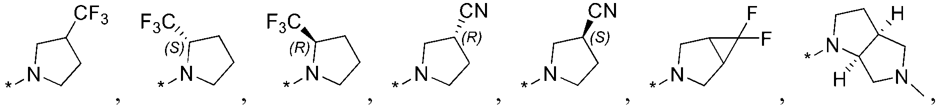 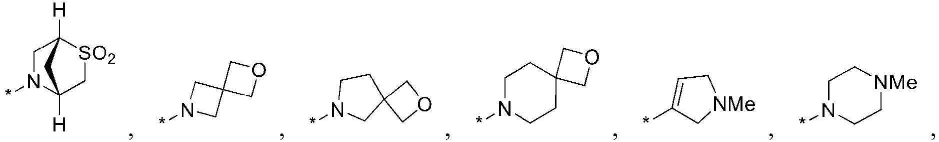 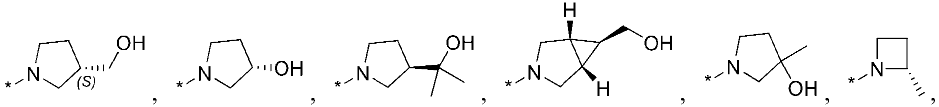 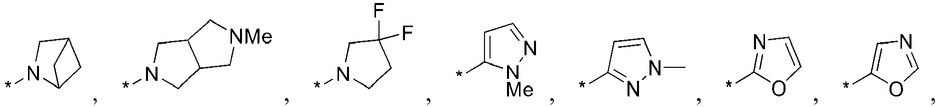 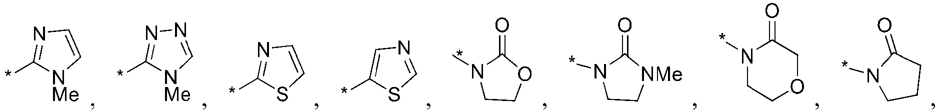 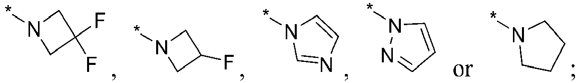 or R₅ is selected from hydrogen, deuterium, fluoro, chloro, bromo, iodo, nitro, cyano, hydroxyl, amino, oxo, methyl, ethyl, propyl, isopropyl, methoxy, ethoxy, propoxy, isopropoxy, cyclopropyl, cyclobutyl, oxiranyl, oxetanyl, aziridinyl, azetidinyl, -NH-(CH₂)₂-OH, -CF₃, - CHF₂, -CH₂F, -NMe₂, -SO₂Me, -SO₂Et, -CONH₂, -CONHMe, or -CO₂Me.

13. The compound, the stereoisomer thereof, or the pharmaceutically acceptable salt thereof according to any one of claims 1-4 or 10, wherein 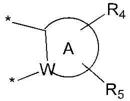 in each of formula (II-A), formula (II-B), formula (II-C), and formula (II-D) is independently selected from 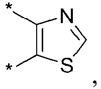 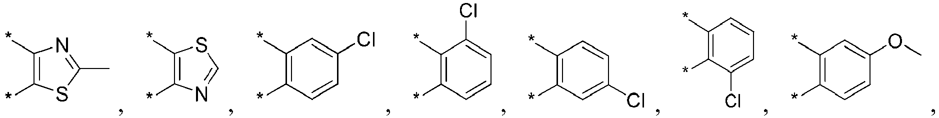 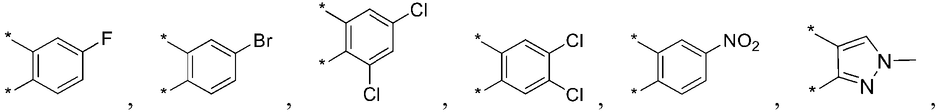 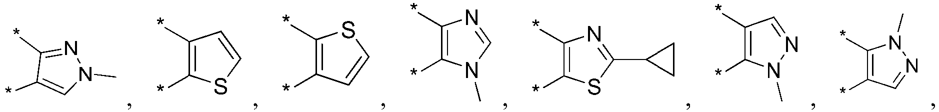 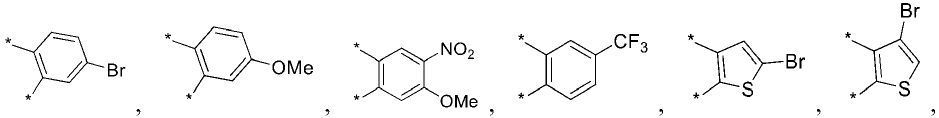 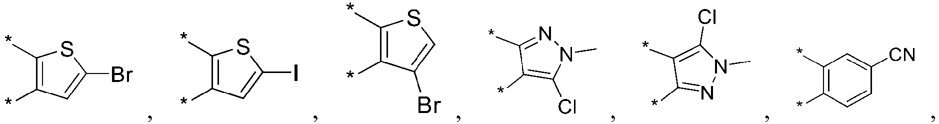 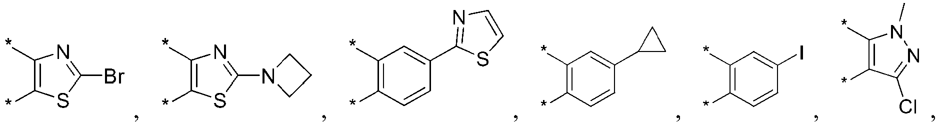 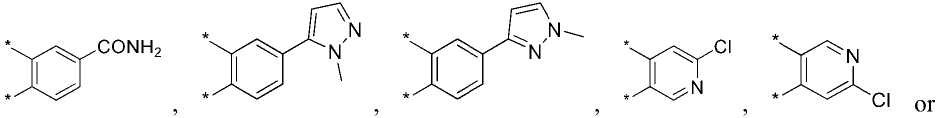 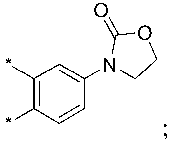 or 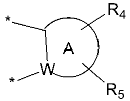 in each of formula (II-A), formula (II-B), formula (II-C), and formula (II-D) is independently selected from

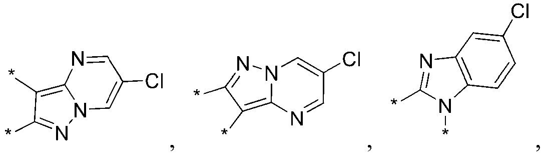

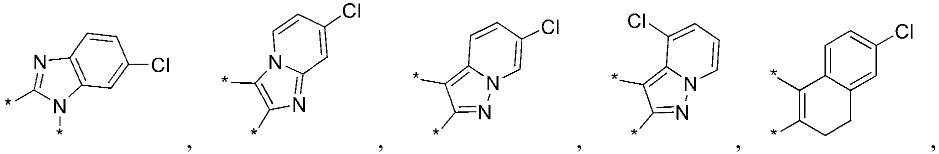

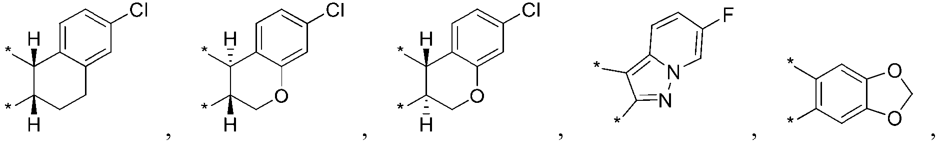

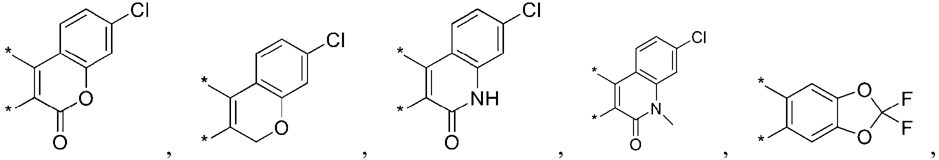

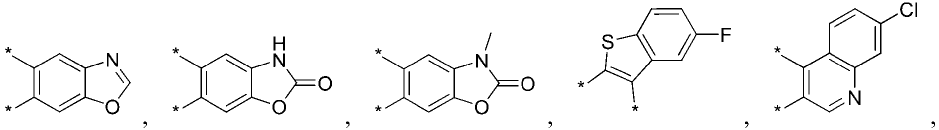

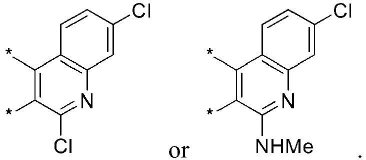

14. The compound, the stereoisomer thereof, or the pharmaceutically acceptable salt thereof according to any one of claims 1-13, wherein the R₁ is selected from hydrogen and C₁₋₃ alkyl;
or the R₂ is selected from the following groups: 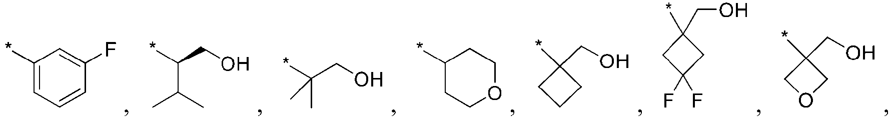 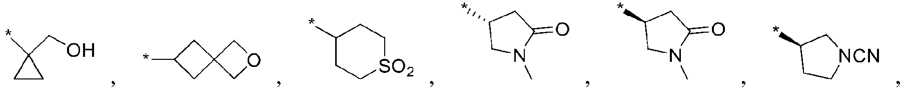 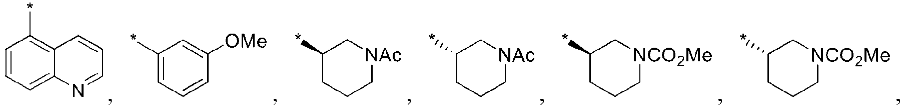 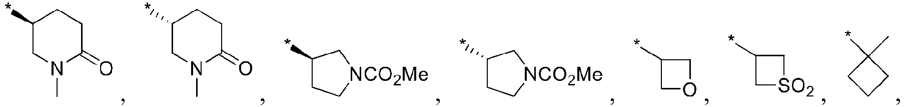 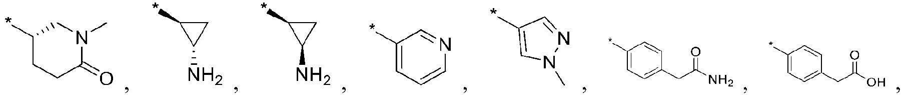 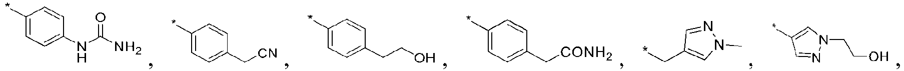 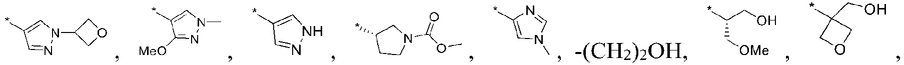 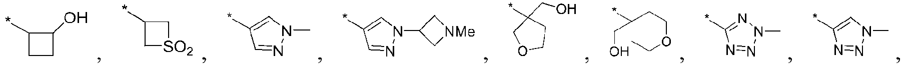 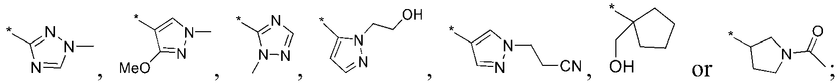
or each R₃ is independently selected from hydrogen, deuterium, fluoro, chloro, bromo, cyano, oxo, methyl, ethyl, propyl, isopropyl, butyl, isobutyl, methoxy, ethoxy, propoxy, isopropoxy, -CF₃, -CHF₂, -CH₂F, cyclopropyl, cyclobutyl, cyclopentyl, oxiranyl, oxetanyl, aziridinyl, azetidinyl, tetrahydropyrrolyl, tetrahydrofuryl, -CH₂OH, -CF₃, -CHF₂, -CH₂F, or - CD₃.

15. The compound, the stereoisomer thereof, or the pharmaceutically acceptable salt thereof according to any one of claims 1-14, wherein the compound is selected from compounds having the following structures:

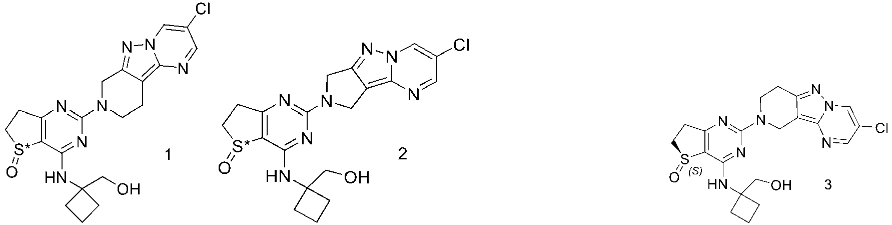

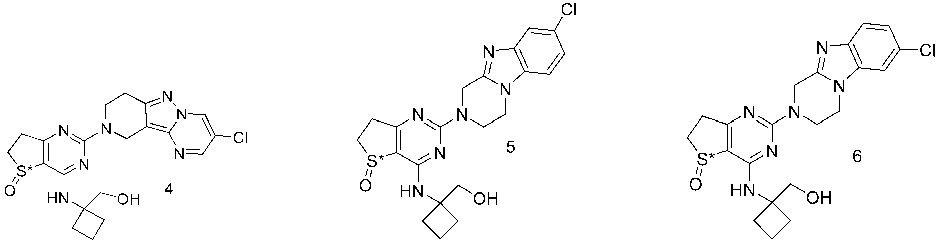

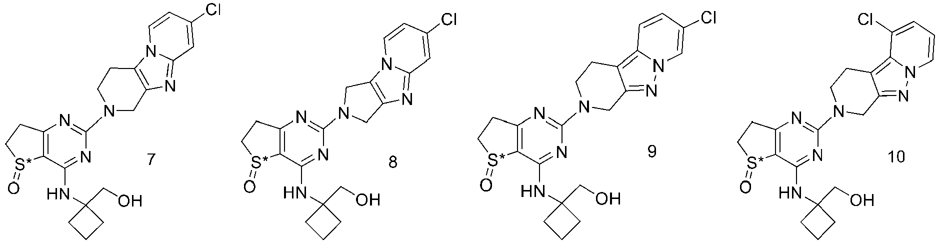

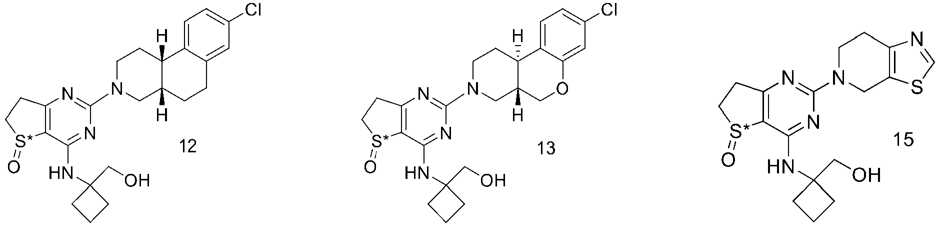

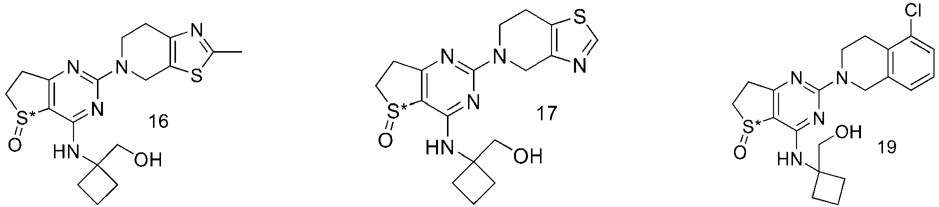

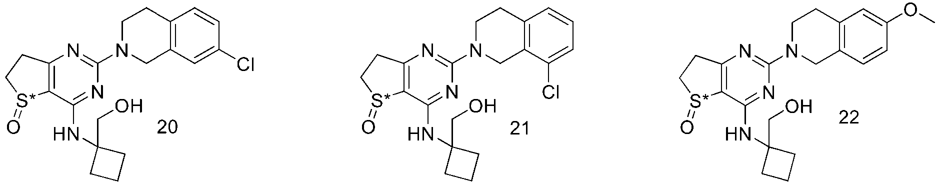

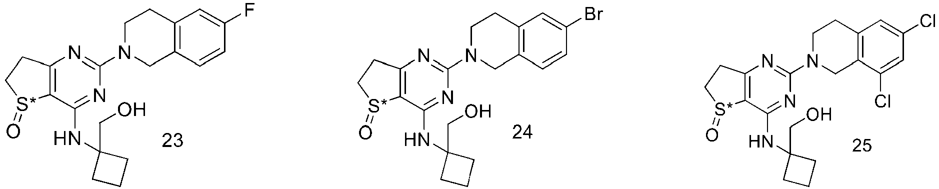

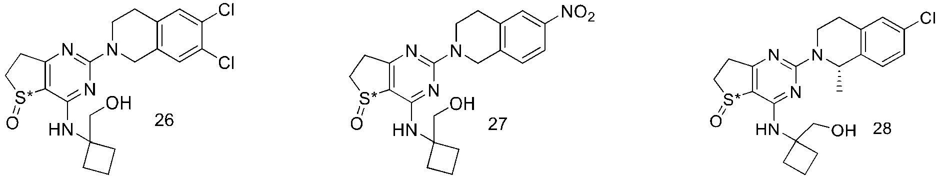

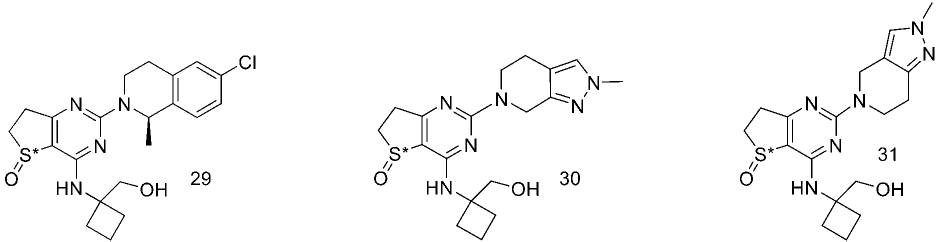

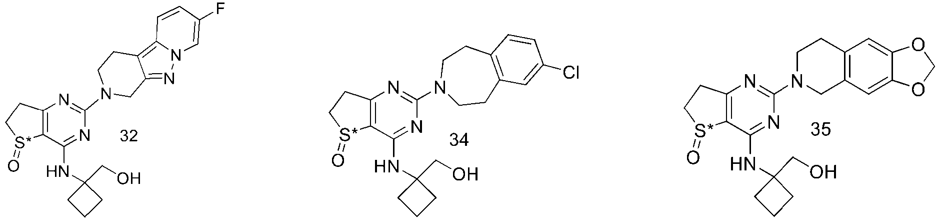

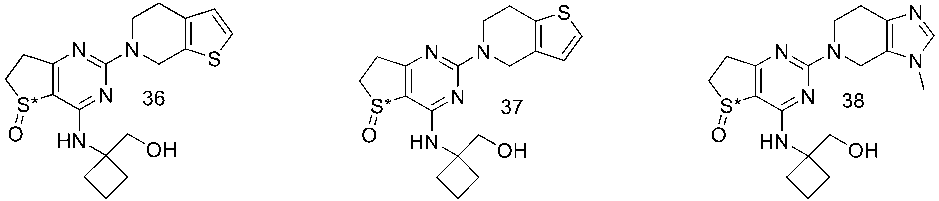

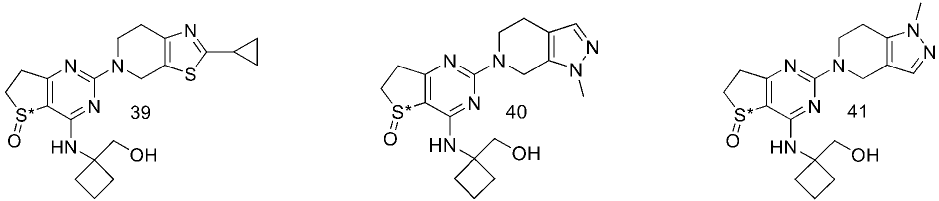

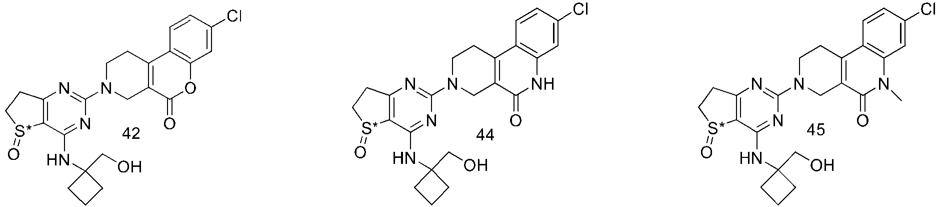

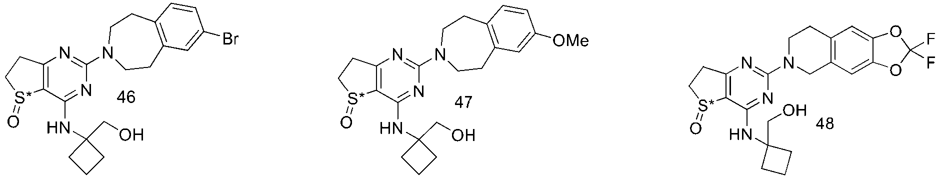

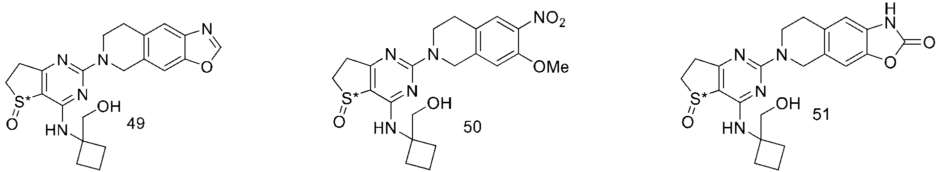

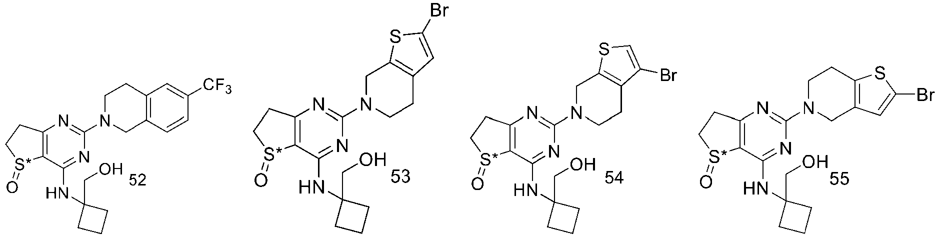

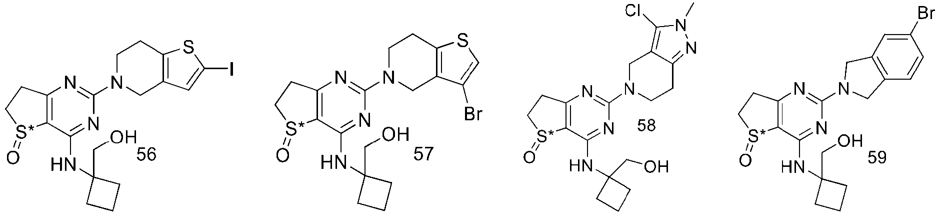

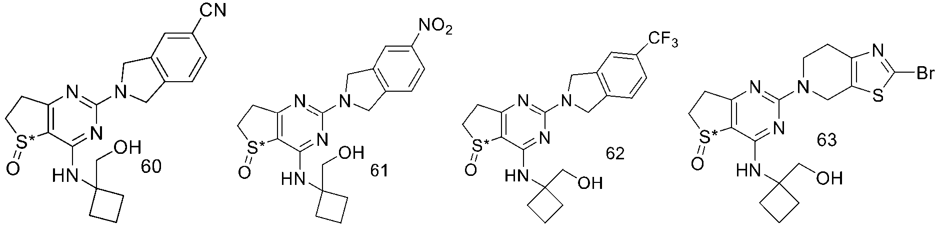

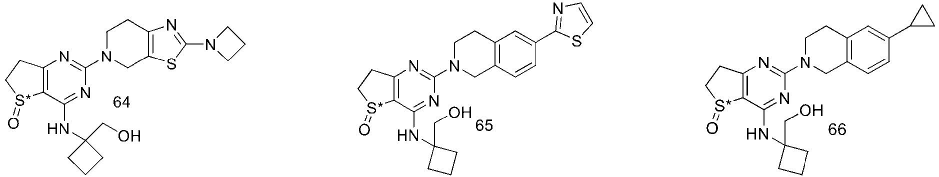

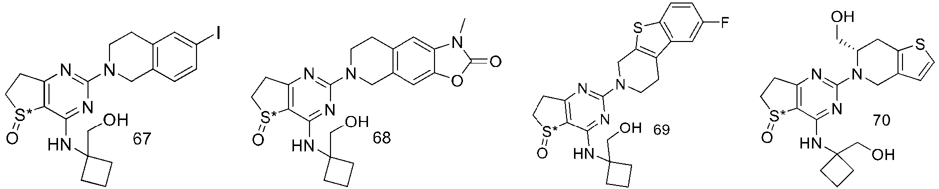

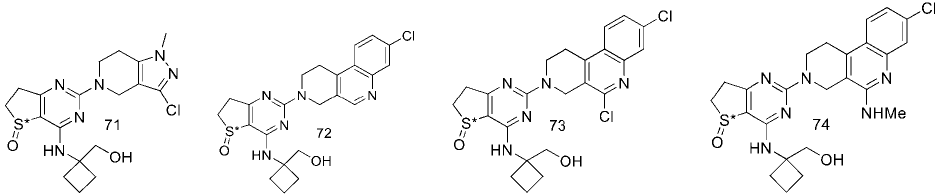

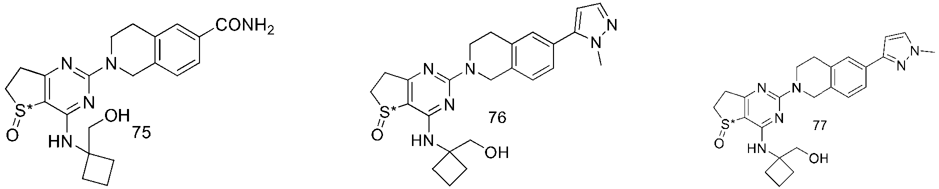

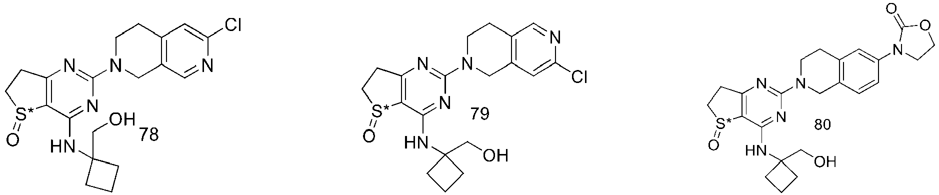

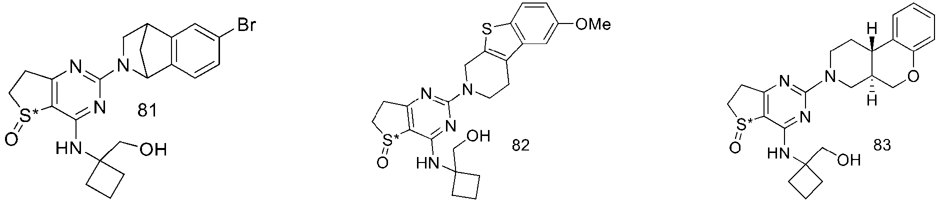

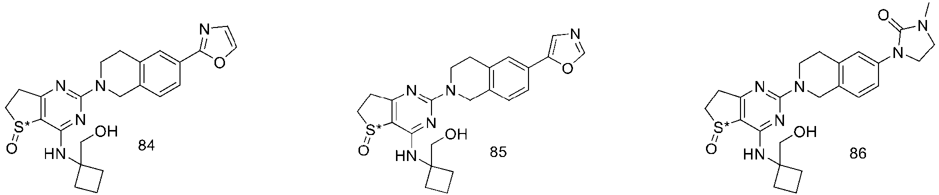

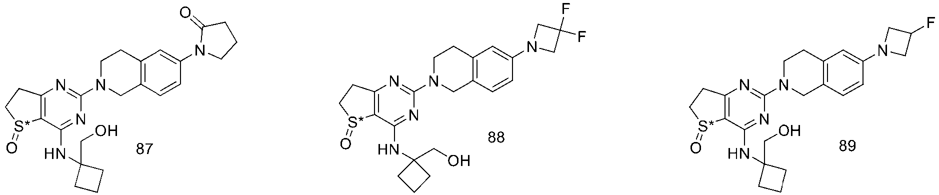

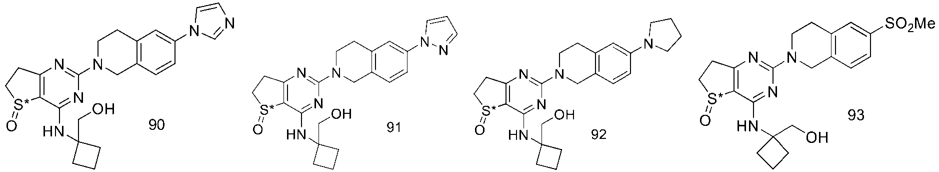

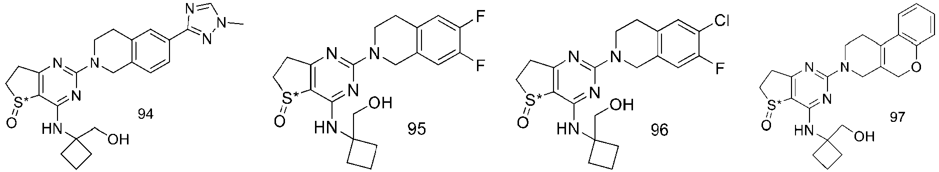

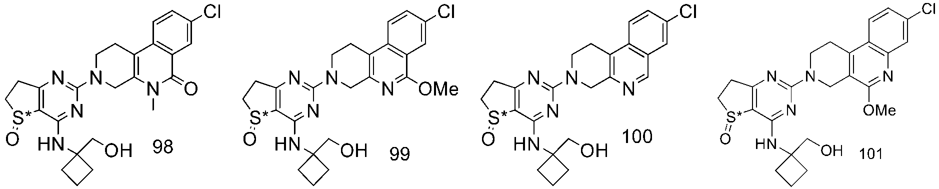

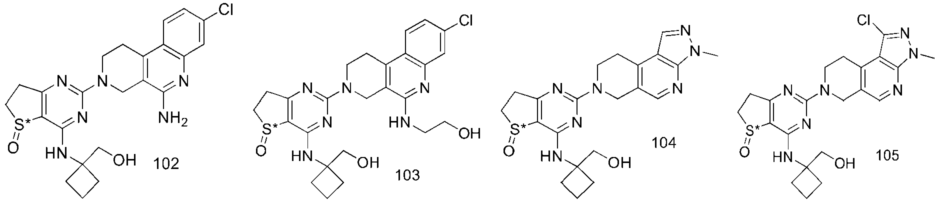

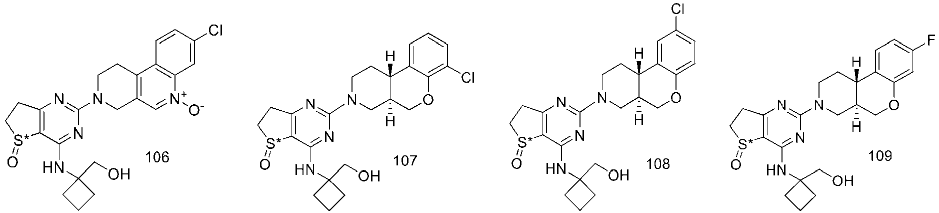

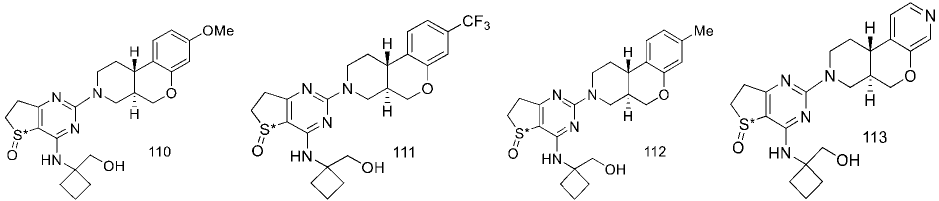

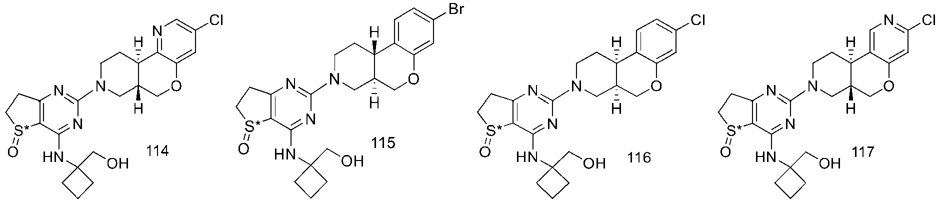

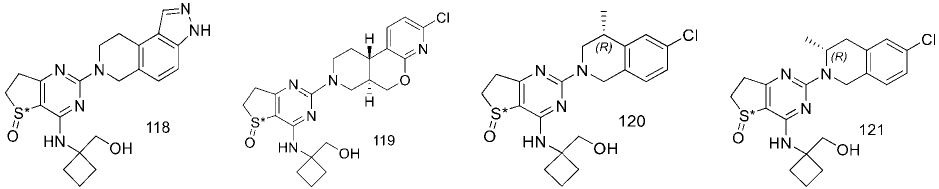

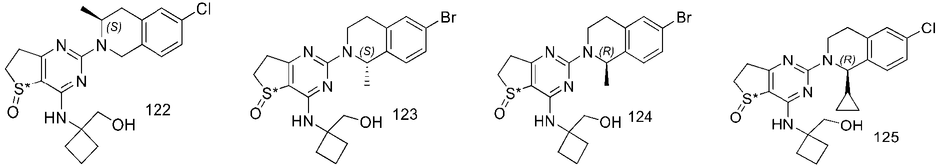

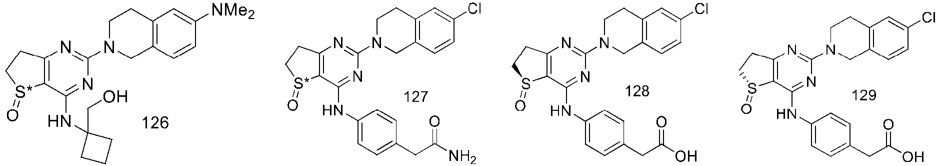

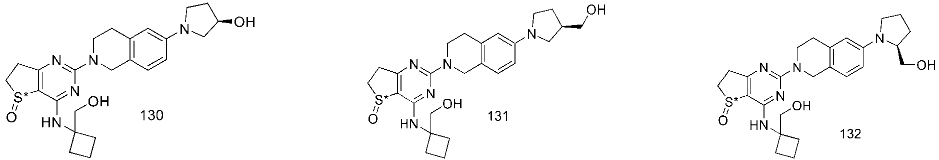

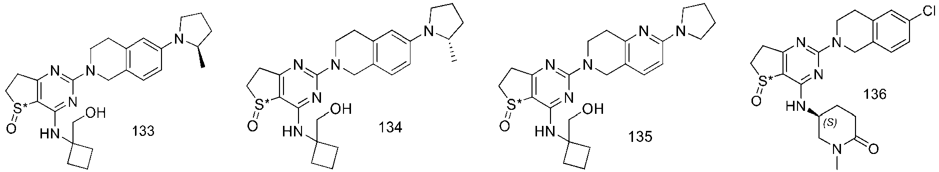

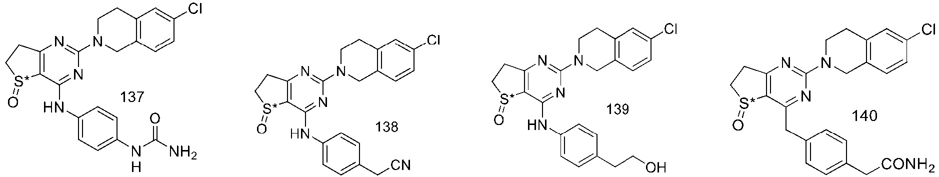

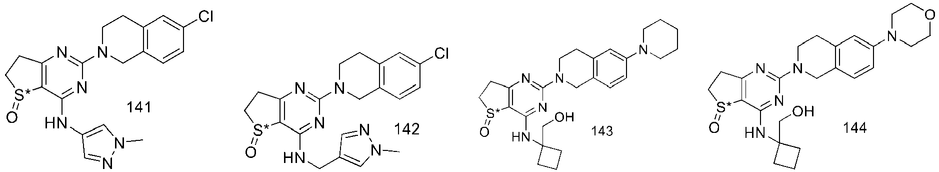

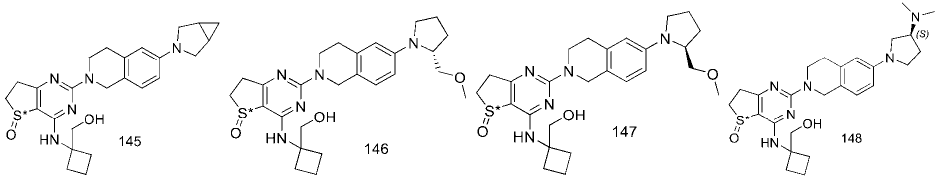

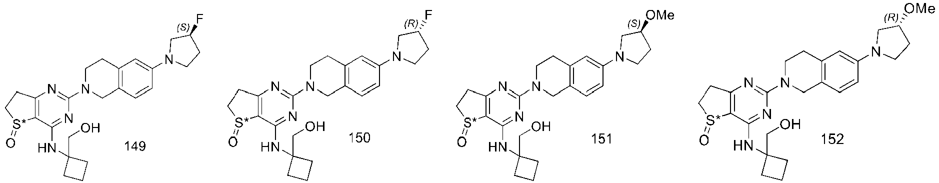

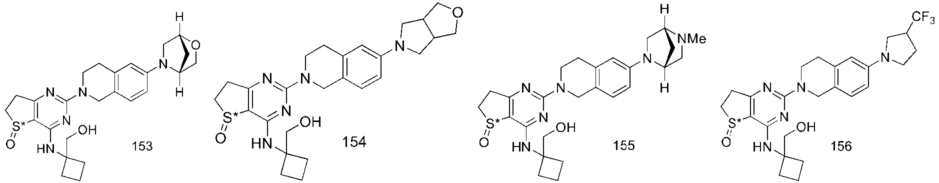

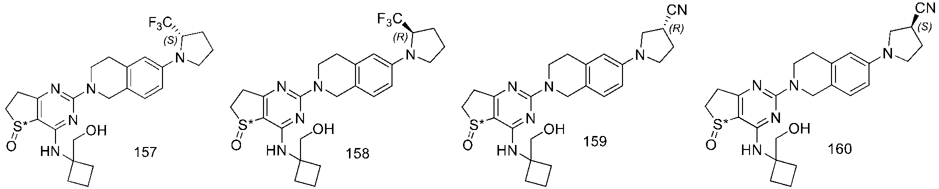

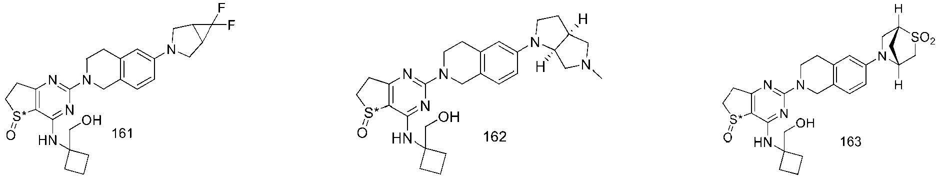

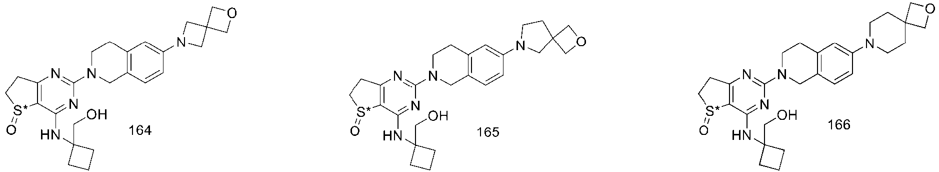

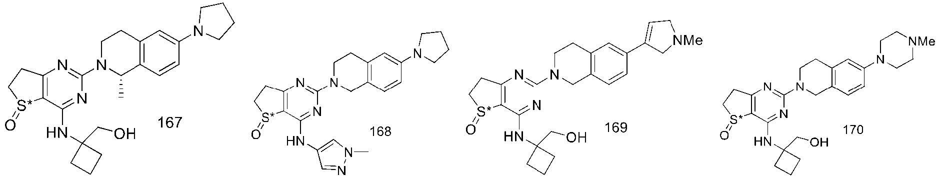

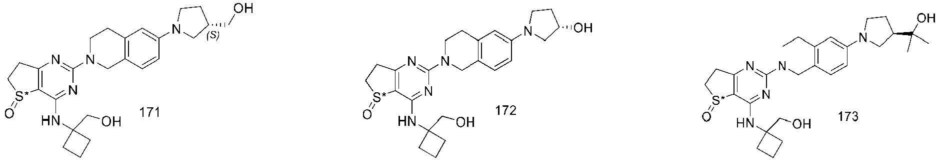

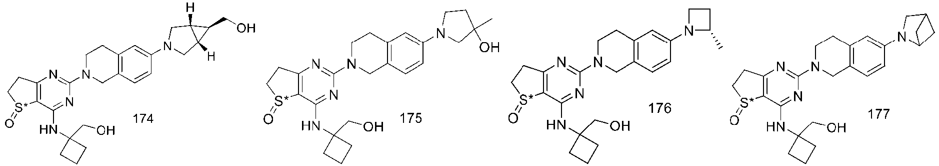

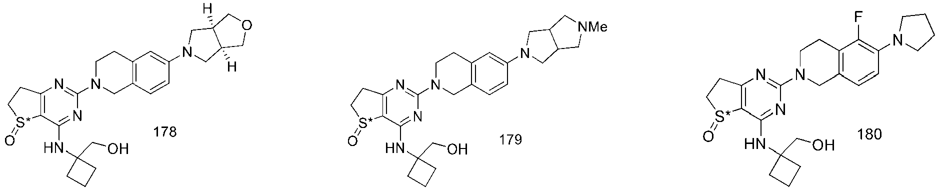

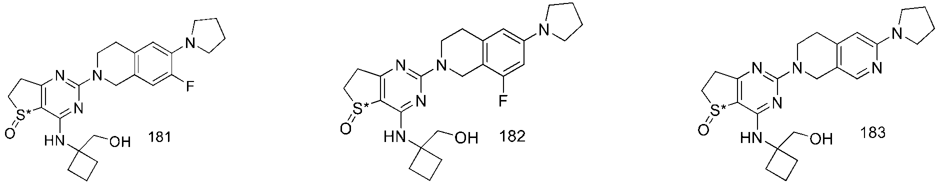

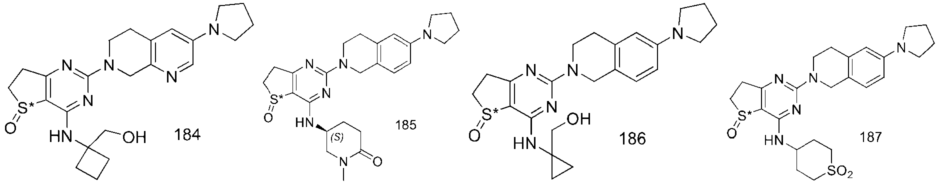

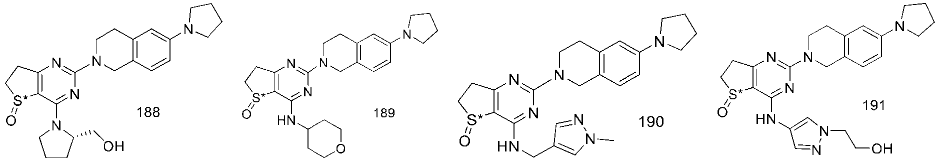

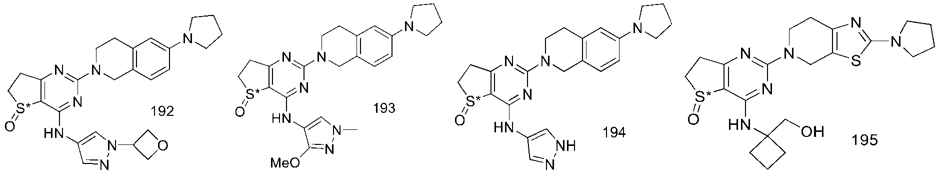

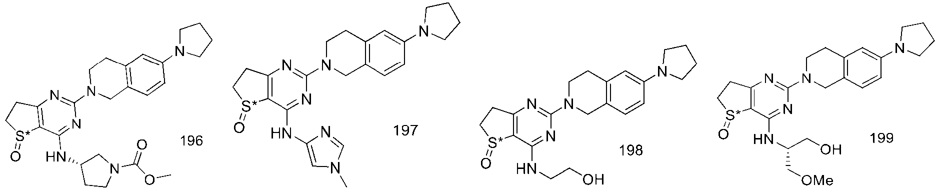

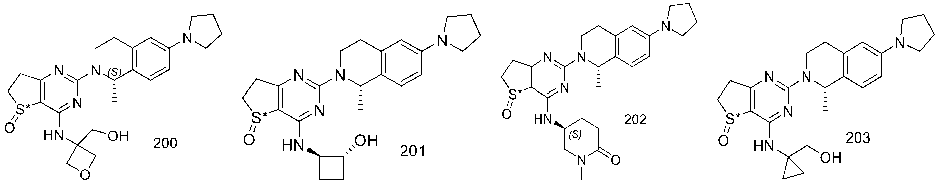

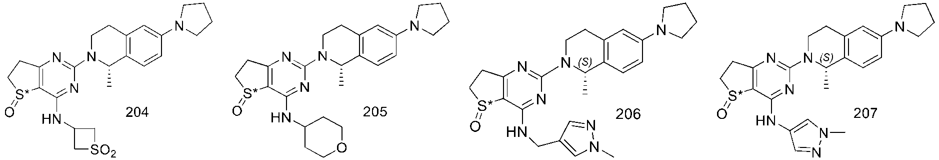

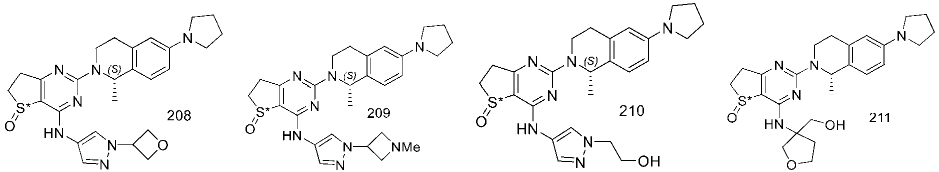

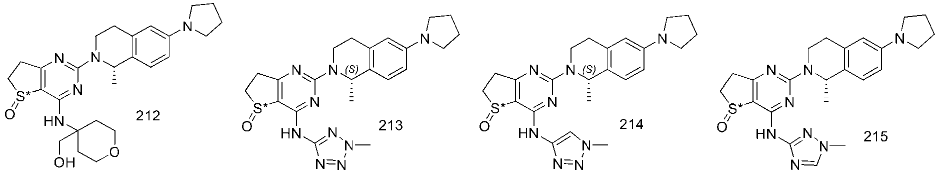

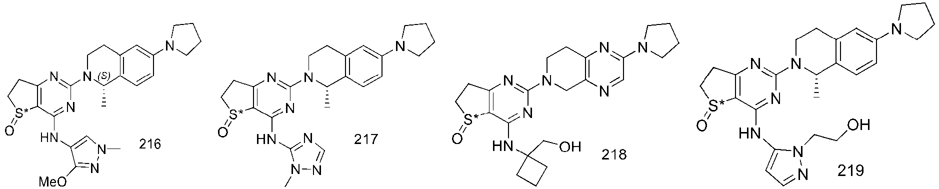

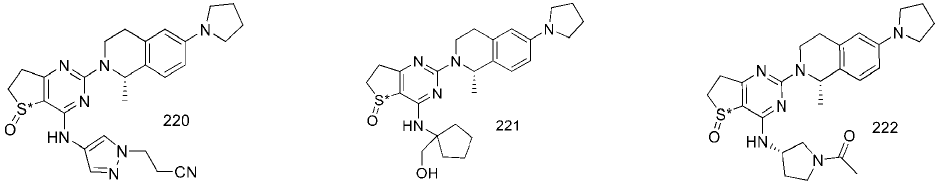

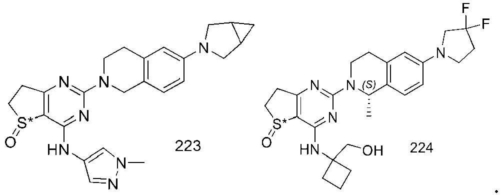

16. A pharmaceutical composition, comprising a therapeutically effective dose of the compound, the stereoisomer thereof, or the pharmaceutically acceptable salt thereof according to any one of claims 1-15, and one or more pharmaceutically acceptable carriers or excipients.

17. Use of the compound, the stereoisomer thereof, or the pharmaceutically acceptable salt thereof according to any one of claims 1-15, or the pharmaceutical composition according to claim 16 in the preparation of a PDE4 inhibitor drug.

18. Use of the compound, the stereoisomer thereof, or the pharmaceutically acceptable salt thereof according to any one of claims 1-15, or the composition according to claim 16 in the preparation of a medicament for treating or preventing inflammatory diseases, autoimmune diseases, metabolic diseases, nervous system diseases, and related diseases.
